(19) Europäisches Patentamt
European Patent Office
Office européen des brevets

(11) **EP 4 379 731 A1**

(12) **EUROPEAN PATENT APPLICATION**
published in accordance with Art. 153(4) EPC


(43) Date of publication:
**05.06.2024 Bulletin 2024/23**

(21) Application number: **22849511.5**

(22) Date of filing: **26.07.2022**

(51) International Patent Classification (IPC):
***G16C 20/80*** (2019.01) ***G01N 33/00*** (2006.01)

(52) Cooperative Patent Classification (CPC):
**G01N 33/00; G16C 20/80;** Y02P 90/30

(86) International application number:
**PCT/JP2022/028846**

(87) International publication number:
**WO 2023/008450 (02.02.2023 Gazette 2023/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2021 JP 2021123593**



(71) Applicant: **Panasonic Intellectual Property Management Co., Ltd.**
**Kadoma-shi, Osaka 571-0057 (JP)**

(72) Inventors:
- **TANAKA, Takehiro**
  **Kadoma-shi, Osaka 571-0057 (JP)**
- **WAKASUGI, Kensuke**
  **Kadoma-shi, Osaka 571-0057 (JP)**

(74) Representative: **Novagraaf International SA**
**Chemin de l'Echo 3**
**1213 Onex, Geneva (CH)**


(54) **INFORMATION DISPLAY METHOD, INFORMATION DISPLAY DEVICE, AND PROGRAM**


(57) An information display method including: obtaining first information for displaying an evaluated property value as a comparison indicator for comparing compounds (step S211); obtaining an evaluated property value of each of the compounds based on the first information (step S213), and generating a map indicating, at a position corresponding to a composition of each of the compounds, the evaluated property value of the compound; generating a first image including the map, and outputting the first image to a displayer (140) (step S214 and S215); and when the first information is changed, changing the map according to the first information changed, generating a second image including the map changed, and outputting the second image to the displayer (140) (step S218 and S219).


FIG. 63

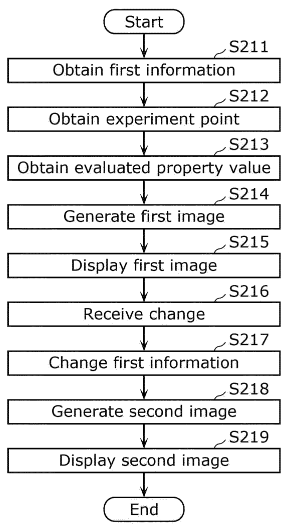


EP 4 379 731 A1

## Description

[Technical Field]

The present disclosure relates to a technique for displaying information regarding compounds.

[Background Art]

Conventionally, in development of materials that are compounds, in order to search for a composition formula or processing conditions of a compound having a desired property, it is common practice to perform experiments while changing blending ratios of raw materials and the like to specify a composition formula or processing conditions having a favorable property. For example, the following methods for displaying differences in properties when changing a composition formula or process conditions of a compound have been disclosed.

Patent Literature 1 discloses a method of displaying material properties on a ternary phase diagram when an organization of a composition formula is changed. Patent Literature 2 discloses a method of displaying properties by arranging, in a matrix form, a plurality of pieces of data illustrated by a pie chart. Patent Literature 3 discloses a method of inputting blending ratios of a huge number of raw materials to output a blending ratio having a targeted property. Patent Literature 4 discloses a method of two-dimensionally displaying a plurality of materials while changing a marker based on prediction accuracy or material properties.

In addition, recently, techniques for predicting material properties of any composition formula using a predictor obtained through machine learning are being developed. For example, Non-Patent Literature 1 discloses a method of predicting thermodynamic stability of perovskite-based materials using a neural network. Furthermore, Non-Patent Literature 2 discloses a method of predicting a probability value that a compound is to exhibit high ionic conductivity. Non-Patent Literature 3 discloses a method of calculating ion diffusion coefficients. Non-Patent Literature 4 discloses a method of optimizing material properties using Bayesian optimization and creating phase diagrams.

[Citation List]

[Patent Literature]

[PTL 1] Japanese Patent No. 6632412

[PTL 2] United States Patent No. 7199809 Specification

[PTL 3] Japanese Patent No. 4009670

[PTL 4] Japanese Unexamined Patent Application Publication No. 2020-128962


[Non Patent Literature]

[NPL 1] Jonathan Schmidt et al., "Predicting the Thermodynamic Stability of Solids Combining Density Functional Theory and Machine Learning", Chemistry of Materials, 29(12), 5090-5103 (2017)

[NPL 2] Austin D. Sendek, et al., "Holistic computational structure screening of more than 12000 candidates for solid lithium-ion conductor materials", Energy & Environmental Science, 10(1), 306-320 (2017)

[NPL 3] Xingfeng He, et al., "Statistical variances of diffusional properties from ab initio molecular dynamics simulations", npj Computational Materials, 4(1), 1-9 (2018)

[NPL 4] Kei Terayama, Koji Tsuda, Ryo Tamura, "Efficient recommendation tool of materials by an executable file based on machine learning", Japanese Journal of Applied Physics, 58(098001)2019.


[Summary of Invention]

[Technical Problem]

However, with the techniques in each of the patent literature and non patent literature described above, it is difficult to appropriately support raw material development.

The present disclosure is intended to solve the aforementioned problem and provides an information display method, and so on, capable of appropriately supporting material development.

[Solution to Problem]

In order to solve the above-described problem, an information display method according to an aspect of the present disclosure includes: obtaining first information for displaying an evaluated property value as a comparison indicator for comparing compounds; obtaining an evaluated property value of each of the compounds based on the first information, and generating a map indicating, at a position corresponding to a composition of each of the compounds, the evaluated property value of the compound; generating a first image including the map, and outputting the first image to a displayer; and when the first information is changed, changing the map according to the first information changed, generating a second image including the map changed, and outputting the second image to the displayer.

It should be noted that these general or specific aspects may be implemented as a system, an integrated circuit, a computer-readable recoding medium such as a CD-ROM, and may be implemented as any combination of an apparatus, a system, a method, an integrated circuit, a computer program, and a recording medium. Furthermore, the recording medium may be a non-transitory recording medium.

[Advantageous Effects of Invention]

According to the present disclosure, material development can be appropriately supported.

Additional benefits and advantages of an aspect of the present disclosure will become apparent from the specification and drawings. The benefits and/or advantages may be individually obtained by the various embodiments and features of the specification and drawings, which need not all be provided in order to obtain one or more of such benefits and/or advantages.

[Brief Description of Drawings]

[FIG. 1]
FIG. 1 is a block diagram illustrating an example of a configuration of a display system in Embodiment 1A.
[FIG. 2]
FIG. 2 is a diagram illustrating an example of a search range in Embodiment 1A.
[FIG. 3]
FIG. 3 is a diagram illustrating an example of formulae represented by combinations of option data in Embodiment 1A.
[FIG. 4]
FIG. 4 is a diagram illustrating an example of predicted property values of respective compounds in Embodiment 1A.
[FIG. 5A]
FIG. 5A is a diagram illustrating an example of a map in Embodiment 1A.
[FIG. 5B]
FIG. 5B is a diagram illustrating an example of a map in Embodiment 1A.
[FIG. 5C]
FIG. 5C is a diagram illustrating an example of a map in Embodiment 1A.
[FIG. 6]
FIG. 6 is a diagram illustrating another example of a map in Embodiment 1A.
[FIG. 7A]
FIG. 7A is a diagram illustrating another example of a map in Embodiment 1A.
[FIG. 7B]
FIG. 7B is a diagram illustrating another example of a map in Embodiment 1A.
[FIG. 7C]
FIG. 7C is a diagram illustrating another example of a map in Embodiment 1A.
[FIG. 7D]
FIG. 7D is a diagram illustrating another example of a map in Embodiment 1A.
[FIG. 8]
FIG. 8 is a diagram illustrating another example of a map in Embodiment 1A.
[FIG. 9A]
FIG. 9A is a diagram illustrating another example of a map in Embodiment 1A.
[FIG. 9B]
FIG. 9B is a diagram illustrating another example of a map in Embodiment 1A.
[FIG. 10]
FIG. 10 is a flowchart illustrating processing operations of the display system in Embodiment 1A.

[FIG. 11]
FIG. 11 is a diagram illustrating an example of a map in Variation 1 of Embodiment 1A.
[FIG. 12]
FIG. 12 is a diagram illustrating an example of processing variables in Variation 2 of Embodiment 1A.
[FIG. 13]
FIG. 13 is a block diagram illustrating an example of a configuration of a display system in Embodiment 1B.
[FIG. 14]
FIG. 14 is a diagram showing an example of experiment data stored in an experiment database in Embodiment 1B.
[FIG. 15]
FIG. 15 is a diagram illustrating an example of a map in Embodiment 1B.
[FIG. 16]
FIG. 16 is a diagram illustrating another example of a map in Embodiment 1B.
[FIG. 17]
FIG. 17 is a diagram illustrating another example of a map in Embodiment 1B.
[FIG. 18]
FIG. 18 is a diagram illustrating another example of a map in Embodiment 1B.
[FIG. 19]
FIG. 19 is a diagram illustrating another example of a map in Embodiment 1B.
[FIG. 20A]
FIG. 20A is a diagram illustrating another example of a map in Embodiment 1B.
[FIG. 20B]
FIG. 20B is a diagram illustrating another example of a map in Embodiment 1B.
[FIG. 21]
FIG. 21 is a diagram illustrating another example of a map in Embodiment 1B.
[FIG. 22A]
FIG. 22A is a diagram illustrating another example of a map in Embodiment 1B.
[FIG. 22B]
FIG. 22B is a diagram illustrating another example of a map in Embodiment 1B.
[FIG. 22C]
FIG. 22C is a diagram illustrating another example of a map in Embodiment 1B.
[FIG. 23]
FIG. 23 is a flowchart illustrating processing operations of the display system in Embodiment 1B.
[FIG. 24A]
FIG. 24A is a diagram illustrating an example of an image map in a variation of Embodiment 1B.
[FIG. 24B]
FIG. 24B is a diagram illustrating another example of an image map in a variation of Embodiment 1B.
[FIG. 25]
FIG. 25 is a block diagram illustrating an example of a configuration of a display system in Embodiment 2A.
[FIG. 26]
FIG. 26 is a diagram illustrating an example of an image element map on which candidate points are superimposed in Embodiment 2A.
[FIG. 27]
FIG. 27 is a diagram illustrating another example of an image element map on which candidate points are superimposed in Embodiment 2A.
[FIG. 28]
FIG. 28 is a diagram illustrating another example of an image element map on which candidate points are superimposed in Embodiment 2A.
[FIG. 29]
FIG. 29 is a diagram illustrating another example of an image element map on which candidate points are superimposed in Embodiment 2A.
[FIG. 30]
FIG. 30 is a diagram illustrating another example of an image element map on which candidate points are superimposed in Embodiment 2A.
[FIG. 31]
FIG. 31 is a diagram illustrating another example of an image element map on which candidate points are superimposed in Embodiment 2A.
[FIG. 32]
FIG. 32 is a diagram illustrating another example of an image element map on which candidate points are superimposed in Embodiment 2A.

[FIG. 33]

FIG. 33 is a flowchart illustrating processing operations of the display system in Embodiment 2A.

[FIG. 34]

FIG. 34 is a diagram illustrating an example of an image map in a variation of Embodiment 2A.

[FIG. 35]

FIG. 35 is a diagram illustrating another example of an image map in a variation of Embodiment 2A.

[FIG. 36]

FIG. 36 is a diagram illustrating another example of an image map in a variation of Embodiment 2A.

[FIG. 37]

FIG. 37 is a block diagram illustrating an example of a configuration of a display system in Embodiment 2B.

[FIG. 38]

FIG. 38 is a diagram illustrating an example of an image element map on which candidate points and experimental property values are superimposed in Embodiment 2B.

[FIG. 39]

FIG. 39 is a diagram illustrating another example of an image element map on which candidate points and experimental property values are superimposed in Embodiment 2B.

[FIG. 40]

FIG. 40 is a diagram illustrating an example of a state transition of an image element map in Embodiment 2B.

[FIG. 41]

FIG. 41 is a diagram illustrating another example of a state transition of an image element map in Embodiment 2B.

[FIG. 42]

FIG. 42 is a flowchart illustrating processing operations of a display system in Embodiment 2B.

[FIG. 43]

FIG. 43 is a block diagram illustrating an example of a configuration of a display system in a variation of Embodiment 2B.

[FIG. 44]

FIG. 44 is a diagram illustrating an example of a history of property display images in a variation of Embodiment 2B.

[FIG. 45]

FIG. 45 is a block diagram illustrating an example of a configuration of a display system in Embodiment 2C.

[FIG. 46]

FIG. 46 is a diagram illustrating an example of candidate point data stored in a candidate point database in Embodiment 2C.

[FIG. 47]

FIG. 47 is a diagram illustrating an example of an image element map on which candidate points and experimental property values are superimposed in Embodiment 2C.

[FIG. 48]

FIG. 48 is a flowchart illustrating processing operations of the display system in Embodiment 2C.

[FIG. 49]

FIG. 49 is a block diagram illustrating an example of a configuration of a display system in Embodiment 3A.

[FIG. 50]

FIG. 50 is a diagram illustrating an example of information stored in an evaluation display database in Embodiment 3A.

[FIG. 51]

FIG. 51 is a diagram illustrating an example of respective data stored in an experiment database in Embodiment 3A.

[FIG. 52]

FIG. 52 is a diagram illustrating an example of compound basic data in Embodiment 3A.

[FIG. 53]

FIG. 53 is a diagram illustrating an example of compound detail data in Embodiment 3A.

[FIG. 54]

FIG. 54 is a diagram illustrating an example of a map in Embodiment 3A.

[FIG. 55]

FIG. 55 is a diagram illustrating an example of a map in Embodiment 3A.

[FIG. 56]

FIG. 56 is a diagram indicating a legend or the like of the map illustrated in FIG. 55

[FIG. 57A]

FIG. 57A is a diagram for describing an example of a transition of images accompanying a change in calculation method information in Embodiment 3A.

[FIG. 57B]

FIG. 57B is a diagram for describing an example of a transition of images accompanying a change in calculation method information in Embodiment 3A.

[FIG. 58A]

FIG. 58A is a diagram for describing an example of image transition following a change in search range information in Embodiment 3A.

[FIG. 58B]

FIG. 58B is a diagram for describing an example of a transition of images accompanying a change in search range information in Embodiment 3A.

[FIG. 59A]

FIG. 59A is a diagram for describing an example of a transition of images accompanying a change in first display target information in Embodiment 3A.

[FIG. 59B]

FIG. 59B is a diagram for describing an example of a transition of images accompanying a change in first display target information in Embodiment 3A.

[FIG. 60A]

FIG. 60A is a diagram for describing an example of a transition of images accompanying a change in display range information in Embodiment 3A.

[FIG. 60B]

FIG. 60B is a diagram for describing an example of a transition of images accompanying a change in display range information in Embodiment 3A.

[FIG. 61A]

FIG. 61A is a diagram for describing an example of a transition of images accompanying a change in search range information in Embodiment 3A.

[FIG. 61B]

FIG. 61B is a diagram for describing an example of a transition of images accompanying a change in search range information in Embodiment 3A.

[FIG. 62]

FIG. 62 is a diagram illustrating an example of a transition of images accompanying a change in map array information in Embodiment 3A.

[FIG. 63]

FIG. 63 is a flowchart illustrating processing operations of the display system in Embodiment 3A.

[FIG. 64]

FIG. 64 is a diagram illustrating an example of a first image in a variation of Embodiment 3A.

[FIG. 65]

FIG. 65 is a diagram illustrating another example of a first image in a variation of Embodiment 3A.

[FIG. 66]

FIG. 66 is a flowchart illustrating a detailed example of generation of a first image by an image processor in a variation of Embodiment 3A.

[FIG. 67]

FIG. 67 is a block diagram illustrating an example of a configuration of a display system in Embodiment 3B.

[FIG. 68]

FIG. 68 is a diagram illustrating an example of information stored in an experiment display database in Embodiment 3B.

[FIG. 69A]

FIG. 69A is a diagram for describing an example of a transition of images accompanying a change in second display target information in Embodiment 3B.

[FIG. 69B]

FIG. 69B is a diagram for describing an example of a transition of images accompanying a change in second display target information in Embodiment 3B.

[FIG. 70A]

FIG. 70A is a diagram for describing an example of a transition of images accompanying a change in display condition information in Embodiment 3B.

[FIG. 70B]

FIG. 70B is a diagram for describing an example of a transition of images accompanying a change in display condition information in Embodiment 3B.

[FIG. 71]

FIG. 71 is a flowchart illustrating processing operations of the display system in Embodiment 3B.

[FIG. 72]

FIG. 72 is a block diagram illustrating an example of a configuration of a display system in Embodiment 3C.
[FIG. 73A]
FIG. 73A is a diagram for describing an example of a transition of images accompanying a change in date and time setting information in Embodiment 3C.
[FIG. 73B]
FIG. 73B is a diagram for describing an example of a transition of images accompanying a change in date and time setting information in Embodiment 3C.
[FIG. 74]
FIG. 74 is a flowchart illustrating processing operations of the display system in Embodiment 3C.
[FIG. 75]
FIG. 75 is a block diagram illustrating an example of a configuration of a display system in Embodiment 3D.
[FIG. 76]
FIG. 76 is a diagram illustrating an example of information stored in an evaluation display database in Embodiment 3D.
[FIG. 77]
FIG. 77 is a diagram illustrating an example of information stored in an experiment display database in Embodiment 3D.
[FIG. 78]
FIG. 78 is a diagram illustrating an example of a transition of images accompanying obtaining position information in Embodiment 3D.
[FIG. 79]
FIG. 79 is a diagram illustrating an example of a composition image indicating a composition formula including a coefficient of an element as a non-visualization variable in Embodiment 3D.
[FIG. 80]
FIG. 80 is a diagram illustrating an example of a composition image when a processing variable is used as a non-visualization variable in Embodiment 3D.
[FIG. 81]
FIG. 81 is a flowchart illustrating processing operations of the display system in Embodiment 3D.

[Description of Embodiments]

[Circumstances Leading to the Present Disclosure]

In recent years, recognition and identification methods based on machine learning and the like have made remarkable progress in fields such as image recognition and natural language processing, and are beginning to be applied to prediction of material properties.
For example, Non-Patent Literature 1 discloses a method of predicting thermodynamic stability of perovskite-based materials using a neural network. Perovskite materials are materials represented by the composition formula ABX_3, in which different elements are designated for A, B, and X. According to the method disclosed in Non-Patent Literature 1, it is possible to predict the thermodynamic stability of a composition formula in which an unsearched combination of elements is assigned to A, B, and X or the thermodynamic stability of a composition formula in which two or more elements are assigned to A (or B or X) such that the sum of the coefficients is 1. The method enables the number of experiments involving man-hours to be reduced and material development to be promoted.
However, although the technique disclosed in Non-Patent Literature 1 is capable of outputting a huge number of prediction results, since each prediction result is presented individually, it is difficult to recognize a relationship among the respective prediction results.
In addition, as described above, Non-Patent Literature 4 discloses a method of optimizing material properties using Bayesian optimization and creating phase diagrams. As a demonstrative example, experimental values that are results of a low-melting point composition search or a phase diagram search in a NaF-KF-LiF system are superimposed on a ternary phase diagram along with predicted values. However, a function of changing display methods is not provided.
Patent Literature 1 discloses a method of displaying properties on a ternary phase diagram when an organization of a composition formula is changed. However, simply displaying a ternary phase diagram as in Patent Literature 1 yields a limited result of presenting a blending ratio of three elements, or in other words, properties of materials within a range that is represented by two variables. For this reason, when there are many types of search variables for a compound, an entire composition range of a compound to be searched cannot be displayed and recognizing a relationship among respective properties of the compound is difficult.
Patent Literature 2 discloses a method of visualizing properties by arranging, in a matrix form, a plurality of pieces of data illustrated by a pie chart. However, with the technique disclosed in Patent Literature 2, in the case of a compound having many types of search variables such as perovskite materials, an entire composition range of a compound to be searched cannot be displayed and recognizing a relationship among respective properties of the compound is difficult.

Patent Literature 3 discloses a method of inputting blending ratios of a huge number of raw materials to output a blending ratio having a targeted property. However, with the technique disclosed in Patent Literature 3, since output results are presented in a localized manner, an entire composition range of a compound to be searched cannot be displayed and recognizing a relationship among respective properties of the compound is difficult. In addition, while a marker is changed and displayed according to a blending ratio, there is no disclosure of a method of changing the marker based on variables not used for display in the event that a composition formula is represented by a plurality of variables, which makes it difficult to comprehend information on variables not used for display.

Patent Literature 4 discloses a method of two-dimensionally displaying a plurality of materials while changing a marker based on prediction accuracy or material properties. However, Patent Literature 4 yields a limited result of presenting a blending ratio of three elements, or in other words, properties of materials within a range that is represented by two variables. For this reason, when there are many types of search variables for a compound, it is difficult to recognize an overall picture of a property of a compound. In addition, there is no disclosure of a method of changing a display method according to more than two search variables. In other words, when there are three or more search variables, predicted values and experimental values cannot be displayed over an entire composition range of a compound to be searched and recognizing a relationship among respective properties of the compound is d ifficu lt.

As described above, with the methods used in the patent literature and the non-patent literature described above, display that contributes to material development is either insufficient or not appropriately performed. As a result, support of material development is inadequate.

In view of this, for example, as indicated in Embodiments 3A to 3D, an information display method according to an aspect of the present disclosure includes: obtaining first information for displaying an evaluated property value as a comparison indicator for comparing compounds; obtaining an evaluated property value of each of the compounds based on the first information, and generating a map indicating, at a position corresponding to a composition of each of the compounds, the evaluated property value of the compound; generating a first image including the map, and outputting the first image to a displayer; and when the first information is changed, changing the map according to the first information changed, generating a second image including the map changed, and outputting the second image to the displayer. For example, the aforementioned first image and the second image are outputted to and displayed on the displayer. Furthermore, the aforementioned evaluated property value may be treated as an indicator of an experiment regarding a property of a compound. Furthermore, the information display method according to the present disclosure may also be referred to as a property display method.

Accordingly, when the first information is changed, a map that is in accordance with the change is displayed by being included in the second image, and thus the evaluated property value of each of the compounds can be displayed from various viewpoints. As a result, material search can be made more efficient, and material development can be appropriately supported.

Furthermore, the first map may include first coloring information indicating at least one of hue, chroma, or brightness as a color attribute of the evaluated property value. In the generating of the map, the map indicating the evaluated property value of each of the compounds using a color having the color attribute indicated by the first coloring information may be generated, and, in the changing of the first information, the color attribute indicated by the first coloring information may be changed. For example, the color attribute is changed according to an input operation by a user.

Accordingly, the color of the evaluated property value indicated on the map is changed to a color having the changed color attribute. Therefore, the color attribute of the evaluated property value indicated on the map can be arbitrarily changed, and a map that is easy to look at for the user can be displayed by being included in the second image. As a result, material search can be made more efficient.

Furthermore, the first information may include search range information indicating variables and items of option data, the variables being used in representing the compositions of the compounds, the items of option data indicating possible values or elements of each of the variables. In the obtaining of the evaluated property value, for each of combinations of option data obtained by selecting one item of option data from the items of option data for each of the variables, the evaluated property value of a compound having a composition corresponding to the combination may be obtained.

Accordingly, since the composition of a compound is represented by a combination of the option data of each of the variables, the composition can be clearly defined, and thus the evaluated property value of each of the compounds can be appropriately obtained. As a result, material search can be made more efficient and material development can be appropriately supported.

Furthermore, the first information may include calculation method information indicating a calculation method of the evaluated property value. The obtaining of the evaluated property value may include calculating, for each of the combinations, the evaluated property value of the compound having the composition corresponding to the combination, by using a specified algorithm that is in accordance with the calculation method indicated by the calculation method information. In the changing of the first information, the calculation method indicated by the calculation method information may be changed.

Accordingly, when the calculation method information is changed, a map that is in accordance with the change is displayed by being included in the second image, and thus the evaluated property of each of the compounds can be displayed from the viewpoints of various calculation methods. As a result, material search can be made more efficient.

Furthermore, the obtaining of the evaluated property value may include calculating, for each of the combinations, a predictive distribution of property values predicted for the compound having the composition corresponding to the combination, by using the specified algorithm that is in accordance with the calculation method indicated by the calculation method information, and calculating the evaluated property value of the compound based on the predictive distribution. For example, a predictive distribution is obtained by Gaussian Process regression and an evaluated property value is obtained by an acquisition function of Bayesian optimization.

Accordingly, since an evaluated property value based on a predictive distribution is calculated, an evaluated property value that includes a prediction variance in a prediction mean can be obtained. As a result, the user can readily confirm an evaluated property value on which a prediction variance is also reflected with respect to each of a plurality of compounds and material search can be made more efficient.

Furthermore, in the changing of the first information, the items of option data that can be taken by each of at least one variable among the variables may be changed.

Accordingly, when the option data is changed, a map that is in accordance with the change is displayed by being included in the second image, and thus evaluated property value of each of the compounds can be displayed from viewpoints of various option data. As a result, material search can be made more efficient.

Furthermore, the first information may include first display target information indicating one or more property types. In the obtaining of the evaluated property value, for each of the compounds, the evaluated property value for each of the one or more property types indicated by the first display target information may be obtained, and, in the changing of the first information, the one or more property types indicated by the first display target information may be changed.

Accordingly, when the type of the one or more property types indicated by the first display target information is changed, a map that is in accordance with the change is displayed by being included in the second image, and thus the evaluated property value of each of the compounds can be displayed from viewpoints of various properties. As a result, material search can be made more efficient. For example, the user can readily perform material development that satisfies a plurality of required properties. As a result, material development by the user can be appropriately supported.

Furthermore, the first information may include display range information indicating a display range for the evaluated property value. In the generating of the map, the display range for the evaluated property value indicated by the display range information may be associated with a scale range of a predetermined color or a predetermined shade of color, and the map indicating the evaluated property value of each of the compounds using a color or a shade of color in the scale range that corresponds to the evaluated property value may be generated. In the changing of the first information, the display range indicated by the display range information may be changed.

Accordingly, when the display range indicated by the display range information is changed, a map that is in accordance with the change is displayed by being included in the second image, and thus the evaluated property value of each of the compounds can be displayed from viewpoints of various display ranges. As a result, material search can be made more efficient. For example, even in a case where a range of evaluated property values indicated on a map changes with the progress of material search, changing a display range enables a plurality of evaluated property values included in the display range to be indicated in colors or shades of color that clearly differ from one another.

Furthermore, the map may include image element maps that are arranged in a matrix along each of a first coordinate axis and a second coordinate axis, and each of the image element maps may include a third coordinate axis and a fourth coordinate axis. The generating of the map may include: associating the first coordinate axis, the second coordinate axis, the third coordinate axis, and the fourth coordinate axis with a first variable, a second variable, a third variable, and a fourth variable, respectively, among the variables; identifying, for each of the compounds, an image element map associated with a value of the first variable and a value of the second variable that are to be used in representing a composition of the compound, among the image element maps; and mapping, for each of the compounds, an evaluated property value of the compound on the image element map identified, at a position indicated by the value of the third variable and the value of the fourth variable that are to be used in representing the composition of the compound.

Accordingly, the map can indicate evaluated property values for a compositions of compounds each of which is represented by four variables, and thus the evaluated property value of a compound can be easily displayed over a wide range. As a result, material search can be made more efficient, and material development can be appropriately supported.

Furthermore, when a total number of the items of option data indicated by the search range information is increased or decreased in the changing of the first information, in the changing of the map, a total number of the image element maps included in the map may be increased or decreased.

Accordingly, when the number of items of option data indicated by the search range information is changed, a number of image element maps that is equal to the changed number is displayed by being included in the second image, and thus the evaluated property value of each of the compounds can be displayed from viewpoints of various of image element maps. As a result, material search can be made more efficient.

Furthermore, the first information may include map array information indicating an array mode of at least one image element map to be displayed. When the map array information is changed, in the changing of the first information, by selection of a position of the at least one image element map to be displayed, in the changing of the map, the map generated may be changed to a map including the at least one image element map arranged according to the array mode indicated by the map array information changed.

Accordingly, when the array mode of at least one image element map indicated by the map array information is changed, the at least one image element map having the array mode that is in accordance with the change is displayed by being included in the second image. Therefore, the evaluated property value of each of the compounds can be displayed from viewpoints of various image element map array modes. As a result, comparison between multiple image element maps is facilitated, and material search can be made more efficient.

Furthermore, in the obtaining of the evaluated property value, the evaluated property value of each of the compounds may be obtained from at least one evaluater for evaluating a property value of a compound, the at least one evaluater being stored in an evaluater database.

Accordingly, an appropriate evaluated property value can be obtained for each of the compounds.

Furthermore, the information display method may further include: obtaining second information for displaying an experimental property value of a compound. The generating of the map may further include: for each of one or more compounds that have been experimented on, obtaining an experimental property value of the compound, based on the second information; and superimposing the experimental property value of the compound at a position, on the map, that corresponds to a composition of the compound, based on the second information.

Accordingly, since the experimental property value is superimposed on the map based on the second information, the evaluated property value and the experimental property value of each of the compounds indicated on the map can be displayed with appropriate contrast.

Furthermore, the information display method may further include: outputting a third image to the displayer when the second information is changed, the third image including the map in which a display mode of the one or more experimental property values superimposed on the map has been changed.

Accordingly, when the second information is changed, a map on which an experimental property value is superimposed in the display mode that is in accordance with the change is displayed by being included in the third image, and thus the experimental property value of each of the one or more compounds that have been experimented on can be displayed from various viewpoints. As a result, material search can be made more efficient, and material development can be appropriately supported.

Furthermore, the second information may include second coloring information indicating at least one of hue, chroma, or brightness as a color attribute of the at least one experimental property. In the generating of the map, the map on which the experimental property value of each of the one or more compounds that have been experimented on may be superimposed using a color having the color attribute indicated by the second coloring information is generated. In the changing of the second information, the color attribute indicated by the second coloring information may be changed.

Accordingly, the color of the experimental property value indicated on the map is changed to a color having the changed color attribute. Therefore, the color attribute of the evaluated property value indicated on the map can be arbitrarily changed, and a map having the experimental property value superimposed that is easy to look at for the user can be displayed by being included in the third image. As a result, material search can be made more efficient.

Furthermore, the second information may include second display target information indicating one or more property types. In the generating of the map: for each of the one or more compounds that have been experimented on, the experimental property value for each of the one or more property types indicated by the second display target information may be obtained; and a mark having a form that is in accordance with the experimental property value for each of the one or more property types may be superimposed on the map. In the changing of the second information, the one or more property types indicated by the second display target information may be changed.

Accordingly, when the second display target information is changed, a map on which an experimental property value is superimposed in the display mode that is in accordance with the change is displayed by being included in the third image, and thus the evaluated property value of each of the one or more compounds that have been experimented on can be displayed from viewpoints of various property types. As a result, material search can be made more efficient.

Furthermore, the second information may include display condition information indicating a display condition of the experimental property value, the display condition being based on a threshold. In the generating of the map, among the one or more experimental values of the one or more compounds that have been experimented on, an experimental property value that satisfies the display condition indicated by the display condition information and an experimental property value that does not satisfy the display condition may be superimposed on the map in mutually different forms. In the changing of the second information, the display condition indicated by the display condition information may be changed by increasing or decreasing the threshold.

Accordingly, when the display condition is changed, a map on which the experimental property value is superimposed in a display mode that is in accordance with the change is displayed by being included in the third image, and thus the evaluated property value of each of the one or more compounds that have been experimented on can be displayed from viewpoints of various display conditions. As a result, material search can be made more efficient. For example, a display mode of an experimental property value that satisfies a display condition that is greater than or equal to a threshold can be emphasized more than a display mode of an experimental property value that does not satisfy the display condition that is greater than or equal to a threshold. Furthermore, for example, by the changing of the threshold by the user, the experimental property value to be emphasized on the map can be changed so as to be easy to look at for the user.

Furthermore, the generating of the first image may include associating, with the first image, additional information indicating a time point at which the first image was generated, and storing the first image to which the additional information has been associated, as a stored image, in a recording medium. The generating of the second image may include associating, with the second image, additional information indicating a time point at which the second image was generated, and storing the second image to which the additional information has been associated, as a stored image, in the recording medium. The information display method may further include: obtaining time point information indicating a first time point; reading out a stored image associated with additional information corresponding to the first time point indicated by the time point information, from the recording medium, as a fourth image, and outputting the fourth image to the displayer; and when the first time point indicated by the time point information is changed to a second time point, reading out a stored image associated with additional information corresponding to the second time point, from the recording medium, as a fifth image, and outputting the fifth image to the display in place of the fourth image. For example, the additional information indicates the date on which the first image or the second image is generated. Furthermore, the first time point and the second time point may be absolute time points such as dates, or may be relative time points for a current time point such as a latest time point. For example, the fourth image and the fifth image are output to and displayed on the displayer.

Accordingly, for example, in accordance with time point information, the first image or the second image generated in the past can be read from a recording medium and displayed as the fourth image. In addition, by changing the time point information, the first image or the second image generated at a time point desired by the user can be read from the recording medium and displayed as the fifth image. As a result, for example, the user can readily comprehend day-to-day progress of experiments and material search can be made more efficient.

Furthermore, the information display method may further include: obtaining position information indicating a position of the evaluated property value or the experimental property value on the map that includes a sixth image that is one of the first image, the second image, or the third image; and changing the sixth image to a seventh image by obtaining composition formula data on a composition formula of a compound corresponding to the position indicated by the position information and superimposing, on the map, a composition image showing the composition formula data.

Accordingly, even when it is difficult to read a composition formula of a compound having an evaluated property value or an experimental property value indicated on a map from coordinate axes of the map, a composition image indicating the composition formula is displayed by position information. Therefore, the user can readily comprehend the composition formula. As a result, material search can be made more efficient and material development can be appropriately supported.

Furthermore, an information display method according to an aspect of the present disclosure includes: obtaining first information for displaying an evaluated property value as a comparison indicator for comparing compounds, the first information including information groups each of which is changeable; obtaining a map indicating, at a position corresponding to a composition of each of the compounds, the evaluated property value of the compound; and outputting an image that includes the map and is generated based on a priority level of each of the information groups.

Accordingly, for example, an information group with a highest priority is displayed by being included in the image and display of other information groups can be suppressed. As a result, the user can readily select and change the information group that is being displayed. In other words, by attaching a higher priority to an information group if the information group is more beneficial to displaying an evaluated property value, the user can readily comprehend a change in a map due to a change in the information group and material development can be made more efficient.

Furthermore, in the outputting of the image, for each of the information groups, the image is generated by using, as the priority level of the information group, the degree of change in the map caused by a change in the group.

Accordingly, the user can readily confirm that a map is to change significantly due to a change in an information group and material development can be made more efficient.

It should be noted that each of the processes or operations included in the above-described information display method is implemented by an information display device or a computer. For example, the information display device or the computer includes a processor and a non-transitory computer-readable recording medium. The recording medium is, for example, a memory, and stores a program for causing the processor to execute the respective processes or operations included in the information display method. The processor reads out the program from the recording medium and executes the program. With this, the respective processes and operations included in the information display method are executed.

Hereinafter, embodiments of the present disclosure will be described with reference to the drawings. Each of the subsequently-described embodiments shows a preferred specific example of the present disclosure. Therefore, numerical values, shapes, materials, elements, the arrangement and connection of the elements, etc., indicated in the following embodiments are mere examples, and thus are not intended to limit the present disclosure. Therefore, among the elements described in the following embodiments, elements not recited in any one of the independent claims that indicate the broadest concepts are described as optional elements.

It should be noted that the respective figures are schematic illustrations and are not necessarily accurate depictions. Furthermore, elements which are substantially the same have the same reference signs in the figures, and duplicate description may be omitted or simplified. The effects of the information display method are likewise realized even with the information display device and the program.

Furthermore, the present disclosure includes Embodiments 1, 2, and 3, and Embodiment 1 includes Embodiments 1A and 1B. Embodiment 2 includes Embodiments 2A, 2B, and 2C, and Embodiment 3 includes Embodiments 3A, 3B, 3C, and 3D. Hereinafter, the respective embodiments will be sequentially described.

[EMBODIMENT 1A]

A display system according to the present embodiment displays a predicted property value of each of a plurality of compounds in the form of a map.

[Configuration of display system 100]

FIG. 1 is a block diagram illustrating an example of a configuration of display system 100 according to the present embodiment. Display system 100 illustrated in FIG. 1 includes input unit 110, predictor database (DB) 120, property display device 130, and displayer 140. Note that property display device 130 is an example of an information display device.

[Input unit 110]

Input unit 110 receives an input operation by a user and outputs an input signal in accordance with the input operation to property display device 130. For example, input unit 110 may be constituted of a keyboard, a touch sensor, a touchpad, a mouse, or the like.

[Predictor database 120]

For example, predictor database 120 is a recording medium storing a predictor that is a computer program. Specifically, predictor database 120 stores predictors for predicting a property value of each of a plurality of compounds. The property value to be predicted is, for example, electrical conductivity, thermal conductivity, a band gap, or the like and will be hereinafter also referred to as a predicted property value. In addition, a predictor is a computer program based on a prescribed calculation algorithm. The prescribed calculation algorithm may be an algorithm obtained by machine learning. For example, predictor database 120 is constituted of a non-volatile memory. With respect to an input of data related to a compound, the predictor outputs a property value of the compound. For example, the data is a composition formula of the compound.

A predictor of any format may be used insofar as the predictor is capable of outputting a predicted property value of a compound. For example, the predicted property value outputted from the predictor may be the values calculated using the predictors described in Non-Patent Literature 1 and Non-Patent Literature 2. Non-Patent Literature 1 discloses a method of predicting the thermodynamic stability of perovskite-based compounds. Non-Patent Literature 2 discloses a method of predicting a probability value that a compound exhibits high ionic conductivity. Note that a compound is synonymous with a material.

In addition, the predicted property value outputted from the predictor may be a property value calculated according to density functional formalism. Processing by density functional formalism requires that a crystal structure be designated. The crystal structure may be designated by an input signal from input unit 110. In addition, even when a crystal structure of a compound to be predicted is unknown, the predictor may output a property value of a compound having a same composition formula as the compound and having a known crystal structure as a predicted property value of the compound to be predicted. Furthermore, when a composition formula of a compound to be predicted differs from composition formulae of compounds that can be predicted, the predictor may create a tentative crystal structure by replacing a part of elements of the composition formula of the compound to be predicted with other elements and perform a calculation of density functional formalism with respect to a compound having the created crystal structure. Alternatively, when one crystal structure of a compound is verified experimentally, the crystal structure may be adopted and density functional formalism may be executed in a similar manner to that described above.

In addition, the predicted property value outputted from the predictor may be a property value calculated using a method called Ab initio molecular dynamics (AIMD) described in, for example, Non-Patent Literature 3. Non-Patent Literature 3 discloses a method of calculating ion diffusion coefficients. Note that the property value is not limited to a predicted value and may also be an experimentally obtained property value such as electrical conductivity or thermal conductivity.

Predictor database 120 outputs, to predicted value obtainer 132, a predictor according to a request by predicted value obtainer 132, or in other words, a predictor suited to predicting a property of a compound.

[Displayer 140]

Displayer 140 obtains an image from property display device 130 and displays the image. Examples of displayer 140 include, but are not limited to, a liquid crystal display, a plasma display, and an organic EL (Electro-Luminescence) display.

Displayer 140 is, for example, a displayer included in an information terminal, an information terminal into which an electronic experiment note is introduced, or the like. Alternatively, displayer 140 may be a displayer included in a synthesis apparatus used to synthesize raw materials or compounds, a reaction apparatus used to cause a reaction of raw materials or compounds, an analysis apparatus used to analyze compounds, an evaluation apparatus used to evaluate compounds, or the like.

[Property display device 130]

Property display device 130 obtains an input signal from input unit 110 and predicts, using a predictor in predictor database 120, property values of a plurality of compounds in accordance with the input signal. In addition, property display device 130 obtains an input signal indicating a display method from input unit 110 as first display method information and generates, according to the display method, an image that indicates a property value having been predicted for each compound as a predicted property value. Furthermore, property display device 130 outputs the generated image to displayer 140.

[Configuration of property display device 130]

Property display device 130 includes search range obtainer 131, predicted value obtainer 132, display method obtainer 133, and image processor 134. Note that property display device 130 may be constituted of, for example, a processor such as a central processing unit (CPU) and a memory. In this case, the processor functions as property display device 130 by, for example, executing a computer program stored in the memory. Note that the memory may be volatile or non-volatile or may be constituted of a volatile memory and a non-volatile memory.

[Search range obtainer 131]

Search range obtainer 131 obtains an input signal indicating a search range of a compound from input unit 110. In addition, search range obtainer 131 outputs the input signal as a search range signal to predicted value obtainer 132. The search range of a compound is a range of a composition formula, a composition, or the like of each compound for which a property value is to be predicted. In the present embodiment, a compound to be predicted is a solid electrolyte and a composition formula of the compound is represented by the following (Formula 1).

$$Li_{2-3a-4b}(M3_{1-x}M3'_{x})a(M4_{1-y}M4'_{y})_{1+b}O_{3} \ldots \qquad \text{(Formula 1)}$$

Search range obtainer 131 obtains, as a search range, each piece of data (in other words, option data to be described later) that may be assumed by each of variables a, b, x, y, M3, M3', M4, and M4' in (Formula 1).

FIG. 2 is a diagram illustrating an example of a search range. A search point included in the search range is represented as one or more pieces of data designated in advance (for example, elements Li and O and coefficients thereof) and one or more range variables and forms a composition formula when a value or an element is designated for every range variable. In other words, when a value or an element is designated for every range variable, a data representation indicated using the range variables forms a single composition or, in other words, a single composition formula of a compound. An example of a data representation is $Li_{2-3a-4b}(M3_{1-xM3'x})_a(M4_{1-yM4'y})_{1+b}O_3$ in (Formula 1) above and illustrated in FIG. 2. A range variable or a search variable is also simply referred to as a variable.

In this case, range variables include categorical variables, discrete variables, and continuous variables. Categorical variables indicate types of elements that constitute a compound. For example, variables M3, M3', M4, and M4' are categorical variables. Each of variables M3 and M3' may assume any one of four pieces of option data "La, Al, Ga, and In". In this case, there are six (${}_4C_2 = 6$) combinations of option data that can be respectively assumed by variables M3 and M3'. Each of variables M4 and M4' may assume any one of three pieces of option data "Ti, Zr, and Hf". In this case, there are three (${}_3C_2 = 3$) combinations of option data that can be respectively assumed by variables M4 and M4'. Note that Li and O are elements fixed in advance and do not correspond to categorical variables.

A discrete variable is a numerical value for determining a composition ratio of a compound. Numerical values of a plurality of pieces of option data which a discrete variable can assume exist in a mutually discrete manner. In addition, the number of pieces of option data in a discrete variable is smaller than that of a continuous variable. For example, variables a and b for determining coefficients of the respective elements are discrete variables. Variable a may assume one of five pieces of option data "0.0, 0.05, 0.1, 0.15, and 0.2". There are five (${}_5C_1 = 5$) combinations of option data that can be assumed by variable a. Variable b may assume one of four pieces of option data "0.0, 0.1, 0.2, and 0.3". There are four (${}_4C_1 = 4$) combinations of option data that can be assumed by variable b.

A continuous variable indicates a numerical value for determining a composition ratio of a compound. The number of pieces of option data in a continuous variable is larger than that in, for example, a discrete variable and a categorical variable and a continuous variable is suited to cases of carrying out an in-depth analysis of a change in a property value with respect to a change in the option data. The number of pieces of option data in a continuous variable is desirably five or more. For example, variables x and y for determining coefficients of the respective elements are continuous variables. Variables x and y may respectively assume any one of 11 pieces of option data indicated by a step width of 0.1 from a minimum value of 0.0 to a maximum value of 1.0. In other words, there are 11 (${}_{11}C_1 = 11$) combinations of option data that can be respectively assumed by variables x and y.

While the 11 pieces of option data of a continuous variable are represented using the minimum value 0.0, the maximum value 1.0, and the step width 0.1 in the example illustrated in FIG. 2, alternatively, the option data may be represented by the 11 coefficients themselves as "0.0, 0.1, 0.2, 0.3, ..., 1.0". In addition, a continuous variable may be set to have a smaller step width than 0.1 so that retrieved data such as a predicted property value changes in a more continuous manner. Examples of smaller step sizes include 0.01 and 0.001. A continuous variable may be designated by a user. For example, the user may designate each of search variables x and y as a continuous variable. In this case, a minimum value and a maximum value for each of search variables x and y may be designated by the user and a value stored in advance in property display device 130 may be used as a step width for each of search variables x and y.

In the composition formula of the data representation illustrated in FIG. 2, a ratio between M3 and M3' represented by continuous variable x has a negative correlation in which changing a proportion of one of M3 and M3' results in changing a proportion of the other. In a similar manner, a ratio between M4 and M4' represented by continuous variable y has a negative correlation in which changing a proportion of one of M4 and M4' results in changing a proportion of the other.

In other words, the respective variables M3, M3', M4, M4' a, b, x, and y are represented as follows using option data.

$$M3 \in \{La, Al, Ga, In\}$$

$$M3' \in \{La, Al, Ga, In\}$$

$$M4 \in \{Ti, Zr, Hf\}$$

$$M4' \in \{Ti, Zr, Hf\}$$

$$a \in \{0.0, 0.05, 0.1, 0.15, 0.2\}$$

$$b \in \{0.0, 0.1, 0.2, 0.3\}$$

$$x \in \{0.0, 0.1, 0.2, 0.3, 0.4, 0.5, 0.6, 0.7, 0.8, 0.9, 1.0\}$$

$$y \in \{0.0, 0.1, 0.2, 0.3, 0.4, 0.5, 0.6, 0.7, 0.8, 0.9, 1.0\}$$

A combination of the option data is then obtained by selecting one piece of option data from a plurality of pieces of option data corresponding to each of variables M3, M3', M4, M4', a, b, x, and y. The combination is (M3, M3', M4, M4', a, b, x, y) = (La, Al, Ti, Zr, 0.1, 0.1, 0.1, 0.1), (La, Al, Ti, Zr, 0.1, 0.1, 0.1, 0.2), (La, Al, Ti, Zr, 0.1, 0.1, 0.1, 0.3), ... or the like. In other words, there are a large number of combinations. Such a combination of option data uniquely represents a composition formula that represents a composition of a compound. In addition, the search range described above is a range that includes all of these combinations and the search point described above can be regarded as indicating one combination or one composition of a compound. Note that a combination of option data that may be assumed by each of a plurality of variables will be hereinafter also referred to as a combination of a plurality of variables. For example, a combination of option data that may be assumed by each of variables x and y will also be referred to as a combination of variables x and y.

When generating search points by changing a value or an element designated for each of the range variables described above, various search points are generated and a set of the search points are to represent a search range.

[Predicted value obtainer 132]

Predicted value obtainer 132 obtains a search range signal from search range obtainer 131. In addition, predicted value obtainer 132 generates all combinations of option data which may be assumed by each of a plurality of variables included in a search range indicated by the search range signal. The combinations represent composition formulae of compounds. Therefore, a plurality of composition formulae or compositions according to the combinations are generated.

FIG. 3 is a diagram illustrating an example of composition formulae represented by combinations of option data. FIG. 3 illustrates the plurality of generated composition formulae and option data of each of variables M3, M3', M4, M4', a, b, x, and y used in each of the plurality of composition formulae. Note that, in the present disclosure, a coefficient of each element included in a composition formula (in other words, a numerical value indicating a composition ratio) is denoted by subscripts in some cases and denoted by ordinary characters in a similar manner to characters denoting elements in other cases.

Note that a data representation of a composition formula may indicate an order of pieces of option data which may be assumed by variables instead of indicating pieces of option data themselves as in FIG. 3. For example, when five pieces of option data of discrete variable a are "0.0, 0.05, 0.1, 0.15, and 0.2", the pieces of option data are specified in this order. In this case, for example, an order "3" of the option data "0.1" of discrete variable a may be used in a data representation of a composition formula in place of discrete variable a = 0.1.

Predicted value obtainer 132 obtains a predictor from predictor database 120 and by inputting the plurality of generated composition formulae into the predictor, obtains predicted property values of compounds having the composition formulae.

FIG. 4 is a diagram illustrating an example of predicted property values of respective obtained compounds.

As illustrated in FIG. 4 (right end of diagram), predicted value obtainer 132 obtains, with respect to each of the plurality of composition formulae generated from a search range, a predicted property value of a compound having the composition formula. With respect to each of the plurality of composition formulae, predicted value obtainer 132 associates the composition formula and the predicted property value obtained with respect to the composition formula with each other and outputs the associated composition formula and the predicted property value to image processor 134. The outputted composition formula may be a combination of the plurality of variables described above.

[Display method obtainer 133]

Display method obtainer 133 obtains an input signal indicating a display method from input unit 110 as first display method information. In addition, display method obtainer 133 outputs the first display method information to image processor 134. The display method indicated by the first display method information is a method of displaying a predicted property value of a compound indicated by each composition formula.

[Image processor 134]

Image processor 134 obtains, for each of a plurality of compounds, a composition formula and a predicted property value of the compound from predicted value obtainer 132. In addition, image processor 134 obtains the first display method information from display method obtainer 133. Image processor 134 generates a map indicating predicted property values of a plurality of compounds according to the display method indicated by the first display method information and displays an image including the map on displayer 140.

[Specific examples of map]

FIGS. 5A to 5C are diagrams illustrating an example of a map.

For example, image processor 134 generates map Ma illustrated in FIG. 5A. Map Ma has coordinate axes A1 and A2. For example, continuous variable x is assigned to coordinate axis A1 and continuous variable y is assigned to coordinate axis A2. A cell of each position indicated by respective numerical values of variables x and y on map Ma is colored in a shade in accordance with a predicted property value of a compound that includes the respective numerical values of variables x and y in its composition formula. Note that the predicted property value is, for example, a band gap, and a scale indicating a relationship between the predicted property value and shades is attached to map Ma.

For example, display method obtainer 133 obtains first display method information in accordance with an input operation with respect to input unit 110 by the user. For example, the first display method information indicates variable x assigned to coordinate axis A1 and variable y assigned to coordinate axis A2. Furthermore, the first display method information indicates option data of each of variables M3, M3', M4, M4', a, and b not assigned to a coordinate axis as designated option data. In addition, display method obtainer 133 outputs the first display method information to image processor 134.

According to the first display method information, image processor 134 assigns variable x to coordinate axis A1 of map Ma and assigns variable y to coordinate axis A2 of map Ma. Furthermore, from predicted property values of a plurality of compounds obtained by predicted value obtainer 132, image processor 134 specifies a predicted property value of each compound for which each of variables M3, M3', M4, M4', a, and b indicates designated option data. In addition, image processor 134 applies a color of a shade in accordance with the specified predicted property value of each compound to a position on map Ma indicated by respective numerical values of variables x and y included in a composition formula of the compound.

For example, when the color is black or white, the larger the predicted property value, the darker the shade of black in the color applied to map Ma with respect to the predicted property value and, conversely, the smaller the predicted property value, the lighter the shade of black in the color applied to map Ma with respect to the predicted property value. However, the color may be other than black or white. Alternatively, image processor 134 may indicate a predicted property value on map Ma by a type of color instead of a shade of color. For example, image processor 134 may assign red to a largest predicted property value, assign orange to a second largest predicted property value, assign yellow to a third largest predicted property value, assign white to a fourth largest predicted property value, assign yellow-green to a fifth largest predicted property value, assign light blue to a sixth largest predicted property value, and assign blue to a seventh largest predicted property value. In addition, image processor 134 may apply a color assigned with respect to a magnitude of the predicted property value obtained from predicted value obtainer 132 to map Ma. In this case, map Ma is displayed like a heat map.

In addition, in a plurality of compounds having predicted property values indicated by map Ma, each of variables other than variables x and y or, in other words, each of variables M3, M3', M4, M4', a, and b not used in coordinate axes of map Ma indicate same option data. More specifically, the option data of each of variables M3, M3', M4, M4', a, and b is fixed to designated option data and a predicted property value of a compound in accordance with a numerical value of each of variables x and y is displayed on map Ma. In other words, on map Ma, combinations of option data of respective variables M3, M3', M4, M4', a, and b are fixed. Pieces of option data of respective variables M3, M3', M4, M4', a, and b are fixed to pieces of designated option data as indicated by an example represented as variables (M3, M3', M4, M4', a, b) = (La, Al, Ti, Zr, 0.05, 0.1).

Note that a variable used in a coordinate axis of map Ma such as variables x and y may be referred to as a variable variable or a visualization variable while a variable not used in a coordinate axis of map Ma such as variables M3 and M4 may be referred to as a fixed variable, a non-visualization variable, a surplus variable, a non-utilized variable, or the like. In addition, map Ma according to the present embodiment is also referred to as image element map Ma.

In addition, as illustrated in FIG. 5B, image processor 134 may interpolate a predicted property value and display map Ma indicating the interpolated predicted property value on displayer 140. In other words, based on predicted property values of 121 (= 11 $\times$ 11) points in accordance with 11 numerical values "0.0, 0.1, 0.2, ..., 10.0" of variable x and 11 numerical values "0.0, 0.1, 0.2, ..., 10.0" of variable y, image processor 134 calculates predicted property values between the points. For example, image processor 134 may interpolate predicted property values using linear interpolation, bilinear interpolation, bicubic interpolation, or the like.

In addition, in FIGS. 5A and 5B, a numerical range of continuous variable x assigned to coordinate axis A1 and a numerical range of continuous variable y assigned to coordinate axis A2 are equal to each other. In other words, the numerical range of continuous variables x and y is from a minimum value 0.0 to a maximum value 1.0. Therefore, a shape of map Ma illustrated in FIGS. 5A and 5B is a square. However, the shape of map Ma is dependent on an assignment method of coordinate axes of map Ma and may be another shape.

For example, the shape of map Ma may be a rectangle or a triangle as illustrated in FIG. 5C.

In the example in FIG. 5C, triangular map Ma indicating a predicted property value of compounds having a composition formula of $IN(N_{1-x-y}Se_xAs_y)$ is displayed. In a similar manner to the examples illustrated in FIGS. 5A and 5B, a cell of each position indicated by respective numerical values of variables x and y on map Ma is colored in a shade in accordance with a predicted property value of a compound that includes the respective numerical values of variables x and y in its composition formula.

As described above, property display device 130 according to the present embodiment includes predicted value obtainer 132, display method obtainer 133, and image processor 134. Predicted value obtainer 132 obtains a predicted property value of each of a plurality of compounds. Display method obtainer 133 obtains first display method information indicating a display method of the predicted property value. Image processor 134 generates, according to the first display method information, map Ma indicating the predicted property value of each of the plurality of compounds. In addition, image processor 134 generates an image including map Ma and outputs the image. In this case, map Ma includes coordinate axes indicating each of at least two variables among a plurality of variables to be used in representing compositions of compounds. For example, the image described above is outputted to and displayed by displayer 140.

Accordingly, first display method information is obtained and, according to a display method indicated by the first display method information, map Ma indicating a predicted property value of each of a plurality of compounds is generated. As a result, if the display method indicated by the first display method information is suitably set for an object of search for a new material, the search for a new material or, in other words, material development can be appropriately supported by displaying an image including the generated map Ma. In addition, since map Ma includes the coordinate axes described above, a predicted property value of each of the plurality of compounds can be displayed at a position corresponding to a composition of the compound. Since respective predicted property values of a large number of compounds to be searched are displayed as map Ma, the predicted property values can be appropriately visualized and a user can readily recognize a relationship between compositions and predicted property values of the compounds. In other words, the user can readily comprehend an overall picture of the predicted property values on map Ma. Note that a search for new materials or new compounds is also referred to as a material search.

Note that property display device 130 need not include display method obtainer 133. In this case, image processor 134 outputs an image including map Ma generated using an obtained predicted property value. Map Ma is a map having a coordinate axis indicating each of at least two variables to be used in representing a composition of a compound and indicates a predicted property value of each of the plurality of compounds. Even with such property display device 130, a similar operation and effect to those described above can be produced.

Furthermore, the first display method information may indicate, as the display method for the predicted property value, at least one of a method for determining a color for indicating the predicted property value or a method for determining a display format for indicating the predicted property value. For example, a color according to a predicted property value is determined by a color determination method and a shade of the color according to the predicted property value is determined by a display format determination method. As a result, image processor 134 according to the present embodiment generates map Ma indicating a color or a shade of color according to a predicted property value of each of a plurality of compounds.

Accordingly, since the predicted property value of each compound is indicated by a color or a shade of color according to the predicted property value, the user can readily comprehend a magnitude of the predicted property value. For example, the user can readily comprehend a difference in predicted property values between compounds with similar compositions.

In addition, property display device 130 according to the present embodiment includes search range obtainer 131. Search range obtainer 131 obtains a plurality of variables to be used in representing compositions of compounds and a plurality of pieces of option data indicating values or elements that can be assumed by each of the plurality of variables. In addition, for each combination of option data obtained by selecting one piece of option data from the plurality of pieces of option data with respect to each of the plurality of variables, predicted value obtainer 132 obtains a predicted property value of a compound having a composition corresponding to the combination. In other words, for each of the combinations described above, search range obtainer 131 obtains a predicted property value of a compound having a composition corresponding to the combination using a prescribed algorithm. For example, the prescribed algorithm is a predictor stored in predictor database 120.

Accordingly, an appropriate predicted property value can be obtained. In other words, property values of a plurality of compounds with respectively different compositions can be appropriately predicted. In addition, when the prescribed algorithm is updated by an experiment on a compound that is carried out as needed, prediction accuracy of the property values can be enhanced.

FIG. 6 is a diagram illustrating another example of map Ma.

As illustrated in FIG. 6, image processor 134 may discretize predicted property values and apply a color of a shade in accordance with the discretized predicted property values to map Ma. For example, image processor 134 discretizes predicted property values "0.5 to 4.0" by intervals of 0.25. Therefore, if a predicted property value obtained from predicted value obtainer 132 is, for example, 0.7, image processor 134 replaces the predicted property value with 0.75.

For example, display method obtainer 133 obtains an input signal indicating discretization of predicted property values and intervals of the discretization from input unit 110 as first display method information and outputs the first display method information to image processor 134. Image processor 134 discretizes, according to the first display method information, a predicted property value of each compound obtained from predicted value obtainer 132.

In this manner, image processor 134 according to the present embodiment discretizes predicted property values and generates map Ma indicating the discretized predicted property values.

In other words, the present embodiment is not limited to applying a color of a shade in accordance with an obtained predicted property value itself to map Ma and, as illustrated in FIG. 6, a color of a shade in accordance with a discretized predicted property value may be applied to map Ma. Accordingly, visibility of a region having similar predicted property values on map Ma is improved, a trend of change or a distribution of predicted property values can be more readily comprehended and man-hours required for comprehending predicted property values can be reduced.

FIGS. 7A to 7D are diagrams illustrating another example of map Ma.

As illustrated in FIG. 7A, the closer a predicted property value is to a reference value, image processor 134 may set a low shade to the predicted property value, and the further a predicted property value is from the reference value, image processor 134 may set a high shade to the predicted property value. For example, display method obtainer 133 obtains first display method information indicating the reference value from input unit 110 and outputs the first display method information to image processor 134. Image processor 134 sets a shade with respect to predicted property values in accordance with the reference value indicated in the first display method information.

In the case of the example illustrated in FIG. 7A, the reference value is a value in a range of 2.65 to 2.85 [eV]. Note that a low shade means a light color and a high shade means a dark color. In addition, image processor 134 may set a color representing a same range with respect to predicted property values in a case where the predicted property values are larger than the reference value and in a case where the predicted property values are smaller than the reference value. For example, as in the example illustrated in FIG. 7A, a predicted property value exceeding 2.85 [eV] is larger than the reference value and a predicted property value under 2.65 [eV] is smaller than the reference value. In addition, on map Ma, region r1 indicating predicted property values exceeding 2.85 [eV] and region r2 indicating predicted property values under 2.65 [eV] may have a color representing a same range. Conversely, image processor 134 may set colors representing mutually different ranges with respect to predicted property values in a case where the predicted property values are larger than the reference value and in a case where the predicted property values are smaller than the reference value. For example, on map Ma, region r1 indicating predicted property values exceeding 2.85 [eV] may be colored in a red range and region r2 indicating predicted property values under 2.65 [eV] may be colored in a blue range. Furthermore, image processor 134 sets white that is determined in advance with respect to a region indicating predicted property values equivalent to the reference value. However, the color set to the region is not limited to white and may be another color such as red. Moreover, predicted property values may be discretized as in the example illustrated in FIG. 6 or may not be discretized. When predicted property values are to be discretized, a plurality of regions in map Ma are to be displayed as though divided by, for example, contour lines.

As illustrated in FIG. 7B, image processor 134 may apply hatchings of a pattern determined in advance with respect to region r2 indicating predicted property values smaller than the reference value on map Ma. The pattern may be a striped pattern or a pattern made up of a plurality of dots as in the example illustrated in FIG. 7B. In other words, image processor 134 may superimpose a plurality of dots with respect to region r2. In addition, instead of applying a color of a shade in accordance with predicted property values to region r2, image processor 134 may only superimpose a plurality of dots.

As illustrated in FIG. 7C, image processor 134 may superimpose isoline L1 corresponding to the reference value on map Ma. For example, as illustrated in FIG. 7C, isoline L1 is superimposed on a boundary between region r3 in which predicted property values are in a range of the reference value and region r2 in which predicted property values are smaller than the range. Alternatively, image processor 134 may superimpose isoline L1 on map Ma without superimposing a plurality of dots on map Ma or only superimpose isoline L1 on map Ma. Accordingly, the user can readily comprehend a region of predicted property values in a vicinity of the reference value or a region of predicted property values exceeding the reference value.

As illustrated in FIG. 7D, image processor 134 may apply a color determined in advance or a color of a shade determined in advance with respect to region r4 (a striped region in the example illustrated in FIG. 7D) indicating predicted property values that do not satisfy a condition on map Ma. The color may be black. Alternatively, image processor 134 may apply hatchings of a pattern determined in advance with respect to region r4. In other words, image processor 134 may superimpose a striped pattern with respect to region r4. The condition described above is, for example, a condition that a predicted property value is equal to or larger than a lower limit value. In the example illustrated in FIG. 7D, the lower limit value is 2.45 [eV].

For example, display method obtainer 133 obtains first display method information indicating the condition or the lower limit value from input unit 110 and outputs the first display method information to image processor 134. Image processor 134 generates, according to the first display method information, map Ma such as that illustrated in FIG. 7D. A region smaller than the lower limit value is, for example, a region that need not be considered in material search. Therefore, in the example illustrated in FIG. 7D, the user can readily comprehend a region that need not be considered in material search.

In this manner, the first display method information according to the present embodiment indicates at least one of (a) to (e) below as a display method of predicted property values. (a) is a method of indicating a predicted property value identical to a reference value on map Ma using a color or a shade of color determined in advance. (b) is a method of indicating a predicted property value that is larger than the reference value using a color representing a first range and indicating a predicted property value that is smaller than the reference value using a color representing a second range. (c) is a method of superimposing a borderline on a boundary between a first region and a second region on map Ma, the first region being a region in which a predicted property value identical to the reference value is indicated and the second region being a region in which a predicted property value different from the reference value is indicated. (d) is a method indicating one of a third region and a fourth region on map Ma using a color or a shade of color determined in advance, the third region being a region in which a predicted property value that satisfies a specified condition is indicated and the fourth region being a region in which a predicted property value that does not satisfy the specified condition is indicated. (e) is a method of superimposing a plurality of dots or a striped pattern on one of the third region and the fourth region. For example, methods (a) and (b) are the display methods illustrated in FIGS. 7A to 7C, method (c) is the display method illustrated in FIG. 7C, method (d) is the display method illustrated in FIG. 7D, and method (e) is the method illustrated in FIGS. 7B to 7D. Note that the borderline in (c) corresponds to isoline L1 in FIG. 7C.

Accordingly, the user can readily comprehend a region indicating predicted property values that are equivalent to a reference value, a region indicating predicted property values that are larger or smaller than the reference value, a boundary between the regions, or a region satisfying a specified condition or a region not satisfying the specified condition.

In addition, in the example described above, the reference value is indicated by the first display method information. In other words, the reference value is a value designated by the user by an input operation with respect to input unit 110. However, the reference value may be a value determined from predicted property values. For example, the reference value may be a mean or a median of all predicted property values indicated on map Ma.

In other words, the reference value in the present embodiment is an average or a median of the predicted property values of the compounds, or is a value designated by a user.

Accordingly, segmentation of an entire map Ma into a plurality of regions or an appropriate reference value for displaying a borderline between the regions can be readily set.

FIG. 8 is a diagram illustrating another example of map Ma.

As illustrated in FIG. 8, image processor 134 may superimpose an arrow indicating a gradient of predicted property values on map Ma. For example, display method obtainer 133 obtains first display method information indicating superimposition of an arrow from input unit 110 and outputs the first display method information to image processor 134. According to the first display method information, image processor 134 calculates a gradient with respect to a predicted property value at each position on map Ma and superimposes an arrow indicating the gradient on the position. The arrow points in an orientation of the gradient and has a length proportional to a magnitude of the gradient. Accordingly, a magnitude and an orientation of a change in predicted property values in accordance with numerical values of respective variables x and y can be readily comprehended.

In other words, image processor 134 according to the present embodiment specifies a gradient of predicted property values indicated on map Ma and superimposes an arrow indicating an orientation and a magnitude of the gradient on map Ma. Accordingly, by looking at an arrow on map Ma, the user can readily comprehend a change with respect to variables x and y of the predicted property values.

FIGS. 9A and 9B are diagrams illustrating another example of map Ma.

As illustrated in FIG. 9A, image processor 134 may indicate a best predicted property value among predicted property values of a plurality of compounds having a same combination of numerical values of respective variables x and y at a position on map Ma corresponding to the same combination. The best predicted property value is, for example, a maximum predicted property value. The best predicted property value is also referred to as a best value and may be a minimum predicted property value or a predicted property value that is closest to a reference value determined in advance.

Specifically, when the same combination of numerical values of respective variables x and y is variables (x, y) = (0.1, 0.2), image processor 134 specifies predicted property values of a plurality of compounds that include variables (x, y) = (0.1, 0.2) in their respective composition formulae. In other words, image processor 134 narrows down predicted property values of a plurality of compounds that include variables (x, y) = (0.1, 0.2) in their respective composition formulae from the predicted property values of all compounds obtained by predicted value obtainer 132. Next, from the plurality of specified predicted property values, image processor 134 determines a best predicted property value as a best value. In addition, image processor 134 indicates the determined best value in a color of a shade in accordance with the best value at a position corresponding to variables (x, y) = (0.1, 0.2) on map Ma. Such a determination of a best value is executed with respect to each position on map Ma and a determined best value is indicated at the position on map Ma.

In addition, image processor 134 may specify predicted property values of a plurality of compounds for which a composition formula includes not only variables (x, y) = (0.1, 0.2) but also variables (M3, M3', M4, M4') = (La, Al, Ti, Zr). In other words, option data of variables (M3, M3', M4, M4') included in the respective composition formulae of the plurality of compounds may be the same or may be fixed. In this case, a plurality of composition formulae are represented by mutually differentiating combinations of respective numerical values of variables (a, b), and a best predicted property value is determined as a best value from among the predicted property values of compounds respectively indicated by the plurality of composition formulae.

For example, as illustrated in FIG. 9A, a best value (in other words, a band gap) at each position of map Ma exceeds 3.0 [eV]. In this case, regardless of the numerical values respectively assumed by variables x and y in a numerical range [0.0 to 1.0], the user can comprehend that a predicted property value exceeding 3.0 [eV] can be obtained by adjusting the respective numerical values of variables a and b. Therefore, a predicted property value can be displayed while focusing on a relationship between important variables used in coordinate axes (for example, variables x and y) and the predicted property value without having to consider variables that are less important in terms of which numerical values are assumed by the variables (for example, variables a and b).

Furthermore, as illustrated in FIG. 9B, image processor 134 may indicate the average value of predicted property values of a plurality of compounds having a same combination of numerical values of respective variables x and y at a position on map Ma corresponding to the same combination.

Specifically, when the same combination of numerical values of respective variables x and y is variables (x, y) = (0.1, 0.2), image processor 134 specifies predicted property values of a plurality of compounds that include variables (x, y) = (0.1, 0.2) in their respective composition formulae. In other words, image processor 134 narrows down predicted property values of a plurality of compounds that include variables (x, y) = (0.1, 0.2) in their respective composition formulae from the predicted property values of all compounds obtained by predicted value obtainer 132. Next, image processor 134 determines the average value of the plurality of specified predicted property values. In addition, image processor 134 indicates the calculated average value in a color of a shade in accordance with the average value at a position corresponding to variables (x, y) = (0.1, 0.2) on map Ma. Such a determination of the average value is executed with respect to each position on map Ma and the calculated average value is indicated at the position on map Ma.

In addition, image processor 134 may specify predicted property values of a plurality of compounds for which a composition formula includes not only variables (x, y) = (0.1, 0.2) but also variables (M3, M3', M4, M4') = (La, Al, Ti, Zr). In other words, option data of variables (M3, M3', M4, M4') included in the respective composition formulae of the plurality of compounds may be the same or may be fixed. In this case, a plurality of composition formulae are represented by mutually differentiating combinations of respective numerical values of variables (a, b), and the average value of the predicted property values of compounds respectively indicated by the plurality of composition formulae is calculated.

Furthermore, respective numerical ranges of variables a and b may be set. For example, display method obtainer 133 obtains first display method information indicating variables (a, b) = (0.05, 0.1) and outputs the first display method information to image processor 134. According to the first display method information, image processor 134 sets respective numerical ranges of variables a and b such as variable a $\in$ {0.0, 0.05, 0.1} and variable b $\in$ {0.0, 0.1, 0.2}. In other words, with respect to a numerical value of each of variables a and b indicated by the first display method information, image processor 134 includes the numerical value, one numerical value preceding the numerical value, and one numerical value following the numerical value in the numerical range. In this case, a plurality of composition formulae are represented by mutually differentiating combinations of respective numerical values of variables (a, b) within the numerical ranges and a mean of predicted property values of compounds respectively indicated by the plurality of composition formulae is calculated. Note that image processor 134 may calculate a differential value, a variance, or the like instead of a mean. Accordingly, a predicted property value can be displayed by also taking into consideration a change in the predicted property value when changing variables a and b and robustness of the predicted property value can be comprehended.

In this manner, in the present embodiment, when there is non-utilized variable a being a variable other than two variables x and y that are used in coordinate axes A1 and A2 of map Ma among the plurality of variables, first display method information indicates displaying a predicted property value using non-utilized variable a as a display method of a predicted property value. Accordingly, since a predicted property value is displayed by also using non-utilized variable a and without being limited to two variables x and y used in coordinate axes A1 and A2 of map Ma, material development can be appropriately supported.

Specifically, when there is non-utilized variable a being a variable other than two variables x and y that are used in coordinate axes A1 and A2 of map Ma among the plurality of variables, the first display method information according to the present embodiment indicates substituting non-utilized variable a with a first value, a second value, or each of numerical values within a prescribed numerical range as the display method of the predicted property value. In this case, in (a) when non-utilized variable a is to be substituted with the first value, image processor 134 generates map Ma indicating a predicted property value of each of a plurality of compounds having a composition represented using non-utilized variable a indicating the first value designated by the user. In addition, in (b) when non-utilized variable a is to be substituted with the second value, image processor 134 determines the second value such that the predicted property value to be indicated on map Ma satisfies a specified condition, and generates map Ma indicating a predicted property value of each of a plurality of compounds having a composition represented using non-utilized variable a indicating the determined second value. Furthermore, in (c) when non-utilized variable a is to be substituted with each of numerical values within a prescribed numerical range, image processor 134 calculates, for each of positions on map Ma, a mean of predicted property values of a plurality of compounds having compositions represented using two variables x and y indicating numerical values corresponding to the position. Image processor 134 generates, for each of the positions on map Ma, map Ma indicating the mean of the predicted property values calculated with respect to the position. In this case, respective non-utilized variables a of the plurality of compounds for which the mean of the predicted property values are to be calculated indicate mutually different numerical values within the prescribed numerical range. Note that a non-utilized variable is a variable that is not utilized in a coordinate axis of map Ma such as discrete variable a and is also referred to as a non-visualization variable. In addition, when there is variable b in addition to variable a as non-utilized variables, each of variables a and b is to be substituted with a first value, a second value, or each of numerical values within a prescribed numerical range. Furthermore, in the case of (a) described above, for example, map Ma illustrated in FIG. 5A is displayed. In other words, in the case of (a) described above, due to an input operation with respect to input unit 110 by the user, option data of a non-utilized variable such as discrete variable a is designated as the first value or fixed as the designated option data described earlier. In addition, the specified condition in the case of (b) described above is, for example, a condition that makes a predicted property value the best value described earlier.

Accordingly, in the case of (a), since a first value is designated for a non-utilized variable by the user, the user can optionally select a composition of a compound having a predicted property value displayed on map Ma. In addition, in the case of (b), by setting a specified condition as a condition that is required of a material search, map Ma satisfying the condition that is required of a material search can be easily displayed without the user having to, for example, designate a value of a non-utilized variable. As a result, material search can be made more efficient. In other words, material development can be appropriately supported. Furthermore, in the case of (c), a change in values that can be assumed by the non-utilized variable can be reflected onto the predicted property value on map Ma and robustness of the predicted property value with respect to the non-utilized variable can be improved.

[Processing operations]

FIG. 10 is a flowchart illustrating processing operations of display system 100 according to the present embodiment.

(Step S111)

First, search range obtainer 131 obtains a search range signal outputted from input unit 110 in accordance with an input operation with respect to input unit 110 by the user. For example, the search range signal is an input signal indicating a search range of compounds as illustrated in FIG. 2. In other words, search range obtainer 131 obtains a search range. In addition, search range obtainer 131 outputs the search range signal to predicted value obtainer 132.

(Step S112)

Predicted value obtainer 132 obtains the search range signal from search range obtainer 131 and combines respective pieces of option data of a plurality of variables according to a search range indicated by the search range signal. For example, all possible combinations in the search range are generated. In addition, by inputting respective composition formulae of the plurality of compounds or, in other words, the respective combinations of the plurality of compounds into a predictor of predictor database 120, predicted value obtainer 132 obtains predicted property values of the compounds. Predicted value obtainer 132 associates the predicted property values of the compounds with composition formulae of the compounds and outputs the associated predicted property values and composition formulae to image processor 134.

In the present embodiment, predicted value obtainer 132 obtains a predictor from predictor database 120 and obtains predicted property values using the predictor. However, predicted value obtainer 132 may obtain a predicted property value of each of the plurality of compounds from at least one predictor for predicting a property value of a compound, the at least one predictor being stored in predictor database 120. Even in this case, an appropriate predicted property value can be obtained for each of the plurality of compounds.

(Step S113)

Display method obtainer 133 obtains first display method information outputted from input unit 110 in accordance with an input operation with respect to input unit 110 by the user. For example, the first display method information indicates a display method of a predicted property value such as those illustrated in FIGS. 5A to 9B. In addition, display method obtainer 133 outputs the first display method information to image processor 134.

(Step S114)

Image processor 134 obtains a predicted property value and a composition formula of each of a plurality of compounds from predicted value obtainer 132 and obtains first display method information from display method obtainer 133. In addition, image processor 134 generates map Ma indicating a predicted property value of each of the plurality of compounds according to the display method indicated by the first display method information and outputs an image including map Ma to displayer 140.

(Step S115)

Displayer 140 obtains the image from image processor 134 and displays the image.
By executing processing of steps S111 to S115 described above, display of a predicted property value or, in other words, property display of a compound being a material is performed.

(Variation 1 of Embodiment 1A)

In the embodiment described above, variables x and y are used but variables a and b are not used in coordinate axes of map Ma. Image processor 134 according to the present variation generates a map by using not only variables x and y but also variables a and b in coordinate axes.
FIG. 11 is a diagram illustrating an example of a map in the present variation.
As illustrated in FIG. 11, image processor 134 generates image map Mb including an array of a plurality of maps Ma and displays image map Mb on displayer 140. In this case, image processor 134 assigns two variables not assigned to coordinate axes A1 and A2 of map Ma to two coordinate axes A3 and A4 of image map Mb. The variables to be assigned to two coordinate axes A3 and A4 are, for example, discrete variables a and b.
Note that map Ma may also be referred to as image element map Ma in order to distinguish map Ma from image map Mb. Furthermore, both image element map Ma and image map Mb may also be simply referred to as maps.
The discrete variable a includes five pieces of option data, namely, 0.0, 0.05, 0.1, 0.15, and 0.2, and the discrete variable b includes four pieces of option data, namely, 0.0, 0.1, 0.2, and 0.3. Therefore, the number of image element maps Ma for each of the combinations of the categorical variables in FIG. 11 is $5 \times 4 = 20$. In other words, image map Mb includes an array of $5 \times 4$-number of image element maps Ma.
In the example described above, when continuous variables x and y and discrete variables a and b are compared with each other, continuous variables x and y which have more combinations are assigned to the coordinate axes of image element maps Ma and discrete variables a and b which have fewer combinations are assigned to the coordinate axes of the array of image element maps Ma in order to facilitate visual understanding by the user. The user may optionally assign each of the variables or image processor 134 may calculate the number of combinations to automatically assign each of the variables.

(Variation 2 of Embodiment 1A)

While the plurality of range variables according to the embodiment described above include categorical variables M3, M3', M4, and M4', discrete variables a and b, and continuous variables x and y, the range variables may also include other variables. For example, the plurality of range variables according to the present variation includes, in addition to the respective variables described earlier, one or more processing variables that indicate a condition used in a generation process of a compound.
FIG. 12 is a diagram illustrating an example of a processing variable according to the present variation.
A processing variable is a variable that is also referred to as a processing condition, and examples of a processing variable include variable Pa indicating a calcination method of a compound, variable Pb indicating a calcination time of a compound, and variable Pc indicating a calcination temperature of a compound. For example, variable Pa indicates a solid reaction method, a ball mill, or the like as option data. For example, variable Pb indicates 1 hour, 2 hours, 3 hours, or the like as option data. For example, variable Pc indicates 100°C, 110°C, 120°C, 130°C, 140°C, 150°C, 160°C, or the like as option data.

For example, search range obtainer 131 obtains each piece of option data which variables M3, M3', M4, M4', a, b, x, and y may respectively assume and each piece of option data which variables Pa, Pb, and Pc may respectively assume. Predicted value obtainer 132 generates all combinations of option data which variables M3, M3', M4, M4', a, b, x, y, Pa, Pb, and Pc may respectively assume. In addition, predicted value obtainer 132 obtains a predictor from predictor database 120 and by inputting all of the generated combinations (in other words, composition formulae and processing condition) into the predictor, obtains a predicted property value of each of the plurality of compounds. Hereinafter, a composition formula of a compound or a composition formula and a processing condition of a compound will also be referred to as a composition of the compound. Furthermore, processing variables are not limited to variables Pa, Pb, and Pc and may be any kind of variables. Image processor 134 may assign one or more variables among variables Pa, Pb, and Pc to coordinate axes of map Ma or Mb.

Accordingly, since not only a composition formula but also a processing condition is to be included in a search range, a predicted property value of a compound generated under a condition that satisfies the processing condition of the search range can be obtained and a map indicating the obtained predicted property value can be displayed.

[EMBODIMENT 1B]

A display system according to the present embodiment displays a predicted property value of each of a plurality of compounds in the form of a map in a similar manner to Embodiment 1A and also superimposes an experimental property value of each of one or more compounds on the map. Note that among the respective components according to the present embodiment, the same components as in Embodiment 1A are given the same reference numerals as in Embodiment 1A and detailed descriptions thereof are omitted.

[Configuration of display system 200]

FIG. 13 is a block diagram illustrating an example of a configuration of display system 200 according to the present embodiment. Display system 200 illustrated in FIG. 13 includes input unit 110, predictor database 120, property display device 230, displayer 140, and experiment database (DB) 150. Note that property display device 230 is an example of an information display device.

Property display device 230 according to the present embodiment obtains an experimental property value that is a property value of a compound obtained by an experiment from experiment database 150, generates a map on which the experimental property value is superimposed, and displays the map on displayer 140. Property display device 230 includes search range obtainer 131, predicted value obtainer 132, display method obtainer 133, experimental value obtainer 232, and image processor 234. Note that property display device 230 may be constituted of, for example, a processor such as a CPU and a memory. In this case, the processor functions as property display device 230 by, for example, executing a computer program stored in the memory. Note that the memory may be volatile or non-volatile or may be constituted of a volatile memory and a non-volatile memory.

[Experiment database 150]

Experiment database 150 is a recording medium that stores composition formulae (in other words, chemical formulae) of compounds, compound identification information of the compounds, and experimental data indicating experimental property values of the compounds. The recording medium is, for example, a hard disk drive, a RAM (Random Access Memory), a ROM (Read Only Memory), or a semiconductor memory. Furthermore, the recording medium may be volatile or non-volatile. When a search range includes a processing variable, experimental data thereof also includes, in advance, experiment process information indicating processing conditions used in experiments.

FIG. 14 is a diagram illustrating an example of experimental data stored in experiment database 150. As in the example illustrated in FIG. 14, experimental data indicates, with respect to each of a plurality of compounds, a composition formula, compound identification information, and an experimental property value of the compound. The compound identification information is a so-called ID and may be any information that enables a compound to be identified such as a name, a sign, or numerals. For example, experimental data indicates a composition formula (formula) "$Li_{1.45}La_{0.045}Ti_{1.1}Al_{0.005}O_3$", of a compound, an ID "000001-00001-001" of the compound, and an experimental property value (exp. data) of the compound "2.349". The composition formula is defined by (M3, M3', M4, M4', a, b, x, y) = (La, Al, Ti, Zr, 0.05, 0.1, 0.1, 0). The ID "000001-00001-001" illustrated in FIG. 14 is made up of numerals (000001, 00001, and 001) of three levels. For example, IDs are registered such that first-level numbers "000001" to "000005" are assigned to five chemical formulae whose composition ranges of the elements Zr and Ti included in a compound differ from each other. By giving an ID that enables a composition formula of a compound to be classified, a plurality of users can easily manage experiment database 150. In addition, a type of an experimental property value illustrated in FIG. 14 is optionally selected in accordance with a compound. For example, an experimental property value is a degree of conductivity in the case of a battery material and an experimental property value is a thermoelectric conversion performance index or the like in the case of a thermoelectric conversion material.

Note that experimental data may indicate a processing condition and analysis information used to generate a compound. Examples of a processing condition include a calcination method, a calcination time, and a calcination temperature. Examples of analysis information include a crystal structure of a compound, a type of a raw material, and a presence or absence of residual raw material. The presence or absence of residual raw material is information indicating whether or not a raw material used for synthesis or generation of a compound remains after the synthesis or the generation of the compound.

[Experimental value obtainer 232]

Experimental value obtainer 232 obtains a composition and an experimental property value of each of one or more compounds that have been experimented on from experiment database 150. The composition may be a composition formula of the compound or a composition formula and a processing condition of the compound. For example, experimental value obtainer 232 obtains a search range from search range obtainer 131 and obtains an experimental property value of a compound having the composition included in the search range together with the composition. Experimental value obtainer 232 outputs the composition and the experimental property value of each of the one or more compounds to image processor 234.

[Display method obtainer 133]

Display method obtainer 133 obtains first display method information indicating a display method of a predicted property value and second display method information indicating a display method of an experimental property value from input unit 110. In addition, display method obtainer 133 outputs the first display method information and the second display method information to image processor 234.

[Image processor 234]

Image processor 234 obtains a composition and a predicted property value of each of a plurality of compounds from predicted value obtainer 132 and obtains a composition and an experimental property value of each of one or more compounds from experimental value obtainer 232. In addition, image processor 234 receives the first display method information and the second display method information from display method obtainer 133. Image processor 234 generates a map indicating predicted property values of the plurality of compounds according to the display method indicated by the first display method information. Furthermore, image processor 234 superimposes the experimental property value of each of the one or more compounds on a map corresponding to a composition of the compound. At this point, image processor 234 superimposes the experimental property value on the map according to the display method indicated by the second display method information. In addition, image processor 234 outputs an image including the map on which experimental property values of the one or more compounds have been superimposed to displayer 140. Note that the map may be image element map Ma or image map Mb.

[Displayer 140]

Displayer 140 obtains an image from image processor 234 and displays the image or, in other words, displays an image including the map on which experimental property values of the one or more compounds have been superimposed.

[Specific examples of map]

FIG. 15 is a diagram illustrating an example of a map.

As an example, image processor 234 superimposes an experimental property value on image element map Ma according to Embodiment 1A as a mark with a circular shape or the like. For example, a color or a shade of a color of the mark with a circular shape or the like indicates an experimental property value. In other words, image processor 234 superimposes a mark having a color or a shade of a color corresponding to an experimental property value of a compound generated by an experiment on image element map Ma at a position corresponding to each of the combinations described above that indicate a composition of the compound. The superimposition of the mark is also called superimposition of an experimental property value. An example of an experimental property value according to the present disclosure is, but not limited to, a band gap of a compound obtained as a result of an experiment.

Accordingly, when an experimental property value deviates from a predicted property value, a difference between a color or a shade of color of a mark corresponding to the experimental property value and a color or a shade of color of a periphery of the mark can be highlighted. As a result, it becomes easy to visually understand the deviation of the experimental property value from the predicted property value. In other words, when a color or a shade of color of a mark corresponding to an experimental property value and a color or a shade of color corresponding to a predicted property value are visually the same, the user can immediately visually understand that the experimental property value and the predicted property value are substantially the same.

In this manner, property display device 230 according to the present embodiment includes experimental value obtainer 232 which obtains an experimental property value of each of one or more compounds that have been experimented on. In addition, image processor 234 superimposes the experimental property value of each of the one or more compounds that have been experimented on onto a position on image element map Ma that corresponds to a composition of the compound and generates an image including image element map Ma on which the experimental property value has been superimposed.

Accordingly, a predicted property value and an experimental property value can be easily compared in a range that is indicated in image element map Ma, and thus material search can be made more efficient.

Furthermore, property display device 230 according to the present embodiment includes display method obtainer 133 which obtains second display method information indicating a display method of an experimental property value. In addition, image processor 234 superimposes an experimental property value on image element map Ma according to the second display method information.

Accordingly, since an experimental property value is superimposed on image element map Ma according to a display method indicated by second display method information, the experimental property value can be displayed in a mode suitable for an object of material search by the user according to settings of the display method. As a result, material search can be made more efficient, and material development can be appropriately supported.

FIG. 16 is a diagram illustrating another example of a map.

As illustrated in FIG. 16, image processor 234 may cause (1) a correspondence relationship between an experimental property value and a shade corresponding to the experimental property value and (2) a correspondence relationship between a predicted property value and a shade corresponding to the predicted property value to coincide with each other. In other words, image processor 234 may cause a scale of shade with respect to experimental property values and a scale of shade with respect to predicted property values to coincide with each other. Accordingly, when a predicted property value and an experimental property value are the same value, a same shade is assigned to the values and a color of the shade is applied to image element map Ma. As a result, a degree of similarity between a predicted property value and an experimental property value can be readily comprehended.

In other words, in property display device 230 according to the present embodiment, a predicted property value is to be displayed on image element map Ma according to a first display mode which is a color or a shade of color that is in accordance with the predicted property value. In addition, an experimental property value is to be superimposed on image element map Ma as a mark having a second display mode which is a color or a shade of color that is in accordance with the experimental property value. In this case, second display method information indicates, as a display method of the experimental property value, matching a scale of the second display mode with respect to the experimental property value with a scale of the first display mode with respect to the predicted property value.

Accordingly, since the scale of the predicted property value and the scale of the experimental property value match each other, comparison between the predicted property value and the experimental property value is further facilitated.

In addition, as illustrated in FIG. 16, while causing a scale of shade with respect to experimental property values and a scale of shade with respect to predicted property values to coincide with each other, image processor 234 may perform discretization with respect to the predicted property values and may not perform discretization with respect to the experimental property values. Experimental property values are often obtained in a focused manner in some regions on image element map Ma. In addition, the experimental property values are often similar to each other. In consideration thereof, due to performing discretization of predicted property values in order to enhance visibility but not performing discretization of experimental property values, minute differences among experimental property values can be readily interpreted.

FIG. 17 is a diagram illustrating another example of a map.

As illustrated in FIG. 17, when marks of a plurality of experimental property values overlap with each other, image processor 234 may arrange experimental property values such that the more favorable an experimental property value, the closer a mark indicating the experimental property value to a front side (in other words, further in front). In other words, image processor 234 may determine an order of superimposition of experimental property values.

For example, display method obtainer 133 obtains second display method information indicating an arrangement method of a plurality of marks as a display method of experimental property values from input unit 110 and outputs the second display method information to image processor 234. When image processor 234 receives the second display method information from display method obtainer 133, in accordance with the second display method information, image processor 234 arranges experimental property values such that the more favorable an experimental property value, the closer a mark indicating the experimental property value to a front side (in other words, further in front). Note that an experimental property value being favorable may mean that the experimental property value is large, mean that the experimental property value is small, or mean that the experimental property value is close to a predicted property value indicated at a same position as the experimental property value on image element map Ma.

Accordingly, information that is beneficial to material search can be presented. In other words, when an experimental property value is superimposed on image element map Ma, as many marks of experimental property values as the variables not used in coordinate axes of image element map Ma overlap with each other at a same position. In addition, a plurality of marks arranged at positions close to each other on image element map Ma overlap with each other. When a plurality of marks overlap with each other in this manner, all of or a part of the marks other than a foremost mark become hidden by the foremost mark. By comparison, in the example illustrated in FIG. 17, since a most favorable experimental property value is displayed in the forefront, information that is beneficial to material search can be presented.

As described above, in property display device 230 according to the present embodiment, when a plurality of experimental property values are to be respectively superimposed on image element map Ma as marks overlapping with each other, the second display method information indicates a rule stipulating an order of overlapping of the marks as the display method of the experimental property values. In addition, image processor 234 superimposes the respective marks of the plurality of experimental property values on image element map Ma so that the marks overlap with each other according to the rule. Furthermore, the rule stipulates that (a) the larger the experimental property value, the closer the mark of the experimental property value is arranged to a front side, (b) the closer the experimental property value is to a prescribed value, the closer the mark of the experimental property value is arranged to a front side, or (c) the closer the experimental property value is to a predicted property value indicated at a position on image element map Ma on which the experimental property value is superimposed, the closer the mark of the experimental property value is arranged to a front side.

Accordingly, a situation where a mark indicating a favorable experimental property value such as a large experimental property value, an experimental property value close to a prescribed value, or an experimental property value close to a predicted property value is hidden behind marks indicating other experimental property values and becomes less visible can be suppressed and material search can be made more efficient.

FIG. 18 is a diagram illustrating another example of a map.

While experimental value obtainer 232 obtains an experimental property value of a compound having a composition included in the search range as described above, alternatively, experimental value obtainer 232 may obtain all experimental property values indicated in experimental data regardless of the search range. In doing so, a composition of a compound corresponding to an experimental property value obtained by experimental value obtainer 232 may not match a composition on image element map Ma. Specifically, as illustrated in FIG. 18, a composition formula of a compound corresponding to an experimental property value obtained by experimental value obtainer 232 is a composition formula of a nitride, an example of which is $Li_{1.45}(La_{0.5}Ga_{0.5})_{0.05}(Ti_{0.5}Zr_{0.5})_{1.1}N_3$. On the other hand, a composition formula indicated by image element map Ma is a composition formula of an oxide, an example of which is $Li_{1.45}(La_{1-x}Ga_x)_{0.05}(Ti_{1-y}Zr_y)_{1.1}O_3$. Focusing on variables x and y, the composition formula "$Li_{1.45}(La_{0.5}Ga_{0.5})_{0.05}(Ti_{0.5}Zr_{0.5})_{1.1}N_3$" corresponds to a position represented by (x, y) = (0.5, 0.5) on image element map Ma. However, the composition formula of the nitride does not match the composition formula of the oxide and a position corresponding to the composition formula of the nitride does not exist on image element map Ma.

In such a case, image processor 234 does not superimpose such a mark indicating an experimental property value corresponding to the composition formula of the nitride on image element map Ma.

In addition, when a processing condition is respectively included in a search range and experimental data, a processing condition of a compound corresponding to an experimental property value obtained by experimental value obtainer 232 may not match a processing condition corresponding to image element map Ma. A processing condition corresponding to image element map Ma is one combination of processing variables used when obtaining all predicted property values indicated on image element map Ma. In a specific example, a calcination time "5 hours" of a compound corresponding to an experimental property value does not match a calcination time "10 hours" of variable Pb used to obtain all predicted property values indicated on image element map Ma.

In such a case, image processor 234 does not superimpose a mark indicating an experimental property value corresponding to the calcination time "5 hours" on image element map Ma that corresponds to the calcination time "10 hours".

In this manner, misunderstanding that may arise due to the superimposition of an experimental property value not corresponding to image element map Ma can be suppressed by not performing the superimposition of the experimental property value.

Alternatively, image processor 234 may superimpose an experimental property value of the nitride described above on a map by setting a new variable for indicating the composition formula of the nitride with respect to the map.

Alternatively, even if a composition formula and a processing condition corresponding to an experimental property value do not match a composition formula and a processing condition corresponding to image element map Ma, image processor 234 may superimpose an experimental property value satisfying a specified condition on image element map Ma. The specified condition may be a condition that, among a composition formula and a processing condition corresponding to an experimental property value, a portion that does not match a composition formula and a processing condition corresponding to image element map Ma is, for example, a portion designated by the user. For example, the element N in the composition formula of the nitride described above differs from the element O in the composition formula corresponding to image element map Ma. Therefore, when the element O is a portion designated by the user, image processor 234 may superimpose an experimental property value corresponding to the composition formula of the nitride on position (x, y) = (0.5, 0.5) on image element map Ma. The position (x, y) = (0.5, 0.5) is a position corresponding to a composition that is closest to the composition formula of the nitride on image element map Ma. Alternatively, the specified condition is a condition that a composition formula and a processing condition corresponding to an experimental property value is within a search range.

Alternatively, image processor 234 may calculate a degree of similarity between a composition formula and a processing condition corresponding to an experimental property value and a composition formula and a processing condition corresponding to image element map Ma and, if the degree of similarity is equal to or greater than a threshold, image processor 234 may superimpose the experimental property value on image element map Ma. For example, image processor 234 may indicate a composition formula and a processing condition corresponding to an experimental property value by a vector, indicate a composition formula and a processing condition corresponding to image element map Ma by a vector, and calculate a norm of a difference between the vectors as a distance. The shorter the distance, the higher the degree of similarity between the composition formula and the processing condition corresponding to the experimental property value and the composition formula and the processing condition corresponding to image element map Ma and, conversely, the longer the distance, the lower the degree of similarity. In addition, when the distance is equal to or shorter than a threshold or, in other words, when the degree of similarity is equal to or higher than a threshold, image processor 234 superimposes the experimental property value on image element map Ma. Note that the vectors may include a coefficient of each of the elements presented in the periodic table and a value of each of a plurality of processing variables as elements of the vectors. Furthermore, image processor 234 may normalize distances such that a maximum value of distances is 1 and a minimum value of distances is 0, determine whether or not a normalized distance is equal to or shorter than a threshold, and when equal to or shorter than the threshold, superimpose the experimental property value on image element map Ma.

As described above, in property display device 230 according to the present embodiment, when a position on image element map Ma corresponding to a composition of a compound having an experimental property value does not exist, second display method information indicates, as a display method of the experimental property value, (a) superimposing the experimental property value on a position corresponding to a composition closest to a composition of a compound having the experimental property value on image element map Ma or (b) not superimposing the experimental property value on image element map Ma. In this case, image processor 234 performs processing regarding superimposition of the experimental property value on image element map Ma according to the second display method information.

Accordingly, in the case of (a), even an experimental property value of a compound having a composition that does not correspond to any of the positions on image element map Ma is superimposed at position corresponding to a composition that is closest to the composition. Therefore, since even an experimental property value of a compound having a composition that does not correspond to image element map Ma is displayed by being suitably associated with image element map Ma, a material search by the user can be made more efficient. In addition, in the case of (b), since superimposition of an experimental property value not corresponding to image element map Ma is not performed, misunderstanding that may arise due to the superimposition of the experimental property value can be suppressed.

In the example described above, image processor 234 determines whether or not the experimental property value obtained by experimental value obtainer 232 is to be superimposed. However, experimental value obtainer 232 may obtain only experimental property values to be superimposed on image element map Ma from among all experimental property values indicated in experimental data in experiment database 150 and output the obtained experimental property values to image processor 234. In this case, in a similar manner to the determination method by image processor 234 described above, experimental value obtainer 232 determines whether or not an experimental property value indicated in experimental data is an experimental property value to be superimposed on image element map Ma. Subsequently, image processor 234 superimposes the experimental property value obtained by experimental value obtainer 232 on image element map Ma without determining whether or not the experimental property value is to be superimposed.

FIG. 19 is a diagram illustrating another example of a map.

Image processor 234 may determine whether or not to superimpose the experimental property value on image element map Ma according to the experimental property value itself. For example, display method obtainer 133 obtains second display method information indicating an experimental threshold from input unit 110 and outputs the second display method information to image processor 234. The experimental threshold is, for example, 2.0 [eV]. For example, image processor 234 changes, according to the second display method information, image element map Ma illustrated in (a) in FIG. 19 to image element map Ma illustrated in (b) in FIG. 19. In image element map Ma illustrated in (a) in FIG. 19, experimental property values within a band gap range of 0.0 to 0.35 [eV] are superimposed. On the other hand, in image element map Ma illustrated in (b) in FIG. 19, experimental property values of band gap 2.0 [eV] or more are superimposed and experimental property values below band gap 2.0 [eV] are not superimposed. In other words, among experimental property values within a band gap range of 0.0 to 3.5 [eV], image processor 234 determines not to superimpose experimental property values under 2.0 [eV] on image element map Ma and determines to superimpose experimental property values equal to or over 2.0 [eV] on image element map Ma. Accordingly, experimental property values are narrowed down. As a result, image processor 234 generates image element map Ma illustrated in (b) in FIG. 19 and displays an image including image element map Ma on displayer 140.

As described above, in property display device 230 according to the present embodiment, second display method information indicates, among the experimental property values of one or more compounds that have been experimented on, superimposing experimental property values equal to or larger than a first threshold determined in advance on image element map Ma and not superimposing experimental property values smaller than the first threshold as a display method of experimental property values. For example, the first threshold is the experimental threshold described above and a specific example thereof is 2.0 [eV].

Accordingly, for example, when a large number of experimental property values are obtained, unimportant experimental property values such as those handled as noise can be prevented from being superimposed on image element map Ma, the large number of obtained experimental property values can be narrowed down to only important experimental property values, and the important experimental property values can be superimposed on image element map Ma. As a result, the important experimental property values superimposed on image element map Ma can be made more conspicuous and material search can be made more efficient.

FIG. 20A is a diagram illustrating another example of a map.

As illustrated in FIG. 20A, image processor 234 may highlight and superimpose an experimental property value satisfying a specified condition. The specified condition is that an experimental property value is obtained by experiments performed during a certain latest period of time or, in other words, by experiments performed between a present time point and a certain period of time ago. The certain period of time is, for example, two weeks. Alternatively, the specified condition is that an experimental property value is obtained by a certain number of experiments performed most recently. The certain most recent number is, for example, three.

In other words, display method obtainer 133 obtains second display method information that satisfies the specified condition described above from input unit 110 and outputs the second display method information to image processor 234. In accordance with the second display method information, image processor 234 superimposes an experimental property value satisfying the specified condition on image element map Ma by emphasizing the experimental property value more than experimental property values not satisfying the specified condition as illustrated in FIG. 20A. Specifically, experimental property values obtained by experiments during latest two weeks or, in other words, experiments between the present time point and two weeks ago are superimposed on image element map Ma as greatly emphasized marks and other experimental property values are superimposed on image element map Ma as small marks. Alternatively, each of three most recent experimental property values or, in other words, each of three experimental property values obtained by three most recent experiments is superimposed on image element map Ma as a greatly emphasized mark. On the other hand, experimental property values obtained by experiments prior to the three experiments are superimposed on image element map Ma as small marks.

FIG. 20B is a diagram illustrating another example of a map.

As illustrated in FIG. 20B, image processor 234 may highlight and superimpose an experimental property value satisfying a condition of being larger than a reference value. In other words, in this case, the specified condition described above is that the experimental property value is larger than the reference value.

For example, display method obtainer 133 obtains second display method information indicating the reference value from input unit 110 and outputs the second display method information to image processor 234. In accordance with the second display method information, image processor 234 superimposes an experimental property value that is larger than the reference value on image element map Ma by emphasizing the experimental property value more than an experimental property value equal to or smaller than the reference value as illustrated in FIG. 20B. Specifically, experimental property values that are larger than the reference value are superimposed on image element map Ma as greatly emphasized marks and experimental property values that are equal to or smaller than the reference value are superimposed on image element map Ma as small marks. Note that, conversely, the specified condition may be the experimental property value being smaller than the reference value. In addition, the reference value may be the reference value used in the examples illustrated in FIGS. 7A to 7B.

Alternatively, as illustrated in FIG. 20B, image processor 234 may emphasize and superimpose an experimental property value satisfying a condition that a difference from a predicted property value is smaller than a difference threshold.

In other words, in this case, the specified condition described above is that a difference between an experimental property value and a predicted property value indicated at a same position on image element map Ma is smaller than a difference threshold. When the predicted property value and the experimental property value are band gaps, the difference threshold is, for example, 0.5 [eV].

For example, display method obtainer 133 obtains second display method information indicating the difference threshold from input unit 110 and outputs the second display method information to image processor 234. In accordance with the second display method information, image processor 234 superimposes an experimental property value that has a difference from a predicted property value that is smaller than the difference threshold on map Ma by emphasizing the experimental property value more than an experimental property value that has the difference that is equal to or larger than the difference threshold as illustrated in FIG. 20B. Specifically, experimental property values that has a difference from a predicted property value that is smaller than the difference threshold are superimposed on image element map Ma as greatly emphasized marks and experimental property values that has a difference from a predicted property value that is equal to or larger than the difference threshold are superimposed on image element map Ma as small marks. Note that, conversely, the specified condition may be that a difference between an experimental property value and a predicted property value indicated at a same position on image element map Ma is equal to or larger than a difference threshold.

As described above, in property display device 230 according to the present embodiment, the second display method information indicates, as the display method for an experimental property value, superimposing an experimental property value that satisfies a specified condition on image element map Ma in a mode where the experimental property value is more emphasized than an experimental property value not satisfying the specified condition among the experimental property values of the one or more compounds that have been experimented on. In this case, image processor 234 superimposes an experimental property value satisfying the specified condition on image element map Ma in a mode of emphasizing the experimental property value according to the second display method information.

Accordingly, the user can visually recognize whether each of the experimental property values which are displayed by being superimposed on image element map Ma satisfies the specified condition. Therefore, material search can be made more efficient.

Furthermore, in property display device 230 according to the present embodiment, the above-described specified condition indicates: (a) that the experimental property value is an experimental property value obtained within a predetermined period from present time; (b) that the experimental property value is one of a predetermined number of experimental property values obtained most recently; (c) that the experimental property value is larger than or equal to a second threshold that is predetermined, or (d) that a difference between the experimental property value and a predicted property value obtained for a compound having a same composition as a compound having the experimental property value is larger than or equal to a third threshold or smaller than the third threshold, the third threshold being predetermined. For example, the second threshold is the aforementioned reference value and the third threshold is the aforementioned difference threshold.

Accordingly, with (a) and (b), the user can easily visually recognize a new experimental property value. Furthermore, in the case of (c), the user can easily visually recognize, for example, an important experimental property value. In the case of (d), the user can easily visually recognize an experimental proper value that is close to the predicted property value.

FIG. 21 is a diagram illustrating another example of a map.

As illustrated in FIG. 21, image processor 234 may superimpose a mark of an experimental property value on image element map Ma in a mode according to a non-utilized variable that corresponds to the experimental property value. For example, a non-utilized variable is associated with an experimental property value of each compound in experimental data in experiment database 150. For example, a non-utilized variable is a variable indicating a processing condition and a more specific example is a calcination temperature. In addition, a non-utilized variable may be a variable indicating a property of a type that differs from an experimental property value. In other words, a non-utilized variable may be described as a variable not used in an coordinate axis of image element map Ma.

For example, display method obtainer 133 obtains second display method information prompting display according to a non-utilized variable to be performed from input unit 110 and outputs the second display method information to image processor 234. Experimental value obtainer 232 outputs a non-utilized variable of each compound together with a composition and an experimental property value of the compound to image processor 234. According to the second display method information, image processor 234 forms a mark of an experimental property value in a mode according to data indicated by the non-utilized variable that corresponds to the experimental property value and superimposes the formed mark on image element map Ma as illustrated in FIG. 21. When the non-utilized variable is a calcination temperature, data indicated by the non-utilized variable is, for example, 500°C, 750°C, or 1000°C. Specifically, image processor 234 forms a mark of an experimental property value of a compound calcined at a calcination temperature of 500°C in a circular shape, forms a mark of an experimental property value of a compound calcined at a calcination temperature of 750°C in a triangular shape, and forms a mark of an experimental property value of a compound calcined at a calcination temperature of 1000°C in a square shape.

In addition, while one non-utilized variable is used in the examples described above, two or more non-utilized variables may be used. In this case, image processor 234 calculates a product of the number of possible outcomes of each of two or more non-utilized variables and determines as many mutually different modes of marks as the number of products. For example, a calcination method, a calcination temperature, and a calcination time exist as three non-utilized variables. When the number of possible outcomes of the calcination method is two, the number of possible outcomes of the calcination time is seven, and the number of possible outcomes of the calcination temperature is three, image processor 234 determines $2 \times 7 \times 3 = 42$ mutually different modes of marks. In addition, among the 42 modes of marks, image processor 234 selects a mode in accordance with a calcination method, a calcination temperature, and a calcination time corresponding to an experimental property value and forms the mark of the experimental property value in the selected mode.

For example, a mode of a mark may be a shape, a color, a size, or the like of the mark, a color, a width, or the like of an edge line of the mark, or the like. Note that the larger the number of mutually different modes of marks, the lower the visibility or distinguishability of the marks. Therefore, a product of the number of possible outcomes of non-utilized variables is preferably a finite number that enables the marks to be distinguishable. For example, the number of mutually different modes of marks is two or more and 15 or less.

As described above, in property display device 230 according to the present embodiment, when at least one non-utilized variable is associated with each of the experimental property values of one or more compounds that have been experimented on, image processor 234 calculates, as a total number of classifications of forms of marks, a product of the number of possible outcomes of each of the at least one non-utilized variable. In addition, for each of the experimental property values of the one or more compounds that have been experimented on, image processor 234 selects a form in accordance with a combination of data indicated by each of the at least one non-utilized variable associated with the experimental property value among forms of the number of types and superimposes a mark of the selected form on image element map Ma. For example, the at least one non-utilized variable indicates, as data, a calcination temperature, a calcination time, and a calcination method used in calcination of a compound having the experimental property value.

Accordingly, the data of each of the at least one non-utilized variable associated with each of the experimental property values to be displayed by being superimposed on image element map Ma can easily be visually recognized by the user from the form of the mark of the experimental property value. Therefore, material search can be made more efficient, and material development can be appropriately supported.

Furthermore, in property display device 230 according to the present embodiment, each of the at least one non-utilized variable indicates a processing condition to be used in generating a compound having the experimental property value or an attribute of the compound having the experimental property value.

Accordingly, the processing condition or the attribute of the compound having the experimental property value to be displayed by being superimposed on image element map Ma can easily be visually recognized by the user from the form of the mark of the experimental property value.

Furthermore, in property display device 230 according to the present embodiment, image processor 234 calculates a number such as at least 2 and at most 15 as the number of types of the form of the mark.

For example, when the number of types of the form of the mark is 16 or more, identifying such forms becomes difficult. Therefore, since the number of types is at least 2 and at most 15, identifiability of such forms can be enhanced. As a result, the data of each of the at least one non-utilized variable associated with each of the experimental property values to be displayed by being superimposed on image element map Ma can more easily be visually recognized by the user. Therefore, material search can further be made more efficient. In the example described above, when the number of possible outcomes of the calcination method is two, the number of possible outcomes of the calcination time is seven, and the number of possible outcomes of the calcination temperature is three, image processor 234 determines $2 \times 7 \times 3 = 42$ mutually different modes of marks. In this case, since the number of types of the form of the marks is 42 which is 16 or more, image processor 234 may omit the calcination temperature among the three non-utilized variables. Accordingly, image processor 234 may determine modes of $2 \times 7 = 14$ mutually different marks to hold the number of types of forms of marks to or below 15.

FIG. 22A is a diagram illustrating another example of a map.

As illustrated in FIG. 22A, image processor 234 may superimpose a mark of an experimental property value on image element map Ma in a mode according to analysis information that corresponds to the experimental property value. As described above, analysis information indicates, for example, a crystal structure of a compound. For example, types of a crystal structure include crystal phase A and crystal phase B. In addition, analysis information is associated with an experimental property value of each compound in experimental data in experiment database 150.

For example, display method obtainer 133 obtains second display method information prompting display according to analysis information to be performed from input unit 110 and outputs the second display method information to image processor 234. Experimental value obtainer 232 outputs analysis information of each compound together with a composition and an experimental property value of the compound to image processor 234. According to the second display method information, image processor 234 forms a mark of an experimental property value in a mode according to analysis information that corresponds to the experimental property value and superimposes the formed mark on image element map Ma as illustrated in FIG. 22A. For example, image processor 234 forms a mark of an experimental property value of a compound having a crystal structure of crystal phase A in a circular shape and forms a mark of an experimental property value of a compound having a crystal structure of crystal phase B in a triangular shape.

FIG. 22B is a diagram illustrating another example of a map.

The analysis information described above may indicate a type of a raw material for synthesizing a compound. For example, types of a raw material include $Li_2O$ (purity 99%) and $Li_2O$ (purity 99.99%). In this case, as illustrated in FIG. 22B, image processor 234 may superimpose a mark of an experimental property value on image element map Ma in a mode according to a type of raw material that corresponds to the experimental property value. For example, image processor 234 forms a mark of an experimental property value of a compound synthesized from a raw material "$Li_2O$ (purity 99%)" in a circular shape and forms a mark of an experimental property value of a compound synthesized from a raw material "$Li_2O$ (purity 99.99%)" in a triangular shape.

FIG. 22C is a diagram illustrating another example of a map.

The analysis information described above may indicate a presence or absence of residual raw material used to synthesize a compound. In this case, as illustrated in FIG. 22C, image processor 234 may superimpose a mark of an experimental property value on image element map Ma in a mode according to the presence or absence of residual raw material that corresponds to the experimental property value. For example, image processor 234 forms a mark of an experimental property value of a compound for which a raw material remains (in other words, residual raw material is present) in a circular shape and forms a mark of an experimental property value of a compound for which a raw material does not remain (in other words, residual raw material is absent) in a triangular shape.

As described above, in property display device 230 according to the present embodiment, an attribute of a compound is: (a) a type of a crystal phase of the compound; (b) a type of a raw material of the compound; or (c) whether or not a raw material of the compound remains upon generation of the compound.

Accordingly, a type of a crystal phase, a type of a raw material, or a presence or absence of residual raw material of the compound having the experimental property value to be displayed by being superimposed on image element map Ma can be readily visually comprehended by the user from a form of a mark of the experimental property value.

[Processing operations]

FIG. 23 is a flowchart illustrating processing operations of display system 200 according to the present embodiment.

Display system 200 according to the present embodiment executes processing of steps S111 to S113 in a similar manner to display system 100 according to Embodiment 1A. Next, display system 200 executes processing of steps S121 to S124.

(Step S121)

Experimental value obtainer 232 obtains a composition and an experimental property value of each compound from experiment database 150 and outputs the composition and the experimental property value to image processor 234. When a processing condition, analysis information, or the like is associated with the composition and the experimental property value of each compound in experiment database 150, experimental value obtainer 232 may obtain the processing condition, the analysis information, or the like together with the experimental property value and output the processing condition, the analysis information, or the like to image processor 234.

(Step S122)

Display method obtainer 133 obtains second display method information that is outputted from input unit 110 in accordance with an input operation with respect to input unit 110 by the user in a similar manner to step S113. For example, the second display method information is information that indicates a display method of an experimental property value such as those illustrated in FIGS. 15 to 22C. In addition, display method obtainer 133 outputs the second display method information to image processor 234.

(Step S123)

Image processor 234 obtains a predicted property value and a composition of each of a plurality of compounds from predicted value obtainer 132 and receives first display method information from display method obtainer 133. In addition, image processor 234 generates, according to the first display method information, a map indicating the predicted property value of each of the plurality of compounds. Note that the map may be image element map Ma or image map Mb. Furthermore, image processor 234 obtains an experimental property value and a composition of each of one or more compounds from experimental value obtainer 232 and receives second display method information from display method obtainer 133. In addition, image processor 234 superimposes, according to the second display method information, the experimental property value of each of the one or more compounds on the map. Accordingly, a map on which the experimental property values of one or more compounds are superimposed is generated. Image processor 234 outputs an image including the map on which the experimental property values of the one or more compounds have been superimposed to displayer 140.

(Step S124)

Displayer 140 obtains an image from image processor 234 and displays the image or, in other words, displays an image including the map on which experimental property values of the one or more compounds have been superimposed.

By executing processing of steps S111 to S113 and steps S121 to S124 described above, property display regarding a predicted property value and an experimental property value of a compound is performed. Accordingly, since experimental property values are superimposed on a map indicating predicted property values, the user can appropriately recognize an overall picture of the experimental property values. Furthermore, the user can readily recognize a relationship between predicted property values and experimental property values.

Note that when an experimental property value is not obtained in step S121, property display device 230 executes steps S114 and S115 instead of steps S121 to S124 in a similar manner to property display device 130 according to Embodiment 1A.

(Variation of Embodiment 1B)

Image processor 234 may generate image map Mb as in Variation 1 of Embodiment 1A and superimpose experimental property values on image map Mb. Note that image map Mb is a map including an array of a plurality of image element maps Ma.

FIGS. 24A and 24B are diagrams illustrating an example of image map Mb according to the present variation.

Image processor 234 superimposes, on any of image element maps Ma included in image map Mb, an experimental property value of a compound having a composition corresponding to image element map Ma. There may be cases where compositions and experimental property values of the respective compounds obtained by experimental value obtainer 232 include a composition and an experimental property value which do not correspond to any of image element maps Ma in image map Mb.

For example, as illustrated in FIG. 24A, image map Mb corresponds to variables (M3, M3', M4, M4') = (La, Ga, Ti, Zr). In addition, each image element map Ma included in image map Mb corresponds to a combination of pieces of option data of each of discrete variables a and b. At this point, for example, a composition formula "$Li_{0.77}(La_{0.5}Ga_{0.5})_{0.15}(Ti_{0.5}Zr_{0.5})_{1.195}O_3$" and an experimental property value of a compound are obtained by experimental value obtainer 232. In this case, since discrete variable b in the composition formula of the compound is 0.195, the composition formula does not correspond to any of image element maps Ma in image map Mb.

In consideration thereof, image processor 234 according to the present variation respectively represents a composition formula of a compound obtained by experimental value obtainer 232 and a composition formula corresponding to each position on image map Mb by vectors. The vector of a composition formula obtained by experimental value obtainer 232 is hereinafter referred to as a first vector and the vector of a composition formula corresponding to each position on image map Mb is hereinafter referred to as a second vector. Note that the vectors are defined by, for example, eight variables (M3, M3', M4, M4', a, b, x, and y). Image processor 234 calculates a norm of a difference between a first vector and a second vector at each position on image map Mb as a distance. Note that the distance may be a normalized distance as described earlier. In addition, as illustrated in FIG. 24A, image processor 234 superimposes an experimental property value of a compound obtained by experimental value obtainer 232 on position 1700 on image map Mb that corresponds to a shortest distance among a plurality of calculated distances. Note that position 1700 is a position on one image element map Ma included in image map Mb.

In addition, image processor 234 may superimpose an experimental property value on a position on image map Mb which is equal to or smaller than a threshold and which corresponds to a shortest distance among the plurality of calculated distances. The threshold is, for example, 0.01. Alternatively, image processor 234 may superimpose an experimental property value on a position on image map Mb which corresponds to a distance equal to or shorter than a threshold among the plurality of calculated distances. When there are a plurality of positions on image map Mb which correspond to a distance equal to or shorter than the threshold, image processor 234 may superimpose an experimental property value on the plurality of positions.

Furthermore, when there are a plurality of shortest distances among the plurality of calculated distances, as illustrated in FIG. 24B, image processor 234 may superimpose an experimental property value on position 1701 on image map Mb which corresponds to each of the plurality of shortest distances. For example, a composition formula "$Li_{1.475}(La_{0.5}Ga_{0.5})_{0.175}(Ti_{0.5}Zr_{0.5})_{1.0}O_3$" and an experimental property value of a compound are obtained by experimental value obtainer 232. In this case, since discrete variable a in the composition formula of the compound is 0.175, the composition formula does not correspond to any of image element maps Ma in image map Mb.

In this case, image processor 234 calculates a norm of a difference between a first vector that corresponds to the composition formula and a second vector at each position on image map Mb as a distance. In addition, in the example illustrated in FIG. 24B, image processor 234 specifies two positions 1701 on image map Mb that correspond to a shortest distance among the plurality of calculated distances. Note that two positions 1701 are respectively included in two image element maps Ma that are adjacent to each other. Image processor 234 superimposes an experimental property value of a compound obtained by experimental value obtainer 232 on two positions 1701. Note that a distance corresponding to position 1700 is equal to or shorter than the threshold "0.01" and a distance corresponding to two positions 1701 is longer than the threshold. Therefore, when a condition of being equal to or shorter than the threshold is applied or, in other words, when an experimental property value is to be superimposed on a position on image map Mb that corresponds to a distance equal to or shorter than the threshold, the experimental property value is not superimposed on two positions 1701 even though the experimental property value is superimposed on position 1700.

As described above, in property display device 230 according to the present embodiment, image map Mb includes a plurality of image element maps Ma that are arrayed in a matrix along each of a first coordinate axis and a second coordinate axis, and each of the plurality of image element maps Ma has a third coordinate axis and a fourth coordinate axis. In addition, image processor 234 respectively associates the first coordinate axis, the second coordinate axis, the third coordinate axis, and the fourth coordinate axis with a first variable, a second variable, a third variable, and a fourth variable among the plurality of variables described earlier. With respect to each of the plurality of compounds, image processor 234 specifies image element map Ma associated with a value of the first variable and a value of a second variable that are to be used in representing a composition of the compound among the plurality of image element maps Ma. Next, image processor 234 maps, at a position corresponding to a value of the third variable and a value of the fourth variable that are to be used in representing a composition of the compound on specified image element map Ma, a predicted property value of the compound. For example, the first variable, the second variable, the third variable, and the fourth variable are variables a, b, x, and y described earlier.

Accordingly, image map Mb, etc., can present predicted property values with respect to compositions of a plurality of compounds respectively represented by four variables and predicted property values of compounds can be displayed in an easily understood manner over a wide range. As a result, material search can be made more efficient, and material development can be appropriately supported.

Furthermore, in property display device 230 according to the present embodiment, for each of the one or more compounds that have been experimented on: when an image element map Ma associated with the first variable and the second variable that are used for representing the composition of the compound is not present among image element maps Ma, image processor 234 identifies, in place of the image element map Ma, an image element map Ma associated with a value closest to a value of the first variable and a value closest to a value of the second variable; and image processor 234 superimposes the experimental property value of the compound on the image element map Ma identified, at a position corresponding to the value of the third variable and the value of the fourth variable that are to be used in representing the composition of the compound.

For example, there may be a case where an image element map Ma associated with a value of a first variable and a value of a second variable does not exist due to the fact that the first variable and the second variable are both discrete variables. However, in the present embodiment, an image element map Ma associated with values that are closest to the values is specified and experimental property values are superimposed on the image element map Ma. Therefore, a situation where an experimental property value is not displayed on an image element map Ma due to a discrete variable can be suppressed.

As described above, in Embodiment 1 including Embodiments 1A and 1B and variations thereof, a display method of a predicted property value can be appropriately set by first display method information. In addition, in Embodiment 1B and the variation thereof, a display method of an experimental property value can be appropriately set by second display method information.

In the present embodiment, display method obtainer 133 obtains first display method information generated in accordance with an input operation with respect to input unit 110 by the user.

Accordingly, a display method of a predicted property value can be optionally set by the user and convenience can be improved. A similar description applies to the second display method information and a display method of an experimental property value can be optionally set by the user and convenience can be improved.

On the other hand, display method obtainer 133 may obtain the first display method information by determining a display method of a predicted property value of a plurality of compounds obtained by predicted value obtainer 132 based on the predicted property value. For example, display method obtainer 133 determines, as a display method of a predicted property value, generating image element map Ma by associating colors or shades of color determined in advance for indicating a maximum value and a minimum value of predicted property values on image element map Ma with a maximum value and a minimum value among predicted property values of a plurality of compounds obtained by predicted value obtainer 132.

Accordingly, since a display method of a predicted property value is determined based on the predicted property value, display suitable for the predicted property value can be performed and material search can be made more efficient.

In a specific example, a maximum value of predicted property values of a plurality of compounds obtained by predicted value obtainer 132 is 3.5 and a minimum value is 0.0. In this case, display method obtainer 133 assigns a maximum shade determined in advance to the maximum value "3.5" of the predicted property values and assigns a minimum shade determined in advance to the minimum value "0.0" of the predicted property values. In other words, display method obtainer 133 determines such a relationship between shades and predicted property values as a display method of a predicted property value. As a result, display method obtainer 133 obtains first display method information indicating the display method. Next, for example, due to a predictor of predictor database 120 being updated, predicted value obtainer 132 newly obtains predicted property values of a plurality of compounds. At this point, for example, a maximum value of predicted property values of the plurality of compounds newly obtained by predicted value obtainer 132 is 1.5 and a minimum value is 1.0. In this case, display method obtainer 133 assigns the maximum shade determined in advance to the maximum value "1.5" of the predicted property values and assigns the minimum shade determined in advance to the minimum value "1.0" of the predicted property values. In other words, display method obtainer 133 determines such a new relationship between shades and predicted property values as a display method of a predicted property value. As a result, display method obtainer 133 obtains first display method information indicating the new display method. In this manner, image processor 234 generates, according to the first display method information that indicates a display method determined based on a predicted property value, image element map Ma indicating the predicted property value. Accordingly, regardless of a range of predicted property values to be obtained being wide or narrow or regardless of predicted property values in the range being large or small, each predicted property value is displayed in a shade in accordance with the range. Therefore, the user can readily comprehend a predicted property value from a shade and efficiency of material search can be improved.

[EMBODIMENT 2A]

A display system according to the present embodiment displays a predicted property value of each of a plurality of compounds in the form of a map in a similar manner to Embodiment 1A and also superimposes one or more candidate points on the map. A candidate point is a point that indicates a composition of a candidate for a compound (in other words a candidate compound) to be used in subsequent experiments. Note that among the respective components according to the present embodiment, the same components as in Embodiments 1A and 1B are given the same reference numerals as in Embodiments 1A and 1B and detailed descriptions thereof are omitted.

[Configuration of display system 300]

FIG. 25 is a block diagram illustrating an example of a configuration of display system 300 according to the present embodiment. Display system 300 illustrated in FIG. 25 includes input unit 110, predictor database 120, property display device 330, displayer 140, and experiment database 150. Note that property display device 330 is an example of an information display device.

Property display device 330 according to the present embodiment determines a candidate point based on a predicted property value of an obtained compound or based on positions of the predicted property value and an experimental property value and superimposes the candidate point on image element map Ma. Note that the position of the experimental property value is a position on image element map Ma that corresponds to a composition of a compound that has been experimented on from which an experimental property value has been obtained. In addition, property display device 330 outputs an image including image element map Ma on which the candidate point has been superimposed to displayer 140. Property display device 330 includes search range obtainer 131, predicted value obtainer 132, display method obtainer 133, experimental value obtainer 232, candidate point determiner 331, and image processor 234. Note that property display device 330 may be constituted of, for example, a processor such as a CPU and a memory. In this case, the processor functions as property display device 330 by, for example, executing a computer program stored in the memory. Note that the memory may be volatile or non-volatile or may be constituted of a volatile memory and a non-volatile memory.

[Predicted value obtainer 132]

With respect to each of a plurality of compounds, predicted value obtainer 132 according to the present embodiment associates a composition of the compound and a predicted property value obtained with respect to the composition with each other, outputs the associated composition and the predicted property value to image processor 334, and also outputs the associated composition and the predicted property value to candidate point determiner 331. Note that the composition of the compound may be a composition formula of the compound or, when the search range includes a processing condition for generating the compound, the composition of the compound may be a composition formula and a processing condition of the compound. Examples of the processing condition include a calcination method, a calcination time, and a calcination temperature.

[Experimental value obtainer 232]

Experimental value obtainer 232 according to the present embodiment obtains a composition and an experimental property value of each of one or more compounds that have been experimented on from experiment database 150. In addition, with respect to each of the one or more compounds that have been experimented on, experimental value obtainer 232 outputs the obtained composition and the experimental property value to candidate point determiner 331.

[Display method obtainer 133]

Display method obtainer 133 obtains third display method information indicating a display method of a candidate point from input unit 110. In addition, display method obtainer 133 outputs the third display method information to candidate point determiner 331 and image processor 334.

[Candidate point determiner 331]

Candidate point determiner 331 obtains a composition and a predicted property value of a compound from predicted value obtainer 132 and obtains a composition and an experimental property value of the compound from experimental value obtainer 232. In addition, candidate point determiner 331 obtains the third display method information from display method obtainer 133. Furthermore, according to the third display method information, candidate point determiner 331 respectively determines one or more positions from among all positions on image element map Ma as candidate points. At this point, candidate point determiner 331 determines a candidate point based on a predicted property value indicated at each position on image element map Ma. Alternatively, candidate point determiner 331 determines a candidate point based on a predicted property value indicated at each position and a position on image element map Ma that corresponds to a composition of each of one or more compounds that have been experimented on or, in other words, a position of an experimental property value.

For example, candidate point determiner 331 determines a first position at which a predicted property value equal to or larger than a threshold is indicated on image element map Ma as a candidate point. Alternatively, candidate point determiner 331 determines a second position at which a predicted property value equal to or smaller than a threshold is indicated on image element map Ma as a candidate point. Alternatively, candidate point determiner 331 determines a third position at which a predicted property value that has a difference from a target value that is equal to or smaller than a threshold is indicated on image element map Ma as a candidate point. The target value may be a value set in accordance with an input operation with respect to input unit 110 by the user. In such a case, candidate point determiner 331 obtains the target value from input unit 110 via display method obtainer 133 and determines a candidate point using the target value.

While candidate point determiner 331 determines a first position at which a predicted property value equal to or larger than a threshold is indicated as a candidate point in the example described above, candidate point determiner 331 may determine first positions respectively indicating K-number of largest predicted property values among all predicted property values on image element map Ma as candidate points. Note that K is an integer determined in advance that is equal to or greater than 1 and the K-number of largest predicted property values are larger than any of the other predicted property values. In addition, while candidate point determiner 331 determines a second position at which a predicted property value equal to or smaller than a threshold is indicated as a candidate point, candidate point determiner 331 may determine second positions respectively indicating L-number of largest predicted property values among all predicted property values that are equal to or smaller than the threshold as candidate points. Note that L is an integer determined in advance that is equal to or greater than 1 and the L-number of largest predicted property values are larger than any of the other predicted property values that are equal to or smaller than the threshold. Furthermore, while candidate point determiner 331 determines a third position at which a predicted property value that has a difference from a target value that is equal to or smaller than a threshold is indicated as a candidate point, candidate point determiner 331 may determine third positions respectively indicating J-number of predicted property values that has a difference from the target value that is smallest among all predicted property values on image element map Ma as candidate points. Note that J is an integer determined in advance that is equal to or greater than 1 and distances from the target value of the J-number of smallest predicted property values are smaller than any of the other predicted property values.

In addition, candidate point determiner 331 may determine each of two or more first positions that are separated from each other by a prescribed distance or more among a plurality of first positions as a candidate point. In a similar manner, candidate point determiner 331 may determine each of two or more positions that are separated from each other by a prescribed distance or more among a plurality of second positions or a plurality of third positions as a candidate point. Furthermore, when compositions and experimental property values of two compounds that have been experimented on are obtained, candidate point determiner 331 may determine a position at a midway point of the positions of the two experimental property values on image element map Ma as a candidate point. In addition, candidate point determiner 331 may determine a first position that is separated from a position of any of the experimental property values by a prescribed distance or more among the plurality of first positions described above as a candidate point. In a similar manner, candidate point determiner 331 may determine a position that is separated from a position of any of the experimental property values by a prescribed distance or more among the plurality of second positions or the plurality of third positions described above as a candidate point.

[Image processor 334]

Image processor 334 generates image element map Ma in a similar manner to Embodiment 1A. In addition, image processor 334 obtains third display method information from display method obtainer 133 and superimposes one or more candidate points determined by candidate point determiner 331 on image element map Ma according to the third display method information. In other words, image processor 334 superimposes a mark indicating each of the one or more candidate points on image element map Ma. Furthermore, image processor 334 outputs an image including image element map Ma on which the one or more candidate points have been superimposed to displayer 140.

[Displayer 140]

Displayer 140 obtains an image from image processor 334 and displays the image or, in other words, displays an image including image element map Ma on which the one or more candidate points have been superimposed.

[Specific examples of map]

FIG. 26 is a diagram illustrating an example of image element map Ma on which candidate points have been superimposed.

According to third display method information, image processor 334 superimposes a candidate point on image element map Ma according to Embodiment 1A as a mark with a star shape or the like. In other words, according to the third display method information, candidate point determiner 331 determines one or more candidate points from among all positions on image element map Ma. In addition, image processor 334 superimposes marks with a star shape on the one or more candidate points.

As described above, property display device 330 according to the present embodiment includes predicted value obtainer 132 and image processor 334. Predicted value obtainer 132 obtains a predicted property value of each of a plurality of compounds. Image processor 334 generates image element map Ma indicating a predicted property value of each of a plurality of compounds at a position corresponding to a composition of the compound. Next, image processor 334 superimposes a candidate point indicating a composition of each of one or more candidate compounds on a position on image element map Ma that corresponds to the composition of the candidate compound. In addition, image processor 334 generates an image including image element map Ma on which the one or more candidate points have been superimposed and outputs the image. In this case, the candidate compound described above is a compound considered to be a candidate of an experiment among a plurality of compounds respectively having a predicted property value indicated on image element map Ma. For example, the image described above is outputted to and displayed by displayer 140.

Accordingly, a relationship between each predicted property value and one or more candidate points on image element map Ma can be readily comprehended and efficiency of material search can be improved. In other words, the user can readily pick out a promising candidate for a next experiment from image element map Ma in the image displayed on displayer 140. As a result, material search can be made more efficient, and material development can be appropriately supported.

Note that property display device 330 need not include predicted value obtainer 132. In addition, while image processor 334 generates image element map Ma, image processor 334 may obtain image element map Ma without generating image element map Ma. In other words, image processor 334 obtains image element map Ma indicating a predicted property value of each of a plurality of compounds at a position corresponding to a composition of the compound. Furthermore, image processor 334 outputs an image including image element map Ma that is generated by superimposing a candidate point indicating a composition of each of one or more candidate compounds on a position on image element map Ma that corresponds to the composition of the candidate compound. In addition, as described above, the candidate compound is a compound considered to be a candidate of an experiment among a plurality of compounds respectively having a predicted property value indicated on image element map Ma. Even with such property display device 330, a similar operation and effect to those described above can be produced.

In addition, property display device 330 according to the present embodiment includes display method obtainer 133 which obtains third display method information indicating a display method of the candidate point. Image processor 334 superimposes one or more candidate points on image element map Ma according to the third display method information.

Accordingly, since a candidate point is superimposed on image element map Ma according to a display method indicated by the third display method information, the candidate point can be displayed in a mode suitable for an object of material search by the user according to settings of the display method. As a result, material search can be made more efficient.

In addition, property display device 330 according to the present embodiment includes candidate point determiner 331 which determines one or more candidate points based on a predicted property value of each of a plurality of compounds. Image processor 334 superimposes one or more candidate points determined by candidate point determiner 331 on image element map Ma.

Accordingly, since a candidate point is determined based on a predicted property value of each of a plurality of compounds, for example, a position where a best predicted property value such as a maximum predicted property value, a minimum predicted property value, or a predicted property value closest to a target value is indicated can be determined as a candidate point. Therefore, efficiency of material search can be further improved.

In addition, property display device 330 according to the present embodiment includes search range obtainer 131 that obtains a search range. Search range obtainer 131 obtains a plurality of variables to be used in representing compositions of compounds and a plurality of pieces of option data indicating values or elements that can be assumed by each of the plurality of variables. For each combination of option data obtained by selecting one piece of option data from the plurality of pieces of option data with respect to each of the plurality of variables, predicted value obtainer 132 obtains a predicted property value of a compound having a composition corresponding to the combination. In other words, for each of the combinations described above, predicted value obtainer 132 obtains a predicted property value of a compound having a composition corresponding to the combination using a prescribed algorithm.

Accordingly, an appropriate predicted property value can be obtained. In other words, property values of a plurality of compounds with respectively different compositions can be appropriately predicted. In addition, when the prescribed algorithm is updated by an experiment on a compound that is carried out as needed, prediction accuracy of the property value can be enhanced.

FIG. 27 is a diagram illustrating another example of image element map Ma on which candidate points have been superimposed.

Candidate point determiner 331 may determine, according to third display method information, one or more candidate points from among a plurality of points (hereinafter, also referred to as grid points) represented by a combination of variables x and y among image element map Ma. For example, the grid points are 11 × 11-number of points represented by variables (x, y) = (0.0, 0.0), (0.0, 0.1), (0.0, 0.2), ..., (1.0, 0.8), (1.0, 0.9), (1.0, 1.0). In addition, image processor 334 superimposes marks on the one or more determined candidate points. In other words, candidate point determiner 331 extracts a candidate point corresponding to a composition (in other words, a composition formula) of a prescribed compound. Alternatively, candidate point determiner 331 can be considered as limiting candidate points to grid points.

For example, as illustrated in FIG. 27, two grid points represented by variables (x, y) = (0.2, 0.2), (0.9, 0.1) among the 11 × 11-number of gird points are respectively determined as candidate points and marks indicating the candidate points are superimposed on image element map Ma.

As described above, in property display device 330 according to the present embodiment, the third display method information indicates superimposing candidate points only on a plurality of positions determined in advance on image element map Ma as a display method of the candidate points.

Accordingly, for example, a point that is difficult to use in an experiment on image element map Ma such as a point represented by variables (x, y) = (0.001, 0.002) or a point not intended by the user can be prevented from being determined and displayed as a candidate point.

FIG. 28 is a diagram illustrating another example of image element map Ma on which candidate points have been superimposed.

Candidate point determiner 331 may limit the number of candidate points to be determined to n-number (where n is an integer equal to or greater than 1) or less according to the third display method information. In the example illustrated in FIG. 28, n = 5. For example, candidate point determiner 331 determines (n + 1)-number or more preliminary candidate points on image element map Ma and specifies priorities of the preliminary candidate points. In addition, candidate point determiner 331 determines each of n-number of preliminary candidate points with highest priorities among the plurality of preliminary candidate points as a candidate point. A preliminary candidate point is a point at, for example, the first position, the second position, or the third position described earlier. Furthermore, for example, when each of (n + 1)-number or more preliminary candidate points is the first position, the priorities may be predicted property values at the first position. In addition, for example, when each of (n + 1)-number or more preliminary candidate points is the second position, the priorities may be predicted property values at the second position. Furthermore, for example, when each of (n + 1)-number or more preliminary candidate points is the third position, the priorities may be reciprocals of differences between predicted property values and a target value at the third position. In addition, the priorities may be a shortest distance among distances from a position of each experimental property value to the preliminary candidate points.

Image processor 334 superimposes n-number or less candidate points determined in this manner on image element map Ma and displays image element map Ma on displayer 140. Accordingly, the user can readily comprehend candidate points to be promising experimental candidates.

In other words, in property display device 330 according to the present embodiment, the third display method information indicates an upper limit number of candidate points to be superimposed on image element map Ma as a display method of the candidate points. In this case, image processor 334 superimposes one or more candidate points equal to or less than the upper limit number on image element map Ma.

Accordingly, even when there are a large number of points that, respectively, may become candidate points on image element map Ma, the number of candidate points to be superimposed on image element map Ma are limited. Therefore, for example, only promising points can be narrowed down as candidate points from a large number of points to be presented to the user and, consequently, efficiency of material search can be improved.

FIG. 29 is a diagram illustrating another example of image element map Ma on which candidate points have been superimposed.

Candidate point determiner 331 may determine each candidate point according to third display method information so that a linear distance between candidate points to be superimposed on image element map Ma is equal to or longer than a prescribed distance as illustrated in FIG. 29. The linear distance between candidate points is a linear distance between positions on image element map Ma that are indicated by continuous variables (x, y) of each of two candidate points. In addition, the prescribed distance is, for example, a distance equal to three times a step width (in other words, 1 scale unit) of each of continuous variables x and y. In other words, the prescribed distance can be considered as being 3 scale units. Note that the prescribed distance is also referred to as a first minimum separation distance.

For example, candidate point determiner 331 determines a preliminary candidate point at which a most favorable predicted property value is indicated from among a plurality of preliminary candidate points on image element map Ma as a first candidate point. The preliminary candidate point at which a most favorable predicted property value is indicated may be a preliminary candidate point for which the priority described above is highest. For example, when each of a plurality of preliminary candidate points is the first position, the preliminary candidate point at which a most favorable predicted property value is indicated is a preliminary candidate point at which a maximum predicted property value is indicated among the plurality of preliminary candidate points.

Next, candidate point determiner 331 deletes preliminary candidate points shorter than a prescribed distance from the first candidate point and determines a preliminary candidate point at which a next favorable predicted property value to the first candidate point is indicated from one or more remaining preliminary candidate points as a second candidate point. By repetitively performing such processing until all preliminary candidate points are deleted, candidate point determiner 331 determines two or more candidate points. A linear distance between the candidate points is equal to or longer than the prescribed distance described above.

Alternatively, candidate point determiner 331 respectively randomly determines two or more preliminary candidate points from among a plurality of preliminary candidate points as candidate points and translocates one of the candidate points to a preliminary candidate point at a position that differs from the candidate point so that a linear distance between the two candidate points becomes equal to or longer than the prescribed distance. In other words, candidate point determiner 331 moves one candidate point. By sequentially performing such a movement of candidate points, candidate point determiner 331 sets a linear distance between each pair of candidate points among the two or more candidate points to be equal to or longer than the prescribed distance described above.

Image processor 334 superimposes a plurality of candidate points separated from each other by the prescribed distance or more and determined by candidate point determiner 331 as described above on image element map Ma.

As described above, in property display device 330 according to the present embodiment, the third display method information indicates the first minimum separation distance determined in advance between a plurality of candidate points as a display method of the candidate points. In this case, image processor 334 superimposes a plurality of candidate points that are separated from each other by the first minimum separation distance or more on image element map Ma.

Accordingly, the user can readily comprehend candidate points that have a certain difference in composition formulae (in other words, compositions) of compounds. In addition, a congestion of candidate points can be avoided and visibility can be improved.

FIG. 30 is a diagram illustrating another example of image element map Ma on which candidate points have been superimposed.

For example, as illustrated in FIG. 30, candidate point determiner 331 determines a plurality of candidate points and an experimental plan in accordance with third display method information. The experimental plan is information indicating which candidate point among the plurality of candidate points is to be experimented on in what order. Candidate point determiner 331 outputs information indicating the determined plurality of candidate points and the determined experimental plan to image processor 334. For example, candidate point determiner 331 determines the plurality of candidate points as described above and, further determines n-number of candidate points in a descending order of priority from the plurality of candidate points as candidate points (hereinafter, referred to as planned candidate points) included in the experimental plan. For example, n = 5. In addition, with respect to each of the n-number of planned candidate points, candidate point determiner 331 assigns an experiment number in a descending order of priority. For example, among the n-number of planned candidate points, candidate point determiner 331 assigns experiment number "1" to the planned candidate point with a highest priority and assigns experiment number "2" to the planned candidate point with a second highest priority. Accordingly, an experimental plan indicating the n-number of planned candidate points and experiment numbers of the planned candidate points is generated.

Image processor 334 obtains information indicating the plurality of candidate points and the experimental plan from candidate point determiner 331. In addition, image processor 334 superimposes the plurality of candidate points on image element map Ma. Furthermore, image processor 334 adds an experiment number of each of the n-number of planned candidate points included in the experimental plan to image element map Ma and further superimposes an arrow pointing from one planned candidate point to a planned candidate point with a next experiment number on image element map Ma. Superimposition of such an arrow and the like is executed in accordance with the third display method information.

In a specific example, with respect to five planned candidate point among a plurality of candidate points, image processor 334 respectively adds experiment numbers (1), (2), (3), (4), and (5). In addition, image processor 334 superimposes an arrow indicating the planned candidate point with the experiment number (2) from the planned candidate point with the experiment number (1) and an arrow indicating the planned candidate point with the experiment number (3) from the planned candidate point with the experiment number (2). Furthermore, image processor 334 superimposes an arrow indicating the planned candidate point with the experiment number (4) from the planned candidate point with the experiment number (3) and an arrow indicating the planned candidate point with the experiment number (5) from the planned candidate point with the experiment number (4). Image processor 334 displays an image including image element map Ma to which experiment numbers and arrows have been added in this manner on displayer 140. Therefore, an experimental plan indicating that experiments are to be conducted with respect to the planned candidate point with the experiment number (1), the planned candidate point with the experiment number (2), the planned candidate point with the experiment number (3), the planned candidate point with the experiment number (4), and the planned candidate point with the experiment number (5) in this order is presented to the user from displayer 140.

As described above, in property display device 330 according to the present embodiment, the third display method information indicates displaying an order of an experiment of a candidate compound corresponding to each of one or more candidate points in the form of an experimental plan as a display method of the candidate points.

Accordingly, when there are a plurality of candidate points, which candidate point is to be experimented on in what order is presented to the user. Therefore, by conducting experiments of candidate points according to the order, the user can carry out the experiments in an efficient manner. In other words, efficiency of material search can be improved and material development can be appropriately supported. While an experimental plan is indicated by experiment numbers and arrows in the example illustrated in FIG. 30, an experimental plan is not limited thereto and may be indicated by other modes.

FIG. 31 is a diagram illustrating another example of image element map Ma on which candidate points have been superimposed.

For example, in accordance with third display method information, candidate point determiner 331 may classify a plurality of candidate points into candidate points for which a predicted property value is equal to or larger than a reference value and candidate points for which a predicted property value is smaller than the reference value as illustrated in FIG. 31. Examples of the reference value are, but not limited to, a mean of predicted property values of respective positions on image element map Ma, a median of the predicted property values, and an arbitrary value. Candidate point determiner 331 obtains the third display method information related to a reference value from input unit 110 via display method obtainer 133 and determines the reference value according to the third display method information. In addition, with respect to each of a plurality of candidate points, candidate point determiner 331 determines whether or not a predicted property value indicated at the candidate point is equal to or larger than the reference value and, when candidate point determiner 331 determines that the predicted property value is equal to or larger than the reference value, candidate point determiner 331 adds a flag indicating, for example, "1" to the candidate point. On the other hand, when candidate point determiner 331 determines that the predicted property value indicated at the candidate point is smaller than the reference value, candidate point determiner 331 adds a flag indicating, for example, "0" to the candidate point. Furthermore, candidate point determiner 331 outputs the plurality of candidate points and flags to image processor 334.

When image processor 334 obtains the plurality of candidate points and flags from candidate point determiner 331, according to the third display method information, image processor 334 assigns, for example, a mark with a star shape with respect to a candidate point with a flag indicating "1" as illustrated in FIG. 31. In addition, image processor 334 assigns, for example, a mark with an inverted triangular shape with respect to a candidate point with a flag indicating "0". Furthermore, image processor 334 superimposes a mark of a form assigned to a candidate point on image element map Ma on the candidate point and outputs an image including image element map Ma on which the mark has been superimposed to displayer 140. As a result, a candidate point indicated by a mark of each mode is superimposed and displayed on image element map Ma.

Therefore, a candidate point at which a predicted property value equal to or larger than a reference value is indicated can be more highlighted than a candidate point at which a predicted property value smaller than the reference value is indicated. In addition, a plurality of notable candidate points can be comprehended while simultaneously comprehending an entire search range.

When the reference value is an arbitrary value that is equal to, for example, a tenth largest predicted property value in image element map Ma, candidate points from a candidate point at which a largest predicted property value is indicated to a candidate point at which a tenth largest predicted property value is indicated can be highlighted. In addition, contrary to the example described above, a candidate point at which a predicted property smaller than a reference value is indicated can be more highlighted than a candidate point at which a predicted property value equal to or larger than the reference value is indicated.

As described above, in property display device 330 according to the present embodiment, the third display method information indicates displaying, among one or more candidate points, a candidate point for which a predicted property value indicated at a position on image element map Ma on which the candidate point is superimposed is equal to or larger than a reference value by highlighting the candidate point more than the remaining candidate points as a display method of the candidate points. For example, in property display device 330 according to the present embodiment, the reference value is a mean or a median of respective predicted property values of a plurality of compounds or a value designated by the user.

Accordingly, the user can readily pick out a candidate point at which a predicted property value equal to or larger than a reference value is indicated from among one or more candidate points superimposed on image element map Ma. As a result, material search can be made more efficient.

FIG. 32 is a diagram illustrating another example of image element map Ma on which candidate points have been superimposed.

For example, in accordance with third display method information, candidate point determiner 331 may classify a plurality of candidate points into three groups as illustrated in FIG. 32. In the example illustrated in FIG. 32, the three groups are candidate point group 1, candidate point group 2, and candidate point group 3. The classification into the groups may be performed by comparing predicted property values with a reference value as in the example illustrated in FIG. 31. For example, when a predicted property value of a candidate point is equal to or larger than a first reference value, candidate point determiner 331 classifies the candidate point into candidate point group 1. In addition, when a predicted property value of a candidate point is smaller than the first reference value and equal to or larger than a second reference value, candidate point determiner 331 classifies the candidate point into candidate point group 2. Furthermore, when a predicted property value of a candidate point is smaller than the second reference value, candidate point determiner 331 classifies the candidate point into candidate point group 3. Examples of each of the first reference value and the second reference value are, but not limited to, a mean of predicted property values of respective positions on image element map Ma, a median of the predicted property values, and an arbitrary value. In addition, the first reference value is larger than the second reference value. In a specific example, the first reference value is 2.5 eV and the second reference value is 1.5 eV.

Alternatively, the priorities described earlier may be used instead of the predicted property values of candidate points for the classification into the respective groups. The priorities used in this case may be predicted property values or reciprocals of a difference between the predicted property values and a target value. For example, when a priority of a candidate point is equal to or larger than a first priority reference value, candidate point determiner 331 classifies the candidate point into candidate point group 1. In addition, when a priority of a candidate point is smaller than the first priority reference value and equal to or larger than a second priority reference value, candidate point determiner 331 classifies the candidate point into candidate point group 2. Furthermore, when a priority of a candidate point is smaller than the second priority reference value, candidate point determiner 331 classifies the candidate point into candidate point group 3. Note that the first priority reference value and the second priority reference value are reference values to be compared with priorities and the first priority reference value is larger than the second priority reference value. In addition, hereinafter, the first priority reference value and the second priority reference value may be collectively referred to as a priority reference value. Examples of the priority reference value are, but not limited to, a mean of priorities of respective candidate points, a median of the priorities, and an arbitrary value.

When candidate point determiner 331 classifies a plurality of candidate points into three groups as described above, candidate point determiner 331 associates the plurality of candidate points and group identification information indicating groups to which the candidate points belong and outputs the associated candidate points and group identification information to image processor 334.

When image processor 334 obtains the plurality of candidate points and the group identification information from candidate point determiner 331, as illustrated in FIG. 32, image processor 334 assigns, for example, a mark with a star shape with respect to a candidate point associated with group identification information indicating candidate point group 1. In addition, image processor 334 assigns, for example, a mark with an inverted triangular shape with respect to a candidate point associated with group identification information indicating candidate point group 2. Furthermore, image processor 334 assigns, for example, a mark with a square shape with respect to a candidate point associated with group identification information indicating candidate point group 3. Assignment of the marks is executed in accordance with the third display method information.

Furthermore, image processor 334 superimposes a mark of a form assigned to a candidate point on image element map Ma on the candidate point and outputs an image including image element map Ma on which the mark has been superimposed to displayer 140. As a result, a candidate point indicated by a mark of each mode is superimposed and displayed on image element map Ma.

As described above, in property display device 330 according to the present embodiment, the third display method information indicates classifying each of one or more candidate points into any one group among a plurality of groups according to a priority of the candidate point and superimposing a mark of a mode associated with the group on image element map Ma as a display method of the candidate points. For example, the priority of a candidate point may be a predicted property value indicated at the candidate point.

Accordingly, the user can readily comprehend a feature of a group to which each candidate point to be superimposed on image element map Ma belongs or, in other words, a feature of a candidate point belonging to the group such as whether a priority is high, intermediate, or low.

[Processing operations]

FIG. 33 is a flowchart illustrating processing operations of display system 300 according to the present embodiment.

Display system 300 according to the present embodiment executes processing of steps S111 and S112 in a similar manner to display system 100 according to Embodiment 1A and executes processing of step S121 in a similar manner to display system 200 according to Embodiment 1B. Next, display system 300 executes processing of steps S131 to S134.

(Step S131)

Display method obtainer 133 obtains third display method information that is outputted from input unit 110 in accordance with an input operation with respect to input unit 110 by the user in a similar manner to step S113. For example, the third display method information is information that indicates a display method of a candidate point such as those illustrated in FIGS. 26 to 32. In addition, display method obtainer 133 outputs the third display method information to candidate point determiner 331 and image processor 334.

(Step S132)

Candidate point determiner 331 obtains a composition and a predicted property value of each of a plurality of compounds from predicted value obtainer 132 and obtains a composition and an experimental property value of each of one or more compounds from experimental value obtainer 232. In addition, candidate point determiner 331 obtains the third display method information from display method obtainer 133. Furthermore, according to the third display method information, candidate point determiner 331 determines one or more candidate points based on the composition and the predicted property value of each of the plurality of compounds and the composition and the experimental property value of each of the one or more compounds.

(Step S133)

Image processor 334 obtains, for each of a plurality of compounds, a composition and a predicted property value of the compound from predicted value obtainer 132. Furthermore, image processor 334 obtains one or more candidate points from candidate point determiner 331. In addition, image processor 334 obtains the third display method information from display method obtainer 133. Note that image processor 334 may receive first display method information indicating a display method of the predicted property value from display method obtainer 133 in a similar manner to Embodiment 1A.

Image processor 334 generates image element map Ma based on a composition and a predicted property value of each of the plurality of obtained compounds. At this point, if image processor 334 has received the first display method information, image processor 334 may generate image element map Ma according to the first display method information. Furthermore, image processor 334 superimposes one or more candidate points obtained from candidate point determiner 331 on image element map Ma according to the third display method information. In other words, image processor 334 superimposes marks indicating one or more candidate points on image element map Ma. Image processor 334 outputs an image including image element map Ma on which the one or more candidate points have been superimposed to displayer 140.

(Step S134)

Displayer 140 obtains the image from image processor 334 and displays the image or, in other words, displays an image including image element map Ma on which one or more candidate points have been superimposed.

By executing processing of steps S111, S112, S121, and S131 to S134 described above, property display regarding predicted property values of compounds and candidate points is performed.

(Variation of Embodiment 2A)

Image processor 334 may generate image map Mb as in Variation 1 of Embodiment 1A and superimpose candidate points on image map Mb. Note that image map Mb is a map including an array of a plurality of maps Ma.

FIG. 34 is a diagram illustrating an example of map Mb according to the present variation.

Image processor 334 superimposes candidate points on image map Mb as illustrated in FIG. 34 according to the third display method information. At this point, when candidate point determiner 331 obtains the third display method information indicating a limit of candidate points from input unit 110 via display method obtainer 133, candidate point determiner 331 determines as many candidate points as the limit. For example, the limit is n (where n is an integer equal to or greater than 1, an example of which is 10) with respect to image map Mb. As a result, for example, image processor 334 superimposes 10 candidate points on image map Mb.

Accordingly, for example, when the number of experiments that can be performed in a week is 10, by setting the limit n to n = 10, the user can readily comprehend candidate points to be experimented on in the week.

FIG. 35 is a diagram illustrating another example of image map Mb according to the present variation.

For example, in accordance with third display method information, candidate point determiner 331 may classify a plurality of candidate points to be superimposed on image map Mb into candidate points for which a predicted property value is equal to or larger than a reference value and candidate points for which a predicted property value is smaller than the reference value as illustrated in FIG. 35 in a similar manner to the example illustrated in FIG. 31. Examples of the reference value used for image map Mb are, but not limited to, a mean of predicted property values of respective positions on image map Mb, a median of the predicted property values, and an arbitrary value.

Specifically, with respect to each of a plurality of candidate points to be superimposed on image map Mb, candidate point determiner 331 determines whether or not a predicted property value indicated at the candidate point is equal to or larger than the reference value and, when candidate point determiner 331 determines that the predicted property value is equal to or larger than the reference value, candidate point determiner 331 adds a flag indicating, for example, "1" to the candidate point. On the other hand, when candidate point determiner 331 determines that the predicted property value indicated at the candidate point is smaller than the reference value, candidate point determiner 331 adds a flag indicating, for example, "0" to the candidate point. Furthermore, candidate point determiner 331 outputs the plurality of candidate points and flags to image processor 334.

As in the example illustrated in FIG. 31, when image processor 334 obtains the plurality of candidate points and flags from candidate point determiner 331, image processor 334 assigns, for example, a mark with a star shape with respect to a candidate point with a flag indicating "1" as illustrated in FIG. 35. In addition, image processor 334 assigns, for example, a mark with an inverted triangular shape with respect to a candidate point with a flag indicating "0". Furthermore, image processor 334 superimposes a mark of a form assigned to a candidate point on image map Mb on the candidate point and outputs an image including image map Mb on which the mark has been superimposed to displayer 140. As a result, a candidate point indicated by a mark of each mode is superimposed and displayed on image map Mb.

In addition, candidate point determiner 331 may use a plurality of reference values to classify a plurality of candidate points into three or more groups in accordance with a comparison result between the reference values and predicted property values of the respective candidate points. Alternatively, in a similar manner to the example illustrated in FIG. 32, candidate point determiner 331 may use the priorities described earlier instead of the predicted property values of candidate points and classify a plurality of candidate points into a plurality of groups in accordance with a comparison result between a priority reference value and priorities of the respective candidate points. For example, when candidate point determiner 331 classifies a plurality of candidate points into two groups as illustrated in FIG. 35, candidate point determiner 331 associates the plurality of candidate points and group identification information indicating groups to which the candidate points belong and outputs the associated candidate points and group identification information to image processor 334.

When image processor 334 obtains the plurality of candidate points and the group identification information from candidate point determiner 331, as illustrated in FIG. 35, image processor 334 assigns, for example, a mark with a star shape with respect to a candidate point corresponding to group identification information indicating a group whose priority is equal to or higher than the priority reference value. In addition, image processor 334 assigns, for example, a mark with an inverted triangular shape with respect to a candidate point corresponding to group identification information indicating a group whose priority is lower than the priority reference value.

Furthermore, image processor 334 superimposes a mark of a form assigned to a candidate point on image map Mb on the candidate point and outputs an image including image map Mb on which the mark has been superimposed to displayer 140. As a result, a candidate point indicated by a mark of each mode is superimposed and displayed on image map Mb.

Accordingly, the user can readily comprehend a feature of a group to which each candidate point to be superimposed on image map Mb belongs or, in other words, a feature of a candidate point corresponding to the group such as whether a predicted property value or a priority is high or low.

FIG. 36 is a diagram illustrating another example of image map Mb according to the present variation.

Candidate point determiner 331 may set the number of candidate points to be superimposed on each image element map Ma included in image map Mb to n (where n is an integer equal to or greater than 1) according to the third display method information. In the example illustrated in FIG. 36, n = 1. For example, input unit 110 outputs third display method information indicating the number of candidate points to display method obtainer 133 in accordance with an input operation with respect to input unit 110 by the user. Display method obtainer 133 obtains the third display method information and outputs the third display method information to candidate point determiner 331. Candidate point determiner 331 receives the third display method information from display method obtainer 133 and determines as many candidate points as the number indicated by the third display method information with respect to each image element map Ma of image map Mb. Accordingly, the user can readily confirm candidate points having been widely dispersed on image map Mb.

As described above, in property display device 330 according to the present variation, image map Mb includes a plurality of image element maps Ma that are arrayed in a matrix along each of a first coordinate axis and a second coordinate axis, and each of the plurality of image element maps Ma has a third coordinate axis and a fourth coordinate axis. Image processor 334 respectively associates the first coordinate axis, the second coordinate axis, the third coordinate axis, and the fourth coordinate axis with a first variable, a second variable, a third variable, and a fourth variable to be used in representing a composition of a compound. In addition, with respect to each of the plurality of compounds, image processor 334 specifies image element map Ma associated with the number of the first variable and a value of a second variable that are to be used in representing a composition of the compound among the plurality of image element maps Ma. Next, image processor 334 maps, at a position corresponding to a value of the third variable and a value of the fourth variable that are to be used in representing a composition of the compound on specified image element map Ma, a predicted property value of the compound. For example, the first variable, the second variable, the third variable, and the fourth variable are variables a, b, x, and y described earlier.

Accordingly, image map Mb, etc., can present predicted property values with respect to compositions of a plurality of compounds respectively represented by four variables and predicted property values of compounds can be displayed in an easily understood manner over a wide range. As a result, material search can be made more efficient.

In addition, in property display device 330 according to the present variation, the third display method information indicates the number of candidate points to be superimposed on each of a plurality of image element maps Ma as a display method of the candidate points. Image processor 334 superimposes as many candidate points as indicated by the third display method information on each of the plurality of image element maps Ma.

Accordingly, candidate points can be evenly superimposed on the plurality of image element maps Ma included in image map Mb. As a result, the user can readily confirm candidate points having been widely dispersed on image map Mb.

[EMBODIMENT 2B]

A display system according to the present embodiment generates a map indicating a predicted property value of each of a plurality of compounds and superimposes candidate points on the map in a similar manner to Embodiment 2A, and also superimposes an experimental property value of each of one or more compounds on the map. Note that among the respective components according to the present embodiment, the same components as in Embodiment 2A are given the same reference numerals as in Embodiment 2A and detailed descriptions thereof are omitted. In addition, while the map according to the present embodiment is image element map Ma, the map may also be image map Mb.

[Configuration of display system 400]

FIG. 37 is a block diagram illustrating an example of a configuration of display system 400 according to the present embodiment. Display system 400 illustrated in FIG. 37 includes input unit 110, predictor database 120, property display device 430, displayer 140, and experiment database 150. Note that property display device 430 is an example of an information display device.

Property display device 430 according to the present embodiment superimposes not only candidate points but also an experimental property value of a compound that is obtained by an experiment on image element map Ma and displays an image including image element map Ma on which the candidate points and the experimental property value have been superimposed on displayer 140. Property display device 430 includes search range obtainer 131, predicted value obtainer 132, display method obtainer 133, experimental value obtainer 232, candidate point determiner 331, and image processor 434. Note that property display device 430 may be constituted of, for example, a processor such as a CPU and a memory. In this case, the processor functions as property display device 430 by, for example, executing a computer program stored in the memory. Note that the memory may be volatile or non-volatile or may be constituted of a volatile memory and a non-volatile memory.

[Experimental value obtainer 232]

Experimental value obtainer 232 according to the present embodiment obtains a composition and an experimental property value of each of one or more compounds that have been experimented on from experiment database 150. In addition, with respect to each of the one or more compounds that have been experimented on, experimental value obtainer 232 outputs the obtained composition and the experimental property value to candidate point determiner 331 and also to image processor 434.

[Image processor 434]

Image processor 434 generates image element map Ma in a similar manner to Embodiment 2A. In addition, in a similar manner to Embodiment 2A, image processor 434 obtains third display method information from display method obtainer 133 and superimposes one or more candidate points determined by candidate point determiner 331 on image element map Ma according to the third display method information. Furthermore, image processor 434 obtains a composition and an experimental property value of each of one or more compounds that have been experimented on from experimental value obtainer 232. Moreover, image processor 434 superimposes, on image element map Ma, the experimental property value of each of the one or more compounds that have been experimented on. In the superimposition of the experimental property value of the compound, image processor 434 superimposes the experimental property value of the compound at a position on image element map Ma that corresponds to a composition of the compound. In other words, image processor 434 superimposes a mark indicating the experimental property value on image element map Ma. In addition, image processor 434 outputs an image including image element map Ma on which experimental property values of the one or more compounds having been experimented on and the one or more candidate points have been superimposed to displayer 140.

[Specific examples of map]

FIG. 38 is a diagram illustrating an example of image element map Ma on which candidate points and experimental property values have been superimposed.

According to third display method information, image processor 434 superimposes a candidate point on image element map Ma as a mark with a star shape or the like. In other words, candidate point determiner 331 determines one or more candidate points from among all positions of image element map Ma and image processor 434 superimposes a mark with a star shape on the one or more candidate points. In addition, image processor 434 superimposes an experimental property value of a compound on image element map Ma as a mark with a circular shape or the like. In other words, when image processor 434 obtains a composition and an experimental property value of each of one or more compounds that have been experimented on from experimental value obtainer 232, image processor 434 superimposes a circular mark with a shade in accordance with the experimental property value of the compound on a position on image element map Ma that corresponds to the composition of the compound.

In this manner, in property display device 430 according to the present embodiment, experimental value obtainer 232 obtains an experimental property value of each of one or more compounds that have been experimented on. In addition, image processor 434 superimposes, on a position on image element map Ma that corresponds to a composition of each of the one or more compounds that have been experimented on, the experimental property value of the compound. Image processor 434 generates an image including image element map Ma on which one or more candidate points and one or more experimental property values have been superimposed.

Accordingly, the user can readily compare a candidate point, an experimental property value, and a position of the experimental property value on image element map Ma. As a result, the user can readily determine, based on the experimental property value and the position of the experimental property value, whether or not to perform an experiment of a compound corresponding to the candidate point. Therefore, efficiency of material search can be improved and material development can be appropriately supported.

FIG. 39 is a diagram illustrating another example of image element map Ma on which candidate points and experimental property values have been superimposed.

According to third display method information, candidate point determiner 331 determines, as a final candidate point, a position that is separated from a position of any of experimental property values by a prescribed distance or more among a plurality of preliminary candidate points described above which may become candidate points. The prescribed distance is, for example, a distance equal to three times a step width (in other words, 1 scale unit) of each of continuous variables x and y. In other words, the prescribed distance can be considered as being 3 scale units. Note that the prescribed distance is also referred to as a second minimum separation distance.

Image processor 434 superimposes one or more candidate points determined as described above by candidate point determiner 331 on image element map Ma according to the third display method information. Furthermore, image processor 434 also superimposes, on a position on image element map Ma that corresponds to a composition of a compound that has been experimented on, the experimental property value of the compound.

Accordingly, a candidate point can be eliminated from a position corresponding to a composition of a compound that has been experimented on (in other words, a position of an experimental property value) and a periphery thereof and an efficient search of a candidate point can be performed.

In other words, in property display device 430 according to the present embodiment, the third display method information indicates the second minimum separation distance determined in advance between a candidate point and a position of an experimental property value as a display method of the candidate points. Image processor 434 separates, from a position of each of one or more experimental property values obtained by experimental value obtainer 232, one or more candidate points by the second minimum separation distance or more and superimposes the one or more candidate points on image element map Ma.

Accordingly, a candidate point can be prevented from being superimposed near a position of an experimental property value. As a result, a situation where similar experimental results are repetitively obtained by performing experiments with respect to compounds (in other words, candidate compounds) having a composition that resembles a compound which has been experimented on and which has an experimental property value can be suppressed and the possibility that a completely new experimental result is to be obtained can be increased. As a result, material search can be made more efficient.

FIG. 40 is a diagram illustrating an example of a state transition of image element map Ma.

For example, when an experimental property value has not been obtained by experimental value obtainer 232, image processor 434 displays image element map Ma illustrated in (a) in FIG. 40 on displayer 140. A plurality of candidate points are superimposed but experimental property values are not superimposed on image element map Ma illustrated in (a) in FIG. 40.

In this case, the user performs an experiment of one candidate point b1 among a plurality of candidate points on image element map Ma. Specifically, the user performs an experiment of a compound having a composition indicated by candidate point b1. Due to the experiment, experimental data of experiment database 150 is updated. In other words, a composition and an experimental property value of a new compound are written into experimental data. As a result, experimental value obtainer 232 obtains the composition and the experimental property value of the new compound and outputs the composition and the experimental property value to candidate point determiner 331 and image processor 434. When image processor 434 obtains the composition and the experimental property value of the new compound from experimental value obtainer 232, image processor 434 superimposes the experimental property value on a position on image element map Ma that corresponds to the composition as illustrated in (b) in FIG. 40. In other words, image element map Ma on which a new experimental property value has been superimposed on the position of candidate point b1 is displayed on displayer 140.

Next, the user operates input unit 110 so that only candidate points separated from the position of the experimental property value by the second minimum separation distance or more are displayed. Accordingly, display method obtainer 133 obtains information in accordance with the input operation by the user from input unit 110. Specifically, display method obtainer 133 obtains third display method information indicating a display method that causes only candidate points separated from the position of the experimental property value by the second minimum separation distance or more to be displayed. In addition, display method obtainer 133 outputs the third display method information to candidate point determiner 331 and image processor 434. As a result, in a similar manner to the example illustrated in FIG. 39, candidate point determiner 331 only retains candidate points that are separated from the position of the experimental property value by the second minimum separation distance or more among the plurality of candidate points superimposed on image element map Ma illustrated in (b) in FIG. 40 and deletes candidate points that are not separated from the position of the experimental property value by the second minimum separation distance or more. In other words, candidate point determiner 331 redetermines and updates candidate points. Image processor 434 displays, on displayer 140, image element map Ma on which one or more candidate points having been redetermined in this manner and experimental property values have been superimposed. Accordingly, the user can carry out experiments more efficiently.

While image element map Ma illustrated in (b) in FIG. 40 is to be displayed on displayer 140 in the example described above, when third display method information has already been obtained before obtaining the experimental property value, image element map Ma illustrated in (b) in FIG. 40 may not be displayed. In other words, after image element map Ma illustrated in (a) in FIG. 40 is displayed and an experiment is subsequently performed, image element map Ma illustrated in (c) in FIG. 40 may be directly displayed. In addition, after image element map Ma illustrated in (c) in FIG. 40 is displayed, when experiments are subsequently repetitively performed and a new experimental property value is obtained, processing similar to that described above is repetitively executed. In other words, candidate point determiner 331 only retains candidate points that are separated from the position of the new experimental property value by the second minimum separation distance or more among the plurality of candidate points superimposed on image element map Ma illustrated in (c) in FIG. 40 and deletes candidate points that are not separated from the position of the new experimental property value by the second minimum separation distance or more. As a result, the plurality of candidate points superimposed on image element map Ma illustrated in (c) in FIG. 40 are updated.

FIG. 41 is a diagram illustrating an example of a state transition of image element map Ma.

In a similar manner to the example illustrated in FIG. 30, candidate point determiner 331 determines a plurality of candidate points and an experimental plan as illustrated in (a) in FIG. 41. Note that the experimental plan is information indicating which candidate point among the plurality of candidate points is to be experimented on in what order. Candidate point determiner 331 outputs information indicating the determined plurality of candidate points and the determined experimental plan to image processor 434.

Image processor 434 obtains information indicating the plurality of candidate points and the experimental plan from candidate point determiner 331. In addition, image processor 434 superimposes the plurality of candidate points on image element map Ma. Furthermore, image processor 434 adds an experiment number of each of n-number of planned candidate points included in the experimental plan to image element map Ma and further superimposes an arrow pointing from one planned candidate point to a planned candidate point with a next experiment number on image element map Ma.

At this point, for example, the user performs an experiment of a compound (in other words, a candidate compound) having a composition indicated by the planned candidate point with experiment number (1). Accordingly, an experimental property value of the compound is obtained and experimental data of experiment database 150 is updated. In other words, a composition and an experimental property value of a new compound obtained by an experiment are written into experimental data. As a result, experimental value obtainer 232 obtains the composition and the experimental property value of the new compound and outputs the composition and the experimental property value to candidate point determiner 331 and image processor 434. When image processor 434 obtains the composition and the experimental property value of the new compound from experimental value obtainer 232, image processor 434 superimposes the experimental property value on a position on image element map Ma that corresponds to the composition or, in other words, on a position of the planned candidate point with experiment number (1) as illustrated in (b) in FIG. 41. In other words, a new experimental property value is superimposed on image element map Ma instead of the planned candidate point with experiment number (1).

Furthermore, candidate point determiner 331 obtains the composition and the experimental property value of the new compound from experimental value obtainer 232. At this point, in a similar manner to the example illustrated in FIG. 40, candidate point determiner 331 only retains candidate points that are separated from the position of the experimental property value by the second minimum separation distance or more among the plurality of candidate points superimposed on image element map Ma illustrated in (a) in FIG. 41. In addition, candidate point determiner 331 deletes candidate points that are not separated from the position of the experimental property value by the second minimum separation distance or more. In other words, by redetermining and updating candidate points, candidate point determiner 331 deletes, for example, three candidate points that are not separated by the second minimum separation distance or more from the position of the experimental property value on which the planned candidate point with experiment number (1) has been superimposed as illustrated in (b) in FIG. 41. Image processor 434 displays, on displayer 140, image element map Ma on which one or more candidate points having been redetermined in this manner and an experimental property value have been superimposed or, in other words, image element map Ma on which a plurality of candidate points including four planned candidate points with experiment numbers (2) to (5) and an experimental property value have been superimposed.

At this point, for example, the user further performs an experiment of a compound (in other words, a candidate compound) having a composition indicated by the planned candidate point with experiment number (2). Accordingly, an experimental property value of the compound is obtained and experimental data of experiment database 150 is updated. As a result, in a similar manner to that described above, experimental value obtainer 232 obtains the composition and the experimental property value of the new compound and outputs the composition and the experimental property value to candidate point determiner 331 and image processor 434. When image processor 434 obtains the composition and the experimental property value of the new compound from experimental value obtainer 232, image processor 434 superimposes the experimental property value on a position on image element map Ma that corresponds to the composition or, in other words, on a position of the planned candidate point with experiment number (2) as illustrated in (c) in FIG. 41. In other words, a new experimental property value is superimposed on image element map Ma instead of the planned candidate point with experiment number (2).

In addition, when experimental data is updated, a predictor of predictor database 120 may be updated to a latest state in accordance with an update result of the experimental data. For example, when the predictor is a model obtained by supervised machine learning, relearning using the composition and the experimental property value of the compound having been newly added to experimental data in training data is executed with respect to the predictor. As a result, predicted value obtainer 132 obtains the updated predictor from predictor database 120 and once again obtains a predicted property value corresponding to a composition of each of a plurality of compounds using the predictor. Candidate point determiner 331 obtains a composition and a predicted property value of each of the plurality of compounds from predicted value obtainer 132 and, based on the obtained composition and predicted property value of each of the plurality of compounds, redetermines one or more candidate points. Accordingly, for example, two new candidate points are added in a periphery of the planned candidate point with experiment number (5) as illustrated in (c) in FIG. 41.

In addition, in a similar manner to the example illustrated in (b) in FIG. 41, candidate point determiner 331 deletes, for example, two candidate points that are not separated by the second minimum separation distance or more from the position of the experimental property value on which the planned candidate point with experiment number (2) has been superimposed as illustrated in (c) in FIG. 41. Image processor 434 displays, on displayer 140, image element map Ma on which one or more candidate points having been redetermined in this manner and an experimental property value have been superimposed or, in other words, image element map Ma on which a plurality of candidate points including three planned candidate points with experiment numbers (3) to (5) and two experimental property values have been superimposed. Accordingly, the user can carry out experiments more efficiently.

As described above, in property display device 430 according to the present embodiment, after image element map Ma is displayed, when processing by experimental value obtainer 232 is subsequently repetitively executed and a new experimental property value is obtained, image processor 434 updates one or more candidate points that have already been superimposed so that all candidate points to be superimposed on image element map Ma are separated by second minimum separation distance or more from the new experimental property value.

Accordingly, every time an experiment of a candidate compound is performed, a new experimental property value is obtained and an appropriate candidate point can be displayed in accordance with the new experimental property value. Accordingly, efficiency of material search can be improved and material development can be appropriately supported.

[Processing operations]

FIG. 42 is a flowchart illustrating processing operations of display system 400 according to the present embodiment.

Display system 400 according to the present embodiment executes processing of steps S111, S112, S121, S131, and S132 illustrated in FIG. 33 in a similar manner to display system 300 according to Embodiment 2A. Next, display system 400 executes processing of steps S141 and S142.

(Step S141)

Image processor 434 obtains a composition and a predicted property value of each of a plurality of compounds from predicted value obtainer 132. In addition, image processor 434 obtains one or more candidate points from candidate point determiner 331. Furthermore, image processor 434 obtains a composition and an experimental property value of each of one or more compounds that have been experimented on from experimental value obtainer 232. In addition, image processor 434 receives the third display method information from display method obtainer 133.

Image processor 434 generates image element map Ma based on a composition and a predicted property value of each of the plurality of obtained compounds. Furthermore, image processor 434 superimposes the one or more candidate points obtained from candidate point determiner 331 on image element map Ma and superimposes the one or more experimental property values obtained from experimental value obtainer 232 on image element map Ma. The superimposition of the candidate points is executed in accordance with the third display method information. Image processor 434 outputs an image including image element map Ma on which the one or more candidate points and the experimental property value have been superimposed to displayer 140.

(Step S142)

Displayer 140 obtains the image from image processor 434 and displays the image or, in other words, displays an image including image element map Ma on which one or more candidate points and an experimental property value have been superimposed.

By executing processing of steps S111, S112, S121, S131, S132, S141, and S142 described above, property display regarding a predicted property value, candidate points, and an experimental property value of a compound is performed. As a result, the user can appropriately recognize an overall picture of experimental property values on image element map Ma while comparing the experimental property values with predicted property values and candidate points.

(Variation of Embodiment 2B)

The display system according to the present variation includes a function of saving and displaying an image generated by property display device 430 or, in other words, an image including image element map Ma on which one or more candidate points and an experimental property value have been superimposed. Note that among the respective components according to the present variation, the same components as in Embodiment 2B are given the same reference numerals as in Embodiment 2B and detailed descriptions thereof are omitted.

[Configuration of display system 401]

FIG. 43 is a block diagram illustrating an example of a configuration of display system 401 according to the present variation. Display system 401 illustrated in FIG. 43 includes all of the components included in display system 400 according to Embodiment 2B and image storage 410.

[Image storage 410]

Image storage 410 is a recording medium for storing, as a property display image, an image which is generated by image processor 434 and which includes image element map Ma on which one or more candidate points and an experimental property value have been superimposed. This recording medium is, for example, a hard disk drive, a RAM, a ROM, a semiconductor memory, or the like. Furthermore, the recording medium may be volatile or nonvolatile.

[Image processor 434]

Image processor 434 according to the present variation generates a property display image in a similar manner to Embodiment 2B and stores the generated property display image in image storage 410. At this point, image processor 434 may add, to the property display image, date and time information indicating a date and time at which the property display image had been generated or a search range or the like used in the generation of the property display image as reference information.

In addition, when image processor 434 receives third display method information indicating a display method regarding past property display images from display method obtainer 133, image processor 434 reads one or more property display images from image storage 410 according to the third display method information. Furthermore, image processor 434 generates a history list image including the one or more property display images and outputs the history list image to displayer 140.

[Specific example of history list image]

FIG. 44 is a diagram illustrating an example of a history of property display images.

Input unit 110 receives an input operation by the user and outputs third display method information that indicates a search range and a time period in accordance with the input operation as a display method regarding past property display images to display method obtainer 133. The time period is, for example, January 2021 to March 2021. Display method obtainer 133 obtains the third display method information and outputs the third display method information to image processor 434. When image processor 434 obtains the third display method information from display method obtainer 133, image processor 434 reads one or more property display images in accordance with the third display method information from image storage 410. In other words, image processor 434 retrieves, from image storage 410, a property display image to which reference information indicating a same search range as the search range indicated by the third display method information has been added and, at the same time, to which date and time information indicating a date and time within a time period indicated by the third display method information has been added.

In addition, image processor 434 generates a history list image by sorting one or more property display images picked out by the retrieval in an order of dates and times of the date and time information added to the property display images and outputs the history list image to displayer 140. As a result, for example, the history list image is displayed on displayer 140 as illustrated in FIG. 44.

For example, the history list image includes a property display image generated on January 20, 2021 illustrated in (a) in FIG. 44, a property display image generated on February 20, 2021 illustrated in (b) in FIG. 44, and a property display image generated on March 20, 2021 illustrated in (c) in FIG. 44. The property display images are arrayed in chronological order of dates and times.

Accordingly, the user can readily comprehend a monthly update status of experimental property values and candidate points during a time period designated by an input operation by the user or, in other words, three months.

While three property display images are included in the history list image in the example illustrated in FIG. 44, when there are four or more property display images corresponding to third display method information, a history list image including the four or more property display images may be displayed. Conversely, when there are one or two property display images corresponding to the third display method information, a history list image including the one or two property display images may be displayed. In addition, when the number of property display images corresponding to the third display method information is a prescribed number (for example, 100) or more, the property display images may be decimated. As a result, a history list image including less than the prescribed number of property display images is displayed. Accordingly, a situation where a large number of property display images are included and displayed in a history list image can be prevented and each property display image can be made more visible.

As described above, in property display device 430 according to the present embodiment, after generation and display of an image including image element map Ma described above are repetitively executed, image processor 434 reads the plurality of images from image storage 410, generates a composite image including the plurality of images, and outputs the composite image to displayer 140. The composite image is the history list image described above. In order to repetitively execute the generation and display of an image described above, obtaining of a predicted property value, generation of image element map Ma, obtaining of third display method information, obtaining of an experimental property value, superimposition of a candidate point, superimposition of the experimental property value, and generation of an image are repetitively executed.

Accordingly, the user can reflect back on a progression of previous experiments and appropriately determine a policy for future material search. As a result, material search can be made more efficient.

[EMBODIMENT 2C]

A display system according to the present embodiment generates a map indicating a predicted property value of each of a plurality of compounds and superimposes candidate points and experimental property values on the map in a similar manner to Embodiment 2B. In this case, a property display device according to the present embodiment obtains the candidate points from a database instead of determining the candidate points from predicted property values or the like. Note that among the respective components according to the present embodiment, the same components as in Embodiments 2A and 2B are given the same reference numerals as in Embodiments 2A and 2B and detailed descriptions thereof are omitted. In addition, while the map according to the present embodiment is image element map Ma, the map may also be image map Mb.

[Configuration of display system 500]

FIG. 45 is a block diagram illustrating an example of a configuration of display system 500 according to the present embodiment. Display system 500 illustrated in FIG. 45 includes input unit 110, predictor database 120, property display device 530, displayer 140, experiment database 150, and candidate point database (DB) 510. Note that property display device 530 is an example of an information display device.

Property display device 530 according to the present embodiment includes search range obtainer 131, predicted value obtainer 132, display method obtainer 133, experimental value obtainer 232, candidate point obtainer 531, and image processor 534. Note that property display device 530 may be constituted of, for example, a processor such as a CPU and a memory. In this case, the processor functions as property display device 530 by, for example, executing a computer program stored in the memory. Note that the memory may be volatile or non-volatile or may be constituted of a volatile memory and a non-volatile memory.

Note that predicted value obtainer 132 according to the present embodiment outputs a composition and a predicted property value of each of a plurality of compounds to image processor 534. In addition, experimental value obtainer 232 according to the present embodiment outputs a composition and an experimental property value of each of one or more compounds to image processor 534. Furthermore, display method obtainer 133 according to the present embodiment outputs third display method information to image processor 534. In other words, predicted value obtainer 132, experimental value obtainer 232, and display method obtainer 133 according to the present embodiment do not output information for determining a candidate point or the like to candidate point obtainer 531.

[Candidate point obtainer 531]

For example, candidate point obtainer 531 obtains a search range signal from search range obtainer 131. In addition, candidate point obtainer 531 retrieves one or more compounds having a composition included in a search range indicated by the search range signal from candidate point database 510. Candidate point obtainer 531 obtains the composition of the compound picked out by the retrieval from candidate point database 510 as a candidate point and outputs the candidate point to image processor 534.

[Image processor 534]

Image processor 534 obtains a composition and a predicted property value of each of a plurality of compounds from predicted value obtainer 132 and obtains a composition and an experimental property value of each of one or more compounds that have been experimented on from experimental value obtainer 232. In addition, image processor 534 obtains one or more candidate points from candidate point obtainer 531 and receives third display method information from display method obtainer 133. Image processor 534 generates image element map Ma indicating predicted property values of a plurality of compounds and superimposes, on image element map Ma, the experimental property value of each of the one or more compounds that have been experimented on. Furthermore, image processor 534 superimposes one or more candidate points on image element map Ma according to the third display method information. Moreover, image processor 534 outputs an image including image element map Ma on which experimental property values of the one or more compounds and the one or more candidate points have been superimposed to displayer 140.

[Candidate point database 510]

Candidate point database 510 stores candidate point data indicating a composition and the like of each of a plurality of compounds.

FIG. 46 is a diagram illustrating an example of candidate point data stored in candidate point database 510. For example, as illustrated in FIG. 46, candidate point data indicates, with respect to each of a plurality of compounds, a management number, a composition formula, a calcination method, a calcination temperature, and a calcination time of the compound as a composition of the compound. Note that a calcination method, a calcination temperature, and a calcination time represent a processing condition for calcining a compound. For example, as illustrated in FIG. 46, candidate point data indicates the management number "1", the composition formula "$Li_{1.45}La_{0.90}Ti_{1.0}Ga_{0.10}O_3$", the calcination method "1: solid-phase method", the calcination temperature "100°C", and the calcination time "3 hours" of a compound.

For example, candidate point obtainer 531 obtains a search range signal from search range obtainer 131 and obtains a composition formula of a compound included in the search range indicated by the search range signal from candidate point data as a candidate point. In other words, candidate point obtainer 531 generates all combinations of respective option data of variables M3, M3', M4, M4', x, y, a, and b included in the search range. In addition, for each of the combinations, candidate point obtainer 531 obtains a composition formula having the combination from candidate point data as a candidate point. Furthermore, when variables Pa, Pb, and Pc that represent a processing condition are included in the search range, candidate point obtainer 531 generates all combinations of respective option data of a plurality of variables including variables Pa, Pb, and Pc. In addition, for each of the combinations, candidate point obtainer 531 obtains a composition formula and a processing condition corresponding to the combination from candidate point data as a candidate point. Alternatively, candidate point obtainer 531 may obtain composition formulae or composition formulae and processing conditions of all compounds indicated in candidate point data as candidate points regardless of the search range.

[Specific examples of map]

FIG. 47 is a diagram illustrating an example of image element map Ma on which candidate points and experimental property values have been superimposed.

Candidate point obtainer 531 obtains a composition of each of one or more compounds from candidate point database 510 as a candidate point. At this point, candidate point obtainer 531 obtains not only the candidate point but also a management number associated with a composition (in other words, a composition formula and the like) that represents the candidate point. In addition, candidate point obtainer 531 outputs the candidate point and the management number to image processor 534.

As illustrated in FIG. 47, image processor 534 generates image element map Ma indicating a predicted property value of each of a plurality of compounds. In addition, image processor 534 superimposes the one or more candidate points obtained by candidate point obtainer 531 and the experimental property value of each of one or more compounds that have been experimented on obtained by experimental value obtainer 232 on image element map Ma. Furthermore, image processor 534 superimposes reference image a1 indicating each of the one or more candidate points and the management number obtained by candidate point obtainer 531 on image element map Ma in association with the candidate point. A reference image may take the form of a word balloon from a candidate point. In addition, image processor 534 outputs an image including image element map Ma on which experimental property values of the one or more compounds having been experimented on, the one or more candidate points, and reference image a1 have been superimposed to displayer 140. Accordingly, image element map Ma is displayed on displayer 140 as illustrated in FIG. 47.

At this point, image processor 534 may display the processing condition on displayer 140. For example, the user selects one candidate point by performing an input operation with respect to input unit 110. More specifically, the user selects the candidate point by pointing a cursor at a desired candidate point using a mouse or the like included in input unit 110 and clicking a button of the mouse. As a result, display method obtainer 133 obtains an input signal in accordance with the input operation from input unit 110 and notifies image processor 534 of a candidate point which is indicated by the input signal and which has been selected by the user. Upon receiving the notification, image processor 534 displays a reference image indicating a processing condition corresponding to the selected candidate point on displayer 140. For example, the processing condition may be displayed by being included in reference image a1 described above.

Alternatively, image processor 534 may superimpose, in advance, a reference image indicating a management number and a processing condition with respect to each of one or more candidate points on image element map Ma regardless of an input operation by the user.

Alternatively, image processor 534 may first display image element map Ma not including reference image a1 on displayer 140 and, after an input operation with respect to input unit 110 by the user is performed, display image element map Ma illustrated in FIG. 47 on displayer 140. In other words, after an input operation by the user, reference image a1 indicating a management number with respect to each candidate point is superimposed on image element map Ma and an image including image element map Ma is displayed on displayer 140. Alternatively, when the input operation by the user is an operation of selecting one candidate point, image processor 534 may superimpose reference image a1 on image element map Ma only with respect to the selected candidate point and display an image including image element map Ma on displayer 140.

As described above, in property display device 530 according to the present embodiment, candidate point obtainer 531 obtains one or more candidate points from candidate point database 510 and image processor 534 superimposes the one or more obtained candidate points on image element map Ma. Candidate point database 510 stores candidate point data indicating a composition of a compound. For example, as illustrated in FIG. 46, candidate point database 510 stores candidate point data indicating a plurality of candidate points (in other words, a plurality of composition formulae).

Accordingly, since candidate points are obtained from a database such as candidate point database 510, the candidate points can be superimposed on image element map Ma regardless of a predicted property value of each of a plurality of compounds obtained by predicted value obtainer 132. Therefore, candidate points that are considered important in advance can be displayed on image element map Ma. As a result, material search can be made more efficient.

[Processing operations]

FIG. 48 is a flowchart illustrating processing operations of display system 500 according to the present embodiment.

Display system 500 according to the present embodiment executes processing of steps S111, S112, S121, and S131 illustrated in FIG. 42 in a similar manner to display system 400 according to Embodiment 2B. Furthermore, display system 500 executes processing of steps S151, S152, and S153.

(Step S151)

Candidate point obtainer 531 obtains each of one or more candidate points and a management number associated with the candidate point from candidate point data in candidate point database 510. In addition, candidate point obtainer 531 outputs the obtained one or more candidate points and the management numbers to image processor 534.

(Step S152)

Image processor 534 obtains a composition and a predicted property value of each of a plurality of compounds from predicted value obtainer 132. In addition, image processor 534 obtains one or more candidate points and management numbers thereof from candidate point obtainer 531. Furthermore, image processor 534 obtains a composition and an experimental property value of each of one or more compounds that have been experimented on from experimental value obtainer 232. In addition, image processor 534 receives third display method information from display method obtainer 133.

Image processor 534 generates image element map Ma based on a composition and a predicted property value of each of the plurality of obtained compounds. In addition, image processor 534 superimposes the one or more candidate points obtained from candidate point obtainer 531 on image element map Ma and superimposes reference image a1 indicating management numbers corresponding to the candidate points on image element map Ma in association with the candidate points. The superimposition of the candidate points is executed in accordance with the third display method information. Furthermore, image processor 534 superimposes the one or more experimental property values obtained from experimental value obtainer 232 on image element map Ma. Image processor 534 outputs an image including image element map Ma on which the one or more candidate points, reference image a1, and the experimental property values have been superimposed to displayer 140.

(Step S153)

Displayer 140 obtains the image from image processor 534 and displays the image or, in other words, displays an image including image element map Ma on which the one or more candidate points, reference image a1, and the experimental property values have been superimposed.

By executing processing of steps S111, S112, S121, S131, and S151 to S153 described above, property display regarding a predicted property value, candidate points, a management number, and an experimental property value of a compound is performed. As a result, the user can appropriately recognize an overall picture of predicted property values, experimental property values, and candidate points on image element map Ma and readily comprehend management numbers regarding the candidate points.

[EMBODIMENT 3A]

A display system according to the present embodiment displays, instead of a predicted property value of each of a plurality of compounds used in Embodiments 1 and 2, evaluated property values of the compounds in the form of a map. Note that an evaluated property value is an evaluated value with respect to a property of a compound having a composition that corresponds to each position on a map and is handled as an indicator of an experiment regarding the property of a compound. Such an evaluated property value indicates a value in accordance with a calculation method to be selected. Therefore, when the calculation method is equivalent to the calculation method for obtaining a predicted property value according to Embodiments 1 and 2, the display system according to the present embodiment displays predicted property values in the form of a map as evaluated property values in a similar manner to Embodiments 1 and 2. Furthermore, the display system according to the present embodiment superimposes a composition of each of one or more compounds that have been experimented on as experiment points on the map. In addition, the display system according to the present embodiment displays an image including the map described above as a first image, and when a display method of evaluated property values is changed, changes the first image to a second image.

Note that among the respective components according to the present embodiment, the same components as in Embodiments 1 and 2 are given the same reference numerals as in Embodiments 1 and 2 and detailed descriptions thereof are omitted.

[Configuration of display system 600]

FIG. 49 is a block diagram illustrating an example of a configuration of display system 600 according to the present embodiment. Display system 600 illustrated in FIG. 49 includes input unit 110, evaluater database (DB) 620, property display device 630, displayer 140, evaluation display database (DB) 640, and experiment database 650. Note that property display device 630 is an example of an information display device.

Property display device 630 according to the present embodiment obtains an evaluated property value of each of a plurality of compounds using evaluater database 620 and evaluation display database 640. In addition, property display device 630 generates a map indicating the obtained evaluated property values. Furthermore, property display device 630 superimposes an experiment point of each of one or more compounds on the map and displays the map on displayer 140. Property display device 630 includes evaluation value obtainer 632, display method obtainer 633, experiment point obtainer 635, and image processor 634. Note that property display device 630 may be constituted of, for example, a processor such as a CPU and a memory. In this case, the processor functions as property display device 630 by, for example, executing a computer program stored in the memory. Note that the memory may be volatile or non-volatile or may be constituted of a volatile memory and a non-volatile memory. Furthermore, the map according to the present embodiment is image map Mb but may be image element map Ma, or may be a map composed of a plurality of image maps Mb.

[Display method obtainer 633]

Display method obtainer 633 obtains an input signal from input unit 110 and obtains first information d10 regarding a display method of evaluated property values from evaluation display database 640 in accordance with the input signal. In addition, display method obtainer 633 outputs first information d10 to evaluation value obtainer 632, experiment point obtainer 635, and image processor 634. Accordingly, an image including a map in accordance with the display method indicated by first information d10 is displayed on displayer 140 as a first image.

In addition, when display method obtainer 633 obtains an input signal indicating a change to the display method from input unit 110, display method obtainer 633 changes first information d10 in accordance with the input signal and outputs first information d10 after the change to evaluation value obtainer 632, experiment point obtainer 635, and image processor 634. Accordingly, an image including a map in accordance with first information d10 after the change is displayed on displayer 140 as the second image.

[Evaluation value obtainer 632]

Evaluation value obtainer 632 obtains first information d10 from display method obtainer 633. In addition, evaluation value obtainer 632 generates all combinations of option data which may be assumed by each of a plurality of variables included in a search range indicated by first information d10. The combinations represent a composition (for example, a composition formula) of a compound. Therefore, a plurality of compositions according to the combinations are generated.

Evaluation value obtainer 632 obtains one or more evaluaters from evaluater database 620 and inputs the plurality of generated compositions in the one or more evaluaters. As a result, for each composition, evaluation value obtainer 632 obtains an evaluated property value with respect to one or more properties of a compound having the composition. At this point, evaluation value obtainer 632 obtains the evaluated property values according to the first information d10 described above.

[Experiment point obtainer 635]

Experiment point obtainer 635 obtains first information d10 from display method obtainer 633. In addition, experiment point obtainer 635 obtains a composition of each of one or more compounds which have been experimented on and which corresponds to a search range indicated by first information d10 from experiment database 650 and outputs the composition of each of the one or more compounds to image processor 634 as an experiment point. Note that regardless of the search range, experiment point obtainer 635 may obtain each of the compositions of all compounds stored in experiment database 650 as an experiment point.

[Image processor 634]

Image processor 634 obtains an evaluated property value of each of a plurality of compounds from evaluation value obtainer 632 and obtains an experiment point of each of one or more compounds that have been experimented on from experiment point obtainer 635. In addition, image processor 634 obtains first information d10 from display method obtainer 633. Image processor 634 generates a map indicating evaluated property values of the plurality of compounds according to first information d10 and further superimposes one or more experiment points obtained by experiment point obtainer 635 on the map. Note that in generation of the map indicating evaluated property values, with respect to each of a plurality of compounds, image processor 634 adds a color of a shade in accordance with an evaluated property value of the compound to a position on the map that corresponds to a composition of the compound. The color of a shade in accordance with an evaluated property value may be a color in accordance with the evaluated property value. In addition, in superimposition of an experiment point, with respect to each of one or more compounds that have been experimented on, image processor 634 superimposes a black circular mark on a position on a map corresponding to an experiment point of the compound. Image processor 634 outputs an image including the map on which the one or more experiment points have been superimposed to displayer 140 as the first image or the second image.

[Evaluater database 620]

Evaluater database 620 is a recording medium storing at least one evaluater for evaluating a property value of a compound. This recording medium is, for example, a hard disk drive, a RAM, a ROM, a semiconductor memory, or the like. Furthermore, the recording medium may be volatile or nonvolatile.

An evaluater is a computer program based on a prescribed calculation algorithm constructed by machine learning or the like. With respect to an input of a composition (in other words, a combination of a plurality of variables) from evaluation value obtainer 632, the evaluater outputs an evaluated value of a property value of a compound having the composition as an evaluated property value. Note that the composition is represented by a combination of option data which may be assumed by each of a plurality of variables included in a search range.

An evaluated property value is an indicator of an experiment and is, for example, a predicted value of a property obtained by a machine learning model such as Least Absolute Shrinkage and Selection Operator (LASSO) regression or Random Forest regression. The predicted value corresponds to the predicted property value according to Embodiments 1 and 2. In addition, an evaluated property value may be an output value of an acquisition function in Bayesian optimization. An acquisition function is a function for determining a next experimental candidate (in other words, a candidate of a composition of a compound to be used in a next experiment or a candidate compound according to Embodiment 2). Examples of the acquisition function include Expected Improvement (EI), Probability of Improvement (PI), Upper Confidence Bound (UCB), or the like using a mean of predicted values and a variance value of predicted values according to Gaussian Process regression.

In addition, an evaluater may output an evaluated property value with respect to various properties of a compound such as an electrochemical property or a thermochemical property. Furthermore, evaluater database 620 may store a plurality of evaluaters and the plurality of evaluaters may be selectively used in accordance with a property of an evaluation object. For example, evaluater database 620 stores evaluater A that outputs an evaluated property value with respect to an electrochemical property and evaluater B that outputs an evaluated property value with respect to a thermochemical property. In this case, for example, when a property of an evaluation object is an electrochemical property, evaluation value obtainer 632 obtains evaluater A and obtains an evaluated property value with respect to an electrochemical property of each of a plurality of compounds using evaluater A. Alternatively, when a property of an evaluation object is a thermochemical property, evaluation value obtainer 632 obtains evaluater B and obtains an evaluated property value with respect to a thermochemical property of each of a plurality of compounds using evaluater B. Accordingly, complication of a calculation algorithm of each evaluater can be suppressed and evaluation accuracy and processing speed in each property can be improved. The present disclosure is not limited to the example described above and an evaluater may be given any format as long as the evaluater can output an evaluated property value with respect to a compound having a composition represented by a combination of a plurality of variables. Such an evaluation of a property value of a compound can also be regarded as a search for the property value of the compound.

[Evaluation display database 640]

Evaluation display database 640 is a recording medium storing information regarding a display method of an evaluated property value. This recording medium is, for example, a hard disk drive, a RAM, a ROM, a semiconductor memory, or the like. Furthermore, the recording medium may be volatile or nonvolatile.

FIG. 50 is a diagram illustrating an example of information stored in evaluation display database 640.

As illustrated in FIG. 50, evaluation display database 640 stores first information d10 that is information regarding a display method of an evaluated property value. First information d10 includes first color information d11, calculation method information d12, map array information d13, search range information d14, first display target information d15, and display range information d16.

First color information d11 indicates at least one of a hue, a chroma, and a brightness of an evaluated property value indicated on a map as a color attribute.

Calculation method information d12 is information regarding a calculation method of an evaluated property value or, in other words, information to be applied to an evaluater in order to calculate an evaluated property value. For example, calculation method information d12 may be information indicating a type of an evaluater used to calculate an evaluated property value or, specifically, information indicating a type of a machine learning model and may be a parameter used in the evaluater. For example, when the evaluater is a neural network, a parameter may be a weight, a bias, or the like to be used in the neural network.

Map array information d13 is information indicating a form of a map to be generated by image processor 634. For example, when the map to be generated by image processor 634 includes a plurality of image element maps Ma having coordinate axes A1 and A2 indicating variables x and y as in the case of image map Mb illustrated in FIG. 11, map array information d13 indicates an array method or the like of image element maps Ma.

Search range information d14 indicates a search range used to calculate an evaluated property value. First display target information d15 indicates a property of a display target regarding an evaluated property value. Display range information d16 indicates a display range of an evaluated property value. Note that details of each piece of information described above that is included in first information d10 will be described later.

[Experiment database 650]

FIG. 51 is a diagram illustrating an example of each piece of data stored in experiment database 650.

As illustrated in FIG. 51, experiment database 650 is a recording medium that respectively stores, as experimental data, compound basic data 651 and one or more pieces of compound detail data 652. This recording medium is, for example, a hard disk drive, a RAM, a ROM, a semiconductor memory, or the like. Furthermore, the recording medium may be volatile or nonvolatile. Compound basic data 651 indicates basic information of each of one or more compounds that have been experimented on. Each of the one or more pieces of compound detail data 652 indicate detailed information of the compound.

FIG. 52 is a diagram illustrating an example of compound basic data 651.

With respect to each of one or more compounds, compound basic data 651 indicates an ID that is compound identification information of the compound, a composition formula of the compound, a processing condition of the compound, one or more properties of the compound, and a crystal phase of the compound. For example, compound basic data 651 illustrated in FIG. 52 indicates an ID "000001-00001-001" of a compound that has been experimented on, a composition formula "$Li_{1.45}La_{0.045}Ti_{1.1}Al_{0.005}O_3$" of the compound, a processing condition "calcination temperature: 400°C, calcination time: 3 hours", properties "property 1: 2.349 eV, property 2: 213°C" of the compound, and a crystal phase "cubic-perovskite" of the compound. Note that a composition including the composition formula and the processing condition of the compound is defined by variables (M3, M3', M4, M4', a, b, Temp, Time, x, y)=(La, Al, Ti, Zr, 0.05, 0.1, 400, 3, 0.1, 0).

The ID that is compound identification information is a name, a sign, a digit sequence, or the like of a compound and may be any kind of information that enables the compound to be identified. The ID 000001-00001-001 illustrated in FIG. 52 is made up of numerals (000001, 00001, and 001) of three levels. For example, IDs are registered such that first-level numbers "000001" to "000005" are assigned to compositions of five compounds whose composition ranges of the elements Zr and Ti included in the compounds differ from each other.

For example, property 1 described above of the compound is a band gap and property 2 described above is a heat-resistance temperature. Property 1, property 2, and the crystal phase are respectively handled as experimental property values. While the band gap, the heat-resistance temperature, and the crystal phase are respectively handled as experimental property values in the example illustrated in FIG. 52, experimental property values are not limited thereto and any property in accordance with the compound may be handled as an experimental property value. For example, electrical conductivity may be handled as an experimental property value with respect to a compound used in a battery material and a thermoelectric conversion performance index may be handled as an experimental property value with respect to a compound used in a thermoelectric conversion material.

FIG. 53 is a diagram illustrating an example of compound detail data 652.

Compound detail data 652 includes an ID that is compound identification information of a compound that has been experimented on and accompanying information of the compound. The accompanying information is so-called metadata and includes, for example, a name of a researcher or an experimenter having performed an experiment of the compound, a date of registration of the accompanying information, air temperature and humidity at the time of the experiment, processing information, and measured data.

The processing information indicates a raw material used in synthesis or generation of the compound and an actual composition of the compound. Note that an actual composition refers to, for example, a composition formula indicating a strict composition ratio or a composition formula indicating a numerical value down to three or four decimal places as a composition ratio such as $Li_{1.451}La_{0.0450}Ti_{1.102}Al_{0.005}O_{3.01}$. In addition, the processing information may indicate an experiment procedure of the compound, a shape and a size of a crucible, a type of an apparatus used in the experiment, a serial number of the apparatus, or the like.

Measured data is data obtained by a measuring instrument for deriving an experimental property value and indicates, for example, electrical resistance, differential scanning calorimetry (DSC), or X-ray diffraction (XRD). Note that measured data is not limited to these examples and may indicate any kind of data in accordance with the compound.

While experiment database 650 according to the present embodiment stores compound basic data 651 and compound detail data 652, experiment database 650 need only store at least compound basic data 651. In the present embodiment, due to compound detail data 652 being stored in experiment database 650, material development can be supported more appropriately. For example, when compound detail data 652 indicates a name of a researcher or an experimenter, a registration date, air temperature and humidity, and the like, credibility of experimental data can be enhanced. In addition, when compound detail data 652 indicates processing information, a more specific search for material properties and improvement of a value of experimental data are expected. Furthermore, when compound detail data 652 indicates measured data, raw data of an experimental property value can be readily comprehended.

[Specific examples of map]

FIGS. 54 and 55 are diagrams illustrating an example of a map according to the present embodiment. Note that FIG. 56 indicates a legend or the like of the map illustrated in FIG. 55. In other words, [A] to [R] in FIG. 55 are composition formulae associated with A to R illustrated in FIG. 56.

FIGS. 54 and 55 illustrate image map Mb for each of combinations of respective pieces of option data of four categorical variables (M3, M3’, M4, and M4’). Image map Mb is a map indicating, for each combination of respective pieces of option data of four variables (x, y, a, and b), an evaluated property value of a compound having a compound represented by the combination. In addition, image map Mb includes an array (or a matrix) of a plurality of image element maps Ma. Continuous variables x and y are assigned to two coordinate axes of image element map Ma, and discrete variables a and b are assigned to two coordinate axes of the array of image element maps Ma (in other words, image map Mb). Note that an assignment method of the variables is not limited to this method. An evaluated property value obtained with respect to each of all combinations of option data included in the search range may be displayed as-is. In addition, interpolation of an evaluated property value may be performed with respect to combinations other than all combinations included in the search range. Such an evaluated property value is indicated by a color or a shade of color in a similar manner to a predicted property value.

FIG. 54 illustrates image maps Mb corresponding to two combinations of four categorical variables, and FIG. 55 illustrates image maps Mb respectively corresponding to 18 combinations of four categorical variables. The number of image element maps Ma included in a map according to the present disclosure is determined on the basis of the number of combinations of respective pieces of option data of two discrete variables a and b and the number of combinations of respective pieces of option data of four categorical variables (in other words, elements). In other words, the wider a search range indicated in search range information d14, the larger the number of image element maps Ma. Therefore, it is important for the user to devise means of display in order to facilitate visual understanding.

As an example of display, a case where the user retrieves an evaluated property value with respect to a composition of a compound represented by a composition formula "$Li_{2-3a-4b}(M3_{1-x}M3'_{x})_{a}(M4_{1-y}M4'_{y})_{1+b}O_{3}$" and a processing condition "a calcination method Pa, a calcination time Pb, and a calcination temperature Pc" will be described with reference to FIGS. 54 and 55.

Image processor 634 obtains first information d10 from evaluation display database 640 via display method obtainer 633 and generates a map in accordance with a form of a map indicated in map array information d13 included in first information d10. Specifically, first, image processor 634 selects four variables a, b, x, and y from continuous variables x and y, discrete variables a and b, and processing variables Pa, Pb, and Pc. This is done because four variables must be assigned to four coordinate axes including the two coordinate axes of image map Mb and the two coordinate axes of image element maps Ma included in image map Mb.

Next, image processor 634 assigns continuous variables x and y to the two coordinate axes of image element map Ma. In a similar manner to Embodiment 1, continuous variables x and y may respectively assume any one of 11 pieces of option data indicated by a step width of 0.1 from a minimum value 0.0 to a maximum value 1.0. In other words, there are 11 combinations of option data that can be respectively assumed by continuous variables x and y. Therefore, image element map Ma is a square. However, in a similar manner to Embodiment 1, the shape of image element map Ma is dependent on an assignment method of coordinate axes of image element map Ma and may be a triangle or another shape.

In addition, the image processor 634 assigns discrete variables a and b to the two coordinate axes of the array of image element maps Ma. The discrete variable a includes five pieces of option data, namely, 0.0, 0.05, 0.1, 0.15, and 0.2, and the discrete variable b includes four pieces of option data, namely, 0.0, 0.1, 0.2, and 0.3. Therefore, the number of image element maps Ma for each of the combinations of the categorical variables in FIG. 54 is $5 \times 4 = 20$. In other words, image map Mb includes an array of $5 \times 4$-number of image element maps Ma.

Furthermore, with respect to the categorical variables, there are six combinations of option data being elements respectively selected for variables M3 and M3’ among the elements La, Al, Ga, and In and there are three combinations of option data being elements respectively selected for variables M4 and M4’ among the elements Ti, Zr, and Hf. As a result, a total of 18 image maps Mb or, in other words, six image maps Mb in a horizontal direction and three image maps Mb in a vertical direction which correspond to combinations of the categorical variables and each of which includes 20 image element maps Ma (a total of 20 × 18 = 360 image element maps Ma) are illustrated in FIG. 55. Image processor 634 may generate a map made up of 18 image maps Mb as illustrated in FIG. 55 or generate a map including two image maps Mb among 18 image maps Mb as illustrated in FIG. 54. In addition, image processor 634 may generate one image map Mb as a map or generate one image element map Ma as a map. In other words, a map according to the present embodiment may be a set of one or more image maps Mb or a set of one or more image element maps Ma.

Evaluation value obtainer 632 obtains first information d10 from evaluation display database 640 via display method obtainer 633 and calculates an evaluated property value based on a search range indicated in search range information d14 included in first information d10. Evaluation value obtainer 632 generates an evaluated property value with respect to all combinations of respective pieces of option data of variables x, y, a, b, Pa, Pb, and Pc. In addition, when obtaining the evaluated property value, evaluation value obtainer 632 uses calculation method information d12 and first display target information d15 included in first information d10. In other words, evaluation value obtainer 632 obtains one or more evaluaters from evaluater database 620 according to a calculation method of an evaluated property value indicated in calculation method information d12 and a property of a display target indicated in first display target information d15 and obtains an evaluated property value using the evaluaters. While an example of a property of the display target is a band gap, the property of the display target is not limited thereto.

In this case, a plurality of variables included in a search range includes visualization variables being variables that are used in coordinate axes of a map and non-visualization variables that are not used in coordinate axes of a map. For example, when the visualization variables are variables x, y, a, and b, the non-visualization variables are variables Pa, Pb, and Pc. 18 image maps Mb illustrated in FIG. 55 are generated with respect to one combination of respective pieces of option data of non-visualization variables Pa, Pb, and Pc. Note that a non-visualization variable is a variable that is not used in coordinate axes of maps and is therefore also referred to as a non-utilized variable.

Therefore, image processor 634 determines one combination of respective pieces of option data of non-visualization variables Pa, Pb, and Pc and generates a map indicating an evaluated property value corresponding to the determined combination of option data. As illustrated in FIG. 12, among the non-visualization variables, variable Pa has two possible pieces of option data, namely, "solid reaction method" and "ball mill", variable Pb has three possible pieces of option data, namely, 1, 3, and 5, and variable Pc has seven possible pieces of option data, namely, 100, 110, 120, 130, 140, 150, and 160. Therefore, image processor 634 determines one combination from among 2 × 3 × 7 = 42 combinations of respective pieces of option data of variables Pa, Pb, and Pc. For example, image processor 634 may determine "solid reaction method" as the option data of variable Pa and determine minimum values as respective pieces of option data of variables Pb and Pc. In other words, image processor 634 determines non-visualization variables (Pa, Pb, Pc) = (solid reaction method, 1, 100). Alternatively, image processor 634 may obtain an input signal from input unit 110 via display method obtainer 633 and determine a combination in accordance with the input signal.

In the example described above, evaluation value obtainer 632 obtains evaluated property values with respect to all combinations of the plurality of variables and image processor 634 determines evaluated property values to be indicated on a map from the obtained evaluated property values. However, evaluation value obtainer 632 may obtain only evaluated property values to be indicated on the map and image processor 634 may indicate all of the evaluated property values obtained by evaluation value obtainer 632 on a map.

In addition, when generating a map, image processor 634 uses first color information d11 and display range information d16 included in first information d10. For example, image processor 634 adds a color of a shade in accordance with an evaluated property value having been set according to display range information d16 and which has a hue or the like indicated by first color information d11 to a position on the map corresponding to a composition of a compound having the evaluated property value. Furthermore, as an additional measure for making it easier to visually understand experimental property values, a data representation, a scale indicating a relationship between evaluated property values and colors or shades of color, or a value of range variables may be displayed above the maps.

In addition, as illustrated in FIGS. 54 and 55, experiment point obtainer 635 obtains one or more experiment points from experiment database 650. In addition, when first display target information d15 included in first information d10 indicates property 1 as a property of a display target, experiment point obtainer 635 extracts a composition of a compound associated with property 1 in compound basic data 651 of the experimental data from compound basic data 651. For example, property 1 is a band gap.

Next, with respect to each of the one or more experiment points obtained by experiment point obtainer 635, image processor 634 determines a position on the map corresponding to a composition of a compound that is the experiment point and superimposes a black circular mark as the experiment point on the position. The superimposition of the mark is also called superimposition of an experiment point. In addition, while an experiment point according to the present embodiment is an experiment point with respect to property 1 and a composition of a compound for which property 1 is registered in compound basic data 651 is extracted as an experiment point, an experiment point is not limited thereto.

As described above, property display device 630 according to the present embodiment generates a map such as image map Mb illustrated in FIG. 54 or 55 according to first information d10. In addition, property display device 630 displays an image including the map on displayer 140 as the first image. In this case, in the present embodiment, display method obtainer 633 changes first information d10 in accordance with an input operation with respect to input unit 110 by the user. Accordingly, property display device 630 changes a map according to the changed first information d10. As a result, property display device 630 changes the first image to the second image including the map after the changes and displays the second image on displayer 140.

Hereafter, specific examples of changing the first image to the second image will be described in detail.

[Specific example 1 of change of images]

FIGS. 57A and 57B are diagrams illustrating an example of a transition of images accompanying a change in calculation method information d12. Specifically, FIG. 57A illustrates calculation method information d12 included in first information d10 and an example of the first image displayed on displayer 140 according to calculation method information d12. FIG. 57B illustrates calculation method information d12 after a change and an example of the second image displayed on displayer 140 according to changed calculation method information d12.

For example, display method obtainer 633 obtains calculation method information d12 illustrated in (b) in FIG. 57A from evaluation display database 640. Calculation method information d12 respectively indicates a machine learning model, Bayesian optimization (acquisition function), and display as setting items. The setting items are also handled as options. The setting item of a machine learning model respectively indicates, as options, a plurality of types of a machine learning model and a plurality of setting parameters with respect to the types of machine learning models.

For example, in FIGS. 57A and 57B, the plurality of types of machine learning models are LASSO (least absolute shrinkage and selection operator) regression, Support Vector regression, Gradient Boosting regression, and Gaussian Process regression. Each of these types represents an option of a type of a machine learning model. Furthermore, the plurality of setting parameters with respect to LASSO regression are, respectively, alphanumeric values and are 0.01, 0.1, 0, 1, and 10. Each of the numerical values represents an option of setting parameters.

In addition, a grid search is included as an option in the setting item of a machine learning model. In a grid search, the machine learning model and the setting parameters are optimized.

For example, the setting item of Bayesian optimization (acquisition function) respectively indicates EI (Expected Improvement), PI (Probability of Improvement), and UCB (Upper Confidence Bound) as options. Note that when UCB is selected, a value of a coefficient of variance thereof is stored and managed as a floating-point number type.

For example, the setting item of display respectively indicates a prediction mean, a prediction variance, and "prediction mean + prediction variance" as options. The options represent types of evaluated property values. Note that when "prediction mean + prediction variance" is selected, a value of a coefficient of variance thereof is stored and managed as a floating-point number type.

In the example illustrated in (b) in FIG. 57A, the setting item of a machine learning model is selected and, further, Gradient Boosting regression is selected. In addition, in Gradient Boosting regression, "100" is selected with respect to a first setting parameter "n_estimators", "3" is selected with respect to a second setting parameter "max_depth", and "0.5" is selected with respect to a third setting parameter "max_features". Furthermore, Bayesian optimization is not selected and prediction mean is selected in the setting item of display.

Evaluation value obtainer 632 obtains such calculation method information d12 from display method obtainer 633. In addition, according to calculation method information d12, evaluation value obtainer 632 obtains one or more evaluaters from evaluater database 620 and obtains an evaluated property value of each of a plurality of compounds using the one or more evaluaters. The evaluated property value obtained at this point corresponds to, for example, the predicted property value according to Embodiments 1 and 2. As illustrated in (a) in FIG. 57A, image processor 634 generates image map Mb indicating the evaluated property value of each compound obtained in this manner. Furthermore, image processor 634 obtains one or more experiment points from experiment point obtainer 635 and superimposes the one or more experiment points on image map Mb. Image processor 634 generates the first image including image map Mb on which the one or more experiment points have been superimposed and displays the first image on displayer 140.

In the first image illustrated in (a) in FIG. 57A, an evaluated property value (in other words, a prediction mean) of image element map Ma1 corresponding to variables (a, b) = (0.0, 0.2) included in image map Mb is larger than other image element maps Ma.

At this point, the user changes calculation method information d12 in first information d10 by performing an input operation with respect to input unit 110. In other words, when display method obtainer 633 obtains an input signal indicating a change of calculation method information d12 from input unit 110, display method obtainer 633 changes calculation method information d12 as illustrated in (b) in FIG. 57B according to the input signal.

In the example illustrated in (b) in FIG. 57B, the setting item of a machine learning model is selected and, further, Gaussian Process regression is selected. Furthermore, "rbf" is selected with respect to a setting parameter "kernel" in the Gaussian Process regression. In addition, the setting item of Bayesian optimization is selected and, furthermore, UCB is selected. Note that the coefficient of variance in UCB is 2.0. Furthermore, in the setting item of display, "prediction mean + prediction variance" is selected. In this manner, calculation method information d12 is changed from the state illustrated in (b) in FIG. 57A to the state illustrated in (b) in FIG. 57B.

Evaluation value obtainer 632 obtains calculation method information d12 changed in such manner from display method obtainer 633. In addition, according to calculation method information d12, evaluation value obtainer 632 obtains one or more evaluaters from evaluater database 620 and obtains an evaluated property value of each of a plurality of compounds using the one or more evaluaters. As illustrated in (a) in FIG. 57B, image processor 634 generates image map Mb indicating the evaluated property value of each compound obtained in this manner. Furthermore, image processor 634 generates the second image including the image map Mb on which the one or more experiment points have been superimposed and displays the first image on displayer 140. Accordingly, the first image is changed to the second image.

In the second image illustrated in (a) in FIG. 57B, an evaluated property value (in other words, prediction mean + prediction variance) of image element map Ma2 corresponding to variables (a, b) = (0.0, 0.0) included in image map Mb is larger than other image element maps Ma. A prediction variance is an indicator representing an uncertainty of a predicted value of a property and, in a region where an experimental property value is not obtained in image map Mb, a variance of a predicted value is large. In other words, since experiment points are absent from image element map Ma2, an evaluated property value (prediction mean + prediction variance) of image element map Ma2 is large.

In this manner, a change in calculation method information d12 causes an image to be displayed on displayer 140 to dynamically change from the first image to the second image. Accordingly, material development by the user can be appropriately supported. For example, "prediction mean + prediction variance" that takes variance into consideration is caused to be displayed on displayer 140 as an evaluated property value in an initial stage of material development while only a prediction mean is caused to be displayed on displayer 140 as an evaluated property value in a final stage of material development.

As described above, property display device 630 according to the present embodiment includes display method obtainer 633, evaluation value obtainer 632, and image processor 634. Display method obtainer 633 obtains first information d10 for displaying an evaluated property value as a comparative indicator for comparing a plurality of compounds. Evaluation value obtainer 632 and image processor 634 obtains an evaluated property value of each of the plurality of compounds based on first information d10 and generates image map Mb indicating an evaluated property value of each of the plurality of compounds at a position corresponding to a composition of the compound. In addition, image processor 634 generates the first image including image map Mb and outputs the first image to displayer 140. Furthermore, when first information d10 is changed, image processor 634 changes image map Mb according to changed first information d10, generates the second image including changed image map Mb, and outputs the second image to displayer 140. For example, the first image and the second image described above are outputted to and displayed by displayer 140.

Accordingly, when first information d10 is changed, since image map Mb in accordance with contents of the change is displayed by being included in the second image, an evaluated property value of each of a plurality of compounds can be displayed from various points of view. As a result, material search can be made more efficient, and material development can be appropriately supported.

In addition, in property display device 630 according to the present embodiment, first information d10 includes search range information d14 which indicates a plurality of variables to be used in representing compositions of compounds and a plurality of pieces of option data indicating values or elements that can be assumed by each of the plurality of variables. For each combination of option data obtained by selecting one piece of option data from the plurality of pieces of option data with respect to each of the plurality of variables, evaluation value obtainer 632 obtains an evaluated property value of a compound having a composition corresponding to the combination.

Accordingly, since a composition of a compound can be represented by a combination of respective pieces of option data of a plurality of variables, the composition can be clearly defined and an evaluated property value of each of a plurality of compounds can be appropriately obtained. As a result, material search can be made more efficient, and material development can be appropriately supported.

Furthermore, in property display device 630 according to the present embodiment, first information d10 includes calculation method information d12 indicating a calculation method of an evaluated property value. For each combination of the option data described above, evaluation value obtainer 632 calculates an evaluated property value of a compound having a composition corresponding to the combination using a prescribed algorithm in accordance with the calculation method indicated by calculation method information d12. In display method obtainer 633, the calculation method indicated by calculation method information d12 is changed.

Accordingly, when calculation method information d12 is changed, since image map Mb in accordance with contents of the change is displayed by being included in the second image, an evaluated property value of each of a plurality of compounds can be displayed from points of view of various calculation methods. As a result, material search can be made more efficient.

In addition, in property display device 630 according to the present embodiment, evaluation value obtainer 632 uses a prescribed algorithm in accordance with the calculation method to calculate, for each combination of the option data described above, a predictive distribution of values of a property that is predicted with respect to a compound having a composition corresponding to the combination and calculates an evaluated property value of the compound based on the predictive distribution. For example, a predictive distribution is obtained by Gaussian Process regression and an evaluated property value is obtained by an acquisition function of Bayesian optimization.

Accordingly, since an evaluated property value based on a predictive distribution is calculated, an evaluated property value that includes a prediction variance in a prediction mean can be obtained. As a result, the user can readily confirm an evaluated property value on which a prediction variance is also reflected with respect to each of a plurality of compounds and efficiency of material search can be improved.

Furthermore, in property display device 630 according to the present embodiment, first information d10 includes first color information d11 that indicates at least one of a hue, a chroma, and a brightness as a color attribute of an evaluated property value. Image processor 634 generates image map Mb that indicates an evaluated property value of each of the plurality of compounds in a color having a color attribute indicated by first color information d11. Image maps Mb illustrated in FIGS. 57A and 57B are generated according to first color information d11. In display method obtainer 633, the color attribute indicated by first color information d11 is changed. For example, display method obtainer 633 changes the color attribute in accordance with an input operation with respect to input unit 110 by the user.

Accordingly, the color of an evaluated property value indicated on image map Mb is changed to a color having the changed color attribute. Therefore, a color attribute of an evaluated property value indicated on image map Mb can be optionally changed and image map Mb readily visible by the user can be displayed by being included in the second image. As a result, material search can be made more efficient.

[Specific example 2 of change of images]

FIGS. 58A and 58B are diagrams illustrating an example of a transition of images accompanying a change in search range information d14. Specifically, FIG. 58A illustrates a part of search range information d14 included in first information d10 and an example of the first image displayed on displayer 140 according to search range information d14. FIG. 58B illustrates a part of search range information d14 after a change and an example of the second image displayed on displayer 140 according to changed search range information d14.

For example, display method obtainer 633 obtains search range information d14 including setting information of a non-visualization variable illustrated in (b) in FIG. 58A from evaluation display database 640. The setting information of a non-visualization variable is information indicating a search range of the non-visualization variable. A non-visualization variable is a processing variable illustrated in FIG. 12 and specific examples include variable Pa indicating a calcination method, variable Pb indicating a calcination time, and variable Pc indicating a calcination temperature. In the examples illustrated in FIGS. 58A and 58B, setting information of a non-visualization variable indicates pieces of option data respectively selected with respect to each of a calcination time (in other words, variable Pb) and a calcination temperature (in other words, variable Pc) among the variables described above. In the present embodiment, a plurality of pieces of option data with respect to variable Pc of calcination temperature include 300°C, 400°C, 500°C, and 600°C, and a plurality of pieces of option data with respect to variable Pb of calcination time include 1 hour, 3 hours, and 5 hours. Furthermore, in the example illustrated in FIG. 58A, the setting information of a non-visualization variable indicates minimum (in other words, 300°C) as option data selected with respect to variable Pc of calcination temperature and minimum (in other words, 1 hour) as option data selected with respect to variable Pb of calcination time.

Note that in FIGS. 58A and 58B, maximum with respect to variable Pc of calcination temperature is a maximum temperature among the four pieces of option data (300°C, 400°C, 500°C, and 600°C) or, in other words, 600°C, and a mean is a mean temperature of the four pieces of option data or, in other words, 450°C. In addition, best with respect to variable Pc of calcination temperature is a temperature at which, for example, a largest evaluated property value is obtained among the four pieces of option data, and a user-defined option refers to a temperature that is designated by the user from the pieces of option data.

In a similar manner, maximum with respect to variable Pb of calcination time is a maximum time among the three pieces of option data (1 hour, 3 hours, and 5 hours) or, in other words, 5 hours, and a mean is a mean time of the three pieces of option data or, in other words, 3 hours. In addition, best with respect to variable Pb of calcination time is a time during which, for example, a largest evaluated property value is obtained among the three pieces of option data, and a user-defined option refers to a time that is designated by the user from the pieces of option data.

Evaluation value obtainer 632 obtains search range information d14 including such setting information of a non-visualization variable from display method obtainer 633. In addition, evaluation value obtainer 632 obtains an evaluated property value in accordance with the setting information of a non-visualization variable. In other words, evaluation value obtainer 632 obtains, using one or more evaluaters, an evaluated property value with respect to each compound having a composition represented using variables (Pb, Pc) = (1, 300) among a plurality of compounds. As illustrated in (a) in FIG. 58A, image processor 634 generates image map Mb indicating the evaluated property value of each compound obtained in this manner. In other words, a compound having each evaluated property value indicated on image map Mb is a compound calcined using variables (Pb, Pc) = (1, 300) and pieces of option data of the variables are fixed with respect to image map Mb. Furthermore, image processor 634 superimposes one or more experiment points on image map Mb. Image processor 634 generates the first image including image map Mb on which the one or more experiment points have been superimposed and displays the first image on displayer 140.

At this point, the user changes setting information of a non-visualization variable included in search range information d14 in first information d10 by performing an input operation with respect to input unit 110. In other words, when display method obtainer 633 obtains an input signal indicating a change of setting information of a non-visualization variable from input unit 110, display method obtainer 633 changes setting information of a non-visualization variable as illustrated in (b) in FIG. 58B according to the input signal.

In the example illustrated in (b) in FIG. 58B, a user-defined option (500°C) is selected with respect to the calcination temperature and minimum (1 hour) is selected with respect to the calcination time. In other words, respective pieces of option data of variables Pb and Pc are changed from (Pb, Pc) = (1, 300) to (Pb, Pc) = (1, 500).

Evaluation value obtainer 632 obtains search range information d14 including such setting information of a non-visualization variable changed in such manner from display method obtainer 633. In addition, evaluation value obtainer 632 obtains an evaluated property value in accordance with the setting information of the changed non-visualization variable. In other words, evaluation value obtainer 632 obtains, using one or more evaluaters, an evaluated property value with respect to each compound having a composition represented using variables (Pb, Pc) = (1, 500) among a plurality of compounds. As illustrated in (a) in FIG. 58B, image processor 634 generates image map Mb indicating the evaluated property value of each compound obtained in this manner. In other words, a compound having each evaluated property value indicated on image map Mb is a compound calcined using variables (Pb, Pc) = (1, 500) and pieces of option data of the variables are fixed with respect to image map Mb. Furthermore, image processor 634 generates the second image including image map Mb on which the one or more experiment points have been superimposed and displays the first image on displayer 140.

On image map Mb illustrated in (a) in FIG. 58B, there are more regions (in other words, image element maps Ma) in which an evaluated property value has improved as compared to image map Mb illustrated in (a) in FIG. 58A. Therefore, the user can determine that conducting an experiment by changing the calcination temperature from 300°C to 500°C is effective.

In this manner, a change in the setting information of a non-visualization variable that is included in search range information d14 causes an image to be displayed on displayer 140 to dynamically change from the first image to the second image. Accordingly, the user can readily confirm an effect of a non-visualization variable that is a variable not used to designate a position on image map Mb and material development by the user can be appropriately supported.

When best is selected with respect to a calcination temperature and a calcination time in the setting information of a non-visualization variable, for example, evaluation value obtainer 632 obtains an evaluated property value with respect to all combinations of respective pieces of option data of variables Pb and Pc. In addition, evaluation value obtainer 632 specifies a calcination temperature and a calcination time corresponding to a maximum evaluated property value. In this case, the user can readily confirm whether or not there is a possibility of improvement of an evaluated property value due to the change in a non-visualization variable.

In addition, when mean is selected with respect to a calcination temperature and a calcination time in the setting information of a non-visualization variable, for example, evaluation value obtainer 632 obtains an evaluated property value that corresponds to a mean of a plurality of pieces of option data of the calcination temperature and a mean of a plurality of pieces of option data of the calcination time. In this case, it becomes easy to argue about a composition of a compound for which an evaluated property value is robust with respect to a change in a non-visualization variable or, in other words, a composition of a compound for which an improvement in stability of material properties during mass production is anticipated.

As described above, in property display device 630 according to the present embodiment, display method obtainer 633 changes a plurality of pieces of option data that can be assumed by each of at least one variable among the plurality of variables described above which are indicated by search range information d14.

Accordingly, when option data is changed, since image map Mb in accordance with contents of the change is displayed by being included in the second image, an evaluated property value of each of a plurality of compounds can be displayed from points of view of various pieces of option data. As a result, material search can be made more efficient.

Note that numerical range data indicating a maximum value and a minimum value of a non-visualization variable may be saved in advance in a database included in display system 600. For example, numerical range may be included in advance in search range information d14 that is saved in evaluation display database 640. Alternatively, when the numerical range data is saved in an external apparatus being a synthesizer that synthesizes materials or a measuring device that measures materials, property display device 630 may obtain the numerical range data from the external apparatus. For example, property display device 630 obtains numerical range data from an external apparatus by being connected to the external apparatus and by communicating with the external apparatus. Accordingly, for example, when the user selects minimum with respect to a non-visualization variable that is a calcination temperature or a calcination time illustrated in FIGS. 58A and 58B, property display device 630 can automatically substitute a minimum value indicated in numerical range data into the non-visualization variable or automatically set the minimum value to the non-visualization variable. In a similar manner, when the user selects maximum with respect to a non-visualization variable that is a calcination temperature or a calcination time, property display device 630 can automatically substitute a maximum value indicated in numerical range data into the non-visualization variable or automatically set the maximum value to the non-visualization variable. Accordingly, the maximum value or the minimum value can be automatically presented to the user by being dependent on an external apparatus or the like.

[Specific example 3 of change of images]

FIGS. 59A and 59B are diagrams illustrating an example of a transition of images accompanying a change in first display target information d15. Specifically, FIG. 59A illustrates first display target information d15 included in first information d10 and an example of the first image displayed on displayer 140 according to first display target information d15. FIG. 59B is a diagram illustrating first display target information d15 after a change and an example of the second image displayed on displayer 140 according to changed first display target information d15.

For example, display method obtainer 633 obtains first display target information d15 illustrated in (b) in FIG. 59A from evaluation display database 640. First display target information d15 respectively indicates property 1, property 2, a crystal phase, and superimposition as options. For example, property 1 is a band gap and property 2 is a heat-resistance temperature. In this case, in first display target information d15, property 1 has been selected.

Evaluation value obtainer 632 obtains such first display target information d15 from display method obtainer 633. In addition, evaluation value obtainer 632 obtains an evaluated property value in accordance with first display target information d15. In other words, evaluation value obtainer 632 obtains an evaluated property value with respect to property 1 of each of a plurality of compounds. Image processor 634 generates image map Mb indicating an evaluated property value with respect to property 1 obtained in this manner and superimposes one or more experiment points on image map Mp.

For example, as illustrated in (a) in FIG. 59A, image processor 634 displays the first image including image map Mb on which one or more experiment points have been superimposed on displayer 140. In the first image, an evaluated property value of image element map Ma3 corresponding to variables (a, b) = (0.0, 0.0) included in image map Mb is larger than other image element maps Ma.

At this point, the user changes first display target information d15 in first information d10 by performing an input operation with respect to input unit 110. In other words, when display method obtainer 633 obtains an input signal indicating a change of first display target information d15 from input unit 110, display method obtainer 633 changes first display target information d15 as illustrated in (b) in FIG. 59B according to the input signal.

In the example illustrated in (b) in FIG. 59B, in first display target information d15, not only property 1 but superimposition is also selected and, furthermore, property 2 is selected as a property regarding superimposition. Furthermore, enabling of a threshold is selected with respect to property 2 and 200 or more is set as the threshold.

Evaluation value obtainer 632 obtains first display target information d15 changed as described above from display method obtainer 633. In addition, evaluation value obtainer 632 obtains an evaluated property value in accordance with first display target information d15. In other words, since an evaluated property value with respect to property 1 of each of a plurality of compounds is already obtained as described above, evaluation value obtainer 632 obtains an evaluated property value with respect to property 2 of each of the plurality of compounds. As illustrated in (a) in FIG. 59B, image processor 634 generates image map Mb indicating an evaluated property value with respect to property 1 and an evaluated property value with respect to property 2 obtained with respect to each of the plurality of compounds.

At this point, since superimposition is selected and property 2 is selected as a property regarding the superimposition in first display target information d15, image processor 634 superimposes an evaluated property value of property 2 with respect to image map Mb illustrated in (a) in FIG. 59A. More specifically, enabling of a threshold is selected with respect to property 2 and 200 or more is set as the threshold in first display target information d15. Therefore, image processor 634 applies hatching to a region in which an evaluated property value of property 2 is less than 200 in image map Mb described above. For example, the region is covered by striped hatching.

In addition, image processor 634 generates the second image including image map Mb on which one or more experiment points have been superimposed and displays the second image on displayer 140. Accordingly, the first image is changed to the second image.

In the second image illustrated in (a) in FIG. 59B, among image map Mb, image element map Ma in which an evaluated property value of property 2 is 200 or more and an evaluated property value of property 1 is maximum is image element map Ma4 corresponding to variables (a, b) = (0.1, 0.0).

Accordingly, the user can readily confirm a region that has a large evaluated property value of property 1 and an evaluated property value of property 2 that is equal to or larger than a threshold on image map Mb. Note that instead of superimposing a plurality of evaluated property values, property display device 630 may calculate a new evaluated property value by weighting and adding up the plurality of evaluated property values. For example, evaluation value obtainer 632 obtains weight parameter p ($0 < p < 1$) from input unit 110 via display method obtainer 633 and calculates a new evaluated property value according to evaluated property value = (1 - p) × (evaluated property value of property 1) + p × (evaluated property value of property 2). Such a new evaluated property value is defined by an input operation with respect to input unit 110 by the user. In this case, since evaluated property values to be displayed are narrowed down to one type, visual understanding is facilitated.

In this manner, a change in first display target information d15 causes an image to be displayed on displayer 140 to dynamically change from the first image to the second image. Accordingly, material development that satisfies a plurality of required properties can be readily performed and material development by the user can be appropriately supported.

In other words, in property display device 630 according to the present embodiment, first information d10 includes first display target information d15 indicating one or more property types. With respect to each of a plurality of compounds, evaluation value obtainer 632 obtains an evaluated property value with respect to each of one or more property types indicated by first display target information d15. In display method obtainer 633, the one or more property types indicated by first display target information d15 is changed.

Accordingly, when the property type indicated by first display target information d15 is changed, since image map Mb in accordance with contents of the change is displayed by being included in the second image, an evaluated property value of each of a plurality of compounds can be displayed from points of view of various properties. As a result, material search can be made more efficient. For example, as described above, the user can readily perform material development that satisfies a plurality of required properties. As a result, material development by the user can be appropriately supported.

[Specific example 4 of change of images]

FIGS. 60A and 60B are diagrams illustrating an example of a transition of images accompanying a change in display range information d16. Specifically, FIG. 60A illustrates display range information d16 included in first information d10 and an example of the first image displayed on displayer 140 according to display range information d16. FIG. 60B illustrates display range information d16 after a change and an example of the second image displayed on displayer 140 according to changed display range information d16.

For example, display method obtainer 633 obtains display range information d16 illustrated in (b) in FIG. 60A from evaluation display database 640. Display range information d16 indicates a display range being a range of evaluated property values that can be represented by a shade of color by an upper limit and a lower limit of the display range. Therefore, an evaluated property value in the display range is assigned a color of a shade in accordance with the value. On the other hand, an evaluated property value equal to or above the display range is assigned a color of a maximum shade within a scale range and an evaluated property value equal to or below the display range is assigned a color of a minimum shade within the scale range. In other words, a color of a maximum or minimum shade does not enable an evaluated property value corresponding to the shade to be uniquely specified and only enabled whether the evaluated property value is equal to or above the display range or equal to or below the display range to be specified. Note that a scale range is a range of a shade of color determined in advance.

Display range information d16 respectively indicates a data maximum value and a user-defined option as options with respect to an upper limit and respectively indicates a data minimum value and a user-defined option as options with respect to a lower limit. A data maximum value is, for example, a maximum evaluated property value on image map Mb, and a data minimum value is, for example, a minimum evaluated property value on image map Mb. A user-defined option is an evaluated property value defined by the user and is designated by, for example, an input operation with respect to input unit 110. In this case, in display range information d16 illustrated in (b) in FIG. 60A, a data maximum value is selected with respect to the upper limit and a data minimum value is selected with respect to the lower limit.

Image processor 634 obtains such display range information d16 from display method obtainer 633. Next, in accordance with the upper limit and the lower limit indicated in display range information d16, image processor 634 assigns a shade of color with respect to an evaluated property value of each of a plurality of compounds obtained from evaluation value obtainer 632. For example, the data maximum value selected with respect to the upper limit is "4.0" and the data minimum value selected with respect to the lower limit is "0.5". In this case, image processor 634 assigns a maximum shade within a scale range to the data maximum value "4.0" and assigns a minimum shade within the scale range to the data minimum value "0.5". Furthermore, with respect to evaluated property values larger than 0.5 and smaller than 4.0, the larger an evaluated property value, the higher a shade within the scale range that is uniformly assigned by image processor 634. In addition, as illustrated in (a) in FIG. 60A, image processor 634 generates image map Mb indicating the evaluated property values by a color of a shade assigned to the evaluated property values and superimposes one or more experiment points on image map Mb. As illustrated in (a) in FIG. 60A, image processor 634 displays the first image including image map Mb on which the one or more experiment points have been superimposed on displayer 140.

In this case, from image map Mb, the user can comprehend an overall trend of evaluated property values on image map Mb. On the other hand, in partial region R1 in image map Mb, the user can only comprehend that evaluated property values are large and it is difficult for the user to comprehend an evaluated property value of each compound or a distribution of the evaluated property values in region R1. Note that region R1 refers to four image element maps Ma corresponding to variables (a, b) = (0.0, 0.0), (0.0, 0.1), (0.005, 0.0), and (0.005, 0.1).

In consideration thereof, the user changes display range information d16 in first information d10 by performing an input operation with respect to input unit 110. When display method obtainer 633 obtains an input signal indicating a change of display range information d16 from input unit 110, display method obtainer 633 changes display range information d16 as illustrated in (b) in FIG. 60B according to the input signal.

In the example illustrated in (b) in FIG. 60B, a user-defined option "4.00" is selected with respect to the upper limit and a user-defined option "3.65" is selected with respect to the lower limit in display range information d16.

Image processor 634 obtains display range information d16 changed in this manner from display method obtainer 633. Next, in accordance with the upper limit "4.00" and the lower limit "3.65" indicated in display range information d16, image processor 634 assigns a shade of color with respect to an evaluated property value of each of a plurality of compounds obtained from evaluation value obtainer 632. In addition, as illustrated in (a) in FIG. 60B, image processor 634 generates image map Mb indicating the evaluated property values by a color of a shade assigned to the evaluated property values. Image processor 634 generates the second image including image map Mb on which one or more experiment points have been superimposed and displays the second image on displayer 140. Accordingly, the first image is changed to the second image.

In this case, as illustrated in (a) in FIG. 60B, the user can readily comprehend an evaluated property value of each compound or a distribution of the evaluated property values in region R1 of image map Mb. In other words, the user can readily confirm differences among evaluated property values in region R1. On the other hand, the user can comprehend that many evaluated property values equal to or below the lower limit "3.65" of the display range are indicated in regions other than region R1 of image map Mb.

In this manner, a change in display range information d16 causes an image to be displayed on displayer 140 to dynamically change from the first image to the second image. Accordingly, even when a range of evaluated property values indicated on Image map Mb is wide, since a display range among the range is set, the user can readily understand differences among evaluated property values in the display range. As a result, material development by the user can be appropriately supported.

In addition, for example, when using a variance of predicted values obtained from a trained machine learning model that is an example of an evaluater, uncertainty of prediction declines or, in other words, the variance of predicted values decreases as material search progresses. Note that in the progress of material search, experimental property values obtained by experiment are repetitively used in learning of a machine learning model. As a result, a range of evaluated property values indicated on image map Mb changes. In consideration thereof, in the examples illustrated in FIGS. 60A and 60B, it is also expected that setting or designating an appropriate display range in accordance with the progress of material search makes it easier to visually understand evaluated property values. While a shade in accordance with an evaluated property value is assigned to the evaluated property value from a range of shades determined in advance in the example described above, alternatively, a color in accordance with the evaluated property value may be assigned to the evaluated property value from a range of colors determined in advance.

As described above, in property display device 630 according to the present embodiment, first information d10 includes display range information d16 indicating a display range of evaluated property values. Image processor 634 associates a display range of evaluated property values indicated by display range information d16 with a scale range of colors or shades of color determined in advance and generates image map Mb indicating an evaluated property value of each of a plurality of compounds in a color or a shade of color in accordance with the evaluated property value in the scale range. In display method obtainer 633, the display range indicated by display range information d16 is changed.

Accordingly, when the display range indicated by display range information d16 is changed, since image map Mb in accordance with contents of the change is displayed by being included in the second image, an evaluated property value of each of a plurality of compounds can be displayed from points of view of various display ranges. As a result, material search can be made more efficient. For example, even in a case where a range of evaluated property values indicated on image map Mb changes with the progress of material search, changing a display range enables a plurality of evaluated property values included in the display range to be indicated in colors or shades of color that clearly differ from one another.

[Specific example 5 of change of images]

FIGS. 61A and 61B are diagrams illustrating an example of a transition of images accompanying a change in search range information d14. Specifically, FIG. 61A illustrates search range information d14 included in first information d10 and an example of the first image displayed on displayer 140 according to search range information d14. FIG. 61B illustrates search range information d14 after a change and an example of the second image displayed on displayer 140 according to changed search range information d14.

For example, display method obtainer 633 obtains search range information d14 illustrated in (b) in FIG. 61A from evaluation display database 640. Search range information d14 is information that indicates a plurality of pieces of option data with respect to each range variable as a search range in a similar manner to, for example, the search ranges illustrated in FIGS. 2 and 12.

Evaluation value obtainer 632 obtains such search range information d14 from display method obtainer 633. In addition, evaluation value obtainer 632 obtains an evaluated property value in accordance with search range information d14. In other words, evaluation value obtainer 632 obtains an evaluated property value with respect to each of a plurality of compounds that has a composition included in the search range. As illustrated in (a) in FIG. 61A, image processor 634 generates image map Mb indicating the obtained evaluated property values. Furthermore, image processor 634 superimposes one or more experiment points on image map Mb and displays the first image including image map Mb on which the one or more experiment points have been superimposed on displayer 140.

At this point, the user confirms region R2 of variable b = 0.0 on image map Mb. Region R2 includes five image element maps Ma corresponding to variables (a, b) = (0.0, 0.0), (0.05, 0.0), (0.1, 0.0), (0.15, 0.0), and (0.2, 0.0). The user comprehends that, in region R2, when variable a is 0.05 or larger, an evaluated property value decrease as a value of variable a increases, and when variable a indicates a numerical value between 0.0 and 0.05, an evaluated property value may possibly become locally maximized.

In consideration thereof, the user changes search range information d14 in first information d10 by performing an input operation with respect to input unit 110. When display method obtainer 633 obtains an input signal indicating a change of search range information d14 from input unit 110, display method obtainer 633 changes search range information d14 as illustrated in (b) in FIG. 61B according to the input signal. In the example illustrated in (b) in FIG. 61B, 0.025 has been added as option data of variable a.

Evaluation value obtainer 632 obtains search range information d14 having been changed in this manner from display method obtainer 633. In addition, evaluation value obtainer 632 obtains an evaluated property value in accordance with search range information d14. In other words, evaluation value obtainer 632 further obtains an evaluated property value of each of a plurality of compounds corresponding to the added option data "0.025" of variable a. Image processor 634 generates image map Mb indicating the obtained evaluated property values. As illustrated in (a) in FIG. 61B, in image map Mb, region R3 including four image element maps Ma has been added to image map Mb illustrated in (a) in FIG. 61A. Note that four image element maps Ma included in region R3 corresponds to variables (a, b) = (0.025, 0.0), (0.025, 0.1), (0.025, 0.2), and (0.025, 0.3). In addition, image processor 634 displays the second image including image map Mb on which one or more experiment points have been superimposed on displayer 140.

The user confirms region R3 of variable a = 0.025 on image map Mb. In addition, for example, the user can confirm that an evaluated property value is locally maximized in image element map Ma corresponding to variables (a, b) = (0.025, 0.0) in region R3 among six image element maps Ma corresponding to variable b = 0.0 included in image map Mb. While the number of image element maps Ma in image map Mb increases in accordance with an addition of option data in the example described above, conversely, the number of image element maps Ma in image map Mb may decrease in accordance with a deletion of option data.

In this manner, a change in search range information d14 causes an image to be displayed on displayer 140 to dynamically change from the first image to the second image. Accordingly, the user can re-examine a search range in accordance with a status of material search and material development by the user can be appropriately supported.

In addition, in property display device 630 according to the present embodiment, image map Mb includes a plurality of image element maps Ma that are arrayed in a matrix along each of a first coordinate axis and a second coordinate axis, and each of the plurality of image element maps Ma has a third coordinate axis and a fourth coordinate axis. Image processor 634 respectively associates the first coordinate axis, the second coordinate axis, the third coordinate axis, and the fourth coordinate axis with a first variable, a second variable, a third variable, and a fourth variable among the plurality of variables described earlier. Furthermore, with respect to each of a plurality of compounds, image processor 634: (a) specifies image element map Ma associated with respective values of the first variable and the second variable that are to be used in representing a composition of the compound among the plurality of image element maps Ma, and (b) maps, at a position indicated by respective values of the third variable and the fourth variable that are to be used in representing a composition of the compound on specified image element map Ma, an evaluated property value of the compound. For example, the first variable, the second variable, the third variable, and the fourth variable are variables a, b, x, and y described earlier.

Accordingly, a map such as image map Mb can represent evaluated property values with respect to compositions of a plurality of compounds respectively represented by four variables and evaluated property values of compounds can be displayed in an easily understood manner over a wide range. As a result, material search can be made more efficient.

In addition, in property display device 630 according to the present embodiment, in display method obtainer 633, when the number of a plurality of pieces of option data indicated by search range information d14 is increased or reduced, image processor 634 increases or reduces the number of image element maps Ma included in image map Mb. Specifically, image processor 634 increases or reduces the number of image element maps Ma included in image map Mb in accordance with changed search range information d14.

Accordingly, when the number of pieces of option data indicated by search range information d14 is changed, since image element maps Ma corresponding to the number in accordance with contents of the change is displayed by being included in the second image, an evaluated property value of each of a plurality of compounds can be displayed from points of view of various image element maps. As a result, material search can be made more efficient.

[Specific example 6 of change of images]

FIG. 62 is a diagram illustrating an example of a transition of images accompanying a change in map array information d13. Specifically, (a) in FIG. 62 illustrates an example of the first image displayed on displayer 140 according to map array information d13 included in first information d10. In FIG. 62, (b) illustrates an example of the second image displayed on displayer 140 according to map array information d13 after the change.

For example, display method obtainer 633 obtains map array information d13 from evaluation display database 640. Map array information d13 indicates arranging 20 image element maps Ma in four rows and five columns. Image processor 634 obtains such map array information d13 from display method obtainer 633. Next, according to map array information d13, image processor 634 generates image map Mb including 20 image element maps Ma arranged in four rows and five columns and superimposes one or more experiment points on image map Mb. As illustrated in (a) in FIG. 62, image processor 634 displays the first image including image map Mb on which the one or more experiment points have been superimposed on displayer 140.

At this point, the user selects one or more image element maps Ma included in image map Mb by performing an input operation with respect to input unit 110. For example, the user selects one or more image element maps Ma on which a large evaluated property value is indicated as one or more image element maps Ma of interest. Such a selection of image element maps Ma is performed by, for example, an input operation such as a click or a drag. For example, the user performs a click with respect to image element maps Ma corresponding to each of (a, b) = (0.0, 0.1) and (0.05, 0.1) and designates region R4 on image map Mb by a drag. Region R4 includes four image element maps Ma corresponding to variables (a, b) = (0.0, 0.0), (0.05, 0.0), (0.1, 0.0), and (0.15, 0.0).

Input unit 110 outputs an input signal in accordance with the input operation to display method obtainer 633. Upon obtaining the input signal, display method obtainer 633 changes map array information d13 in accordance with the input signal and outputs changed map array information d13 to image processor 634. Changed map array information d13 may indicate a position on image map Mb of each of, for example, six image element maps Ma selected by the user. Note that positions are designated by variables a and b.

According to map array information d13, image processor 634 generates image map Mb including six image element maps Ma selected by the user. In addition, as illustrated in (b) in FIG. 62, image processor 634 displays the second image including image map Mb on which one or more experiment points have been superimposed on displayer 140.

In this manner, a change in map array information d13 causes an image to be displayed on displayer 140 to dynamically change from the first image to the second image. Accordingly, a plurality of image element maps Ma can be readily compared with one another and material development by the user can be appropriately supported.

Note that display method obtainer 633 may change an array of a plurality of image element maps Ma indicated by map array information d13 in accordance with the input signal. In other words, display method obtainer 633 may change map array information d13 so that the plurality of image element maps Ma are rearranged. In this case, the plurality of image element maps can be more readily compared with one another. Furthermore, when displaying the second image on displayer 140, image processor 634 may change a size of selected image element maps Ma in accordance with the number of selected image element maps Ma. For example, when the number of selected image element maps Ma is small, image processor 634 enlarges image element maps Ma. In this case, an improvement in visibility of the user is anticipated.

In addition, in property display device 630 according to the present embodiment, first information d10 includes map array information d13 indicating an array mode of image element maps Ma to be displayed. In display method obtainer 633, when map array information d13 is changed due to selection of a position of image element map Ma to be displayed, image processor 634 changes image map Mb that has already been generated to image map Mb including at least one image element map Ma arranged in accordance with the array mode indicated by changed map array information d13.

Accordingly, when the array mode of image element maps Ma indicated by map array information d13 is changed, image element maps Ma in the array mode in accordance with contents of the change is displayed by being included in the second image. Therefore, an evaluated property value of each of a plurality of compounds can be displayed from points of view of various array modes of image element maps Ma. As a result, a plurality of image element maps Ma can be readily compared with one another and material search can be made more efficient.

[Processing operations]

FIG. 63 is a flowchart illustrating processing operations of display system 600 according to the present embodiment.

(Step S211)

Display method obtainer 633 obtains first information d10 regarding a display method of an evaluated property value from evaluation display database 640 and outputs first information d10 to evaluation value obtainer 632, experiment point obtainer 635, and image processor 634.

(Step S212)

Experiment point obtainer 635 obtains first information d10 from display method obtainer 633. In addition, experiment point obtainer 635 obtains an experiment point of each of one or more compounds which have been experimented on and which corresponds to a search range indicated by search range information d14 in first information d10 from experiment database 650. Experiment point obtainer 635 outputs the experiment point of each of the one or more compounds which have been experimented on to image processor 634.

(Step S213)

Evaluation value obtainer 632 obtains first information d10 from display method obtainer 633. In addition, evaluation value obtainer 632 obtains an evaluated property value of each of a plurality of compounds in accordance with calculation method information d12, search range information d14, and the like in first information d10. At this point, evaluation value obtainer 632 obtains an evaluated property value of each of a plurality of compounds using one or more evaluaters in evaluater database 620. Evaluation value obtainer 632 outputs a composition and an evaluated property value corresponding to the composition of each of the plurality of compounds to image processor 634.

Note that in the present embodiment, evaluation value obtainer 632 obtains an evaluater from evaluater database 620 and obtains evaluated property values using the evaluater. However, evaluation value obtainer 632 may obtain an evaluated property value of each of a plurality of compounds from at least one evaluater for evaluating a property value of a compound, the at least one evaluater being stored in evaluater database 620. Even in this case, an appropriate evaluated property value can be obtained with respect to each of the plurality of compounds.

(Step S214)

Image processor 634 generates the first image including image map Mb. At this point, image processor 634 generates image map Mb using first information d10 obtained in step S211 and a composition and an evaluated property value of each of the plurality of compounds obtained in step S213. Furthermore, image processor 634 superimposes an experiment point of each of one or more compounds which have been experimented on and obtained in step S212 on image map Mb. In addition, image processor 634 generates the first image including image map Mb on which one or more experiment points have been superimposed and outputs the first image to displayer 140.

(Step S215)

Displayer 140 obtains the first image from image processor 634 and displays the first image.

(Step S216)

The user performs an input operation with respect to input unit 110. In accordance with the input operation by the user, input unit 110 outputs an input signal indicating a change of display of the first image to display method obtainer 633 of property display device 630. By obtaining the input signal, display method obtainer 633 receives the change of display of the first image.

(Step S217)

In accordance with the input signal obtained in step S216, display method obtainer 633 changes first information d10 obtained in step S211. In addition, display method obtainer 633 outputs changed first information d10 to evaluation value obtainer 632, experiment point obtainer 635, and image processor 634.

(Step S218)

Upon receiving changed first information d10 from display method obtainer 633, evaluation value obtainer 632 and experiment point obtainer 635 once again obtains an evaluated property value and an experiment point in accordance with changed contents of first information d10. In addition, image processor 634 generates the second image in accordance with changed first information d10. For example, the second image indicates the evaluated property value obtained once again and includes image map Mb on which the experiment point obtained once again has been superimposed. Image processor 634 outputs the second image to displayer 140.

(Step S219)

Displayer 140 obtains the second image from image processor 634 and displays the second image.

(Variation of Embodiment 3A)

In the embodiment described above, in order to receive a change to first information d10, all pieces of information such as calculation method information d12 included in first information d10 are displayed on displayer 140. Specifically, an entirety of calculation method information d12 is displayed as illustrated in FIGS. 57A and 57B and an entirety of search range information d14 is displayed as illustrated in FIGS. 58A and 58B. When many setting items or many non-visualization variables are included in such information, the user may find it difficult to select a best option among the setting items or the non-visualization variables. In other words, it is difficult for the user to readily find a setting item or a non-visualization variable having a plurality of options when a change in options to be selected causes a significant change in a map.

In consideration thereof, image processor 634 according to the present variation calculates a priority with respect to each setting item or each non-visualization variable. The priority indicates a degree by which a map changes due to a change in an option to be selected from a plurality of options included in a setting item or a non-visualization variable. In addition, the higher the priority of a setting item or a non-visualization variable, the more preferentially image processor 634 displays the setting item or the non-visualization variable on displayer 140.

FIG. 64 is a diagram illustrating an example of the first image according to the present variation.

When generating the first image including image map Mb based on calculation method information d12, image processor 634 includes calculation method image d121 indicating a part of calculation method information d12 in first image as illustrated in, for example, FIG. 64. In addition, image processor 634 displays the first image on displayer 140. Calculation method image d121 only represents a setting item of Bayesian optimization among three setting items indicated by calculation method information d12, namely, a setting item of a machine learning model, a setting item of Bayesian optimization, and a setting item of display. In other words, image processor 634 preferentially displays the setting item of Bayesian optimization over the setting item of a machine learning model and the setting item of display. More specifically, image processor 634 preferentially displays a plurality of options included in the setting item of Bayesian optimization over the pluralities of options respectively included in the setting item of a machine learning model and the setting item of display.

When such the first image illustrated in FIG. 64 is displayed on displayer 140, image processor 634 first calculates a priority of each of a plurality of setting items. In the example illustrated in FIG. 64, the plurality of setting items are the setting item of a machine learning model, the setting item of Bayesian optimization, and the setting item of display. For example, image processor 634 selects a setting item from the plurality of setting items as a processing object item. Next, image processor 634 repetitively executes selection processing of selecting a combination of one or more options from a plurality of options included in the processing object item. In each step of selection processing, mutually different combinations are selected. In addition, every time selection processing is performed, image processor 634 generates a map (for example, image map Mb) based on a combination of the one or more options selected by the selection processing. Next, based on the plurality of generated image maps Mb, for each position on image maps Mb, image processor 634 calculates a variance value of an evaluated property value indicated at the position. Furthermore, image processor 634 calculates a mean of variance values at each position on image map Mb as a priority of the processing object item described above. By sequentially re-selecting each of a plurality of setting items as a processing object item, image processor 634 calculates a priority of each of the plurality of setting items.

While a priority is calculated as a mean of variance values in the example described above, a priority is not limited thereto. For example, when calculating a priority of a processing object item, based on the plurality of image maps Mb generated in a similar manner to that described above, for each position on image maps Mb, image processor 634 calculates a mean of evaluated property values indicated at the position as an evaluated mean. In addition, image processor 634 calculates a sum of absolute values of differences between an evaluated property value indicated at the position and an evaluated mean calculated with respect to the position of each of plurality of image maps Mb as a difference absolute value sum. Furthermore, image processor 634 calculates a sum of difference absolute value sums at respective positions on image map Mb as a priority of a processing object item.

Accordingly, a setting item in which a change to an option to be selected causes a map to change significantly or, in other words, a setting item for which an evaluated property value changes significantly and a plurality of options included in the setting item are preferentially displayed. Therefore, the user can readily find a setting item that is effective for material development. In other words, the user can readily visually comprehend that a map is to change significantly due to a change in an option to be selected and efficiency of material development can be improved.

FIG. 65 is a diagram illustrating another example of the first image according to the present variation.

When generating the first image including image map Mb based on search range information d14, image processor 634 includes search range image d141 indicating a part of search range information d14 in the first image as illustrated in, for example, FIG. 65. In addition, image processor 634 displays the first image on displayer 140. Search range image d141 represents only variable Pc that indicates a calcination temperature among two non-visualization variables indicated by search range information d14 or, in other words, variable Pb that indicates a calcination time and variable Pc that indicates a calcination temperature. In other words, image processor 634 preferentially displays variable Pc that indicates a calcination temperature over variable Pb that indicates a calcination time. More specifically, image processor 634 preferentially displays a plurality of pieces of option data that may be assumed by variable Pc that indicates a calcination temperature over a plurality of pieces of option data that may be assumed by variable Pb that indicates a calcination time.

When such the first image illustrated in FIG. 65 is displayed on displayer 140, image processor 634 first calculates a priority of each of a plurality of non-visualization variables in a similar manner to the example illustrated in FIG. 64. Means of variance values or a sum of difference absolute value sums described earlier may be used to calculate the priorities. In addition, image processor 634 may calculate a priority of each of a plurality of non-visualization variables based on one or more experiment points superimposed on image map Mb.

Specifically, image processor 634 selects one non-visualization variable as a processing object variable from a plurality of non-visualization variables. Next, image processor 634 repetitively executes selection processing of selecting one piece of option data from a plurality of pieces of option data that may be assumed by the processing object variable. Note that the plurality of pieces of option data is at least two pieces of option data among minimum, maximum, best, mean, a user-defined option, and the like indicated in, for example, FIGS. 58A, 58B, and 65. In addition, in each execution of the selection processing, mutually different pieces of option data are selected. Furthermore, every time selection processing is performed, image processor 634 specifies one or more experiment points to be superimposed on a map (for example, image map Mb) based on the option data selected by the selection processing and experimental data stored in experiment database 650.

Next, image processor 634 calculates a variance value of values of the processing object variable associated with all experiment points specified by the selection processing having been repetitively executed as a first variance value. For example, when the processing object variable is variable Pc indicating a calcination temperature, in compound basic data 651 illustrated in FIG. 52, a variance value of a calcination temperature associated with each specified experiment point (for example, a composition formula of the experiment point) is calculated as the first variance value.

Furthermore, image processor 634 calculates a variance value of all values of the processing object variable indicated in experimental data in experiment database 650 as a second variance value. For example, when the processing object variable is variable Pc indicating a calcination temperature, a variance value of all calcination temperatures indicated in compound basic data 651 illustrated in FIG. 52 is calculated as the second variance value.

In addition, image processor 634 calculates a ratio of the first variance value to the second variance value as a priority of the processing object variable described above. By sequentially re-selecting each of a plurality of non-visualization variables as a processing object variable, image processor 634 calculates a priority of each of the plurality of non-visualization variables.

Accordingly, a non-visualization variable in which a change to option data to be selected causes a map to change significantly or, in other words, a non-visualization variable for which an evaluated property value changes significantly and a plurality of pieces of option data that may be assumed by the non-visualization variable are preferentially displayed. Alternatively, a non-visualization variable for which a value tends to vary significantly with respect to one or more experiment points to be superimposed on a map and a plurality of pieces of option data that the non-visualization variable may assume are preferentially displayed. Therefore, the user can readily find a non-visualization variable that is effective for material development. In addition, the user can compare respective experiment points with each other in an efficient manner. As a result, material development can be made more efficient.

While image map Mb and an image of first information d10 such as calculation method image d121 or search range image d141 are displayed in a state of being included in the first image in the example illustrated in FIGS. 64 and 65, image map Mb and the image of first information d10 may not be displayed at the same time. In other words, image map Mb and the image of first information d10 may be displayed on displayer 140 at mutually different timings.

FIG. 66 is a flowchart illustrating an detailed example of generation of the first image by image processor 634 according to the present variation. Note that FIG. 66 illustrates a detailed processing operation of step S214 in FIG. 63.

(Step S214a)

First, image processor 634 divides first information d10 into a plurality of groups. Specifically, image processor 634 divides calculation method information d12, search range information d14, and the like included in first information d10 into a plurality of groups. For example, calculation method information d12 is divided into groups corresponding to each of a plurality of setting items included in calculation method information d12. In other words, one group includes a setting item and a plurality of options included in the setting item. In addition, search range information d14 is divided into groups corresponding to each of a plurality of non-visualization variables included in search range information d14. In other words, one group includes a non-visualization variable and a plurality of pieces of option data that may be assumed by the non-visualization variable.

(Step S214b)

Next, image processor 634 calculates a priority of each of the plurality of groups. As described above, the priority is calculated based on a mean of variance values, a sum of difference absolute value sums, a variance value based on experiment points, or the like.

(Step S214c)

Next, image processor 634 preferentially displays a group with a high priority on displayer 140 by including the group in the first image. For example, as illustrated in FIG. 64 or 65, image processor 634 displays only a group with a highest priority among a plurality of groups by including the group in the first image. The first image includes a map such as image map Mb.

While only one group is displayed in the example described above, image processor 634 may array a plurality of groups in an order of priorities and display the groups on displayer 140. In other words, image processor 634 arrays the plurality of groups such that the higher a priority of a group, the more forward or upward the group is displayed. In addition, image processor 634 may only display, on displayer 140, groups corresponding to a specified number determined in advance with higher priorities among the plurality of groups. Note that the specified number is an integer equal to or greater than 2 and a priority of groups corresponding to the specified number is higher than priorities of other groups. Even in such a case, groups whose contribution towards supporting material development is relatively low can be prevented from being displayed. As a result, a similar effect to that off the examples illustrated in FIGS. 64 and 65 can be produced.

In addition, image processor 634 may determine whether or not the number of groups included in information is equal to or larger than a threshold and, when the number of groups is equal to or larger than a threshold, image processor 634 may divide the information into a plurality of groups according to a flowchart illustrated in FIG. 66. Accordingly, when the number of groups is small, an entire piece of information is to be displayed on displayer 140 and the user can change first information d10 without hesitation. Furthermore, even when the number of groups is large, since a group with high priority is to be preferentially displayed on displayer 140, the user can change first information d10 without hesitation.

In addition, in the flowcharts illustrated in FIGS. 63 and 66, an evaluated property value is obtained in step S213 based on default first information d10 obtained in step S211 before a priority of each group included in first information d10 is calculated. However, after the priority of each group is calculated, an evaluated property value based on the priority may be obtained. For example, in step S213, when calculation method information d12 among first information d10 obtained in step S211 is to be used, evaluation value obtainer 632 calculates a priority of each of a plurality of setting items included in calculation method information d12. Next, evaluation value obtainer 632 changes calculation method information d12 so that an option of a setting item with highest priority is selected. Furthermore, evaluation value obtainer 632 obtains an evaluated property value of each of a plurality of compounds in accordance with changed calculation method information d12. Even in such a case, in step S215, image processor 634 may display the first image illustrated in FIG. 64 on displayer 140.

As described above, in property display device 630 according to the present embodiment, display method obtainer 633 obtains first information d10 including a plurality of respectively-changeable information groups for displaying an evaluated property value as a comparative indicator for comparing a plurality of compounds. Evaluation value obtainer 632 and image processor 634 obtain image map Mb indicating an evaluated property value of each of a plurality of compounds at a position corresponding to a composition of the compound. In addition, image processor 634 outputs the first image including image map Mb that is generated based on a priority of each of the plurality of information groups. For example, the first image is outputted to and displayed by displayer 140. Note that information groups correspond to the groups illustrated in FIG. 66.

Accordingly, for example, an information group with a highest priority is displayed by being included in the first image and display of other information groups can be suppressed. As a result, the user can readily select and change the information group that is being displayed. In other words, by attaching a higher priority to an information group if the information group is more beneficial to displaying an evaluated property value, the user can readily comprehend a change in a map due to a change in the information group and an efficiency of material development can be improved.

In addition, in property display device 630 according to the present embodiment, with respect to each of the plurality of information groups, image processor 634 generates the first image using a degree of change in image map Mb due to a change in the information group as a priority of the information group. Accordingly, the user can readily confirm that image map Mb is to change significantly due to a change in an information group and efficiency of material development can be improved.

[EMBODIMENT 3B]

A display system according to the present embodiment displays an evaluated property value of each of a plurality of compounds in the form of a map in a similar manner to Embodiment 3A. Furthermore, the display system according to the present embodiment superimposes an experimental property value on the map in a similar manner to Embodiments 1 and 2 instead of an experiment point of each of one or more compounds that have been experimented on. In addition, the display system according to the present embodiment superimposes an experimental property value on the map according to second information to be described later.

Note that among the respective components according to the present embodiment, the same components as in Embodiments 1, 2, and 3A are given the same reference numerals as in Embodiments 1, 2, and 3A and detailed descriptions thereof are omitted. In addition, while image map Mb is described as an example of a map in the present embodiment, the map according to the present embodiment is not limited to image map Mb and may be image element map Ma or a set of a plurality of image maps Mb.

[Configuration of display system 700]

FIG. 67 is a block diagram illustrating an example of a configuration of display system 700 according to the present embodiment. Display system 700 illustrated in FIG. 67 includes input unit 110, evaluater database 620, property display device 730, displayer 140, evaluation display database 640, experiment database 650, and experiment display database (DB) 740. Note that property display device 730 is an example of an information display device.

Property display device 730 according to the present embodiment obtains an evaluated property value of each of a plurality of compounds using evaluater database 620 and evaluation display database 640 in a similar manner to property display device 630 according to Embodiment 3A. In addition, property display device 730 generates a map indicating the obtained evaluated property values. Furthermore, property display device 730 superimposes, on a map, an experimental property value of each of one or more compounds that have been experimented on using experiment database 650 and experiment display database 740. Property display device 730 includes evaluation value obtainer 632, display method obtainer 733, experimental value obtainer 732, and image processor 734. Note that property display device 630 may be constituted of, for example, a processor such as a CPU and a memory. In this case, the processor functions as property display device 730 by, for example, executing a computer program stored in the memory. Note that the memory may be volatile or non-volatile or may be constituted of a volatile memory and a non-volatile memory.

[Display method obtainer 733]

Display method obtainer 733 obtains an input signal from input unit 110 and, in accordance with the input signal, obtains first information d10 from evaluation display database 640 and further obtains second information d20 regarding a display method of experimental property values from experiment display database 740. In addition, display method obtainer 733 outputs first information d10 to evaluation value obtainer 632, experimental value obtainer 732, and image processor 734. Furthermore, display method obtainer 733 outputs second information d20 to experimental value obtainer 732 and image processor 734. Accordingly, an image including a map in accordance with first information d10 and second information d20 is displayed on displayer 140 as the first image.

Furthermore, when display method obtainer 733 obtains an input signal indicating a change to the display method of evaluated property values from input unit 110, display method obtainer 733 changes first information d10 in accordance with the input signal and outputs first information d10 after the change to evaluation value obtainer 632, experimental value obtainer 732, and image processor 734. In addition, when display method obtainer 733 obtains an input signal indicating a change to the display method of experimental property values from input unit 110, display method obtainer 733 changes second information d20 in accordance with the input signal and outputs second information d20 after the change to experimental value obtainer 732 and image processor 734. Accordingly, an image including a map in accordance with respective changed display methods of evaluated property values and experimental property values is displayed on displayer 140 as a third image. Note that the third image according to the present embodiment is an image generated according to a change in second information d20.

[Experimental value obtainer 732]

Experimental value obtainer 732 obtains first information d10 and second information d20 from display method obtainer 733. In addition, experimental value obtainer 732 obtains a composition and an experimental property value of each of one or more compounds which have been experimented on and which corresponds to search range information d14 of first information d10 and to second information d20 from experiment database 650 and outputs the composition and the experimental property value to image processor 734. Note that regardless of search range information d14, experimental value obtainer 732 may obtain a composition and an experimental property value of each of all compounds stored in experiment database 650.

[Image processor 734]

Image processor 734 obtains a composition and an evaluated property value of each of a plurality of compounds from evaluation value obtainer 632 and obtains a composition and an experimental property value of each of one or more compounds that have been experimented on from experimental value obtainer 732. Furthermore, image processor 734 obtains first information d10 and second information d20 from display method obtainer 733. Image processor 734 generates a map indicating an evaluated property value of each of a plurality of compounds according to display methods indicated by first information d10 and second information d20 and superimposes, on the map, the experimental property value of each of the one or more compounds that have been experimented on. Note that in generation of the map indicating evaluated property values, with respect to each of a plurality of compounds, image processor 734 adds a color of a shade in accordance with an evaluated property value of the compound to a position on the map that corresponds to a composition of the compound. In addition, in superimposition of experimental property values, with respect to each of one or more compounds that have been experimented on, image processor 734 superimposes a mark with a color of a shade in accordance with an experimental property value of the compound to a position on the map that corresponds to a composition of the compound. The color of a shade in accordance with the evaluated property value or the experimental property value may be a color in accordance with the evaluated property value or the experimental property value. Image processor 734 outputs an image including the map on which the one or more experimental property values have been superimposed to displayer 140 as the first image, the second image, or the third image.

[Experiment display database 740]

Experiment display database 740 is a recording medium storing information regarding a display method of an experimental property value. This recording medium is, for example, a hard disk drive, a RAM, a ROM, a semiconductor memory, or the like. Furthermore, the recording medium may be volatile or nonvolatile.

FIG. 68 is a diagram illustrating an example of information stored in experiment display database 740.

As illustrated in FIG. 68, experiment display database 740 stores second information d20 that is information regarding a display method of an experimental property value. Second information d20 includes second color information d21, second display target information d22, and display condition information d23.

Second color information d21 indicates at least one of a hue, a chroma, and a brightness of an experimental property value indicated on a map as a color attribute. Second display target information d22 indicates a property of a display target regarding an experimental property value. Display condition information d23 indicates a presence or absence of a condition regarding display of an experimental property value and, when the condition is present, contents of the condition. For example, the contents of the condition are that the experimental property value is equal to or larger than a threshold.

[Specific example 1 of change of images]

FIGS. 69A and 69B are diagrams illustrating an example of a transition of images accompanying a change in second display target information d22. Specifically, FIG. 69A illustrates second display target information d22 included in second information d20 and an example of the first image displayed on displayer 140 according to second display target information d22. FIG. 69B illustrates second display target information d22 after a change and an example of the third image displayed on displayer 140 according to changed second display target information d22. Note that in each specific example according to the present embodiment, the first image is changed to the third image by keeping first information d10 unchanged but changing the second information d20.

For example, display method obtainer 733 obtains second display target information d22 illustrated in (b) in FIG. 69A from experiment display database 740. Second display target information d22 indicates property 1, property 2, and a crystal phase as options with respect to each of a shape and a shade of color used in a mark for indicating an experimental property value. For example, property 1 is a band gap and property 2 is a heat-resistance temperature. In other words, in second display target information d22, property 1, property 2, and a crystal phase are respectively indicated as options as a property of an experimental property value that is displayed by a shape of a mark. Furthermore, in second display target information d22, property 1, property 2, and a crystal phase are respectively indicated as options as a property of an experimental property value that is displayed by a shade of color of a mark. In this case, in second display target information d22, property 1 has been selected with respect to each of a shape and a shade of color of a mark.

Experimental value obtainer 732 obtains such second display target information d22 from display method obtainer 733. In addition, for example, experimental value obtainer 732 retrieves a compound associated with an experimental property value of property 1 selected in second display target information d22 among compounds having a composition included in a search range indicated by search range information d14 in first information d10 from experimental data in experiment database 650. Experimental value obtainer 732 obtains a composition and an experimental property value of property 1 of each of the one or more retrieved compounds that have been experimented on from experiment database 650 and outputs the composition and the experimental property value to image processor 734.

Image processor 734 generates image map Mb indicating an evaluated property value with respect to property 1 obtained by evaluation value obtainer 632 in a similar manner to image processor 634 according to Embodiment 3A. Furthermore, image processor 734 obtains second display target information d22 from display method obtainer 733 and obtains a composition and an experimental property value of property 1 of each of one or more compounds that have been experimented on from experimental value obtainer 732. In addition, with respect to each of the one or more compounds that have been experimented on, image processor 734 superimposes a mark indicating the experimental property value of property 1 of the compound on a position on image map Mb that corresponds to a composition of the compound. At this point, image processor 734 superimposes the mark according to second display target information d22. In other words, image processor 734 superimposes a mark with a circular shape in accordance with property 1 and a color of a shade in accordance with the experimental property value of property 1 on image map Mb.

As a result, image processor 734 displays the first image illustrated in (a) in FIG. 69A on displayer 140. The first image includes image map Mb and a mark with a circular shape and a color of a shade in accordance with the experimental property value is superimposed on image map Mb. In other words, the experimental property value is superimposed on image map Mb as the mark. When an evaluated property value is represented by a same dimension or unit as an experimental property value, image processor 734 may make a relationship between experimental property values and shades and a relationship between evaluated property values and shades the same. In other words, image processor 734 may harmonize a scale of shade with respect to evaluated property values and a scale of shade with respect to experimental property values. Accordingly, when an evaluated property value and an experimental property value with respect to one compound significantly differ from each other, a shade of color at a position corresponding to the compound on image map Mb and a shade of color added to the mark of the experimental property value differs significantly. As a result, for example, as in the example illustrated in (a) in FIG. 69A, mark e1 of the experimental property value that significantly differs from the evaluated property value can be highlighted. In other words, it becomes easy to visually understand the deviation of the experimental property value from the evaluated property value. Specifically, when a shade of color of a mark of an experimental property value and a shade of color in an evaluated property value are visually the same, the user can visually understand that the experimental property value and the evaluated property value are substantially the same.

At this point, the user changes second display target information d22 in second information d20 by performing an input operation with respect to input unit 110. For example, when display method obtainer 733 obtains an input signal indicating a change of second display target information d22 from input unit 110, display method obtainer 733 changes second display target information d22 as illustrated in (b) in FIG. 69B according to the input signal.

In the example illustrated in (b) in FIG. 69B, a crystal phase is selected with respect to a shape of a mark and property 1 is selected with respect to a shade of color of the mark.

Experimental value obtainer 732 obtains second display target information d22 changed as described above from display method obtainer 733. In addition, for example, experimental value obtainer 732 retrieves a compound associated with an experimental property value of each of property 1 and a crystal phase selected in second display target information d22 among compounds having a composition included in the search range from experimental data in experiment database 650. Note that in the present embodiment, a type of a crystal phase is indicated as an experimental property value. With respect to each of the one or more retrieved compounds that have been experimented on, experimental value obtainer 732 obtains a composition of the compound, an experimental property value of property 1 of the compound, and an experimental property value of a crystal phase (in other words, a type of crystal phase) of the compound from experiment database 650 and outputs the composition and the experimental property values to image processor 734.

Image processor 734 obtains changed second display target information d22 from display method obtainer 733 and obtains, with respect to each of the one or more compounds that have been experimented on, the composition of the compound, the experimental property value of property 1, and the experimental property value of a crystal phase. In addition, with respect to each of the one or more compounds that have been experimented on, image processor 734 superimposes a mark indicating the experimental property value of property 1 of the compound and the experimental property value of a crystal phase (in other words, a type of crystal phase) of the compound on a position on image map Mb that corresponds to the composition of the compound. At this point, image processor 734 superimposes the mark according to second display target information d22. In other words, image processor 734 superimposes a mark with a shape in accordance with the experimental property value of a crystal phase of the compound and a color of a shade in accordance with the experimental property value of property 1 of the compound on image map Mb.

As a result, image processor 734 displays the third image illustrated in (a) in FIG. 69B on displayer 140. The third image includes image map Mb, and mark e2 with a circular shape and a color of a shade in accordance with the experimental property value of property 1 and mark e3 with a triangular shape and a color of a shade in accordance with the experimental property value of property 1 are superimposed on image map Mb. Mark e2 with a circular shape indicates that a crystal phase of a compound having a composition corresponding to a position of mark e2 is a first type and mark e3 with a triangular shape indicates that a crystal phase of a compound having a composition corresponding to a position of mark e3 is a second type.

In this manner, a shape of a mark is set to a circular shape, a triangular shape, or the like in accordance with an experimental property value of a crystal phase or, in other words, a type of a crystal phase. In addition, a shade of color of a mark is set in accordance with an experimental property value of property 1. As a result, in the third image illustrated in (a) in FIG. 69B, the shade of color of mark e3 significantly differs from a shade of color of a position of mark e3 and a periphery thereof on image element map Ma and a shape of mark e3 also differs from shapes of other marks e2 in a similar manner to the example illustrated in (a) in FIG. 69A. Therefore, the user having viewed the third image can visually understand that, with respect to a compound having a composition corresponding to the position of mark e3, an experimental property value of property 1 of the compound significantly differs from an evaluated property value and a type of a crystal phase of the compound differs from a type of a crystal phase of a compound corresponding to another mark e2. In other words, in display system 700 according to the present embodiment, it can be suggested to the user that, for example, there is a possibility that a processing condition is not optimal. In this manner, a change in second display target information d22 causes an image to be displayed on displayer 140 to dynamically change from the first image to the third image. Accordingly, for example, experimental property values and crystal phases can be readily compared among a plurality of compounds and material development by the user can be appropriately supported.

In other words, property display device 730 according to the present embodiment includes experimental value obtainer 732, display method obtainer 733, and image processor 734. Display method obtainer 733 obtains second information d20 for displaying an experimental property value of a compound. Based on second information d20, with respect to each of the one or more compounds that have been experimented on, experimental value obtainer 732 and image processor 734 obtain an experimental value obtainer of the compound and superimpose the experimental property value of the compound on a position on image map Mb that corresponds to a composition of the compound.

Accordingly, since experimental property values are superimposed on image map Mb based on second information d20, an evaluated property value and an experimental property value of each of a plurality of compounds to be indicated on image map Mb can be appropriately displayed so that the evaluated property value and the experimental property value can be compared with each other.

In addition, in property display device 730 according to the present embodiment, second information d20 is changed by display method obtainer 733. Image processor 734 outputs the third image including image map Mb in which a display mode of one or more experimental property values superimposed on image map Mb has been changed to displayer 140. Specifically, by changing a display mode of one or more experimental property values superimposed on image map Mb according to changed second information d20, image processor 734 changes image map Mb, generates the third image including changed image map Mb, and outputs the third image to displayer 140. For example, the third image is outputted to and displayed by displayer 140.

Accordingly, when second information d20 is changed, since image map Mb on which an experimental property value has been superimposed in a display mode in accordance with contents of the change is displayed by being included in the third image, an experimental property value of each of one or more compounds that have been experimented on can be displayed from various points of view. As a result, material search can be made more efficient, and material development can be appropriately supported.

In addition, in property display device 730 according to the present embodiment, second information d20 includes second display target information d22 indicating one or more property types. With respect to each of a plurality of compounds that have been experimented on, experimental value obtainer 732 obtains an experimental property value with respect to each of one or more property types indicated by second display target information d22. With respect to each of a plurality of compounds that have been experimented on, image processor 734 superimposes a mark in a mode according to an experimental property value with respect to each of the one or more property types on image map Mb. In display method obtainer 733, the one or more property types indicated by second display target information d22 is changed.

Accordingly, when second display target information d22 is changed, since image map Mb on which an experimental property value has been superimposed in a display mode in accordance with contents of the change is displayed by being included in the third image, an evaluated property value of each of one or more compounds that have been experimented on can be displayed from points of view of various kinds of properties. As a result, material search can be made more efficient.

In addition, in property display device 730 according to the present embodiment, second information d20 includes second color information d21 that indicates at least one of a hue, a chroma, and a brightness as a color attribute of an experimental property value. Image processor 734 generates image map Mb on which an experimental property value of each of one or more compounds that have been experimented on is superimposed in a color having a color attribute indicated by second color information d21. In display method obtainer 733, the color attribute indicated by second color information d21 is changed. For example, display method obtainer 733 changes the color attribute in accordance with an input operation with respect to input unit 110 by the user.

Accordingly, the color of an experimental property value indicated on image map Mb is changed to a color having the changed color attribute. Therefore, a color attribute of an evaluated property value indicated on image map Mb can be optionally changed and image map Mb on which an experimental property value that is readily visible by the user has been superimposed can be displayed by being included in the third image. As a result, material search can be made more efficient.

[Specific example 2 of change of images]

FIGS. 70A and 70B are diagrams illustrating an example of a transition of images accompanying a change in display condition information d23. Specifically, FIG. 70A illustrates display condition information d23 included in second information d20 and an example of the first image displayed on displayer 140 according to display condition information d23. FIG. 70B illustrates display condition information d23 after a change and an example of the third image displayed on displayer 140 according to changed display condition information d23.

For example, display method obtainer 733 obtains display condition information d23 illustrated in (b) in FIG. 70A from experiment display database 740. Display condition information d23 indicates, with respect to a threshold being a condition regarding display of an experimental property value, threshold setting "present" and threshold setting "absent" as options. In this case, in display condition information d23, threshold setting "absent" is selected.

Image processor 734 generates image map Mb indicating an evaluated property value obtained by evaluation value obtainer 632 in a similar manner to that described above. Furthermore, image processor 734 obtains display condition information d23 from display method obtainer 733 and obtains a composition and an experimental property value of each of one or more compounds that have been experimented on from experimental value obtainer 732. In addition, with respect to each of the one or more compounds that have been experimented on, image processor 734 superimposes a mark indicating the experimental property value of the compound on a position on image map Mb that corresponds to a composition of the compound. At this point, image processor 734 superimposes the mark according to display condition information d23. In other words, since threshold setting "absent" is selected in display condition information d23, image processor 734 superimposes a mark with a same shape regardless of a magnitude of the experimental property value obtained by experimental value obtainer 732 and a color of a shade in accordance with the experimental property value on image map Mb.

As a result, image processor 734 displays the first image illustrated in (a) in FIG. 70A on displayer 140. The first image includes image map Mb and, for example, each of all experimental property values obtained by experimental value obtainer 732 is superimposed on image map Mb as a mark with a circular shape and a color of a shade in accordance with the experimental property value.

At this point, the user changes display condition information d23 in second information d20 by performing an input operation with respect to input unit 110. For example, when display method obtainer 733 obtains an input signal indicating a change of display condition information d23 from input unit 110, display method obtainer 733 changes display condition information d23 as illustrated in (b) in FIG. 70B according to the input signal.

In the example illustrated in (b) in FIG. 70B, threshold setting "present" is selected. Furthermore, display condition information d23 indicates a threshold "3.5" as the numerically-designated threshold. In other words, display condition information d23 indicates that an experimental property value is equal to or larger than the threshold "3.5" as a content of the condition. In display condition information d23, a threshold that designates the number of values may be set or a threshold that designates a date and time may be set. When a threshold that designates the number of values is set, display condition information d23 indicates that the number of experimental property values is equal to or larger than the threshold or equal to or smaller than the threshold as a content of the condition. When a threshold that designates a date and time is set, display condition information d23 indicates that a date and time at which an experimental property value has been obtained by an experiment is later than the threshold date and time or before the threshold date and time as a content of the condition.

Image processor 734 obtains changed display condition information d23 from display method obtainer 733 and obtains a composition and an experimental property value of each of one or more compounds that have been experimented on from experimental value obtainer 732. In addition, with respect to each of the one or more compounds that have been experimented on, image processor 734 superimposes a mark indicating the experimental property value of the compound on a position on image map Mb that corresponds to a composition of the compound. At this point, image processor 734 superimposes the mark according to changed display condition information d23. In other words, display condition information d23 indicates that threshold setting "present" has been selected and that an experimental property value is equal to or larger than the threshold "3.5". Therefore, as illustrated in (a) in FIG. 70B, when the experimental property value obtained by experimental value obtainer 732 is equal to or larger than the threshold "3.5", image processor 734 superimposes mark e4 with a star shape and a color of a shade in accordance with the experimental property value on image map Mb as the experimental property value. On the other hand, when the experimental property value obtained by experimental value obtainer 732 is smaller than the threshold "3.5", image processor 734 superimposes mark e5 with a circular shape and a color of a shade in accordance with the experimental property value on image map Mb as the experimental property value. In other words, image processor 734 highlights mark e4 that corresponds to an experimental property value equal to or larger than the threshold "3.5" more than mark e5 that corresponds to an experimental property value smaller than the threshold "3.5". In addition, as illustrated in (a) in FIG. 70B, image processor 734 displays the third image including image map Mb on which marks e4 and e5 have been superimposed on displayer 140.

In this manner, a change in display condition information d23 causes an image to be displayed on displayer 140 to dynamically change from the first image to the third image. Accordingly, for example, the user can more readily visually understand experimental data and material development by the user can be appropriately supported.

In the example illustrated in FIG. 70B, image processor 734 highlights an experimental property value equal to or larger than the threshold "3.5" by differentiating a shape of mark e4 that corresponds to the experimental property value equal to or larger than the threshold "3.5" from mark e5 that corresponds to an experimental property value smaller than the threshold "3.5". However, image processor 734 may highlight an experimental property value equal to or larger than the threshold "3.5" by means other than the shape of a mark. For example, a mark or an experimental property value corresponding to the mark may be highlighted by applying edging to the mark, decorating the mark by applying luster, or the like.

In addition, when a threshold that designates the number of values is set in display condition information d23, for example, experimental property values in a descending order of magnitude corresponding to the number designated by the threshold are highlighted. Accordingly, the user can readily understand a desirable experimental property value from a large number of experimental property values. In addition, when a threshold that designates a date and time is set in display condition information d23, for example, experimental property values obtained by an experiment after the threshold date and time are highlighted. Accordingly, the user can readily comprehend a status of recent experiments or a status of experiments from day to day.

As described above, in property display device 730 according to the present embodiment, second information d20 includes display condition information d23 indicating a display condition of experimental property values based on a threshold. Among experimental property values of each of one or more compounds that have been experimented on, image processor 734 superimposes an experimental property value satisfying a display condition indicated by display condition information d23 and an experimental property value not satisfying the display condition in mutually different modes on a map. In display method obtainer 733, a display condition indicated by display condition information d23 is changed by increasing or reducing the threshold.

Accordingly, when display condition information d23 is changed, since image map Mb on which an experimental property value has been superimposed in a display mode in accordance with contents of the change is displayed by being included in the third image, an evaluated property value of each of one or more compounds that have been experimented on can be displayed from points of view of various display conditions. As a result, material search can be made more efficient. For example, a display mode of an experimental property value that satisfies the display condition of being equal to or larger than a threshold can be more highlighted than a display mode of an experimental property value that does not satisfy the display condition of being equal to or larger than a threshold. Furthermore, for example, by changing the threshold, experimental property values to be highlighted on image map Mb can be changed so as to become more visible by the user.

While first information d10 is not changed in the specific examples of a change to images illustrated in FIGS. 69A to 70B, first information d10 may also be changed in a similar manner to Embodiment 3A. In this case, the third image includes image map Mb on which respective changes to first information d10 and second information d20 are reflected.

[Processing operations]

FIG. 71 is a flowchart illustrating processing operations of display system 700 according to the present embodiment.

(Step S211)

Display method obtainer 733 obtains first information d10 regarding a display method of an evaluated property value from evaluation display database 640 and outputs first information d10 to evaluation value obtainer 632, experimental value obtainer 732, and image processor 734.

(Step S221)

Display method obtainer 733 further obtains second information d20 regarding a display method of an experimental property value from experiment display database 740 and outputs second information d20 to experimental value obtainer 732 and image processor 734.

(Step S222)

Experimental value obtainer 732 obtains first information d10 and second information d20 from display method obtainer 733. In addition, experimental value obtainer 732 obtains an experimental property value of each of one or more compounds which have been experimented on and which is included in the search range indicated by search range information d14 of first information d10, the experimental property value being of a type indicated by second display target information d22 of second information d20, from experiment database 650 together with a composition of the compound. Experimental value obtainer 732 outputs the composition and the experimental property value of each of the one or more compounds which have been experimented on to image processor 734.

(Step S213)

Evaluation value obtainer 632 obtains first information d10 from display method obtainer 733. In addition, evaluation value obtainer 632 obtains an evaluated property value of each of a plurality of compounds in accordance with calculation method information d12, search range information d14, and the like in first information d10. At this point, evaluation value obtainer 632 obtains an evaluated property value of each of a plurality of compounds using one or more evaluaters in evaluater database 620. Evaluation value obtainer 632 outputs a composition and an evaluated property value corresponding to the composition of each of the plurality of compounds to image processor 734.

(Step S223)

Image processor 734 generates the first image including image map Mb. At this point, image processor 734 generates image map Mb using first information d10 obtained in step S211 and a composition and an evaluated property value of each of the plurality of compounds obtained in step S213. Furthermore, image processor 734 superimposes an experimental property value of one or more compounds which have been experimented on and obtained in step S222 on a position on image map Mb that corresponds to the composition of the compound. At this point, image processor 734 generates a mark in a form corresponding to the experimental property value in accordance with second information d20 obtained in step S221 and superimposes the mark as the experimental property value. Accordingly, the first image including image map Mb on which the experimental property value of each of one or more compounds which have been experimented on is superimposed is generated. Image processor 734 outputs the first image to displayer 140.

(Step S224)

Displayer 140 obtains the first image from image processor 734 and displays the first image.

(Step S225)

The user performs an input operation with respect to input unit 110. In accordance with the input operation by the user, input unit 110 outputs an input signal indicating a change of display of the first image to display method obtainer 733 of property display device 630. By obtaining the input signal, display method obtainer 733 receives the change of display of the first image.

(Step S226)

In accordance with the input signal obtained in step S225, display method obtainer 733 changes at least one piece of information among first information d10 obtained in step S211 and second information d20 obtained in step S221. In addition, display method obtainer 733 outputs the at least one piece of changed information to at least two of evaluation value obtainer 632, experimental value obtainer 732, and image processor 734. In other words, when first information d10 has been changed, display method obtainer 733 outputs changed first information d10 to evaluation value obtainer 632, experimental value obtainer 732, and image processor 734. In addition, when second information d20 has been changed, display method obtainer 733 outputs changed second information d20 to experimental value obtainer 732 and image processor 734.

(Step S227)

Upon receiving changed first information d10 from display method obtainer 733, evaluation value obtainer 632 once again obtains an evaluated property value in accordance with changed contents of first information d10. Upon receiving changed first information d10 and changed second information d20 from display method obtainer 733, experimental value obtainer 732 once again obtains an experimental property value in accordance with changed contents of first information d10 and second information d20. In addition, image processor 734 generates the third image in accordance with changed first information d10 and changed second information d20. For example, the third image indicates the evaluated property value obtained once again and includes image map Mb on which the experimental property value obtained once again has been superimposed. Image processor 734 outputs the third image to displayer 140.

(Step S228)

Displayer 140 obtains the third image from image processor 734 and displays the third image.

[EMBODIMENT 3C]

A display system according to the present embodiment generates and displays an image including a map on which an experimental property value of each of one or more compounds which have been experimented on is superimposed in a similar manner to Embodiment 3B. Furthermore, the display system according to the present embodiment saves one or more images generated in the past and displays saved images.

Note that among the respective components according to the present embodiment, the same components as in Embodiments 1, 2, 3A, and 3B are given the same reference numerals as in Embodiments 1, 2, 3A, and 3B and detailed descriptions thereof are omitted. In addition, while image map Mb is described as an example of a map in the present embodiment, the map according to the present embodiment is not limited to image map Mb and may be image element map Ma or a set of a plurality of image maps Mb.

[Configuration of display system 800]

FIG. 72 is a block diagram illustrating an example of a configuration of display system 800 according to the present embodiment. Display system 800 illustrated in FIG. 72 includes input unit 110, evaluater database 620, property display device 830, displayer 140, evaluation display database 640, experiment database 650, experiment display database 740, and image storage 820. Note that property display device 830 is an example of an information display device.

Property display device 830 according to the present embodiment generates a map indicating an evaluated property value of each of a plurality of compounds and superimposes, on the map, an experimental property value of each of one or more compounds which have been experimented on in a similar manner to property display device 730 according to Embodiment 3B. In addition, property display device 830 generates an image including the map and displays the image on displayer 140. Property display device 830 according to the present embodiment adds a date and time at which the image has been generated to the image and saves the image to which the date and time have been added in image storage 820. Furthermore, when property display device 830 receives an input signal indicating a date and time from input unit 110, property display device 830 obtains an image in accordance with the date and time from image storage 820 and displays the image on displayer 140.

Property display device 830 includes evaluation value obtainer 632, display method obtainer 833, experimental value obtainer 732, and image processor 834. Note that property display device 830 may be constituted of, for example, a processor such as a CPU and a memory. In this case, the processor functions as property display device 830 by, for example, executing a computer program stored in the memory. Note that the memory may be volatile or non-volatile or may be constituted of a volatile memory and a non-volatile memory.

[Display method obtainer 833]

Display method obtainer 833 has a similar function to display method obtainer 733 according to Embodiment 3B and further obtains an input signal indicating a date and time from input unit 110 as date and time setting information d30. Date and time setting information d30 is information prompting display of an image to which a date and time set by the user has been added. When obtaining date and time setting information d30, display method obtainer 833 outputs date and time setting information d30 to image processor 834. Accordingly, an image to which a date and time indicated by date and time setting information d30 has been added is displayed on displayer 140 as a fourth image.

Furthermore, when display method obtainer 833 obtains an input signal indicating a change to the date and time from input unit 110, display method obtainer 833 changes date and time setting information d30 in accordance with the input signal and outputs date and time setting information d30 after the change to image processor 834. Accordingly, an image to which a date and time indicated by date and time setting information d30 after the change has been added is displayed on displayer 140 as a fifth image.

[Image processor 834]

Image processor 834 has a similar function to image processor 734 according to Embodiment 3B and, further, stores an image including a generated map in image storage 820. In this case, image processor 834 adds a date and time at which the image had been generated to the image and stores the image in image storage 820. The image may be any of the first image, the second image, and the third image. In addition, the image to which the date and time has been added is stored in image storage 820 as a saved image.

When image processor 834 obtains date and time setting information d30 from display method obtainer 833, image processor 834 retrieves a saved image to which a date and time indicated by date and time setting information d30 has been added from image storage 820. In addition, image processor 834 obtains the saved image to which the date and time has been added from image storage 820 and outputs the saved image to displayer 140 as fourth image or fifth image.

[Image storage 820]

Image storage 820 is a recording medium for storing, as a saved image, an image including a map generated by image processor 834. This recording medium is, for example, a hard disk drive, a RAM, a ROM, a semiconductor memory, or the like. Furthermore, the recording medium may be volatile or nonvolatile. A date and time at which a saved image to be stored in image storage 820 had been generated are added to the saved image as accompanying information.

[Specific example of change of images]

FIGS. 73A and 73B are diagrams illustrating an example of a transition of images accompanying a change in date and time setting information d30. Specifically, FIG. 73A illustrates a setting image for receiving date and time setting information d30 and an example of the fourth image displayed on displayer 140 according to date and time setting information d30. FIG. 73B illustrates a setting image for receiving date and time setting information d30 after a change and an example of the fifth image displayed on displayer 140 according to date and time setting information d30 after the change.

For example, display method obtainer 833 obtains date and time setting information d30 from input unit 110. Specifically, as illustrated in (b) in FIG.73A, image processor 834 displays a setting image for receiving date and time setting information d30 on displayer 140. The setting image respectively indicates ON and OFF as options with respect to a date and time setting. The user performs an input operation with respect to input unit 110 while viewing the setting image. For example, the user selects OFF by the input operation. Input unit 110 outputs date and time setting information d30 that is an input signal in accordance with the input operation to display method obtainer 833. The date and time setting information d30 indicates OFF with respect to the date and time setting. Display method obtainer 833 outputs date and time setting information d30 to image processor 834.

When image processor 834 obtains date and time setting information d30 from display method obtainer 833, since OFF is indicated in date and time setting information d30, image processor 834 obtains a latest saved image from image storage 820. In addition, image processor 834 outputs the latest saved image to displayer 140 as the fourth image. In other words, image processor 834 displays the fourth image illustrated in (a) in FIG. 73A on displayer 140.

At this point, the user changes date and time setting information d30 by performing an input operation with respect to input unit 110. Specifically, as illustrated in (b1) to (b3) in FIG. 73B, image processor 834 displays a setting image for receiving a change to date and time setting information d30 on displayer 140. The user performs an input operation with respect to input unit 110 while viewing the setting image. For example, the user selects ON by the input operation. Accordingly, when image processor 834 receives an input signal indicating ON from input unit 110 via display method obtainer 833, image processor 834 includes a slider and a slider bar for setting a date and time and a continuous playback button in the setting image. By performing an input operation with respect to input unit 110, for example, as illustrated in (b1) in FIG, 73B, the user moves the slider and sets the date and time to April 1, 2018. As a result, date and time setting information d30 is changed. In other words, date and time setting information d30 is changed from information indicating OFF with respect to the date and time setting to information indicating April 1, 2018 with respect to the date and time setting. Display method obtainer 833 obtains date and time setting information d30 after the change from input unit 110 and outputs date and time setting information d30 after the change to image processor 834.

When image processor 834 obtains date and time setting information d30 after the change from display method obtainer 833, since April 1, 2018 is indicated in date and time setting information d30 after the change, image processor 834 retrieves a saved image to which April 1, 2018 is added as a date and time from image storage 820. In addition, image processor 834 obtains the saved image from image storage 820 and outputs the saved image to displayer 140 as the fifth image. In other words, image processor 834 displays the fifth image illustrated in (a1) in FIG. 73B on displayer 140.

In a similar manner, by performing an input operation with respect to input unit 110, for example, as illustrated in (b2) or (b3) in FIG, 73B, the user moves the slider and sets the date and time to April 2, 2018 or April 3, 2018. Even in this case, date and time setting information d30 is changed in a similar manner to that described above. As a result, when image processor 834 obtains date and time setting information d30 after the change from display method obtainer 833, image processor 834 retrieves a saved image to which a date and time corresponding to date and time setting information d30 after the change is added from image storage 820. In addition, image processor 834 obtains the saved image from image storage 820 and outputs the saved image to displayer 140 as the fifth image. In other words, image processor 834 displays the fifth image illustrated in (a2) or (a3) in FIG. 73B on displayer 140.

While the user moves a slider in the example described above, the user may select the continuous playback button instead. In this case, image processor 834 obtains respective saved images from a saved image with an old date and time to a saved image with a new date and time from image storage 820 and displays the saved images in chronological order of the saved images on displayer 140 as the fifth image.

In this manner, a change in date and time setting information d30 causes an image to be displayed on displayer 140 to dynamically change from the fourth image to the fifth image. Accordingly, the user can readily comprehend day-to-day progress of experiments and material development by the user can be appropriately supported.

When the user sets a plurality of dates and times, display method obtainer 833 may output date and time setting information d30 indicating the plurality of dates and times to image processor 834. In this case, image processor 834 may sort saved images respectively generated on the plurality of dates and times in chronological order and display the sorted saved images on displayer 140. Accordingly, the user can readily compare a plurality of saved images with each other.

In addition, image processor 834 may convert an image including image map Mb into an image in a change operation-disabled state and store the image in the change operation-disabled state in image storage 820 as a saved image. Furthermore, image processor 834 may obtain the saved image from image storage 820 and display the saved image on displayer 140 as-is or reconstruct an image in a change operation-enabled state from the saved image and display the reconstructed image on displayer 140. An image in a change operation-disabled state may be an image in a format such as JPEG (Joint Photographic Experts Group), GIF (Graphics Interchange Format) or PNG (Portable Network Graphics). Such an image in a change operation-disabled state is an image that cannot be updated in accordance with a change to first information d10, second information d20, date and time setting information d30, and the like. On the other hand, an image in a change operation-enabled state is an image that can be updated in accordance with a change to first information d10, second information d20, date and time setting information d30, and the like. Reconstruction is processing of returning a state of an image from a change operation-disabled state to a change operation-enabled state. Accordingly, when an image in a change operation-disabled state has a smaller data amount than an image in a change operation-enabled state, storage capacity of image storage 820 can be suppressed. In addition, since the image in a change operation-disabled state is read from image storage 820 and reconstructed into an image in a change operation-enabled state and subsequently displayed on displayer 140, the user can update the displayed image by changing first information d10, second information d20, date and time setting information d30, or the like. Accordingly, since images generated in the past can be additionally analyzed, discussions are expected to be advanced smoothly and research and development are expected to proceed with greater efficiency.

As described above, in property display device 830 according to the present embodiment, image processor 834 associates accompanying information indicating a time point at which the first image had been generated with the first image and stores the first image with which the accompanying information has been associated as a saved image in image storage 820. In addition, when generating the second image, image processor 834 associates accompanying information indicating a time point at which the second image had been generated with the second image and stores the second image with which the accompanying information has been associated as a saved image in image storage 820. Display method obtainer 833 obtains time point information indicating a first time point. Image processor 834 reads a saved image associated with accompanying information corresponding to the first time point indicated by the time point information from image storage 820 as the fourth image and outputs the fourth image to displayer 140. In addition, in display method obtainer 833, the first time point indicated by time point information is changed to a second time point. At this point, image processor 834 reads a saved image associated with accompanying information corresponding to the second time point from image storage 820 as the fifth image and outputs the fifth image in place of the fourth image to displayer 140. For example, the accompanying information indicates a date and time at which the first image or the second image had been generated and the time point information corresponds to date and time setting information d30 described earlier. In addition, the first time point and the second time point may be absolute time points such as a date and time or relative time points such as a latest time point. For example, the fourth image and the fifth image are outputted to and displayed by displayer 140.

Accordingly, for example, in accordance with time point information that is date and time setting information d30, the first image or the second image generated in the past can be read from image storage 820 and displayed as the fourth image. In addition, by changing the time point information, the first image or the second image generated at a desired time point of the user can be read from a recording medium and displayed as the fifth image. As a result, material search can be made more efficient.

[Processing operations]

FIG. 74 is a flowchart illustrating processing operations of display system 800 according to the present embodiment.

(Step S230)

First, display system 800 executes image generation processing. In other words, in image generation processing in present step S230, display system 800 executes each step indicated in the flowchart illustrated in FIG. 71 according to Embodiment 3B or steps S211, S213, and S221 to S224 included in the flowchart. Accordingly, at least the first image among the first image, the second image, and the third image is generated and displayed.

(Step S231)

Next, image processor 834 of property display device 830 saves the image generated by the image generation processing in step S230 as a saved image. At this point, image processor 834 adds a date and time at which the image had been generated to the image and stores the image to which the date and time have been added as a saved image in image storage 820.

(Step S232)

Next, display method obtainer 833 of property display device 830 determines whether or not date and time setting information d30 has been received from input unit 110. When display method obtainer 833 determines that date and time setting information d30 has not been received (No in step S232), display system 800 repetitively executes processing steps from step S230.

(Step S233)

On the other hand, when display method obtainer 833 determines that date and time setting information d30 has been received in step S232 (Yes in step S232), image processor 834 obtains date and time setting information d30 from display method obtainer 833. In addition, image processor 834 obtains a saved image to which a date and time indicated by date and time setting information d30 has been added from image storage 820 as the fourth image.

(Step S234)

Image processor 834 displays the fourth image obtained in step S233 on displayer 140 by outputting the fourth image to displayer 140.

(Step S235)

Next, display method obtainer 833 determines whether or not a change to date and time setting information d30 has been received from input unit 110. When display method obtainer 833 determines that a change to date and time setting information d30 has not been received from input unit 110 (No in step S235), property display device 830 executes processing of step S238.

(Step S236)

On the other hand, when display method obtainer 833 determines that a change to date and time setting information d30 has been received from input unit 110 (Yes in step S235), image processor 834 obtains the fifth image. In other words, image processor 834 obtains a saved image to which a date and time indicated by date and time setting information d30 after the change has been added from image storage 820 as the fifth image.

(Step S237)

Image processor 834 displays the fifth image obtained in step S236 on displayer 140 by outputting the fifth image to displayer 140.

(Step S238)

Next, property display device 830 determines whether or not an end condition of processing is satisfied. For example, when an input signal is obtained from input unit 110 and the input signal indicates an end of processing, property display device 830 determines that the end condition of processing is satisfied. When property display device 830 determines that the end condition of processing is not satisfied (No in step S238), processing steps from step S235 are repetitively executed. On the other hand, when property display device 830 determines that the end condition of processing is satisfied (Yes in step S238), all processing regarding property display is ended.

[EMBODIMENT 3D]

A display system according to the present embodiment generates and displays an image including a map on which an experimental property value of each of one or more compounds which have been experimented on is superimposed in a similar manner to Embodiment 3B. Furthermore, when an evaluated property value or an experimental property value on a map is selected, the display system according to the present embodiment displays a composition formula of a compound having the selected evaluated property value or the selected experimental property value.

Note that among the respective components according to the present embodiment, the same components as in Embodiments 1, 2, and 3A to 3C are given the same reference numerals as in Embodiments 1, 2, and 3A to 3C and detailed descriptions thereof are omitted. In addition, while image map Mb is described as an example of a map in the present embodiment, the map according to the present embodiment is not limited to image map Mb and may be image element map Ma or a set of a plurality of image maps Mb.

[Configuration of display system 900]

FIG. 75 is a block diagram illustrating an example of a configuration of display system 900 according to the present embodiment. Display system 900 illustrated in FIG. 75 includes input unit 110, evaluater database 620, property display device 930, displayer 140, evaluation display database 640a, experiment database 650, and experiment display database 740a. Note that property display device 930 is an example of an information display device.

Property display device 930 according to the present embodiment generates a map indicating an evaluated property value of each of a plurality of compounds and superimposes, on the map, an experimental property value of each of one or more compounds which have been experimented on in a similar manner to property display device 730 according to Embodiment 3B. In addition, property display device 930 generates an image including the map and displays the image on displayer 140. At this point, when an evaluated property value on the map is selected, property display device 930 according to the present embodiment specifies a composition formula of a compound having the evaluated property value using evaluation display database 640a and displays the composition formula on displayer 140. In a similar manner, when an experimental property value on the map is selected, property display device 930 according to the present embodiment specifies a composition formula of a compound having the experimental property value using experiment display database 740a and displays the composition formula on displayer 140.

Property display device 930 includes evaluation value obtainer 632, display method obtainer 933, experimental value obtainer 732, and image processor 934. Note that property display device 930 may be constituted of, for example, a processor such as a CPU and a memory. In this case, the processor functions as property display device 930 by, for example, executing a computer program stored in the memory. Note that the memory may be volatile or non-volatile or may be constituted of a volatile memory and a non-volatile memory.

[Display method obtainer 933]

Display method obtainer 933 has a similar function to display method obtainer 733 according to Embodiment 3B. Furthermore, display method obtainer 933 specifies a composition formula of a compound having an evaluated property value or an experimental property value selected by the user. For example, the user views an image being displayed on displayer 140 and selects an evaluated property value indicated on a map in the image by an input operation with respect to input unit 110. Input unit 110 outputs an input signal indicating a position on the map of an evaluated property value selected in accordance with the input operation by the user to display method obtainer 933 as position information d40. When display method obtainer 933 obtains position information d40 from input unit 110, display method obtainer 933 specifies a composition formula corresponding to a position indicated by position information d40 using first composition information to be described later which is included in evaluation display database 640a.

In a similar manner, for example, the user views an image being displayed on displayer 140 and selects an experimental property value indicated on a map in the image by an input operation with respect to input unit 110. Input unit 110 outputs an input signal indicating a position on the map of an experimental property value selected in accordance with the input operation by the user to display method obtainer 933 as position information d40. When display method obtainer 933 obtains position information d40 from input unit 110, display method obtainer 933 specifies a composition formula corresponding to a position indicated by position information d40 using second composition information to be described later which is included in experiment display database 740a.

In addition, display method obtainer 933 outputs the specified composition formula to image processor 934.

[Image processor 934]

Image processor 934 has a similar function to image processor 734 according to Embodiment 3B. Furthermore, when image processor 934 receives a composition formula from display method obtainer 933, image processor 934 outputs a composition image indicating the composition formula to displayer 140. Accordingly, the composition image is displayed on displayer 140.

[Evaluation display database 640a]

FIG. 76 is a diagram illustrating an example of information stored in evaluation display database 640a.

Evaluation display database 640a according to the present embodiment is a recording medium that stores first information d10 in a similar manner to evaluation display database 640 according to Embodiments 3A to 3C and further stores first composition information d18. First composition information d18 is information indicating a composition formula corresponding to a position of each evaluated property value on a map.

[Experiment display database 740a]

FIG. 77 is a diagram illustrating an example of information stored in experiment display database 740a.

Experiment display database 740a according to the present embodiment is a recording medium that stores second information d20 in a similar manner to experiment display database 740 according to Embodiments 3B and 3C and further stores second composition information d24. Second composition information d24 is information indicating a composition formula corresponding to a position of each experimental property value on a map.

[Specific example of change of images]

FIG. 78 is a diagram illustrating an example of a transition of images accompanying obtaining position information d40. Specifically, (a) in FIG. 78 illustrates an example of a sixth image displayed on displayer 140 and (b) in FIG. 78 illustrates an example of a seventh image displayed on displayer 140 according to position information d40. Note that the sixth image is the first image, the second image, or the third image.

For example, as illustrated in (a) in FIG. 78, image processor 934 displays the sixth image including image map Mb on displayer 140 in a similar manner to Embodiment 3B. In addition, one or more experimental property values have been superimposed on image map Mb.

In this case, the user selects any one of one or more experimental property values by performing an input operation with respect to input unit 110. As a result, in accordance with the input operation, input unit 110 outputs position information d40 indicating a position on image map Mb of the selected experimental property value to display method obtainer 933. When display method obtainer 933 obtains position information d40 from input unit 110, display method obtainer 933 obtains second composition information d24 from experiment display database 740a. In addition, display method obtainer 933 retrieves a composition formula associated with a position on image map Mb indicated in position information d40 from second composition information d24. Display method obtainer 933 outputs the retrieved composition formula to image processor 934.

When image processor 934 receives the composition formula from display method obtainer 933, image processor 934 generates composition image f1 indicating the composition formula as illustrated in (b) in FIG. 78. In addition, image processor 934 generates the seventh image by superimposing composition image f1 on image map Mb and outputs the seventh image to displayer 140. As a result, as illustrated in (b) in FIG. 78, the seventh image on which composition image f1 has been superimposed is displayed on displayer 140. In other words, an image to be displayed on displayer 140 dynamically changes from the sixth image to the seventh image.

While an experimental property value is selected in the example illustrated in FIG. 78, an evaluated property value may be selected. In this case, display method obtainer 933 obtains first composition information d18 from evaluation display database 640a. In addition, display method obtainer 933 retrieves a composition formula associated with a position on image map Mb indicated in position information d40 from first composition information d18. Display method obtainer 933 outputs the retrieved composition formula to image processor 934. As a result, in a similar manner to the example illustrated in (b) in FIG. 78, composition image f1 indicating a composition formula corresponding to the selected evaluated property value is superimposed on image map Mb and the seventh image including image map Mb is displayed on displayer 140.

In addition, when input unit 110 is a mouse, the selection of an experimental property value or an evaluated property value by the user may be performed by a click or by a click and hold of the mouse. In the case of a click and hold, image processor 934 may display composition image f1 on displayer 140 while the click and hold is being performed.

Accordingly, the user can comprehend a composition formula of a compound corresponding to the selected evaluated property value or the selected experimental property value in a simple manner without having to obtain the composition formula from option data of a range variable. As a result, discussions are expected to be advanced smoothly and research and development are expected to proceed with greater efficiency. In addition, when an evaluated property value is selected, the composition formula indicated in composition image f1 need not be a strict composition formula. In other words, when an evaluated property value at a position other than a grid point of image element map Ma is selected, a composition formula corresponding to a grid point nearest to the position may be displayed by being included in a composition image.

As described above, in property display device 930 according to the present embodiment, display method obtainer 933 obtains position information d40 indicating a position of an evaluated property value or an experimental property value on image map Mb included in the sixth image that is the first image, the second image, or the third image. In addition, display method obtainer 933 obtains composition formula data regarding a composition formula of a compound corresponding to the position indicated by position information d40. Image processor 934 changes the sixth image to the seventh image by superimposing composition image f1 indicating the composition formula data on image map Mb. For example, the seventh image is outputted to and displayed by displayer 140. Although composition formula data may be data indicating a composition formula of a compound itself, composition formula data is not limited to a composition formula itself and may indicate any kind of contents as long as the contents are related to the composition formula.

Accordingly, even when it is difficult to read a composition formula of a compound having an evaluated property value or an experimental property value indicated on image map Mb from coordinate axes of image map Mb, composition image f1 indicating the composition formula is displayed by position information d40. Therefore, the user can easily recognize the composition formula. As a result, material search can be made more efficient, and material development can be appropriately supported.

While the sixth image that is the first image, the second image, or the third image is changed to the seventh image in the present embodiment, the sixth image may be the fourth image or the fifth image according to Embodiment 3C.

In addition, while composition image f1 indicating a composition formula is displayed in the example illustrated in FIG. 78, the composition formula may include a coefficient of an element as a non-visualization variable. In this case, the non-visualization variable is displayed in various modes.

FIG. 79 is a diagram illustrating an example of a composition image indicating a composition formula including a coefficient of an element as a non-visualization variable.

For example, the seventh image includes image map Mb of a compound with a composition formula "$Li_{2-3a-4b}(Al_{1-x}Ga_x)_a(Ti_{1-y}Zr_y)_{1+b}O_c$". The composition formula includes a coefficient c of element O as a non-visualization variable (in other words, a non-utilized variable). For example, the first value described earlier is substituted into coefficient c. The first value is a constant. For example, the first value is 3 (in other words, c = 3). In this case, image map Mb indicates an evaluated property value of each of a plurality of compounds with the composition formula "$Li_{2-3a-4b}(Al_{1-x}Ga_x)_a(Ti_{1-y}Zr_y)_{1+b}O_3$". At this point, the user selects an evaluated property value indicated at a position of variables (a, b, x, y) = (0.2, 0.1, 0.6, 0.3) among image map Mb by, for example, an operation of a mouse being input unit 110. As a result, image processor 934 superimposes composition image f11 that is the same as composition image f1 on image map Mb as illustrated in (a) in FIG. 79. Note that when the first value is substituted into coefficient c, regardless of which position on image map Mb is selected, the first value (in other words, 3) is displayed as coefficient c on a composition image superimposed on the position.

In addition, the second value described earlier may be substituted into coefficient c. The second value is a value that satisfies, at each position on image map Mb, a condition that an evaluated property value indicated at the position is a best value. In this case, the user selects an evaluated property value indicated at a position of variables (a, b, x, y) = (0.2, 0.1, 0.6, 0.3) among image map Mb by an operation of a mouse. As a result, image processor 934 superimposes composition image f12 on image map Mb as illustrated in (b) in FIG. 79. A composition formula indicated by composition image f12 includes "3.1" as coefficient c. In other words, composition image f12 indicates that a composition formula of a compound that produces an evaluated property value being a best value among a plurality of compounds with a composition formula "$Li_{1.0}Al_{0.08}Ga_{0.12}Ti_{0.77}Zr_{0.33}O_c$" includes "3.1" being a second value as coefficient c. Furthermore, the user selects an evaluated property value indicated at a different position such as a position of variables (a, b, x, y) = (0.2, 0.1, 0.6, 0.2) among image map Mb by an operation of a mouse. As a result, image processor 934 superimposes composition image f13 on image map Mb as illustrated in (c) in FIG. 79. A composition formula indicated by composition image f13 includes "2.7" as coefficient c. In other words, composition image f13 indicates that a composition formula of a compound that produces an evaluated property value being a best value among a plurality of compounds with a composition formula "$Li_{1.0}Al_{0.08}Ga_{0.12}Ti_{0.88}Zr_{0.22}O_c$" includes "2.7" being a second value as coefficient c. In this manner, when the second value is substituted into coefficient c, different second values are displayed on a composition image in accordance with a position selected on image map Mb.

In addition, each numerical value within the prescribed numerical range described earlier may be substituted into coefficient c. In this case, a mean of evaluated property values of a plurality of compounds is indicated at each position indicated by variables a, b, x, and y on image map Mb. Each of the plurality of compounds has a composition formula expressed by each numerical value to be substituted into coefficient c of composition formula "$Li_{2-3a-4b}(Al_{1-x}Ga_x)_a(Ti_{1-y}Zr_y)_{1+b}O_c$". Note that variables a, b, x, and y included in the composition formula indicate values in accordance with positions on image map Mb.

At this point, the user selects an evaluated property value indicated at a position of variables (a, b, x, y) = (0.2, 0.1, 0.6, 0.3) among image map Mb by an operation of a mouse. As a result, image processor 934 superimposes composition image f14 on image map Mb as illustrated in (d) in FIG. 79. Composition image f14 indicates an English word "mean" denoting arithmetic processing instead of coefficient c. In other words, composition image f14 indicates that a mean of evaluated property values based on a coefficient of element O is represented on image map Mb. Alternatively, image processor 934 superimposes composition image f15 on image map Mb as illustrated in (e) in FIG. 79. Composition image f15 indicates a numerical range used to calculate a mean of evaluated property values or, in other words, a numerical range "2.7 to 3.0" of respective numerical values to be substituted into coefficient c. Note that when each numerical value in a prescribed numerical range is substituted into coefficient c, regardless of which position on image map Mb is selected, "mean" or the numerical range "2.7 to 3.0" is displayed on a composition image superimposed on the position.

While an evaluated property value is selected by an operation of a mouse in the example illustrated in FIG. 79, a predicted property value may be selected or an experimental property value or an experiment point may be selected. Even when a predicted property value, an experimental property value, or an experiment point is selected as a selection, a composition image indicating a composition formula corresponding to the selection is superimposed on image map Mb. When the selection is a predicted property value, a composition image indicating a composition formula including coefficient c used in the calculation of the predicted property value is superimposed. When the selection is an experimental property value, a composition image indicating a composition formula of a compound having the experimental property value is superimposed, and when the selection is an experiment point, a composition image indicating a composition formula of a compound associated with the experiment point is superimposed. In the examples illustrated in FIGS. 24A and 24B, even when a position on a map corresponding to a composition formula of a compound having the experimental property value does not exist, the experimental property value is superimposed on the map. Even in the case of such examples, when an experimental property value is selected, a composition image indicating a composition formula of a compound having the selected experimental property value is superimposed on a map in a similar manner to that described above. However, in the case of such examples, there is a possibility that the composition formula of the composition image to be superimposed differs from a composition formula that corresponds to a position of an experimental property value on the map or an evaluated property value in a periphery of the position. For example, a composition image indicating a composition formula including a numerical value that differs from "3" as coefficient c may be superimposed on image map Mb corresponding to coefficient c = 3.

As described above, in property display device 930 according to the present embodiment, display method obtainer 933 obtains position information d40 indicating a position of a predicted property value or an experimental property value on image map Mb. In addition, image processor 934 obtains composition formula data regarding a composition formula of a compound corresponding to the position indicated by the position information d40 and a composition image indicating the composition formula data is superimposed on image map Mb. The composition formula data includes a non-utilized variable associated with a compound having the predicted property value or the experimental property value. Note that the non-utilized variable is, among a plurality of variables, a variable other than variables a, b, x, and y to be used in the coordinate axes of image map Mb. Furthermore, composition formula data is data indicating, for example, a composition formula included in first composition information d18 or second composition information d24. In addition, due to the selection of an evaluated property value or an experimental property value by an operation of a mouse described above, display method obtainer 933 obtains position information d40 indicating a position of the evaluated property value or the experimental property value.

Accordingly, even if a non-utilized variable of a compound corresponding to a position of a predicted property value or an experimental property value cannot be comprehended from the position, since a composition image including the non-utilized variable is superimposed on image map Mb, the non-utilized variable can be readily comprehended by looking at the displayed composition image.

In addition, a variable not included in a composition formula may be represented as a non-visualization variable in a composition image. A specific example of the variable not included in a composition formula may be a processing variable.

FIG. 80 is a diagram illustrating an example of a composition image when a processing variable is used as a non-visualization variable.

For example, the seventh image includes image map Mb of a compound with a composition formula "$Li_{2-3a-4b}(Al_{1-x}Ga_x)_a(Ti_{1-y}Zr_y)_{1+b}O_3$". In the generation of image map Mb, three processing variables, namely, variable Pa indicating a calcination method, variable Pb indicating a calcination time, and variable Pc indicating a calcination temperature are used as non-visualization variables. At this point, the user selects an evaluated property value indicated at a position of variables (a, b, x, y) = (0.2, 0.1, 0.6, 0.3) among image map Mb by an operation of a mouse. As a result, image processor 934 superimposes composition image f16 on image map Mb as illustrated in FIG. 80. Composition image f16 not only indicates a composition formula of a compound having an evaluated property value selected by the mouse but also indicates option data of each of variables Pa, Pb, and Pc used to calculate the evaluated property value. For example, "calcination method Pa = ball mill", "calcination time Pb = 1 hour", "calcination temperature Pc = 100°C", and the like are indicated by composition image f16.

Note that the selections using a mouse in the examples illustrated in FIGS. 79 and 80 may be a click of the mouse, a click and hold of the mouse, or a mouseover. Alternatively, the selections described above may be made using an input device that differs from a mouse.

[Processing operations]

FIG. 81 is a flowchart illustrating processing operations of display system 900 according to the present embodiment.

(Step S230)

First, display system 900 executes image generation processing. In other words, in image generation processing in present step S230, display system 900 executes each step indicated in the flowchart illustrated in FIG. 71 according to Embodiment 3B or steps S211, S213, and S221 to S224 included in the flowchart. Accordingly, the first image, the second image, or the third image is displayed as the sixth image.

(Step S241)

Next, display method obtainer 933 of property display device 930 determines whether or not position information d40 has been received from input unit 110. At this point, when display method obtainer 933 determines that position information d40 has not been received (No in step S241), display system 900 ends processing regarding property display.

(Step S242)

On the other hand, in step S241, when display method obtainer 933 determines that position information d40 has been received (Yes in step S241), display method obtainer 933 obtains first composition information d18 or second composition information d24 from evaluation display database 640a or experiment display database 740a. For example, when a position of a selected evaluated property value is indicated in position information d40, display method obtainer 933 obtains first composition information d18 from evaluation display database 640a. In addition, when a position of a selected experimental property value is indicated in position information d40, display method obtainer 933 obtains second composition information d24 from experiment display database 740a. Furthermore, display method obtainer 933 retrieves a composition formula of a compound having the selected evaluated property value or the selected experimental property value from first composition information d18 or second composition information d24. In addition, image processor 934 generates composition image f1 including the retrieved composition formula.

(Step S243)

Image processor 934 generates the seventh image by superimposing composition image f1 generated in step S242 on image map Mb of the sixth image having been generated and displayed in step S230.

(Step S244)

Image processor 934 displays the seventh image generated in step S243 on displayer 140 by outputting the seventh image to displayer 140.

<Other aspects>

Although the various display systems according to the present disclosure have been described on the basis of embodiments, the present disclosure is not limited to these embodiments. Embodiments obtained by applying various modifications that may occur to a person skilled in the art, embodiments formed by combining the constituent elements in different embodiments, and the like are also included in the scope of the present disclosure insofar as such embodiments do not depart from the gist of the present disclosure.

For example, in the respective embodiments described above, while a map such as image element map Ma or image map Mb is generated, image map Mb may be generated instead of image element map Ma or image element map Ma may be generated instead of image map Mb. In addition, a map to be generated may be a set of a plurality of image maps Mb.

Furthermore, in the respective embodiments described above, experimental value obtainer 232, experimental value obtainer 732, and experiment point obtainer 635 obtain an experimental property value or an experiment point of compounds which have been experimented on and which have a composition included in a search range. However, experimental value obtainer 232, experimental value obtainer 732, and experiment point obtainer 635 may also obtain an experimental property value or an experiment point of compounds which have been experimented on and which have a composition included in not only the search range but also in ranges similar to the search range. Alternatively, experimental value obtainer 232, experimental value obtainer 732, and experiment point obtainer 635 may obtain experimental property values or experiment points of all compounds included in experiment database 150 or 650 regardless of the search range.

In addition, predictor database 120 and evaluater database 620 according to the respective embodiments described above may be updated every time an experimental result of a new compound is obtained based on the experimental result. In other words, predictor database 120 and evaluater database 620 may be updated every time a new experimental result is reflected on experiment database 150 or 650.

Furthermore, while a predicted property value, an experimental property value, and an evaluated property value are mainly indicated by a shade of color in the respective embodiments described above, the values may be indicated by a mode other than a shade of color as long as the mode is visually expressed. For example, a predicted property value, an experimental property value, and an evaluated property value may be indicated by color.

In addition, first information d10 or second information d20 is changed in Embodiments 3A to 3D described above. In this case, a property display device may display first information d10 or second information d20 on displayer 140 and the user may change first information d10 or second information d20 by performing an input operation with respect to input unit 110 while viewing displayer 140.

Furthermore, any one of Embodiments 1A, 1B, 2A to 2C, and 3A to 3D described above may be combined with another embodiment. For example, display modes of maps and experimental property values according to Embodiments 1A and 1B may be applied to display modes of evaluated property values and experimental property values according to Embodiments 3A to 3D. In addition, for example, display modes of maps and experimental property values according to Embodiments 1A and 1B may be applied to display modes of candidate points according to Embodiments 2A to 2C. More specifically, in Embodiments 2A to 2C, a property display device may obtain first display method information and second display method information according to Embodiment 1 and perform processing according to the pieces of information.

For example, while the property display devices according to the respective embodiments described above constitute a part of a display system, the property display devices may include all of the components of the display system. For example, property display device 230 illustrated in FIG. 13 may include input unit 110, predictor database 120, experiment database 150, and the like. In addition, the property display device may also include a plurality of processors. Furthermore, the property display device may be implemented as a single computer apparatus or may be implemented as computer apparatuses communicably connected to each other. In other words, the plurality of constituent elements included in the property display devices according to the respective embodiments described above need not be included in a same single apparatus and may be distributed among and arranged in mutually different apparatuses.

Furthermore, while each constituent element is constituted of dedicated hardware in the respective embodiments described above, each constituent element may be realized by executing a software program suitable for each constituent element. Each of the constituent elements may be realized by having a program executer such as a CPU or a processor read and execute a software program recorded in a recording medium such as a hard disk or a semiconductor memory. In this case, the program for achieving the property display devices and the like according to the respective embodiments described above may cause a processor to execute the respective steps included in at least one of the flowcharts illustrated in FIGS. 10, 23, 42, 48, 63, 66, 71, 74, and 81.

(Hardware configuration)

Specifically, each of display systems 100 to 900 described above may be configured by a computer system composed of, for example, a microprocessor, a ROM, a RAM, a hard disk drive, a display unit, a keyboard, and a mouse. The RAM or the hard disk drive stores a display program. The microprocessor operates according to the display program, so that the functions of each of display systems 100 to 900 are achieved. Here, the display program includes a plurality of instruction codes indicating instructions to be given to the computer so as to achieve a specific function.

In addition, some or all of the structural components included in each of display systems 100 to 900 described above may be realized as a single system large scale integration (LSI). The system LSI is a super multifunctional LSI manufactured by integrating a plurality of structural components onto a signal chip. To be more specific, the system LSI is a computer system configured with a microprocessor, a ROM, and a RAM, for example. The RAM stores a computer program. The microprocessor operates according to the computer program, so that a function of the system LSI is achieved.

Furthermore, some or all of the components included in each of display systems 100 to 900 described above may be implemented as an IC card or standalone module that can be inserted into and removed from a computer. The IC card or the module is a computer system configured with a microprocessor, a ROM, a RAM, and the like, for example. The IC card or the module may include the aforementioned super multifunctional LSI. The microprocessor operates according to the computer program, so that a function of the IC card or the module is achieved. The IC card or the module may be tamper-resistant.

Furthermore, the present disclosure may be a display method executed by display systems 100 to 900 described above. Furthermore, this display method may be realized by a computer executing a display program, or may be realized by a digital signal of the display program.

Moreover, the present disclosure may be may be the display program or the aforementioned digital signal recorded on a non-transitory computer-readable recording medium. The recording medium is, for example, a flexible disk, a hard disk, a CD-ROM, an MO, a DVD, a DVD-ROM, a DVD-RAM, a Blu-ray (registered trademark) disc (BD), or a semiconductor memory. The display program may be the digital signal recorded on a non-transitory recording medium.

Furthermore, the present disclosure may be the aforementioned display program or digital signal transmitted via a telecommunication line, a wireless or wired communication line, a network represented by the Internet, data broadcasting, or the like.

Moreover, the present disclosure may be a computer system including a microprocessor and a memory. The memory may store the aforementioned display program and the microprocessor may operate according to the display program.

Furthermore, by transferring the recording medium having the aforementioned display program or digital signal recorded thereon or by transferring the aforementioned display program or digital signal via the aforementioned network or the like, the present disclosure may be implemented by a different independent computer system.

Furthermore, the display system may be implemented by a server and a terminal that is in the possession of a user and is connected to the server via a network.

[Industrial Applicability]

The present disclosure produces the advantageous effect of being able to display information related to properties of compounds in an easily understandable manner.

[Reference Signs List]

100, 200, 300, 400, 401, 500, 600, 700, 800, 900 display system
110 input unit
120 predictor database
130, 230, 330, 430, 530, 630, 730, 830, 930 property display device
131 search range obtainer
132 predicted value obtainer
133, 633, 733, 833, 933 display method obtainer
134, 234, 334, 434, 534, 634, 734, 834, 934 image processor
140 displayer
150, 650 experiment database
232, 732 experimental value obtainer
331 candidate point determiner
410, 820 image storage
510 candidate point database
531 candidate point obtainer
620 evaluater database
632 evaluation value obtainer
635 experiment point obtainer
640, 640a evaluation display database
651 compound basic data
652 compound detail data
740, 740a experiment display database
a1 reference image
b1 candidate point
d10 first information
d11 first color information
d12 calculation method information
d121 calculation method image
d13 map array information
d14 search range information
d141 search range image
d15 first display target information
d16 display range information
d18 first composition information
d20 second information
d21 second color information
d22 second display target information
d23 display condition information
d24 second composition information
d30 date and time setting information
d40 position information
e1, e2, e3, e4, e5 mark (experimental property value)

f1, f11-f16 composition image
L1 isoline
Ma, Mal-Ma4 image element map (map)
Mb image map
r1, r2, r3, r4 region
R1, R2, R3, R4 region

## Claims

**1.** An information display method comprising:

obtaining first information for displaying an evaluated property value as a comparison indicator for comparing compounds;
obtaining an evaluated property value of each of the compounds based on the first information, and generating a map indicating, at a position corresponding to a composition of each of the compounds, the evaluated property value of the compound;
generating a first image including the map, and outputting the first image to a displayer; and
when the first information is changed, changing the map according to the first information changed, generating a second image including the map changed, and outputting the second image to the displayer.

**2.** The information display method according to claim 1, wherein

the first information includes first coloring information indicating at least one of hue, chroma, or brightness as a color attribute of the evaluated property value,
in the generating of the map, the map indicating the evaluated property value of each of the compounds using a color having the color attribute indicated by the first coloring information is generated, and
in the changing of the first information, the color attribute indicated by the first coloring information is changed.

**3.** The information display method according to claim 1 or 2, wherein

the first information includes search range information indicating variables and items of option data, the variables being used in representing the compositions of the compounds, the items of option data indicating possible values or elements of each of the variables, and
in the obtaining of the evaluated property value, for each of combinations of option data obtained by selecting one item of option data from the items of option data for each of the variables, the evaluated property value of a compound having a composition corresponding to the combination is obtained.

**4.** The information display method according to claim 3, wherein

the first information includes calculation method information indicating a calculation method of the evaluated property value,
the obtaining of the evaluated property value includes calculating, for each of the combinations, the evaluated property value of the compound having the composition corresponding to the combination, by using a specified algorithm that is in accordance with the calculation method indicated by the calculation method information, and
in the changing of the first information, the calculation method indicated by the calculation method information is changed.

**5.** The information display method according to claim 4, wherein
the obtaining of the evaluated property value includes calculating, for each of the combinations, a predictive distribution of property values predicted for the compound having the composition corresponding to the combination, by using the specified algorithm that is in accordance with the calculation method indicated by the calculation method information, and calculating the evaluated property value of the compound based on the predictive distribution.

**6.** The information display method according to any one of claims 3 to 5, wherein
in the changing of the first information, the items of option data that can be taken by each of at least one variable among the variables are changed.

**7.** The information display method according to any one of claims 3 to 6, wherein

the first information includes first display target information indicating one or more property types,
in the obtaining of the evaluated property value, for each of the compounds, the evaluated property value for each of the one or more property types indicated by the first display target information is obtained, and
in the changing of the first information, the one or more property types indicated by the first display target information are changed.

**8.** The information display method according to any one of claims 3 to 7, wherein

the first information includes display range information indicating a display range for the evaluated property value,
in the generating of the map, the display range for the evaluated property value indicated by the display range information is associated with a scale range of a predetermined color or a predetermined shade of color, and
the map indicating the evaluated property value of each of the compounds using a color or a shade of color in the scale range that corresponds to the evaluated property value is generated, and
in the changing of the first information, the display range indicated by the display range information is changed.

**9.** The information display method according to claim 6, wherein the map includes image element maps that are arranged in a matrix along each of a first coordinate axis and a second coordinate axis,

each of the image element maps includes a third coordinate axis and a fourth coordinate axis, and
the generating of the map includes:

associating the first coordinate axis, the second coordinate axis, the third coordinate axis, and the fourth coordinate axis with a first variable, a second variable, a third variable, and a fourth variable, respectively, among the variables;
identifying, for each of the compounds, an image element map associated with a value of the first variable and a value of the second variable that are to be used in representing a composition of the compound, among the image element maps; and
mapping, for each of the compounds, an evaluated property value of the compound on the image element map identified, at a position indicated by the value of the third variable and the value of the fourth variable that are to be used in representing the composition of the compound.

**10.** The information display method according to claim 9, wherein when a total number of the items of option data indicated by the search range information is increased or decreased in the changing of the first information,
in the changing of the map, a total number of the image element maps included in the map is increased or decreased.

**11.** The information display method according to claim 9 or 10, wherein

the first information includes map array information indicating an array mode of at least one image element map to be displayed, and
when the map array information is changed, in the changing of the first information, by selection of a position of the at least one image element map to be displayed,
in the changing of the map, the map generated is changed to a map including the at least one image element map arranged according to the array mode indicated by the map array information changed.

**12.** The information display method according to any one of claims 1 to 11, wherein
in the obtaining of the evaluated property value, the evaluated property value of each of the compounds is obtained from at least one evaluater for evaluating a property value of a compound, the at least one evaluater being stored in an evaluater database.

**13.** The information display method according to any one of claims 1 to 12, further comprising:

obtaining second information for displaying an experimental property value of a compound, wherein
the generating of the map further includes:

for each of one or more compounds that have been experimented on, obtaining an experimental property value of the compound, based on the second information; and

superimposing the experimental property value of the compound at a position, on the map, that corresponds to a composition of the compound, based on the second information.

**14.** The information display method according to claim 13, further comprising:
outputting a third image to the displayer when the second information is changed, the third image including the map in which a display mode of the one or more experimental property values superimposed on the map has been changed.

**15.** The information display method according to claim 14, wherein

the second information includes second coloring information indicating at least one of hue, chroma, or brightness as a color attribute of the at least one experimental property,
in the generating of the map, the map on which the experimental property value of each of the one or more compounds that have been experimented on is superimposed using a color having the color attribute indicated by the second coloring information is generated, and
in the changing of the second information, the color attribute indicated by the second coloring information is changed.

**16.** The information display method according to claim 14 or 15, wherein

the second information includes second display target information indicating one or more property types,
in the generating of the map:

for each of the one or more compounds that have been experimented on, the experimental property value for each of the one or more property types indicated by the second display target information is obtained; and a mark having a form that is in accordance with the experimental property value for each of the one or more property types is superimposed on the map,

in the changing of the second information, the one or more property types indicated by the second display target information is changed.

**17.** The information display method according to any one of claims 14 to 16, wherein

the second information includes display condition information indicating a display condition of the experimental property value, the display condition being based on a threshold,
in the generating of the map, among the one or more experimental values of the one or more compounds that have been experimented on, an experimental property value that satisfies the display condition indicated by the display condition information and an experimental property value that does not satisfy the display condition are superimposed on the map in mutually different forms, and
in the changing of the second information, the display condition indicated by the display condition information is changed by increasing or decreasing the threshold.

**18.** The information display method according to any one of claims 14 to 17, wherein

the generating of the first image includes associating, with the first image, additional information indicating a time point at which the first image was generated, and storing the first image to which the additional information has been associated, as a stored image, in a recording medium,
the generating of the second image includes associating, with the second image, additional information indicating a time point at which the second image was generated, and storing the second image to which the additional information has been associated, as a stored image, in the recording medium,
the information display method further comprises:

obtaining time point information indicating a first time point;
reading out a stored image associated with additional information corresponding to the first time point indicated by the time point information, from the recording medium, as a fourth image, and outputting the fourth image to the displayer; and
when the first time point indicated by the time point information is changed to a second time point, reading out a stored image associated with additional information corresponding to the second time point, from the recording medium, as a fifth image, and outputting the fifth image to the display in place of the fourth image.

**19.** The information display method according to any one of claims 14 to 18, further comprising:

obtaining position information indicating a position of the evaluated property value or the experimental property value on the map that includes a sixth image that is one of the first image, the second image, or the third image; and
changing the sixth image to a seventh image by obtaining composition formula data on a composition formula of a compound corresponding to the position indicated by the position information and superimposing, on the map, a composition image showing the composition formula data.

**20.** An information display method comprising:

obtaining first information for displaying an evaluated property value as a comparison indicator for comparing compounds, the first information including information groups each of which is changeable;
obtaining a map indicating, at a position corresponding to a composition of each of the compounds, the evaluated property value of the compound; and
outputting an image including the map, the image being generated based on a priority level of each of the information groups.

**21.** The information display method according to claim 20, wherein
in the outputting of the image, for each of the information groups, the image is generated by using, as the priority level of the information group, the degree of change in the map caused by a change in the group.

**22.** An information display device comprising:

a display method obtainer that obtains first information for displaying an evaluated property value as a comparison indicator for comparing compounds; and
an image processor that obtains an evaluated property value of each of the compounds based on the first information, generates a map indicating, at a position corresponding to a composition of each of the compounds, the evaluated property value of the compound, generates a first image including the map, and outputs the first image to a displayer, wherein
when the first information is changed by the display method obtainer, the image processor generates a second image different from the first image, and outputs the second image to the displayer.

**23.** A program for causing a computer to execute:

obtaining first information for displaying an evaluated property value as a comparison indicator for comparing compounds;
obtaining an evaluated property value of each of the compounds based on the first information, and generating a map indicating, at a position corresponding to a composition of each of the compounds, the evaluated property value of the compound;
generating a first image including the map, and outputting the first image to a displayer; and
when the first information is changed, generating a second image different from the first image, and outputting the second image to the displayer.

**24.** A program for causing a computer to execute:

obtaining first information for displaying an evaluated property value as a comparison indicator for comparing compounds, the first information including information groups each of which is changeable;
obtaining a map indicating, at a position corresponding to a composition of each of the compounds, the evaluated property value of the compound; and
outputting a first image including the map, the image being generated based on a priority level of each of the information groups.

FIG. 1

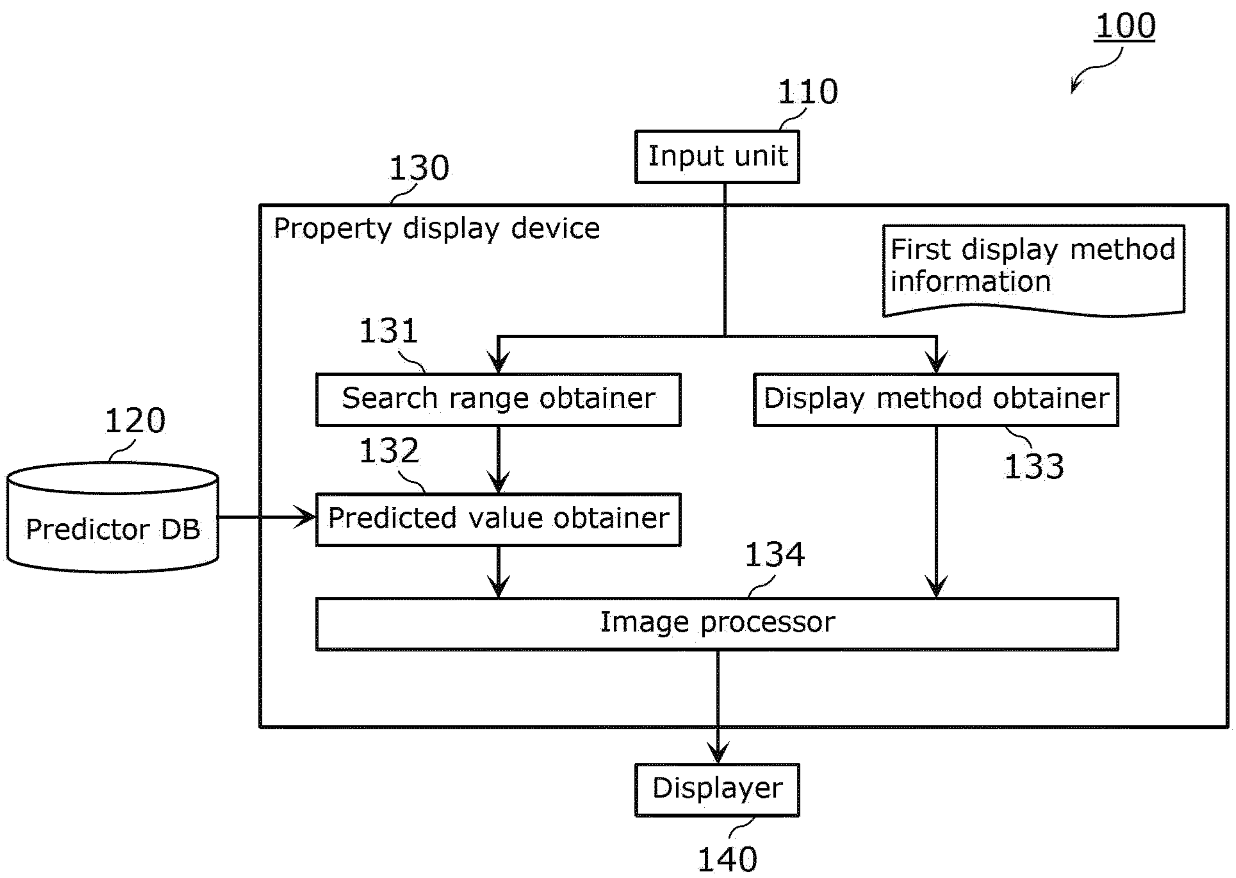
100
110
Input unit
130
Property display device
First display method information
131
Search range obtainer
Display method obtainer
120
132
133
Predictor DB
Predicted value obtainer
134
Image processor
Displayer
140

# FIG. 2

Data representation: $Li_{2-3a-4b}(M3_{1-x}M3'_{x})_{a}(M4_{1-y}M4'_{y})_{1+b}O_{3}$

Range variable

Categorical variable

| Variable name | Option data | Number of possible outcomes |
|---|---|---|
| M3, M3' | La, Al, Ga, In | 6 |
| M4, M4' | Ti, Zr, Hf | 3 |

Discrete variable

| Variable name | Option data | Number of possible outcomes |
|---|---|---|
| a | 0.0, 0.05, 0.1, 0.15, 0.2 | 5 |
| b | 0.0, 0.1, 0.2, 0.3 | 4 |

Continuous variable

| Variable name | Minimum value | Maximum value | Step width | Number of possible outcomes |
|---|---|---|---|---|
| x | 0.0 | 1.0 | 0.1 | 11 |
| y | 0.0 | 1.0 | 0.1 | 11 |

# FIG. 3

| formula | M3 | M3' | M4 | M4' | a | b | x | y |
|---|---|---|---|---|---|---|---|---|
| Li1.45La0.05Ti1.1O3 | La | Al | Ti | Zr | 0.05 | 0.1 | 0 | 0 |
| Li1.45La0.05Zr0.11Ti0.99O3 | La | Al | Ti | Zr | 0.05 | 0.1 | 0 | 0.1 |
| Li1.45La0.05Zr0.22Ti0.88O3 | La | Al | Ti | Zr | 0.05 | 0.1 | 0 | 0.2 |
| Li1.45La0.05Zr0.33Ti0.77O3 | La | Al | Ti | Zr | 0.05 | 0.1 | 0 | 0.3 |
| Li1.45La0.05Zr0.44Ti0.66O3 | La | Al | Ti | Zr | 0.05 | 0.1 | 0 | 0.4 |
| Li1.45La0.05Zr0.55Ti0.55O3 | La | Al | Ti | Zr | 0.05 | 0.1 | 0 | 0.5 |
| Li1.45La0.05Zr0.66Ti0.44O3 | La | Al | Ti | Zr | 0.05 | 0.1 | 0 | 0.6 |
| Li1.45La0.05Zr0.77Ti0.33O3 | La | Al | Ti | Zr | 0.05 | 0.1 | 0 | 0.7 |
| Li1.45La0.05Zr0.88Ti0.22O3 | La | Al | Ti | Zr | 0.05 | 0.1 | 0 | 0.8 |
| Li1.45La0.05Zr0.99Ti0.11O3 | La | Al | Ti | Zr | 0.05 | 0.1 | 0 | 0.9 |
| Li1.45La0.05Zr1.1O3 | La | Al | Ti | Zr | 0.05 | 0.1 | 0 | 1 |
| Li1.45La0.045Ti1.1Al0.005O3 | La | Al | Ti | Zr | 0.05 | 0.1 | 0.1 | 0 |
| Li1.45La0.045Zr0.11Ti0.99Al0.005O3 | La | Al | Ti | Zr | 0.05 | 0.1 | 0.1 | 0.1 |
| Li1.45La0.045Zr0.22Ti0.88Al0.005O3 | La | Al | Ti | Zr | 0.05 | 0.1 | 0.1 | 0.2 |
| Li1.45La0.045Zr0.33Ti0.77Al0.005O3 | La | Al | Ti | Zr | 0.05 | 0.1 | 0.1 | 0.3 |
| Li1.45La0.045Zr0.44Ti0.66Al0.005O3 | La | Al | Ti | Zr | 0.05 | 0.1 | 0.1 | 0.4 |
| Li1.45La0.045Zr0.55Ti0.55Al0.005O3 | La | Al | Ti | Zr | 0.05 | 0.1 | 0.1 | 0.5 |
| Li1.45La0.045Zr0.66Ti0.44Al0.005O3 | La | Al | Ti | Zr | 0.05 | 0.1 | 0.1 | 0.6 |
| Li1.45La0.045Zr0.77Ti0.33Al0.005O3 | La | Al | Ti | Zr | 0.05 | 0.1 | 0.1 | 0.7 |
| Li1.45La0.045Zr0.88Ti0.22Al0.005O3 | La | Al | Ti | Zr | 0.05 | 0.1 | 0.1 | 0.8 |
| Li1.45La0.045Zr0.99Ti0.11Al0.005O3 | La | Al | Ti | Zr | 0.05 | 0.1 | 0.1 | 0.9 |
| Li1.45La0.045Zr1.1Al0.005O3 | La | Al | Ti | Zr | 0.05 | 0.1 | 0.1 | 1 |

FIG. 4

| formula | M3 | M3' | M4 | M4' | a | b | x | y | predict |
|---|---|---|---|---|---|---|---|---|---|
| Li1.45La0.05Ti1.1O3 | La | Al | Ti | Zr | 0.05 | 0.1 | 0 | 0 | 2.350 |
| Li1.45La0.05Zr0.11Ti0.99O3 | La | Al | Ti | Zr | 0.05 | 0.1 | 0 | 0.1 | 2.366 |
| Li1.45La0.05Zr0.22Ti0.88O3 | La | Al | Ti | Zr | 0.05 | 0.1 | 0 | 0.2 | 2.437 |
| Li1.45La0.05Zr0.33Ti0.77O3 | La | Al | Ti | Zr | 0.05 | 0.1 | 0 | 0.3 | 2.565 |
| Li1.45La0.05Zr0.44Ti0.66O3 | La | Al | Ti | Zr | 0.05 | 0.1 | 0 | 0.4 | 2.599 |
| Li1.45La0.05Zr0.55Ti0.55O3 | La | Al | Ti | Zr | 0.05 | 0.1 | 0 | 0.5 | 2.573 |
| Li1.45La0.05Zr0.66Ti0.44O3 | La | Al | Ti | Zr | 0.05 | 0.1 | 0 | 0.6 | 2.561 |
| Li1.45La0.05Zr0.77Ti0.33O3 | La | Al | Ti | Zr | 0.05 | 0.1 | 0 | 0.7 | 2.622 |
| Li1.45La0.05Zr0.88Ti0.22O3 | La | Al | Ti | Zr | 0.05 | 0.1 | 0 | 0.8 | 2.586 |
| Li1.45La0.05Zr0.99Ti0.11O3 | La | Al | Ti | Zr | 0.05 | 0.1 | 0 | 0.9 | 2.639 |
| Li1.45La0.05Zr1.1O3 | La | Al | Ti | Zr | 0.05 | 0.1 | 0 | 1 | 2.619 |
| Li1.45La0.045Ti1.1Al0.005O3 | La | Al | Ti | Zr | 0.05 | 0.1 | 0.1 | 0 | 2.349 |
| Li1.45La0.045Zr0.11Ti0.99Al0.005O3 | La | Al | Ti | Zr | 0.05 | 0.1 | 0.1 | 0.1 | 2.371 |
| Li1.45La0.045Zr0.22Ti0.88Al0.005O3 | La | Al | Ti | Zr | 0.05 | 0.1 | 0.1 | 0.2 | 2.462 |
| Li1.45La0.045Zr0.33Ti0.77Al0.005O3 | La | Al | Ti | Zr | 0.05 | 0.1 | 0.1 | 0.3 | 2.558 |
| Li1.45La0.045Zr0.44Ti0.66Al0.005O3 | La | Al | Ti | Zr | 0.05 | 0.1 | 0.1 | 0.4 | 2.607 |
| Li1.45La0.045Zr0.55Ti0.55Al0.005O3 | La | Al | Ti | Zr | 0.05 | 0.1 | 0.1 | 0.5 | 2.578 |
| Li1.45La0.045Zr0.66Ti0.44Al0.005O3 | La | Al | Ti | Zr | 0.05 | 0.1 | 0.1 | 0.6 | 2.559 |
| Li1.45La0.045Zr0.77Ti0.33Al0.005O3 | La | Al | Ti | Zr | 0.05 | 0.1 | 0.1 | 0.7 | 2.612 |
| Li1.45La0.045Zr0.88Ti0.22Al0.005O3 | La | Al | Ti | Zr | 0.05 | 0.1 | 0.1 | 0.8 | 2.572 |
| Li1.45La0.045Zr0.99Ti0.11Al0.005O3 | La | Al | Ti | Zr | 0.05 | 0.1 | 0.1 | 0.9 | 2.621 |
| Li1.45La0.045Zr1.1Al0.005O3 | La | Al | Ti | Zr | 0.05 | 0.1 | 0.1 | 1 | 2.594 |

# FIG. 5A

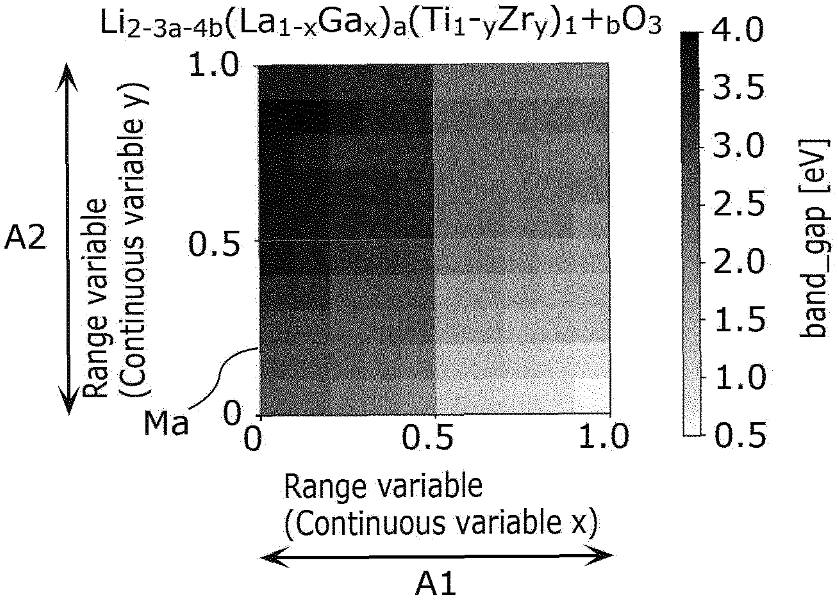
Li2-3a-4b(La1-xGax)a(Ti1-yZry)1+bO3
A2
Range variable
(Continuous variable y)
Ma
1.0
0.5
0
0
0.5
1.0
Range variable
(Continuous variable x)
A1
4.0
3.5
3.0
2.5
2.0
1.5
1.0
0.5
band_gap [eV]

# FIG. 5B

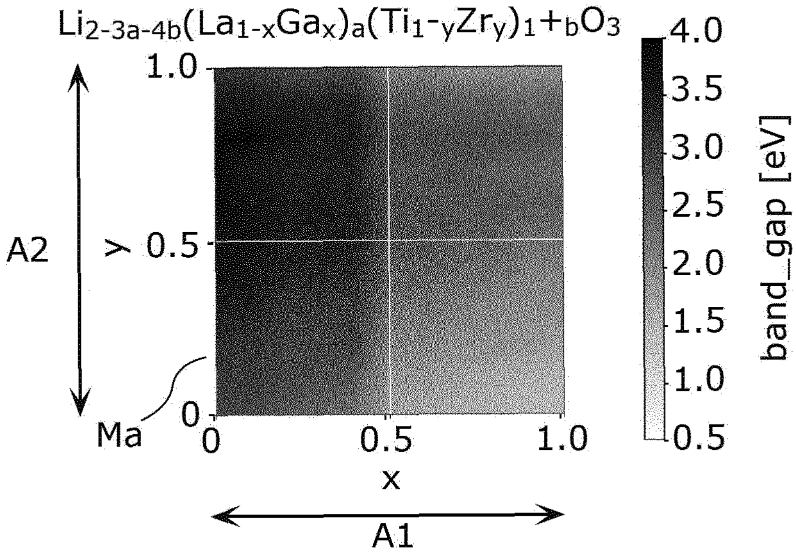
Li2-3a-4b(La1-xGax)a(Ti1-yZry)1+bO3
A2
y
Ma
1.0
0.5
0
0
0.5
1.0
x
A1
4.0
3.5
3.0
2.5
2.0
1.5
1.0
0.5
band_gap [eV]

FIG. 5C

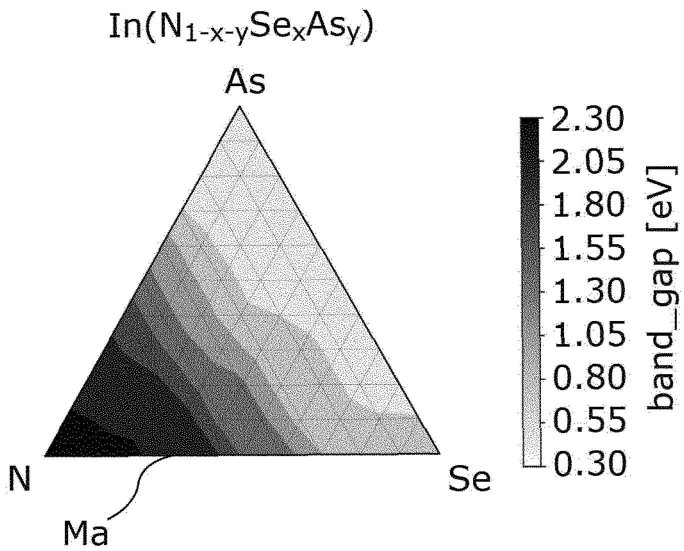
In(N1-x-ySexAsy)
As
N
Se
Ma
2.30
2.05
1.80
1.55
1.30
1.05
0.80
0.55
0.30
band_gap [eV]

FIG. 6

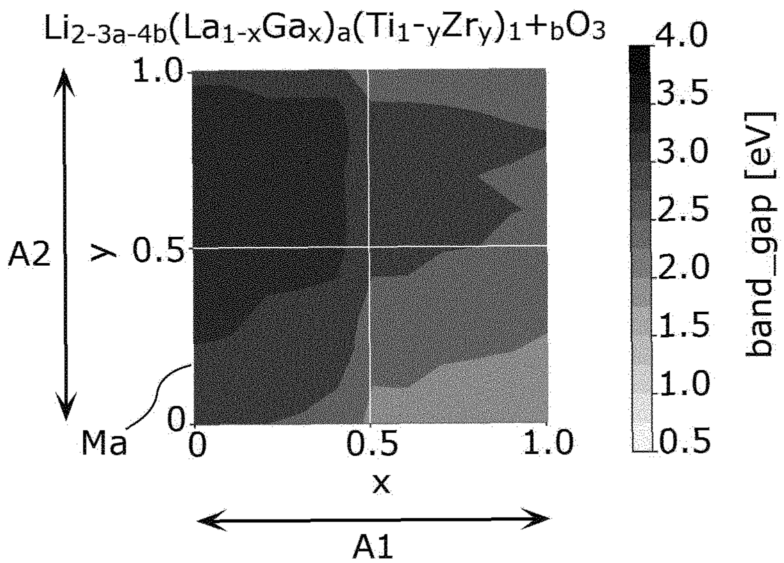
Li2-3a-4b(La1-xGax)a(Ti1-yZry)1+bO3
1.0
y 0.5
0
A2
Ma
0
0.5
1.0
x
A1
4.0
3.5
3.0
2.5
2.0
1.5
1.0
0.5
band_gap [eV]

FIG. 7A

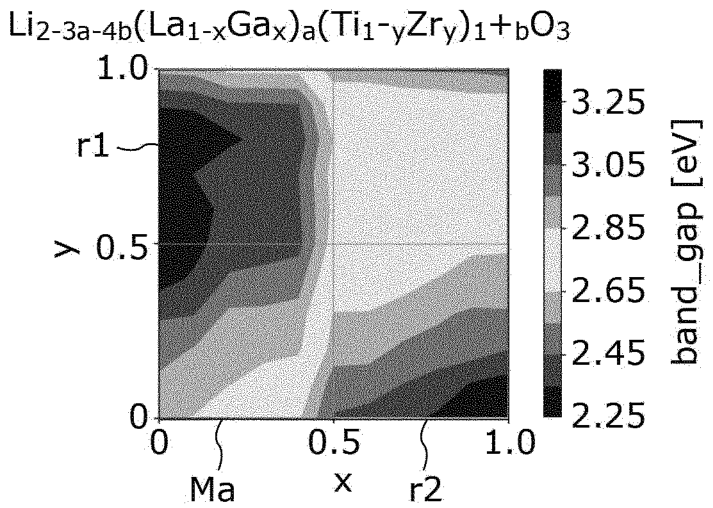
Li2-3a-4b(La1-xGax)a(Ti1-yZry)1+bO3
1.0
r1
y 0.5
0
0
Ma
0.5
x
r2
1.0
3.25
3.05
2.85
2.65
2.45
2.25
band_gap [eV]

FIG. 7B

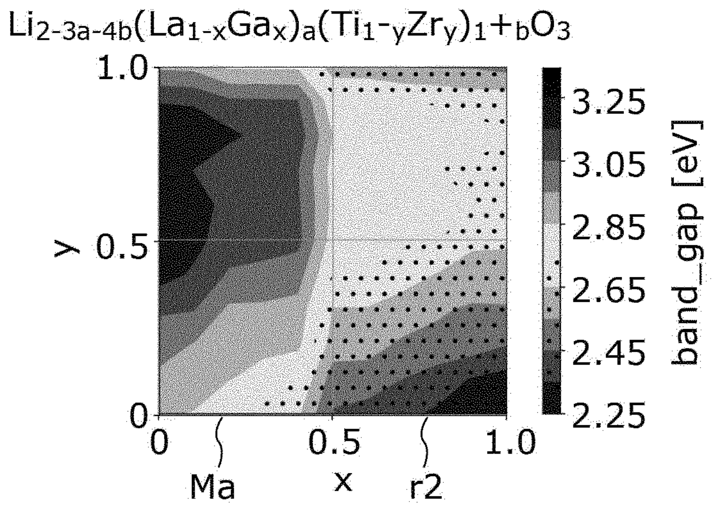
Li2-3a-4b(La1-xGax)a(Ti1-yZry)1+bO3
1.0
y 0.5
0
0
Ma
0.5
x
r2
1.0
3.25
3.05
2.85
2.65
2.45
2.25
band_gap [eV]

FIG. 7C

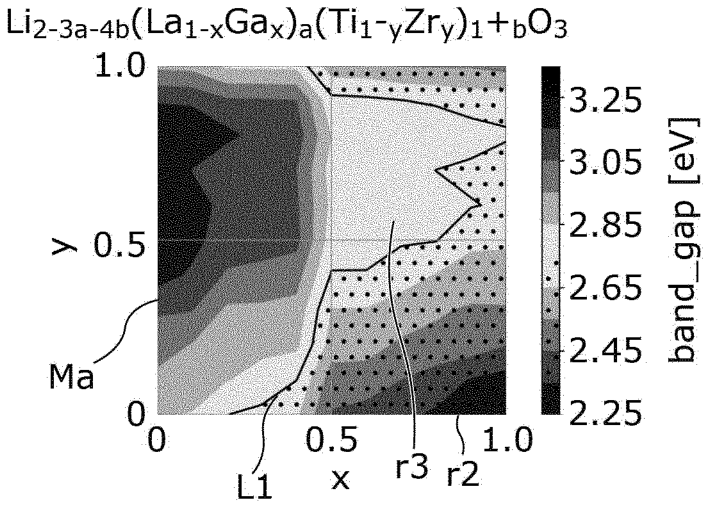
$Li_{2-3a-4b}(La_{1-x}Ga_x)_a(Ti_{1-y}Zr_y)_{1+b}O_3$
1.0
y 0.5
0
Ma
0
0.5
1.0
x
L1
r3
r2
3.25
3.05
2.85
2.65
2.45
2.25
band_gap [eV]

FIG. 7D

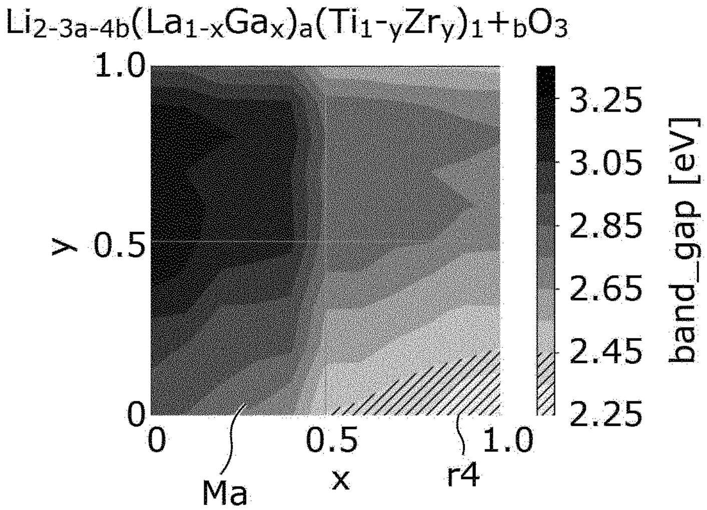
$Li_{2-3a-4b}(La_{1-x}Ga_x)_a(Ti_{1-y}Zr_y)_{1+b}O_3$
1.0
y 0.5
0
0
0.5
1.0
x
Ma
r4
3.25
3.05
2.85
2.65
2.45
2.25
band_gap [eV]

FIG. 8

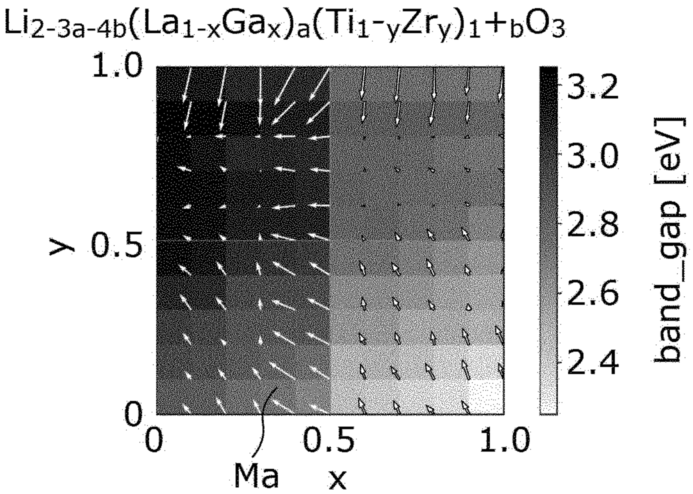
Li2-3a-4b(La1-xGax)a(Ti1-yZry)1+bO3
1.0
y 0.5
0
0
0.5
1.0
Ma
x
3.2
3.0
2.8
2.6
2.4
band_gap [eV]

FIG. 9A

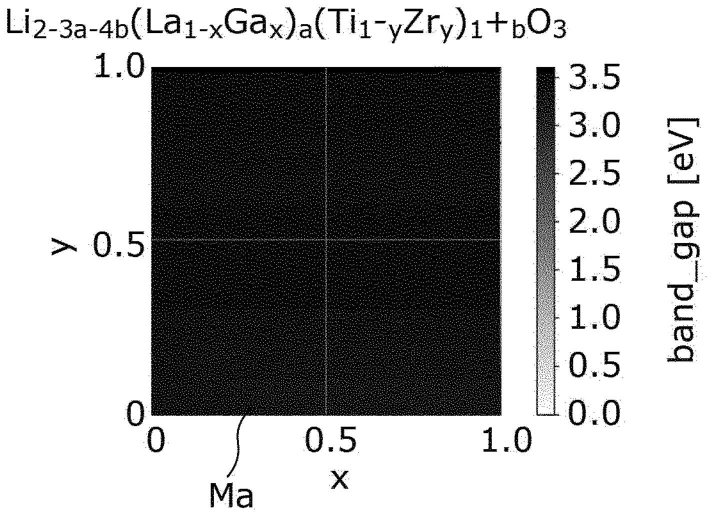


Predicted property value = best value when changing option data of each variable other than x and y

FIG. 9B

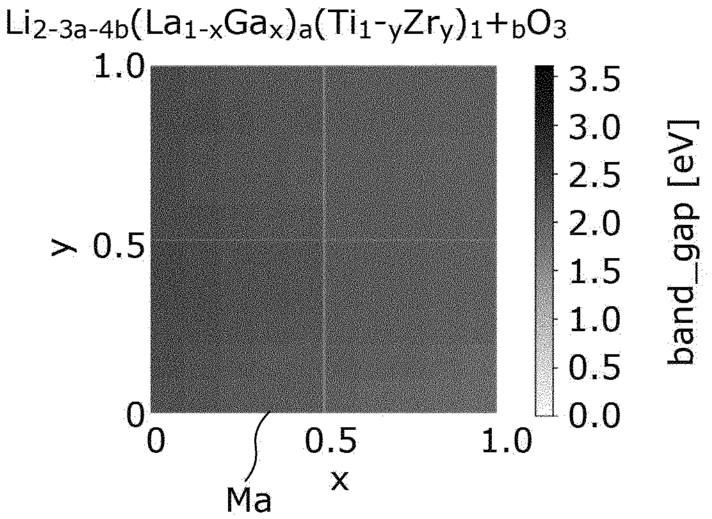


Predicted property value = mean in range of a ∈ {0.0, 0.05, 0.1} and b ∈ {0.0, 0.1, 0.2}

FIG. 10

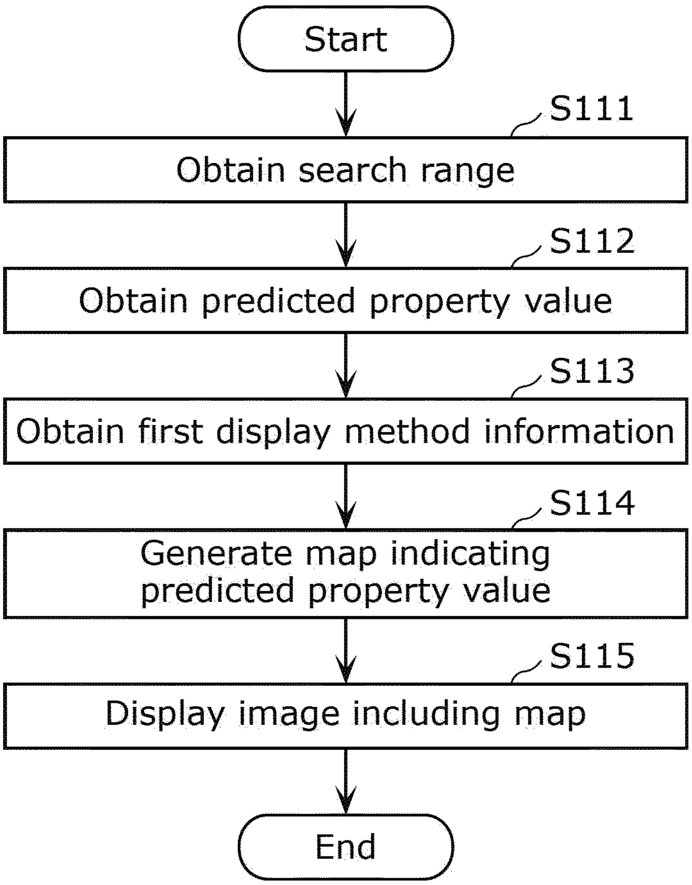
Start
S111
Obtain search range
S112
Obtain predicted property value
S113
Obtain first display method information
S114
Generate map indicating predicted property value
S115
Display image including map
End

FIG. 11

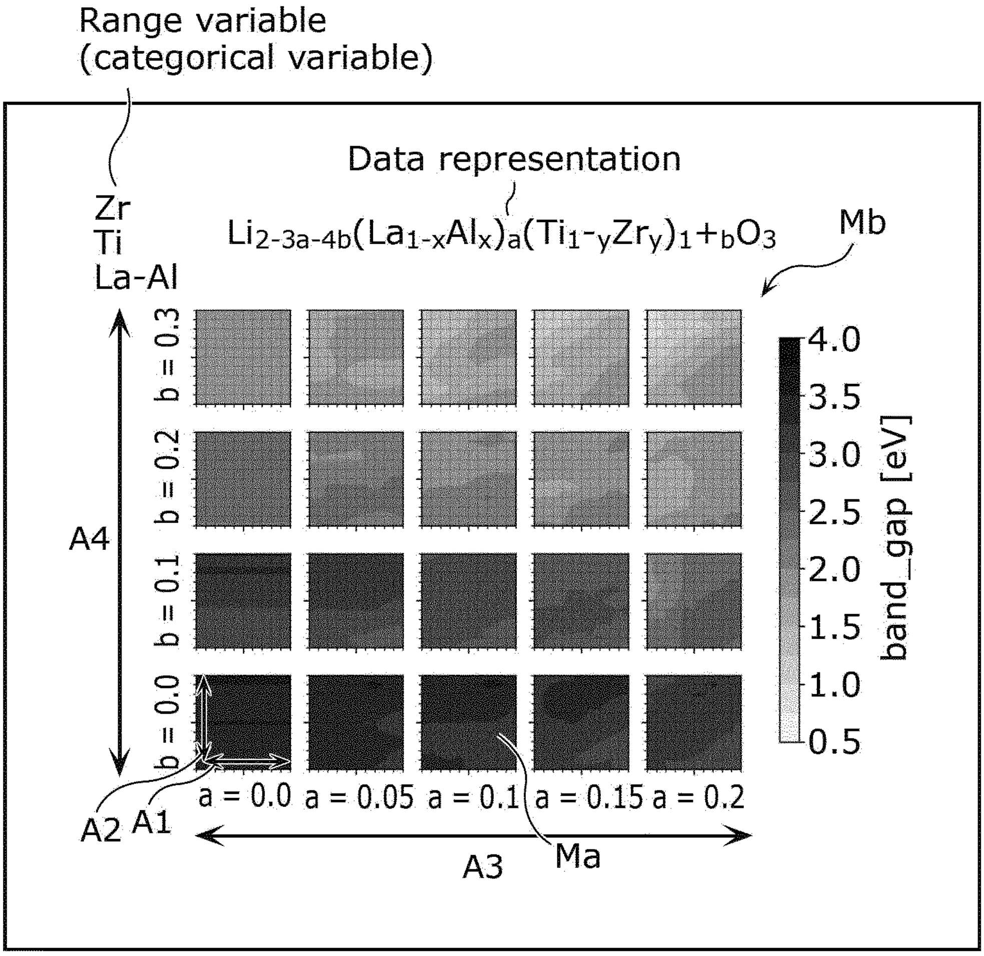


A1: Range variable (continuous variable x)
A2: Range variable (continuous variable y)
A3: Range variable (discrete variable a)
A4: Range variable (discrete variable b)

# FIG. 12

Processing variable (processing condition)

Range variable

| Variable name | Option data | Number of possible outcomes |
|---|---|---|
| Pa (calcination method) | Solid reaction method, ball mill | 2 |
| Pb (calcination time (h)) | 1, 2, 3 | 3 |
| Pc (calcination temperature (°C)) | 100,110,120,130,140,150,160 | 7 |

FIG. 13

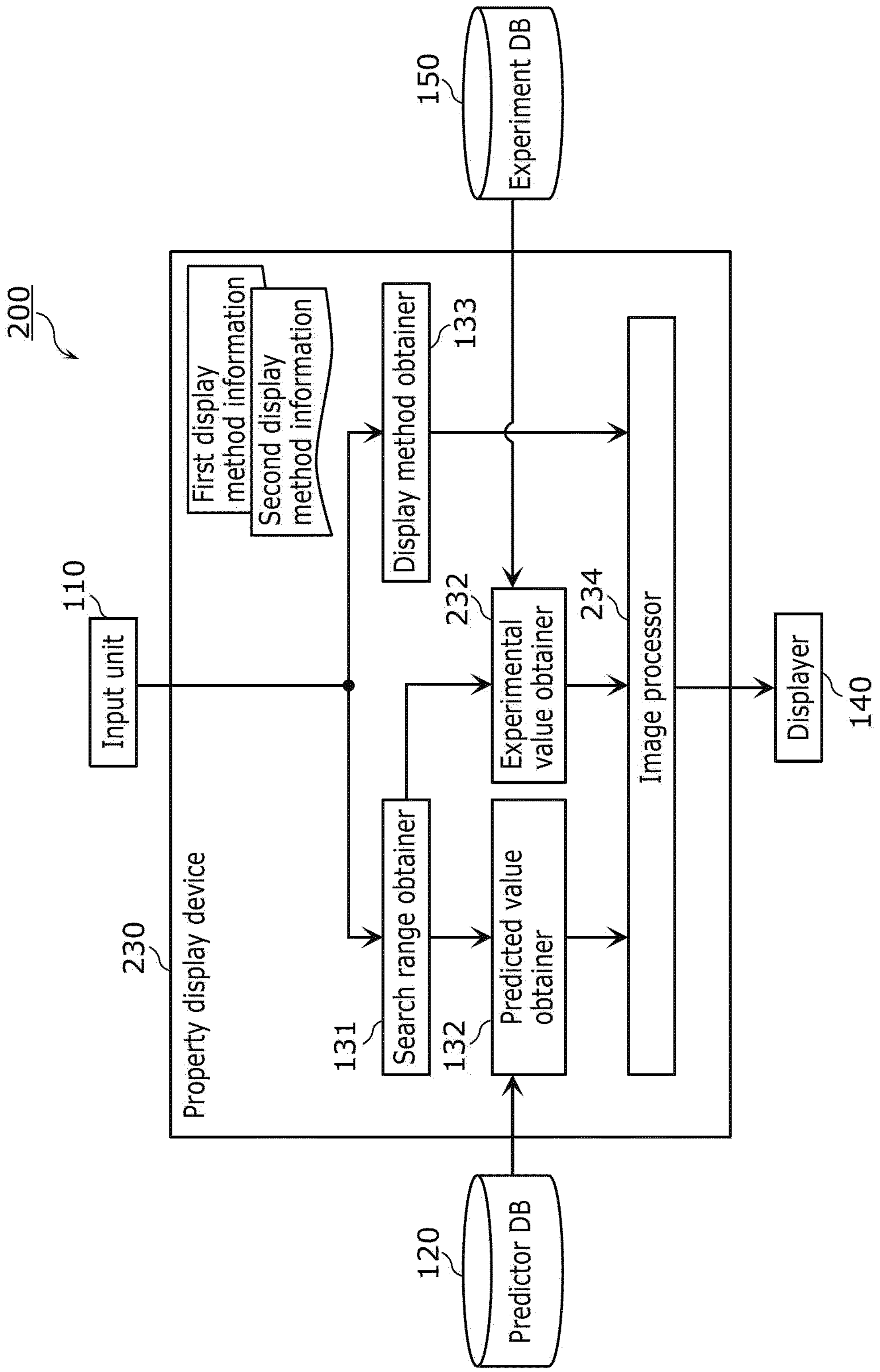
200
110
Input unit
230
Property display device
First display method information
Second display method information
Display method obtainer
133
131
Search range obtainer
132
Predicted value obtainer
232
Experimental value obtainer
234
Image processor
Displayer
140
150
Experiment DB
120
Predictor DB

FIG. 14

Experimental data

| formula | ID | exp.data |
|---|---|---|
| Li1.45La0.045Ti1.1Al0.005O3 | 000001-00001-001 | 2.349 |
| Li1.45La0.045Zr0.22Ti0.88Al0.005O3 | 000002-00001-001 | 2.462 |
| Li1.45La0.045Zr0.55Ti0.55Al0.005O3 | 000003-00001-001 | 2.578 |
| Li1.45La0.045Zr0.99Ti0.11Al0.005O3 | 000004-00001-001 | 2.621 |
| Li1.45La0.045Zr1.1Al0.005O3 | 000005-00001-001 | 2.594 |

FIG. 15

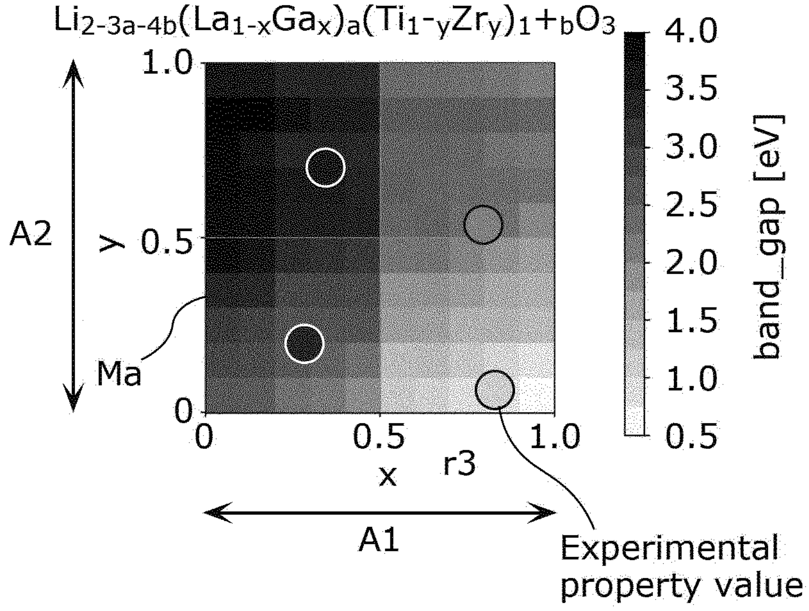
Li2-3a-4b(La1-xGax)a(Ti1-yZry)1+bO3
1.0
y 0.5
0
0
0.5
1.0
x
r3
A1
A2
Ma
4.0
3.5
3.0
2.5
2.0
1.5
1.0
0.5
band_gap [eV]
Experimental property value

FIG. 16

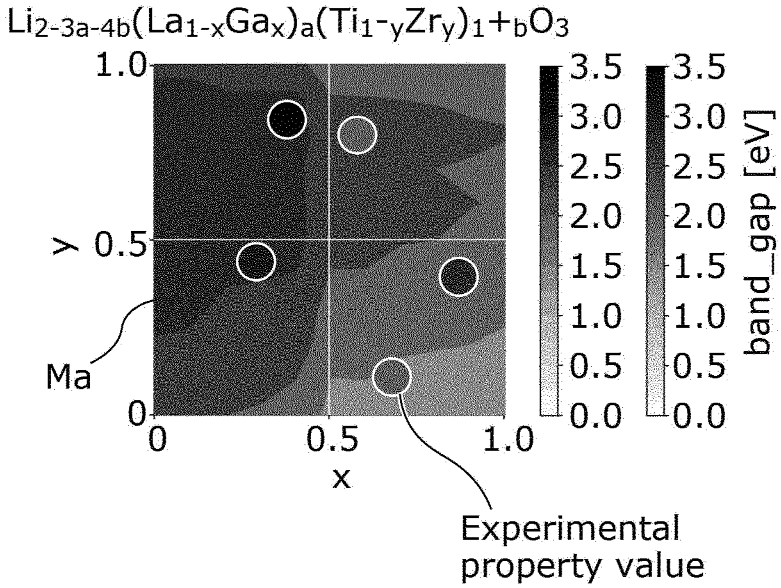
Li2-3a-4b(La1-xGax)a(Ti1-yZry)1+bO3
1.0
y 0.5
0
0
0.5
1.0
x
Ma
3.5
3.0
2.5
2.0
1.5
1.0
0.5
0.0
3.5
3.0
2.5
2.0
1.5
1.0
0.5
0.0
band_gap [eV]
Experimental property value

FIG. 17

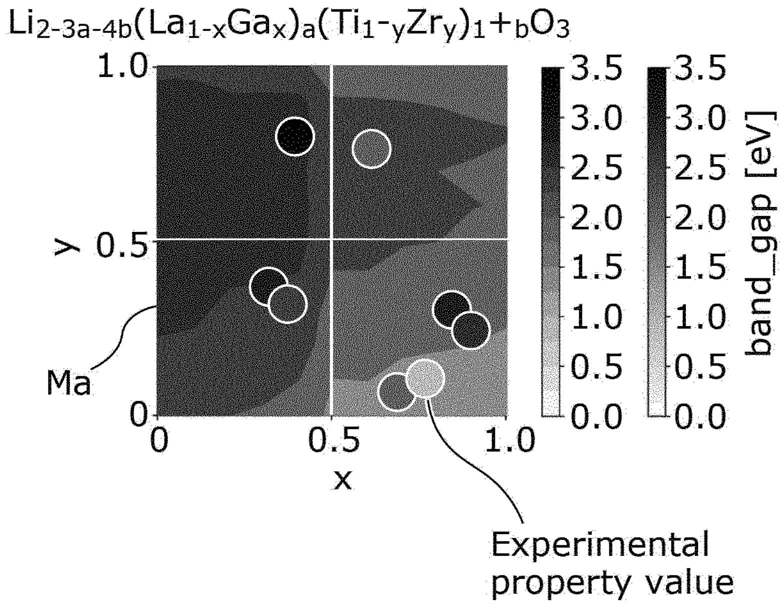
Li2-3a-4b(La1-xGax)a(Ti1-yZry)1+bO3
1.0
y 0.5
0
0
0.5
1.0
x
Ma
3.5
3.0
2.5
2.0
1.5
1.0
0.5
0.0
band_gap [eV]
Experimental property value

FIG. 18

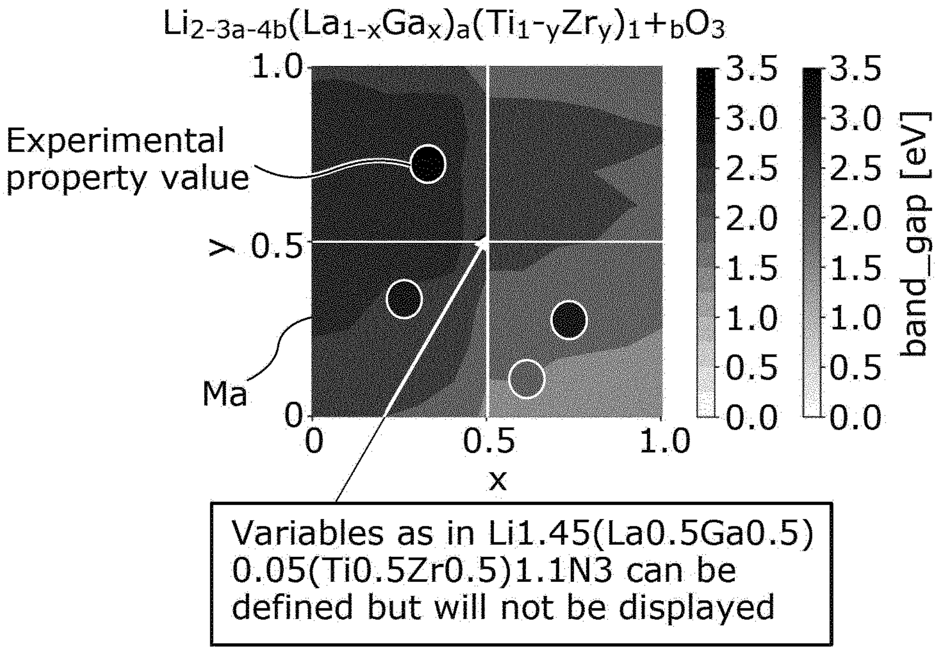
Li2-3a-4b(La1-xGax)a(Ti1-yZry)1+bO3
1.0
y 0.5
0
0
0.5
1.0
x
Ma
Experimental property value
3.5
3.0
2.5
2.0
1.5
1.0
0.5
0.0
band_gap [eV]
Variables as in Li1.45(La0.5Ga0.5) 0.05(Ti0.5Zr0.5)1.1N3 can be defined but will not be displayed

FIG. 19

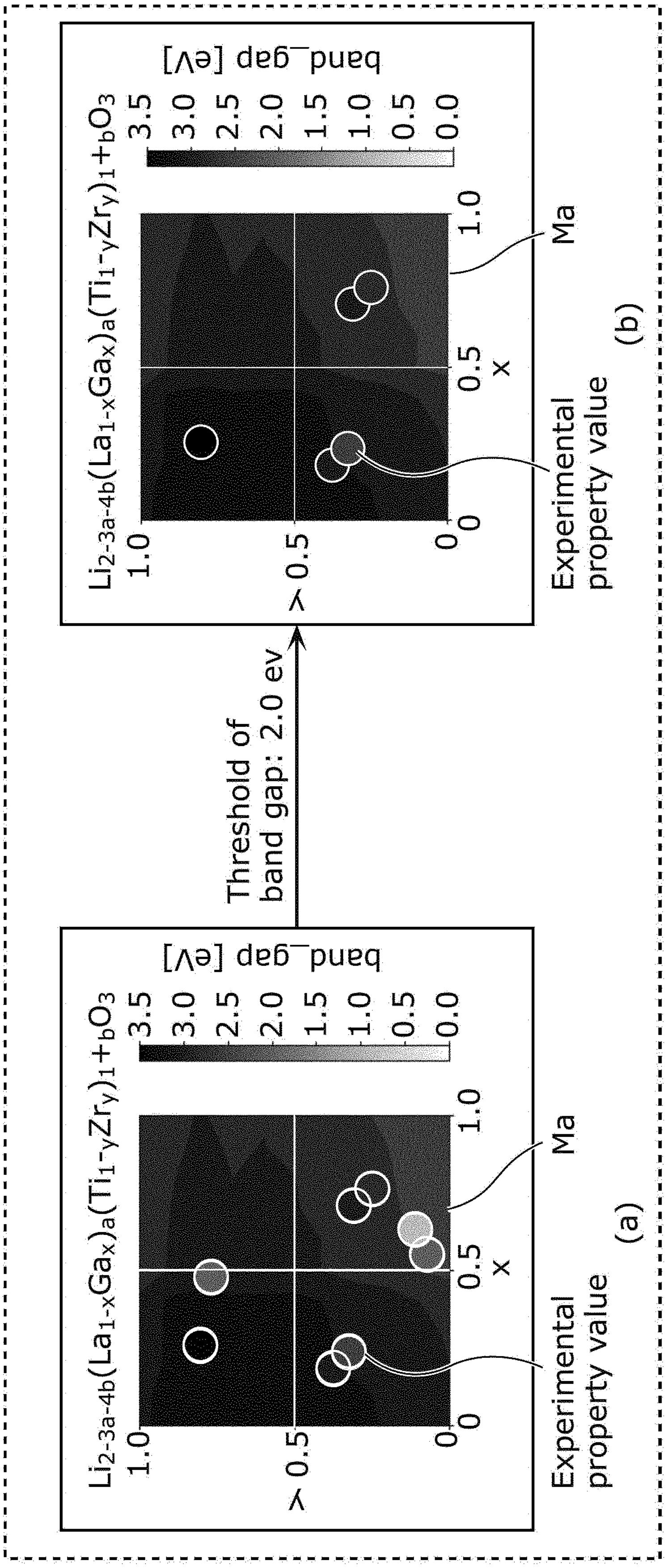
$Li_{2-3a-4b}(La_{1-x}Ga_x)_a(Ti_{1-y}Zr_y)_{1+b}O_3$
band_gap [eV]
3.5
3.0
2.5
2.0
1.5
1.0
0.5
0.0
y
1.0
0.5
0
x
Ma
Experimental property value
(a)
Threshold of band gap: 2.0 ev
$Li_{2-3a-4b}(La_{1-x}Ga_x)_a(Ti_{1-y}Zr_y)_{1+b}O_3$
band_gap [eV]
Ma
Experimental property value
(b)

FIG. 20A

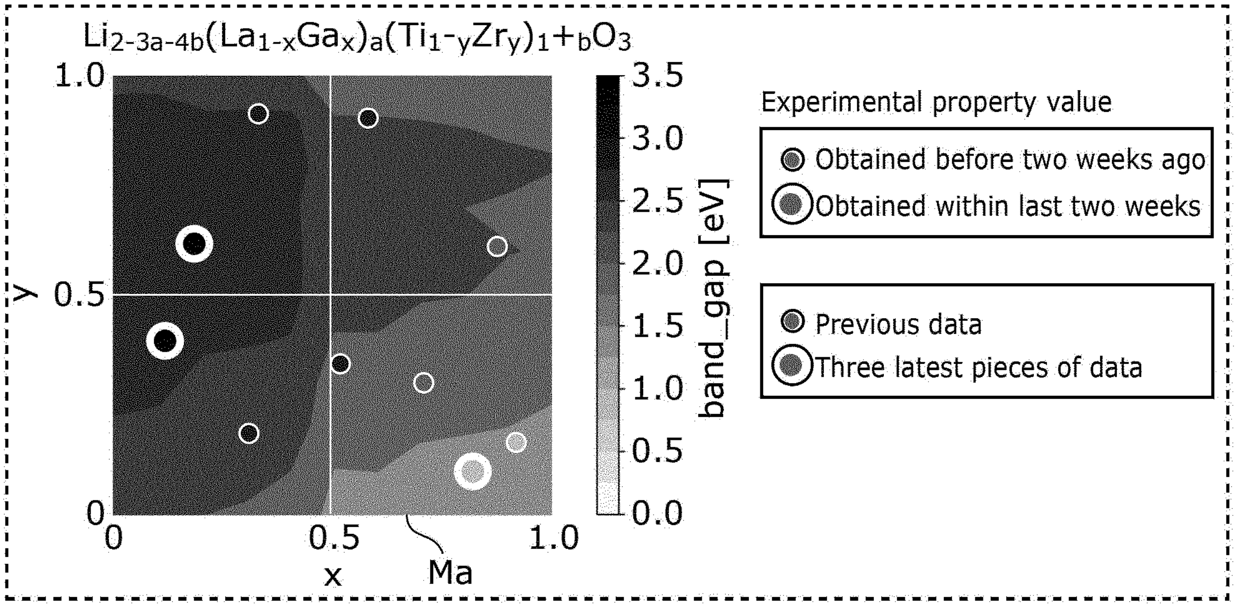


FIG. 20B

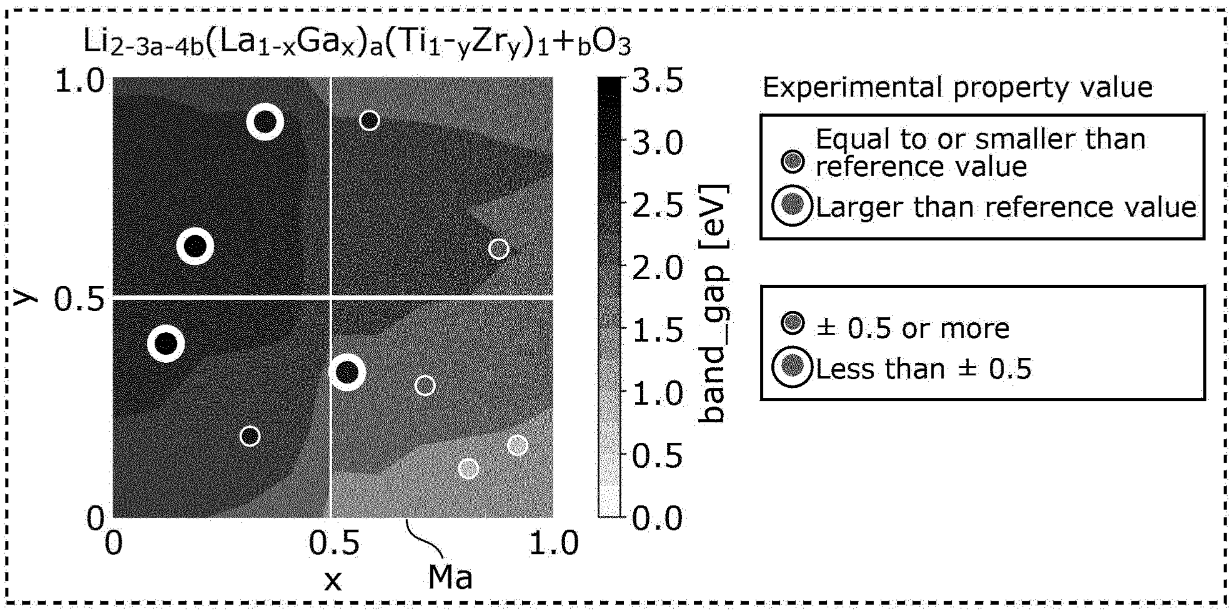

FIG. 21

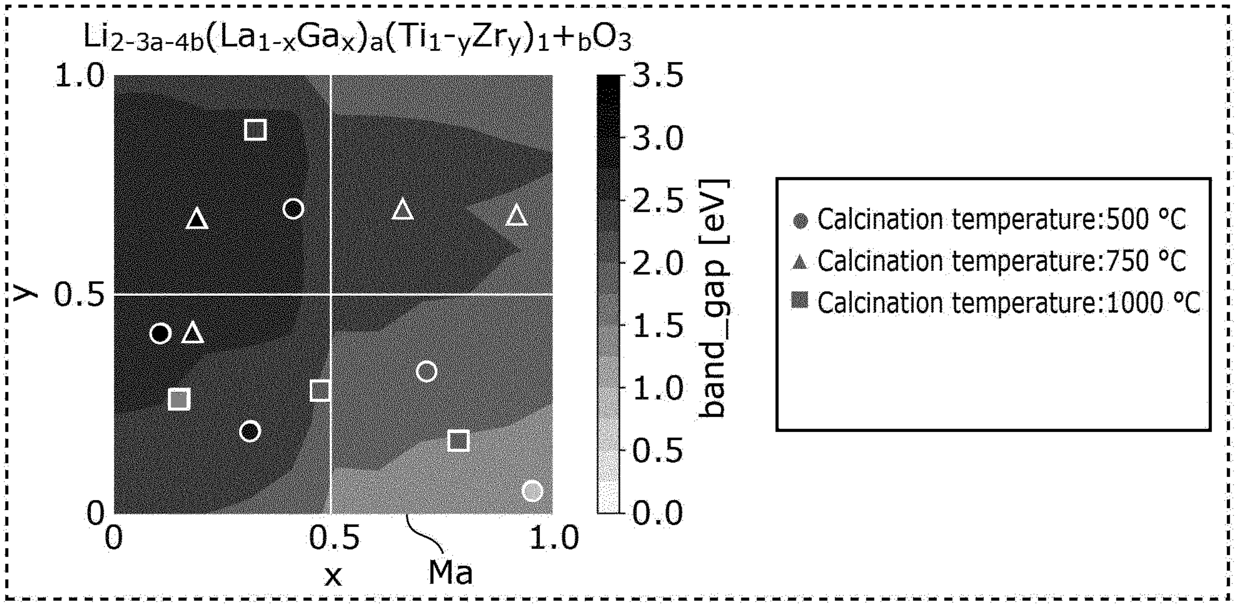


FIG. 22A

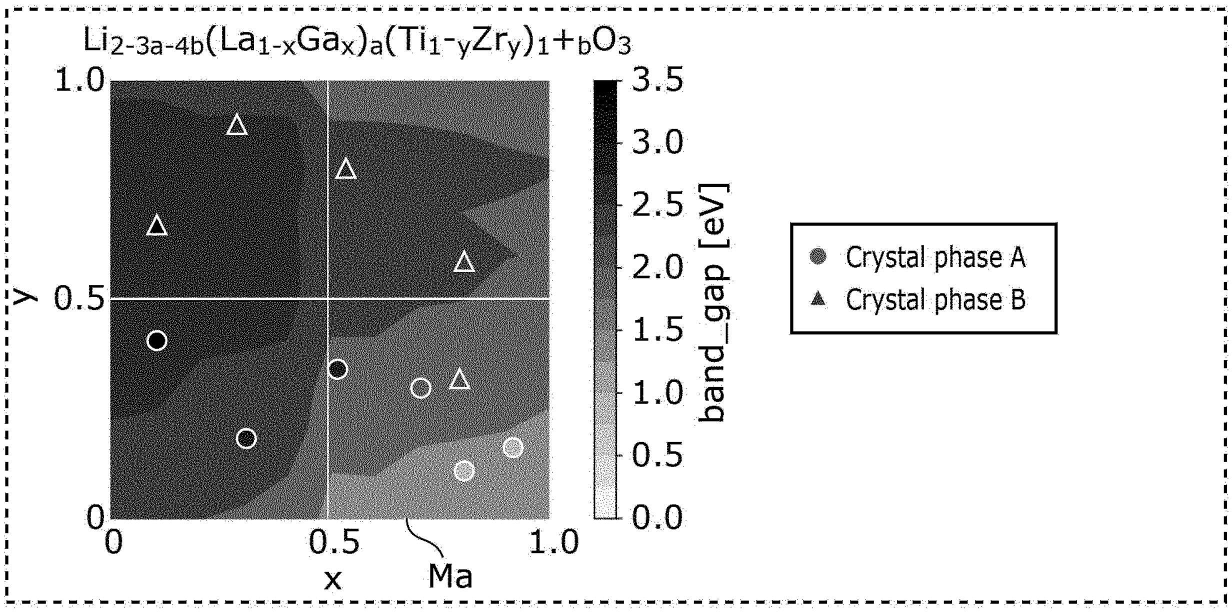

FIG. 22B

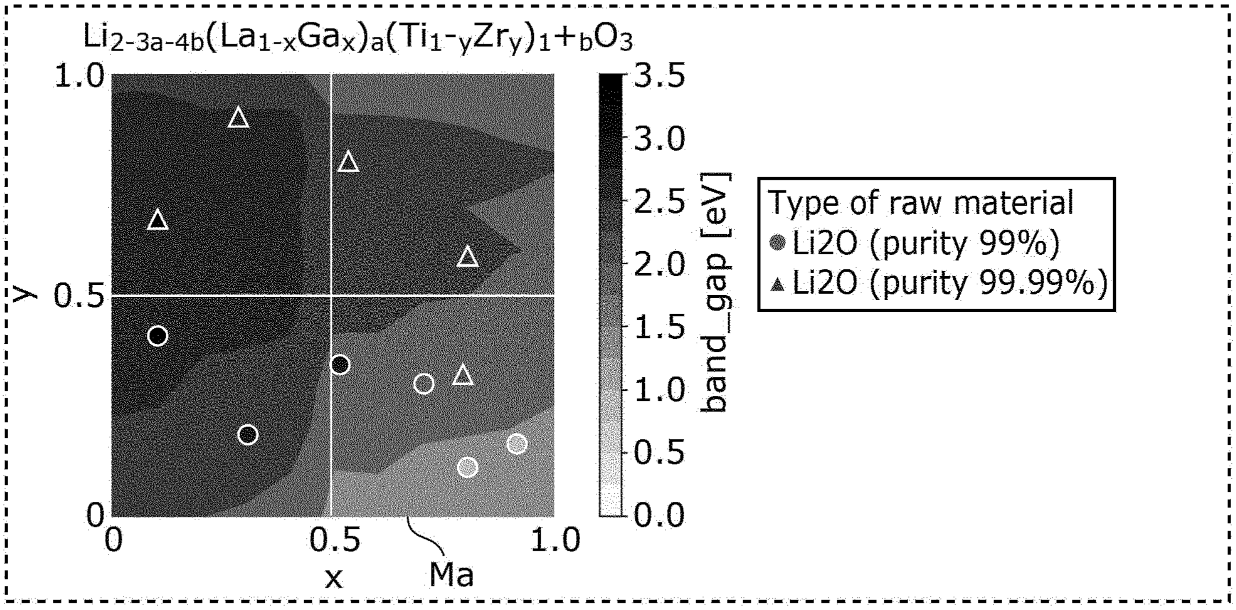


FIG. 22C

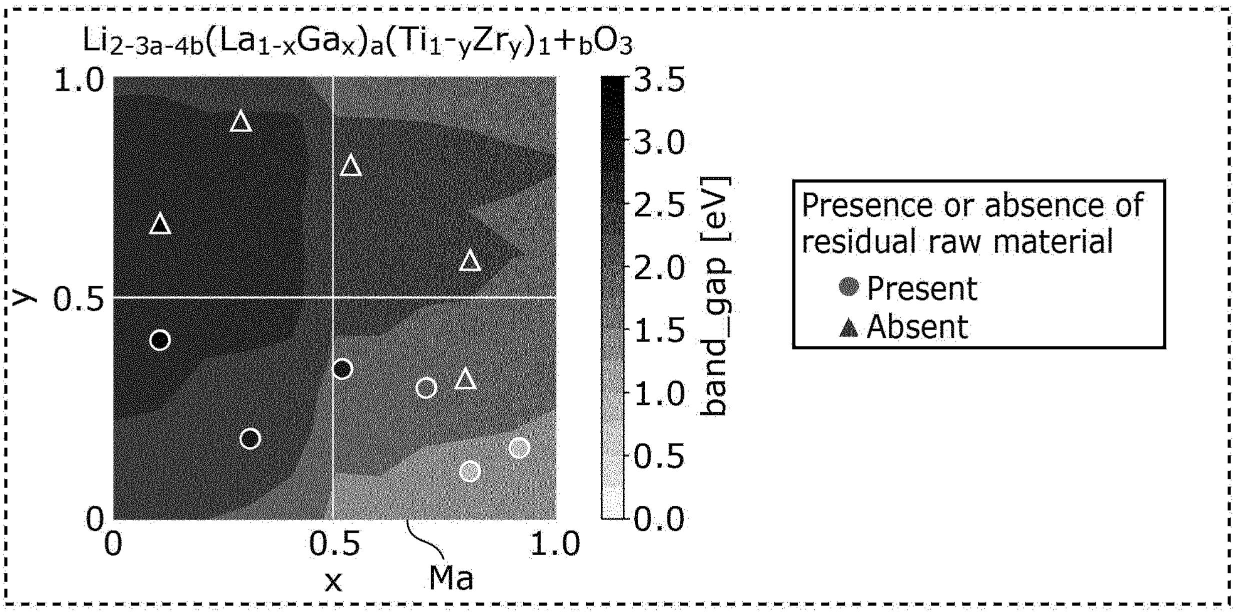

FIG. 23

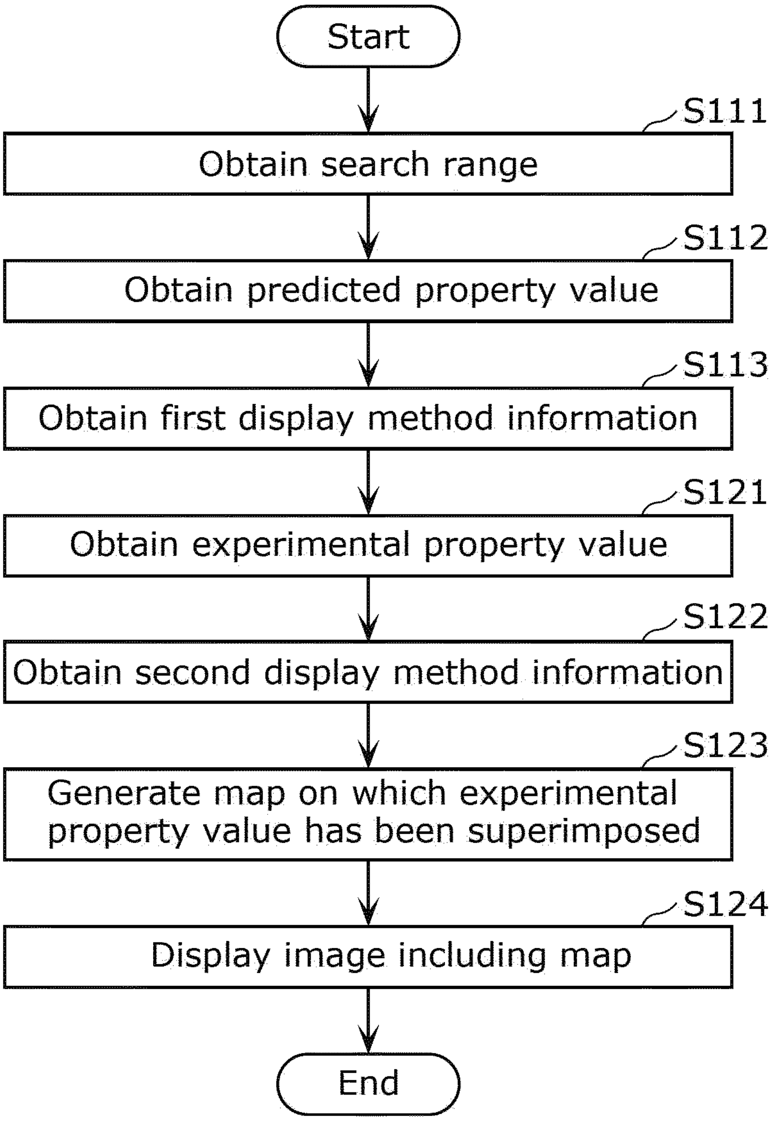
Start
S111
Obtain search range
S112
Obtain predicted property value
S113
Obtain first display method information
S121
Obtain experimental property value
S122
Obtain second display method information
S123
Generate map on which experimental property value has been superimposed
S124
Display image including map
End

FIG. 24A

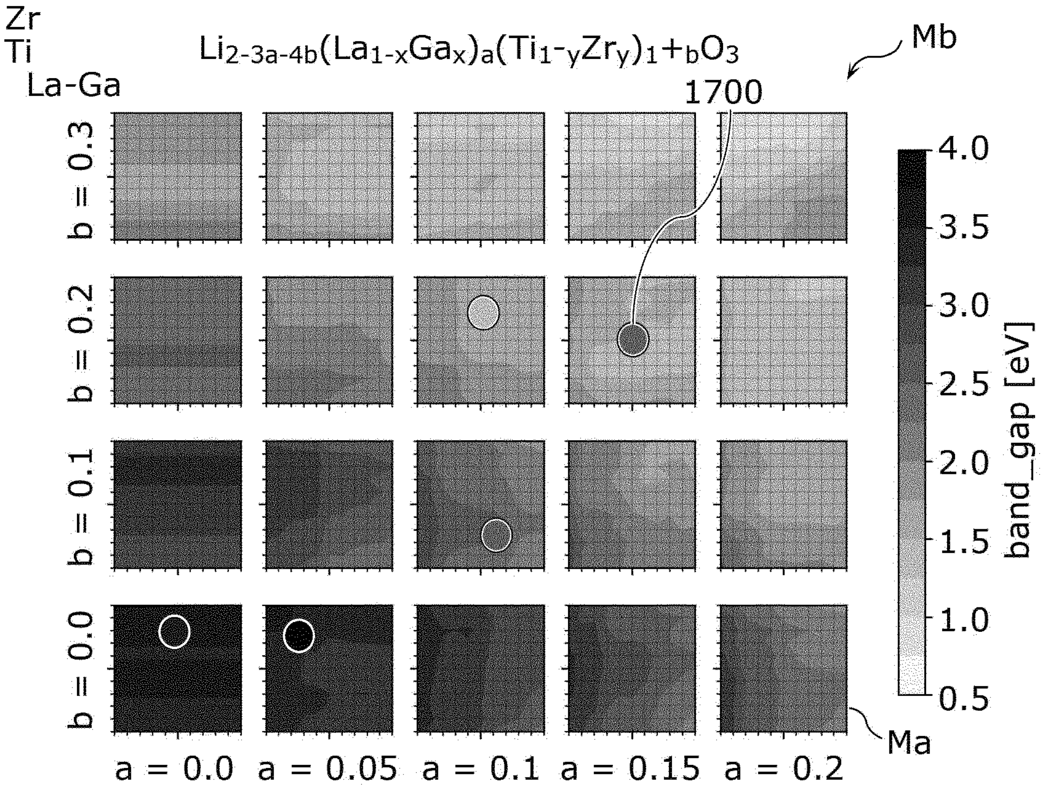


FIG. 24B

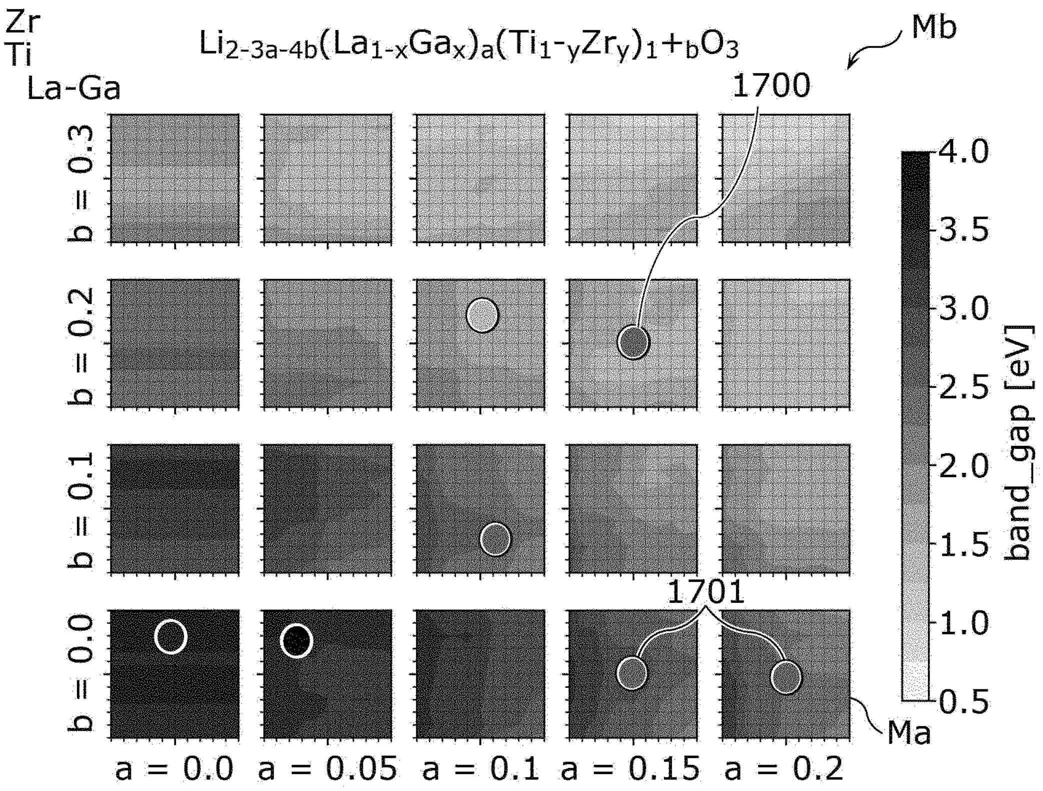

FIG. 25

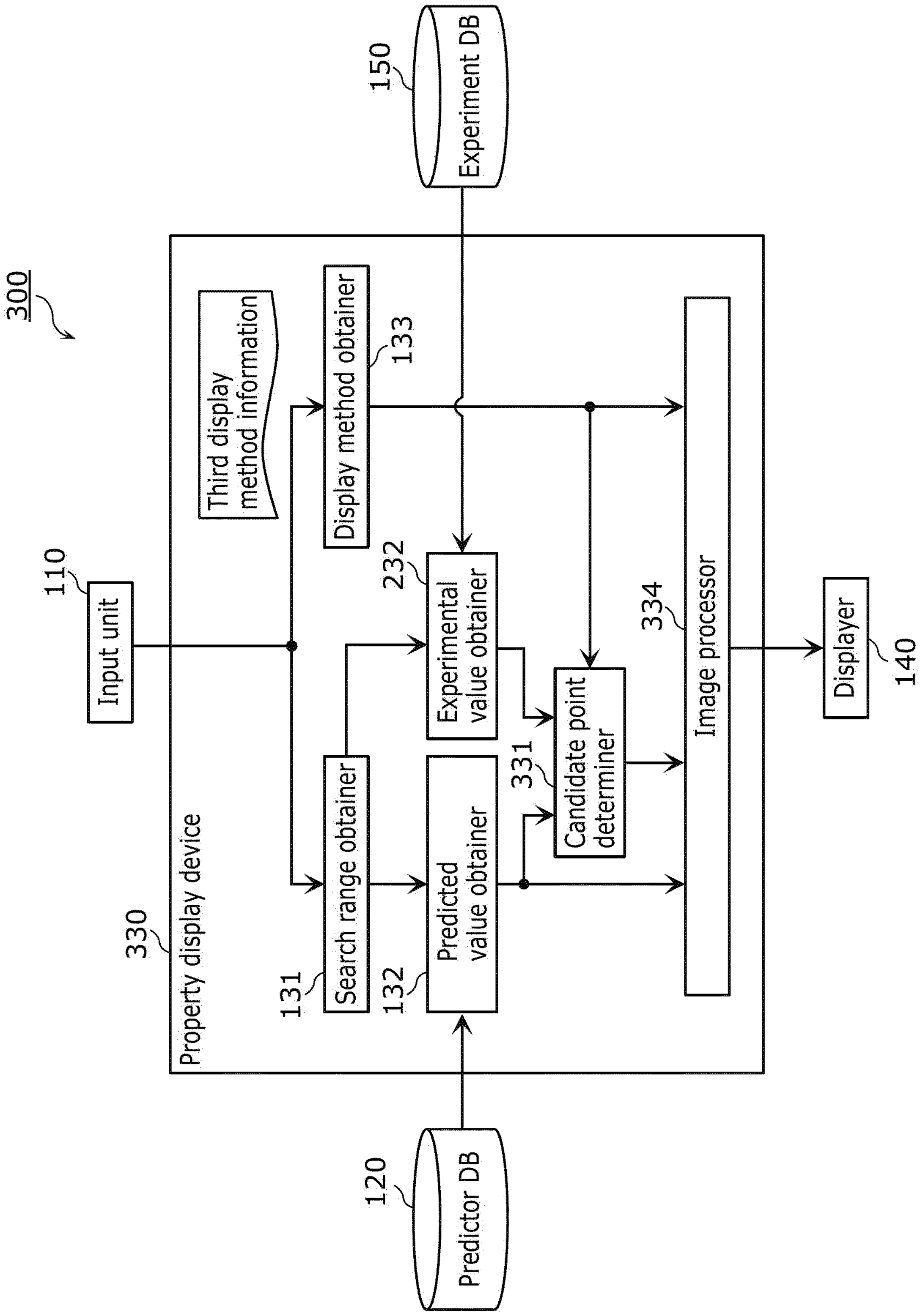
300
110
Input unit
330
Property display device
Third display method information
133
Display method obtainer
131
Search range obtainer
132
Predicted value obtainer
232
Experimental value obtainer
331
Candidate point determiner
334
Image processor
Displayer
140
120
Predictor DB
150
Experiment DB

FIG. 26

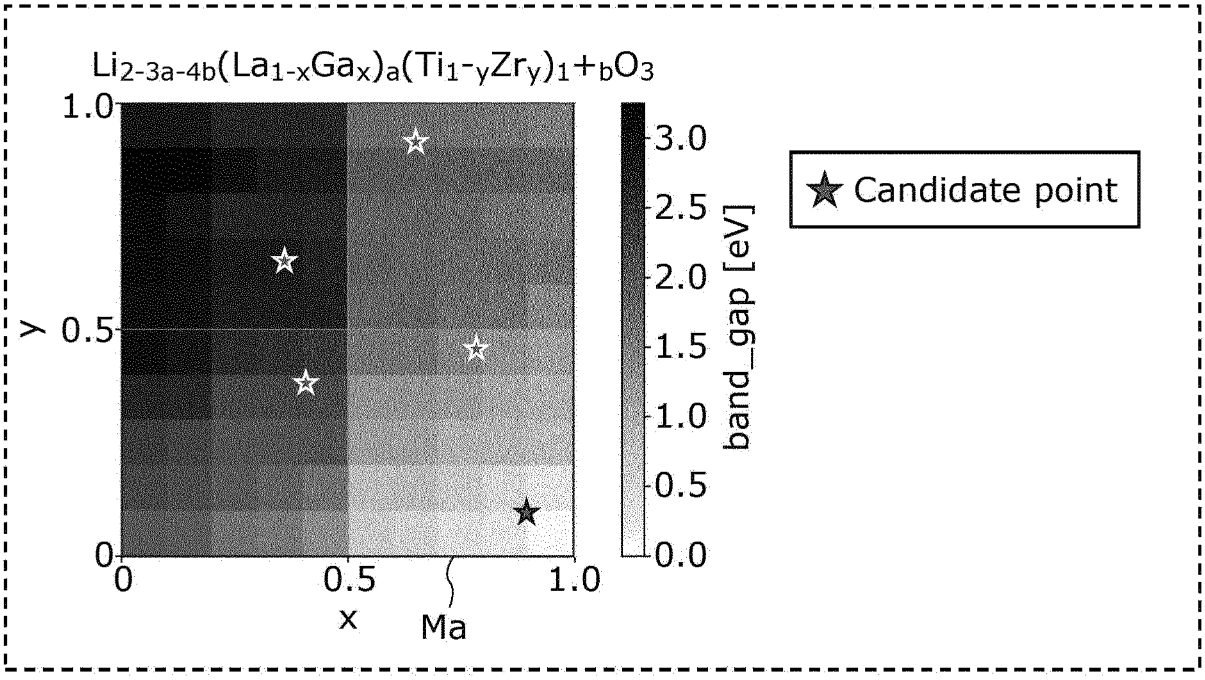

$Li_{2-3a-4b}(La_{1-x}Ga_x)_a(Ti_{1-y}Zr_y)_{1+b}O_3$
1.0
y 0.5
0
0
0.5
1.0
x
Ma
3.0
2.5
2.0
1.5
1.0
0.5
0.0
band_gap [eV]
Candidate point


FIG. 27

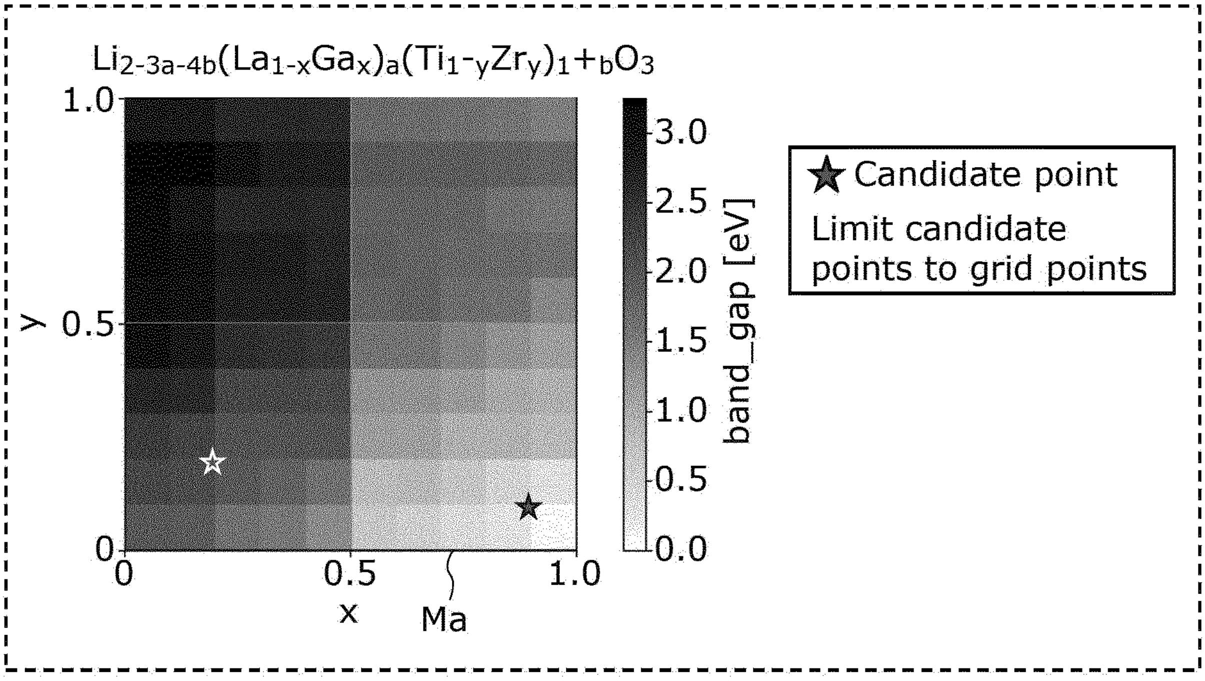

$Li_{2-3a-4b}(La_{1-x}Ga_x)_a(Ti_{1-y}Zr_y)_{1+b}O_3$
1.0
y 0.5
0
0
0.5
1.0
x
Ma
3.0
2.5
2.0
1.5
1.0
0.5
0.0
band_gap [eV]
Candidate point
Limit candidate points to grid points

FIG. 28

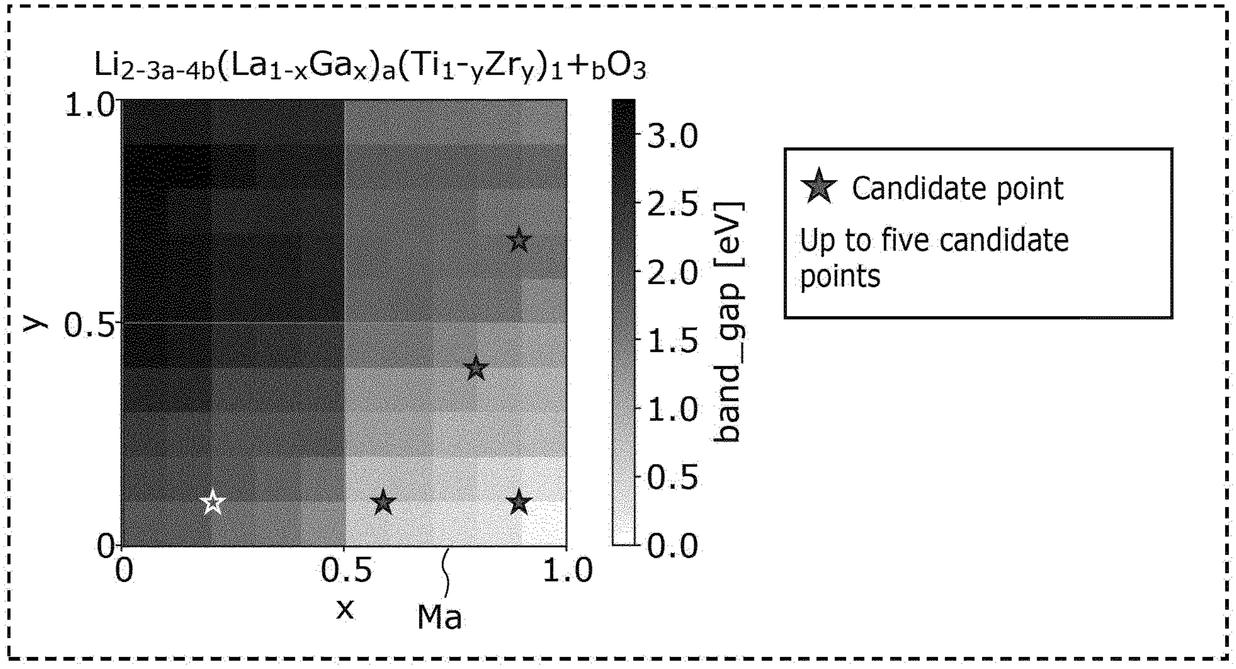
Li2-3a-4b(La1-xGax)a(Ti1-yZry)1+bO3
1.0
0.5
0
y
0
0.5
1.0
x
Ma
3.0
2.5
2.0
1.5
1.0
0.5
0.0
band_gap [eV]
Candidate point
Up to five candidate points

FIG. 29

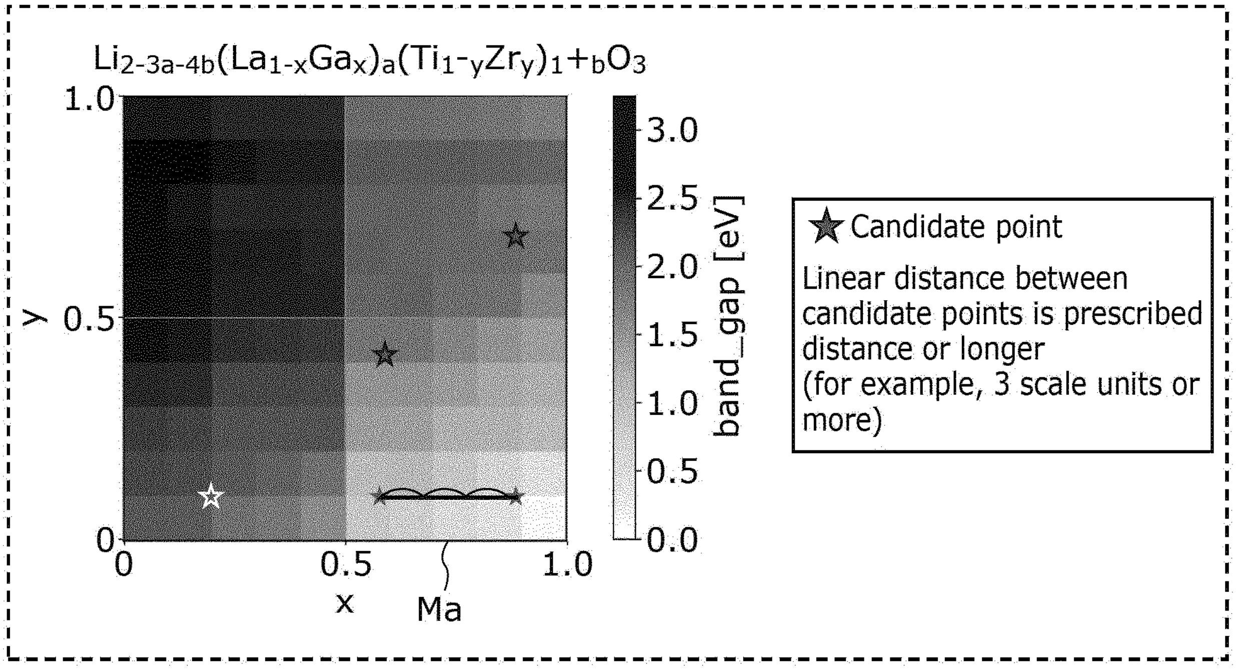
Li2-3a-4b(La1-xGax)a(Ti1-yZry)1+bO3
1.0
0.5
0
y
0
0.5
1.0
x
Ma
3.0
2.5
2.0
1.5
1.0
0.5
0.0
band_gap [eV]
Candidate point
Linear distance between candidate points is prescribed distance or longer
(for example, 3 scale units or more)

FIG. 30

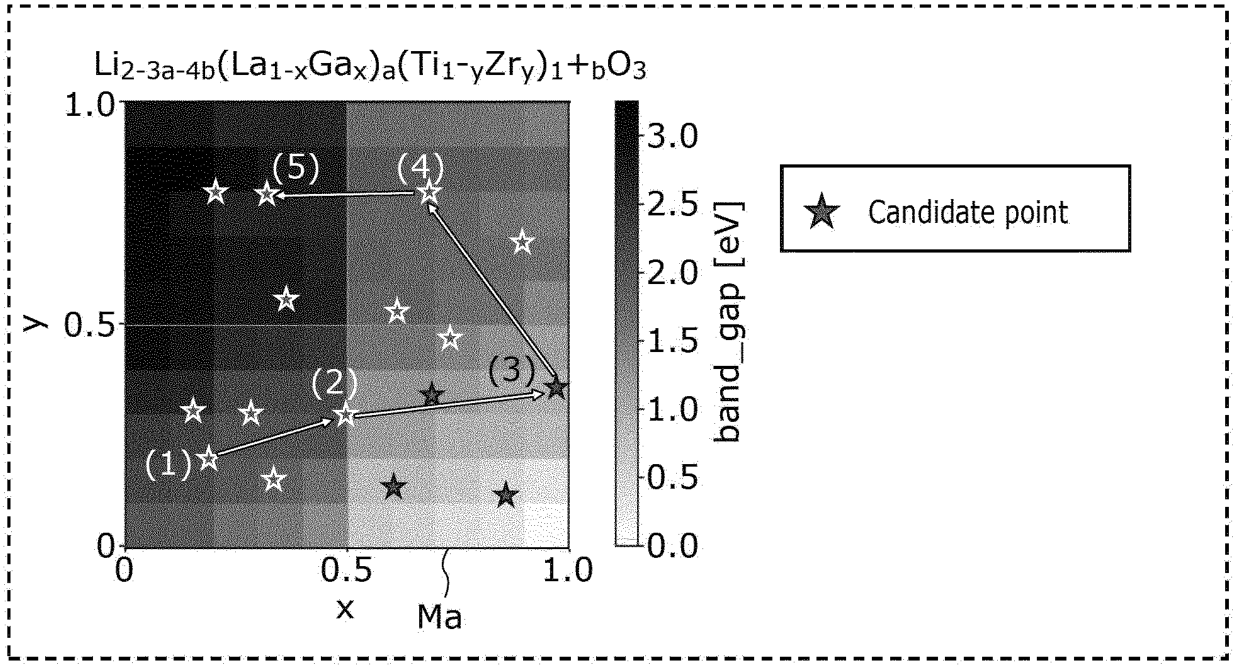
Li2-3a-4b(La1-xGax)a(Ti1-yZry)1+bO3
1.0
y 0.5
0
0
0.5
1.0
x
Ma
(1)
(2)
(3)
(4)
(5)
3.0
2.5
2.0
1.5
1.0
0.5
0.0
band_gap [eV]
Candidate point

FIG. 31

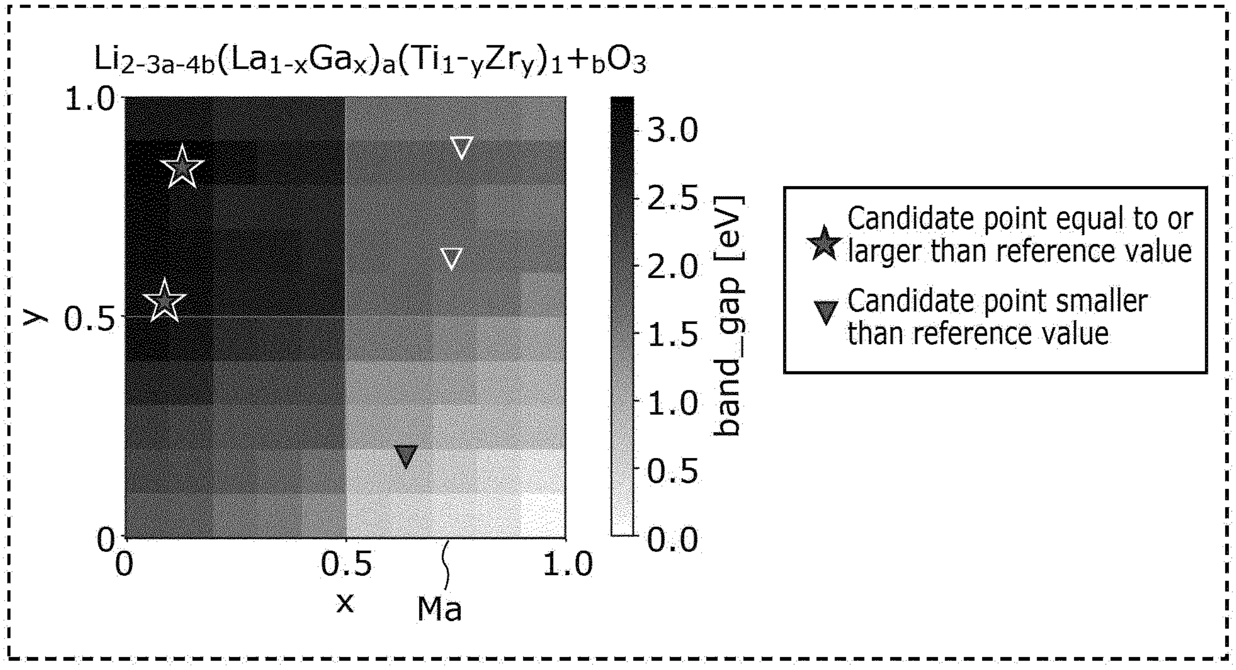
Li2-3a-4b(La1-xGax)a(Ti1-yZry)1+bO3
1.0
y 0.5
0
0
0.5
1.0
x
Ma
3.0
2.5
2.0
1.5
1.0
0.5
0.0
band_gap [eV]
Candidate point equal to or larger than reference value
Candidate point smaller than reference value

FIG. 32

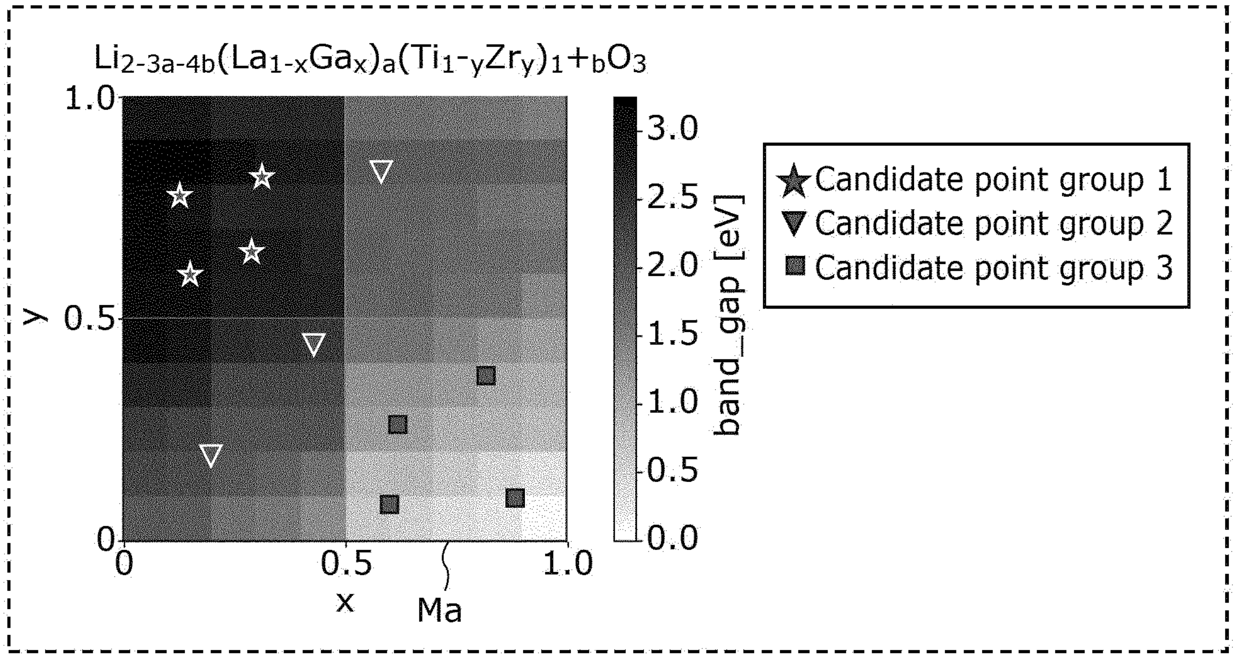
Li2-3a-4b(La1-xGax)a(Ti1-yZry)1+bO3
1.0
y 0.5
0
0
0.5
1.0
x
Ma
3.0
2.5
2.0
1.5
1.0
0.5
0.0
band_gap [eV]
Candidate point group 1
Candidate point group 2
Candidate point group 3

FIG. 33

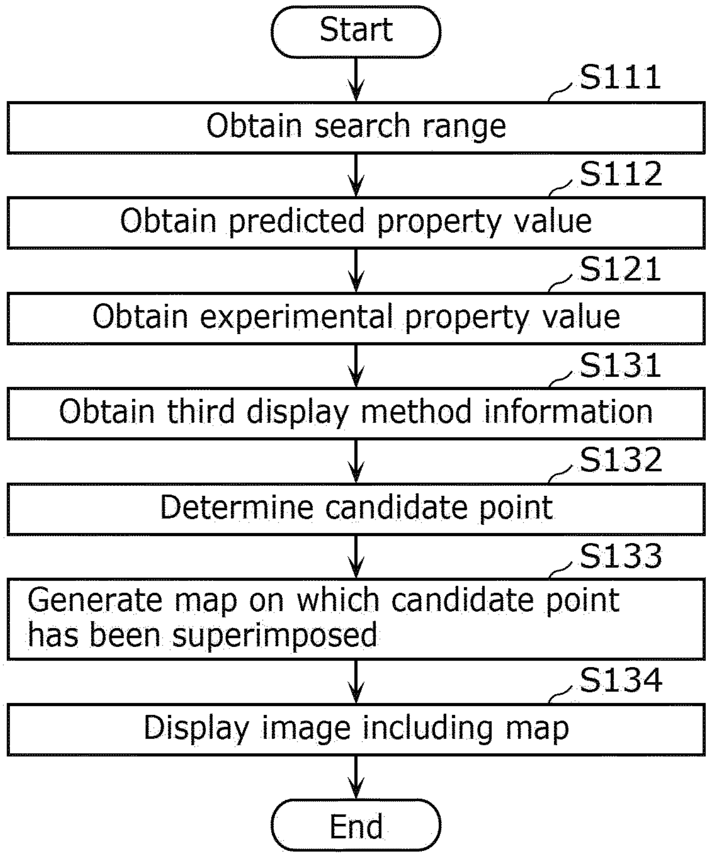
Start
S111
Obtain search range
S112
Obtain predicted property value
S121
Obtain experimental property value
S131
Obtain third display method information
S132
Determine candidate point
S133
Generate map on which candidate point has been superimposed
S134
Display image including map
End

FIG. 34

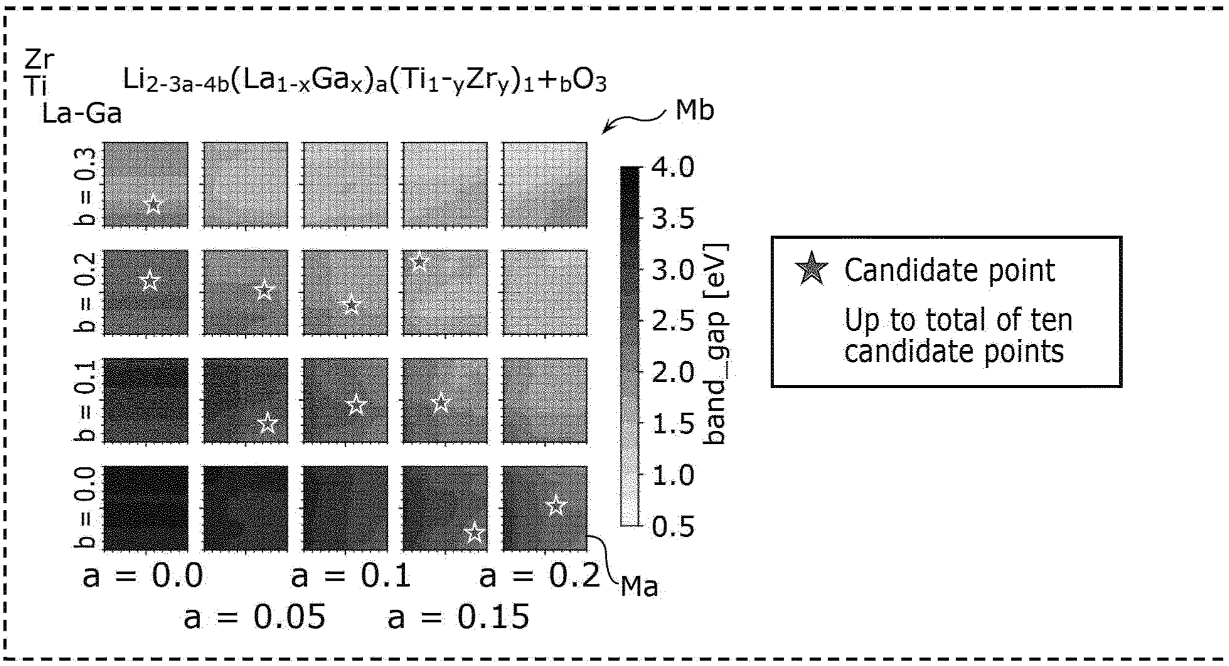
Zr
Ti
La-Ga
Li2-3a-4b(La1-xGax)a(Ti1-yZry)1+bO3
Mb
b = 0.3
b = 0.2
b = 0.1
b = 0.0
a = 0.0
a = 0.05
a = 0.1
a = 0.15
a = 0.2
Ma
4.0
3.5
3.0
2.5
2.0
1.5
1.0
0.5
band_gap [eV]
Candidate point
Up to total of ten candidate points

FIG. 35

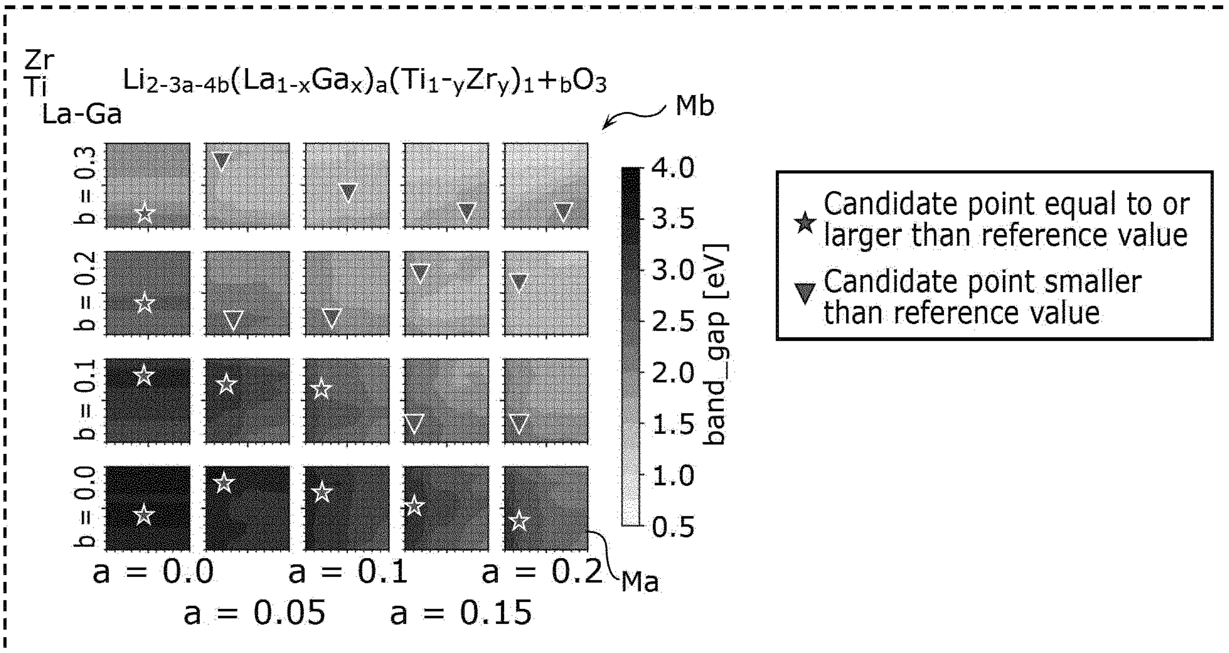
Zr
Ti
La-Ga
Li2-3a-4b(La1-xGax)a(Ti1-yZry)1+bO3
Mb
b = 0.3
b = 0.2
b = 0.1
b = 0.0
a = 0.0
a = 0.05
a = 0.1
a = 0.15
a = 0.2
Ma
4.0
3.5
3.0
2.5
2.0
1.5
1.0
0.5
band_gap [eV]
Candidate point equal to or larger than reference value
Candidate point smaller than reference value

FIG. 36

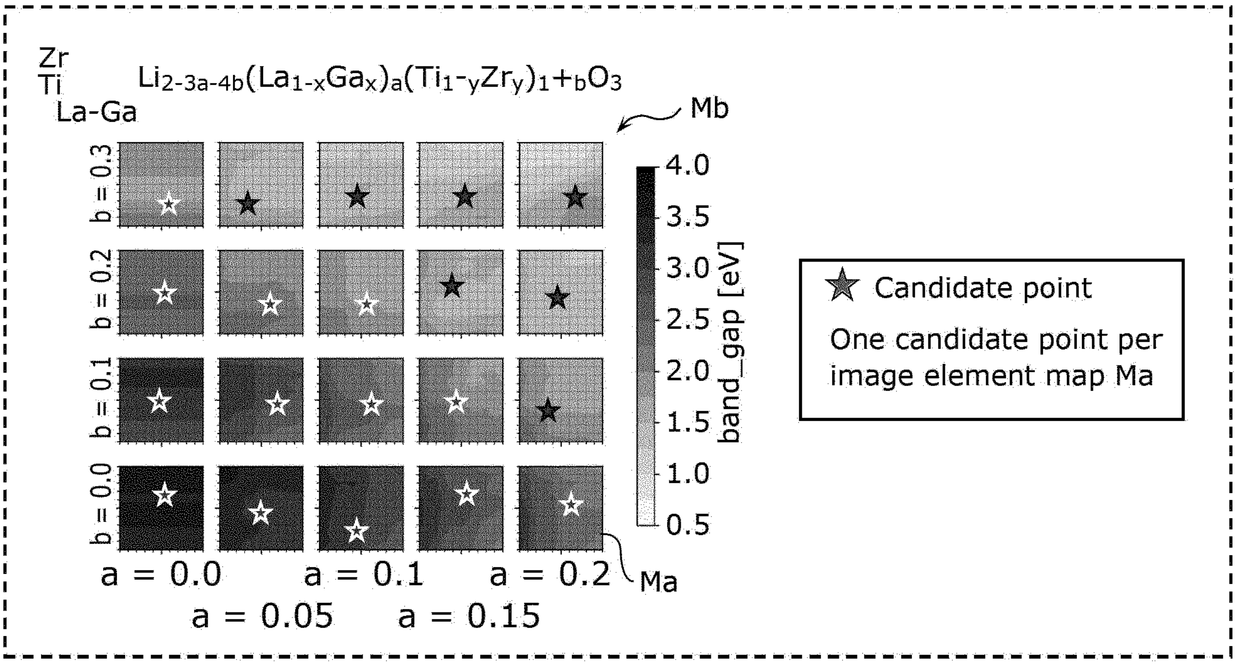
Zr
Ti
La-Ga
$Li_{2-3a-4b}(La_{1-x}Ga_x)_a(Ti_{1-y}Zr_y)_{1+b}O_3$
Mb
b = 0.3
b = 0.2
b = 0.1
b = 0.0
a = 0.0
a = 0.05
a = 0.1
a = 0.15
a = 0.2
Ma
4.0
3.5
3.0
2.5
2.0
1.5
1.0
0.5
band_gap [eV]
Candidate point
One candidate point per image element map Ma

FIG. 37

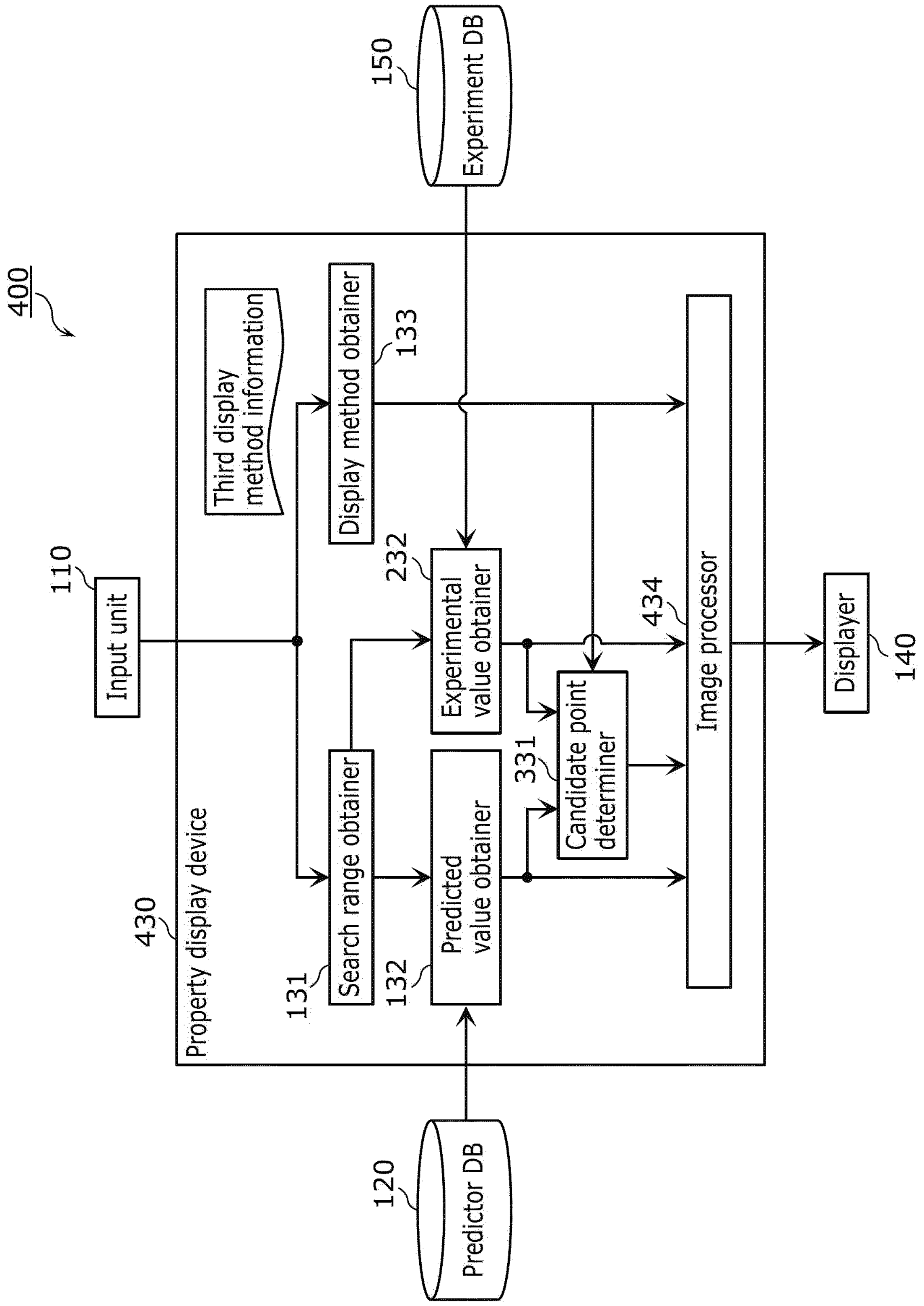
400
Input unit
110
Experiment DB
150
Predictor DB
120
Property display device
430
Third display method information
Display method obtainer
133
Search range obtainer
131
Predicted value obtainer
132
Experimental value obtainer
232
Candidate point determiner
331
Image processor
434
Displayer
140

FIG. 38

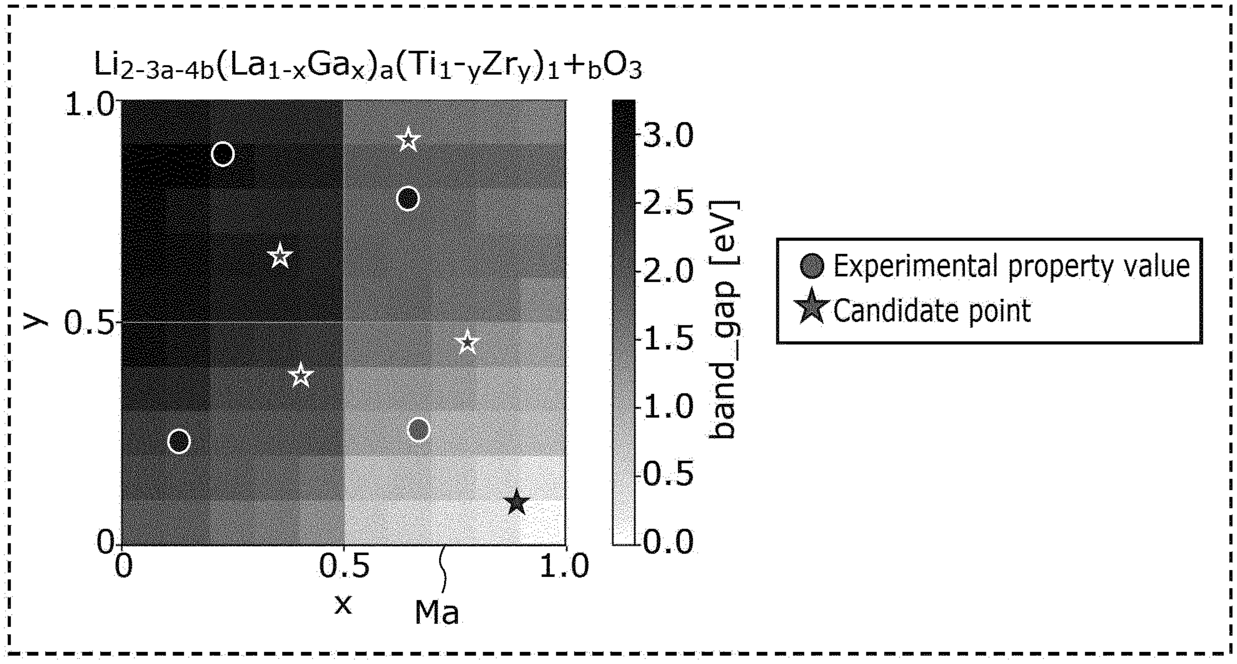

$Li_{2-3a-4b}(La_{1-x}Ga_x)_a(Ti_{1-y}Zr_y)_{1+b}O_3$
1.0
y 0.5
0
0
0.5
1.0
x
Ma
3.0
2.5
2.0
1.5
1.0
0.5
0.0
band_gap [eV]
Experimental property value
Candidate point


FIG. 39

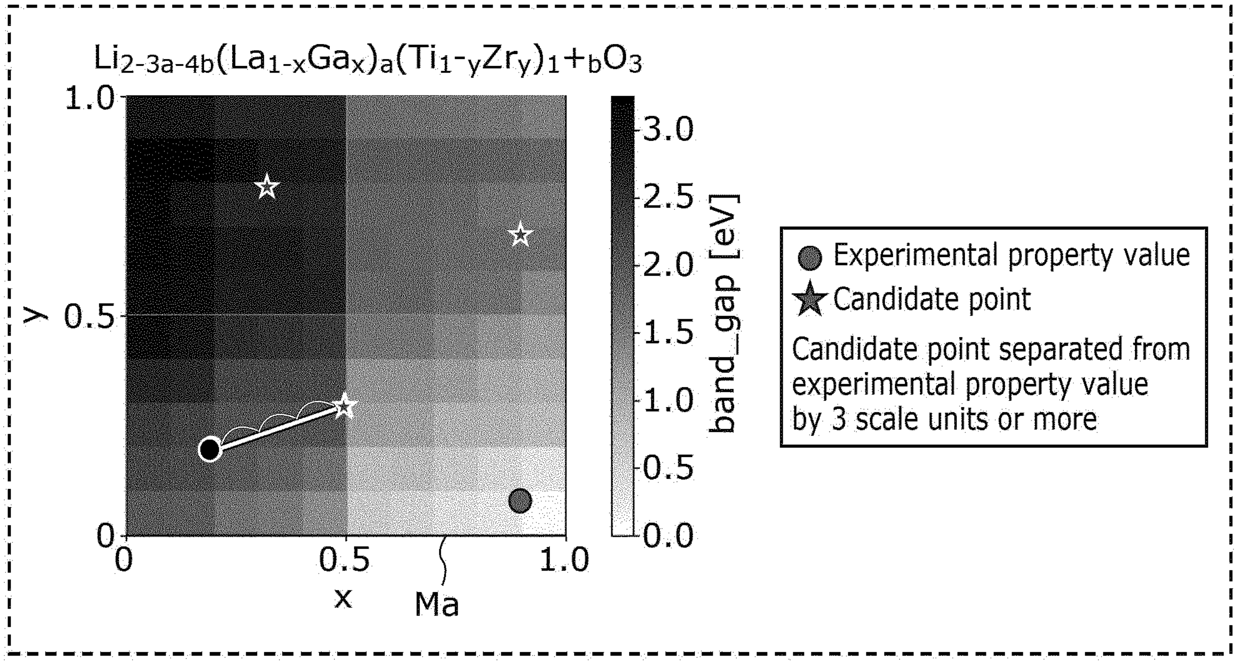

$Li_{2-3a-4b}(La_{1-x}Ga_x)_a(Ti_{1-y}Zr_y)_{1+b}O_3$
1.0
y 0.5
0
0
0.5
1.0
x
Ma
3.0
2.5
2.0
1.5
1.0
0.5
0.0
band_gap [eV]
Experimental property value
Candidate point
Candidate point separated from experimental property value by 3 scale units or more

FIG. 40

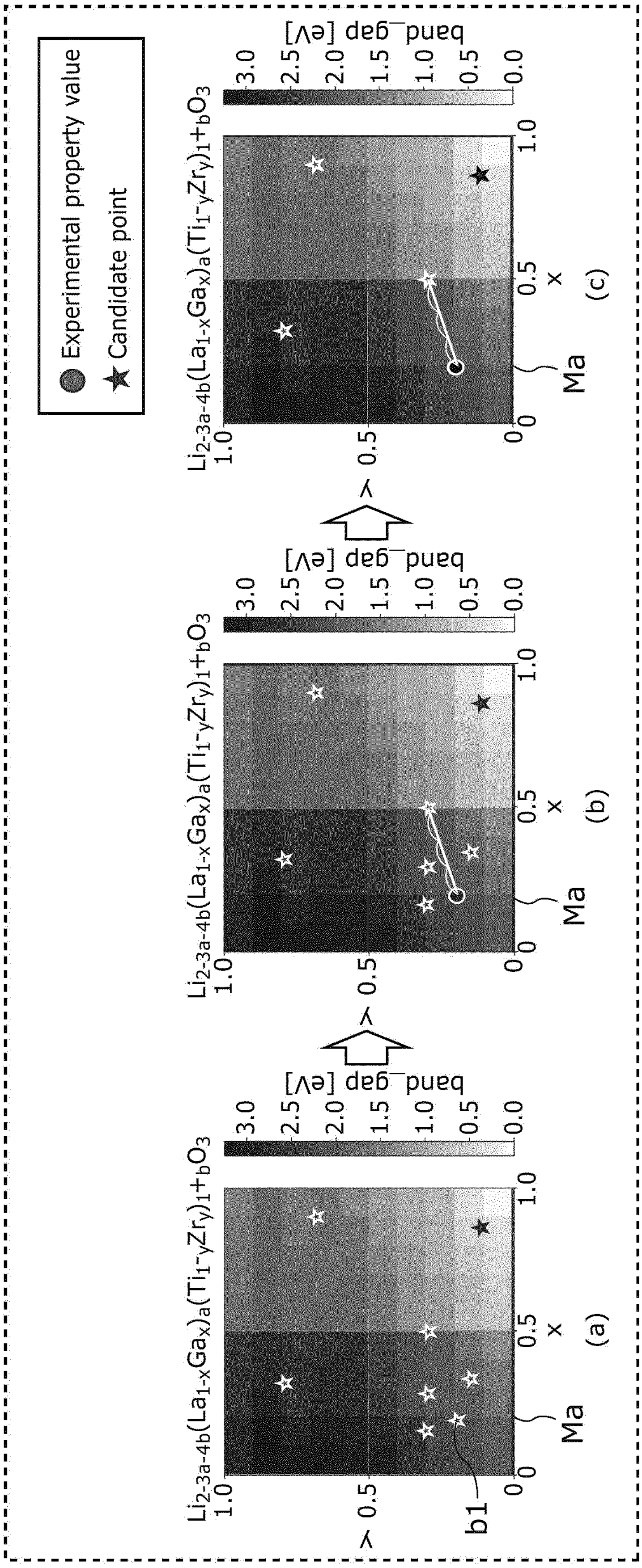

Experimental property value
Candidate point
$Li_{2-3a-4b}(La_{1-x}Ga_x)_a(Ti_{1-y}Zr_y)_{1+b}O_3$
band_gap [eV]
3.0
2.5
2.0
1.5
1.0
0.5
0.0
y
x
Ma
b1
(a)
(b)
(c)

FIG. 41

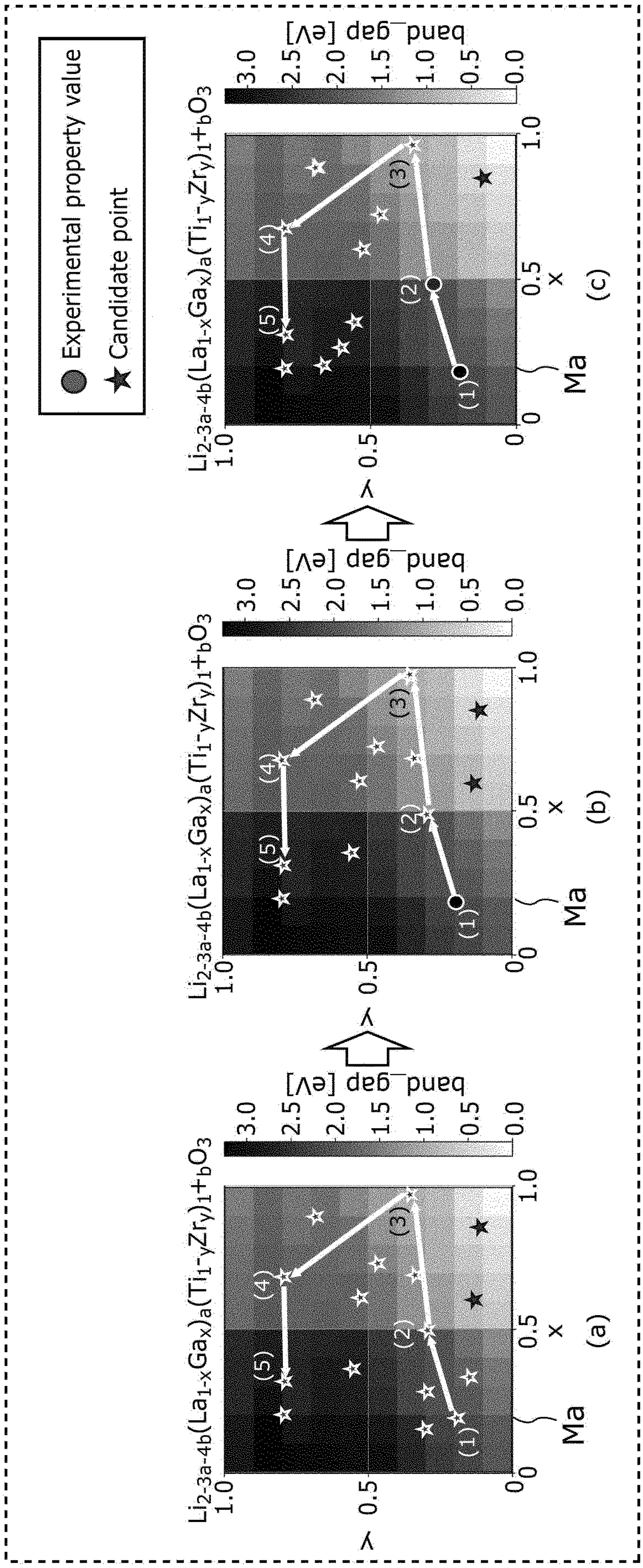
Experimental property value
Candidate point
$Li_{2-3a-4b}(La_{1-x}Ga_x)_a(Ti_{1-y}Zr_y)_{1+b}O_3$
band_gap [eV]
y
x
Ma
(a)
(b)
(c)

FIG. 42

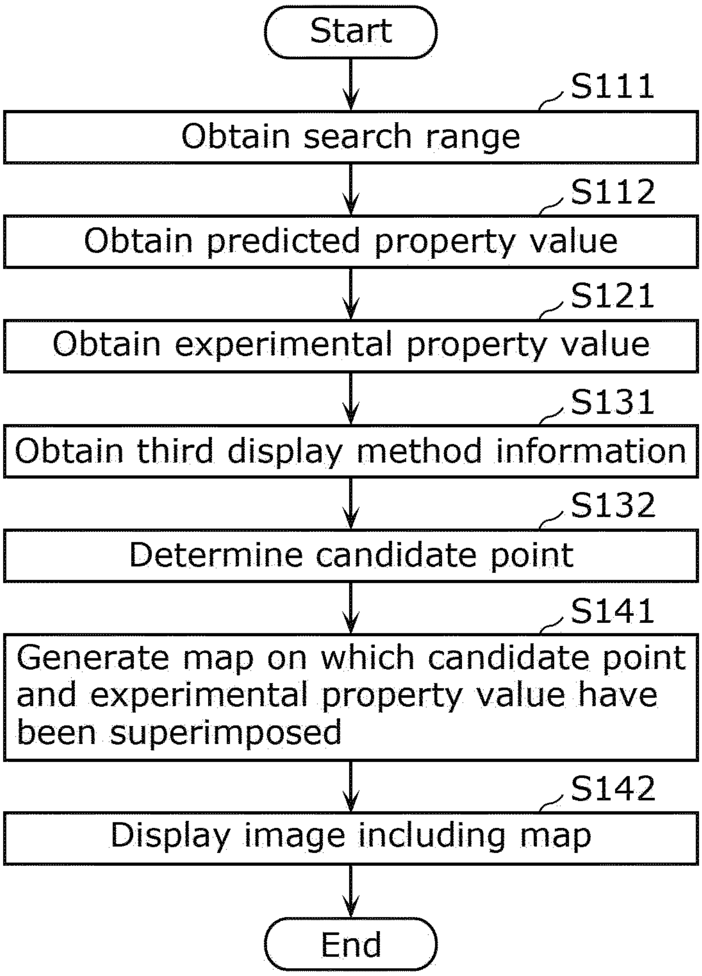
Start
S111
Obtain search range
S112
Obtain predicted property value
S121
Obtain experimental property value
S131
Obtain third display method information
S132
Determine candidate point
S141
Generate map on which candidate point and experimental property value have been superimposed
S142
Display image including map
End

FIG. 43

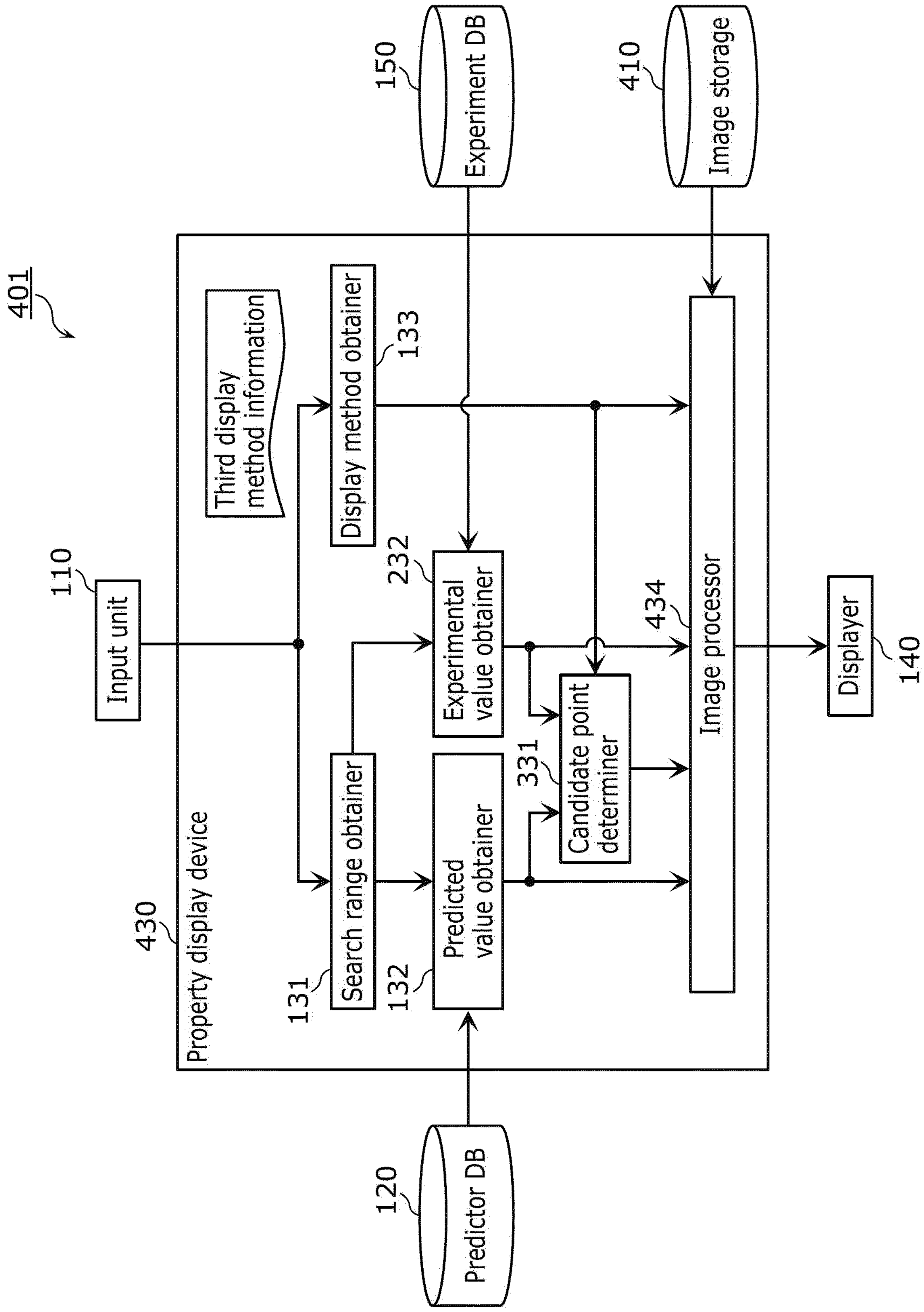
401
Input unit
110
Property display device
430
Third display method information
Display method obtainer
133
Search range obtainer
131
Experimental value obtainer
232
Predicted value obtainer
132
Candidate point determiner
331
Image processor
434
Displayer
140
Experiment DB
150
Image storage
410
Predictor DB
120

FIG. 44

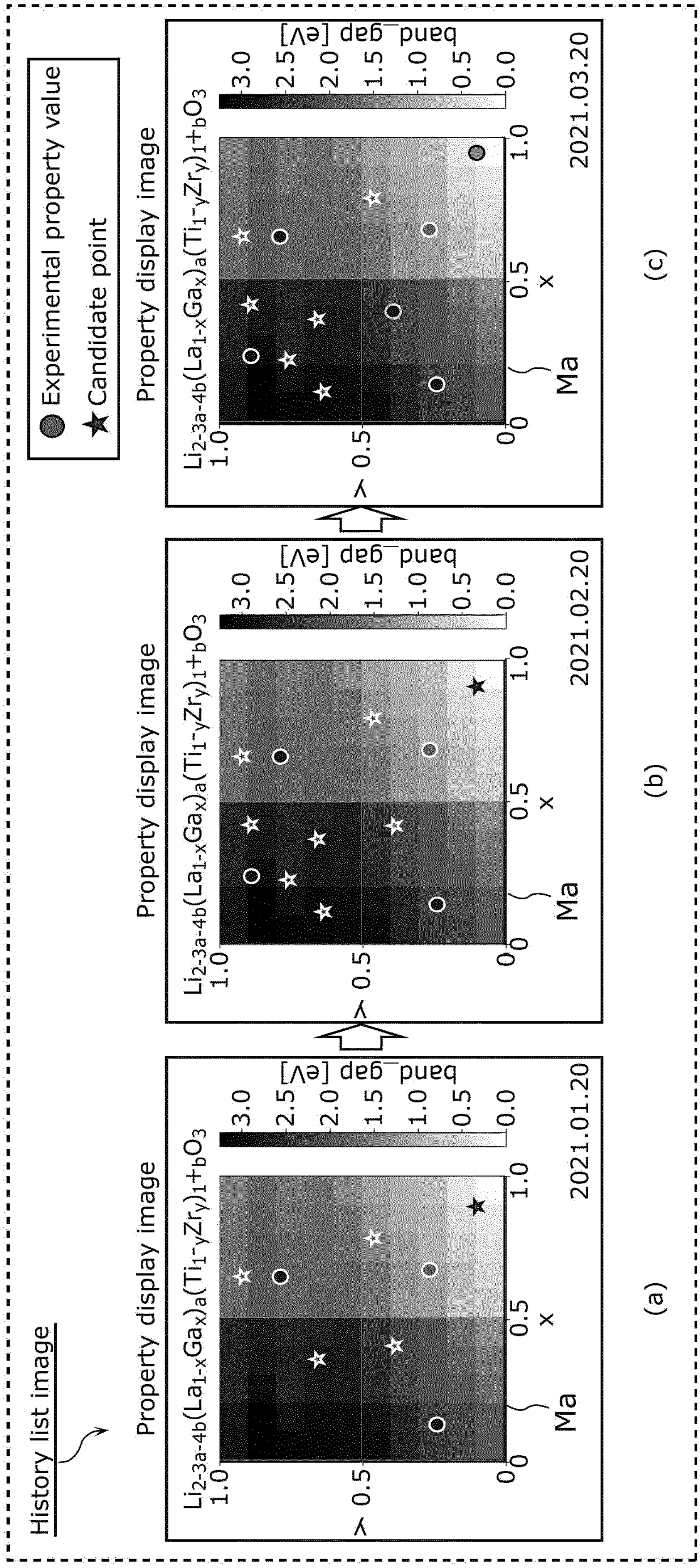
History list image
Experimental property value
Candidate point
Property display image
$Li_{2-3a-4b}(La_{1-x}Ga_x)_a(Ti_{1-y}Zr_y)_{1+b}O_3$
band_gap [eV]
3.0
2.5
2.0
1.5
1.0
0.5
0.0
y
x
Ma
2021.01.20
2021.02.20
2021.03.20
(a)
(b)
(c)

FIG. 45

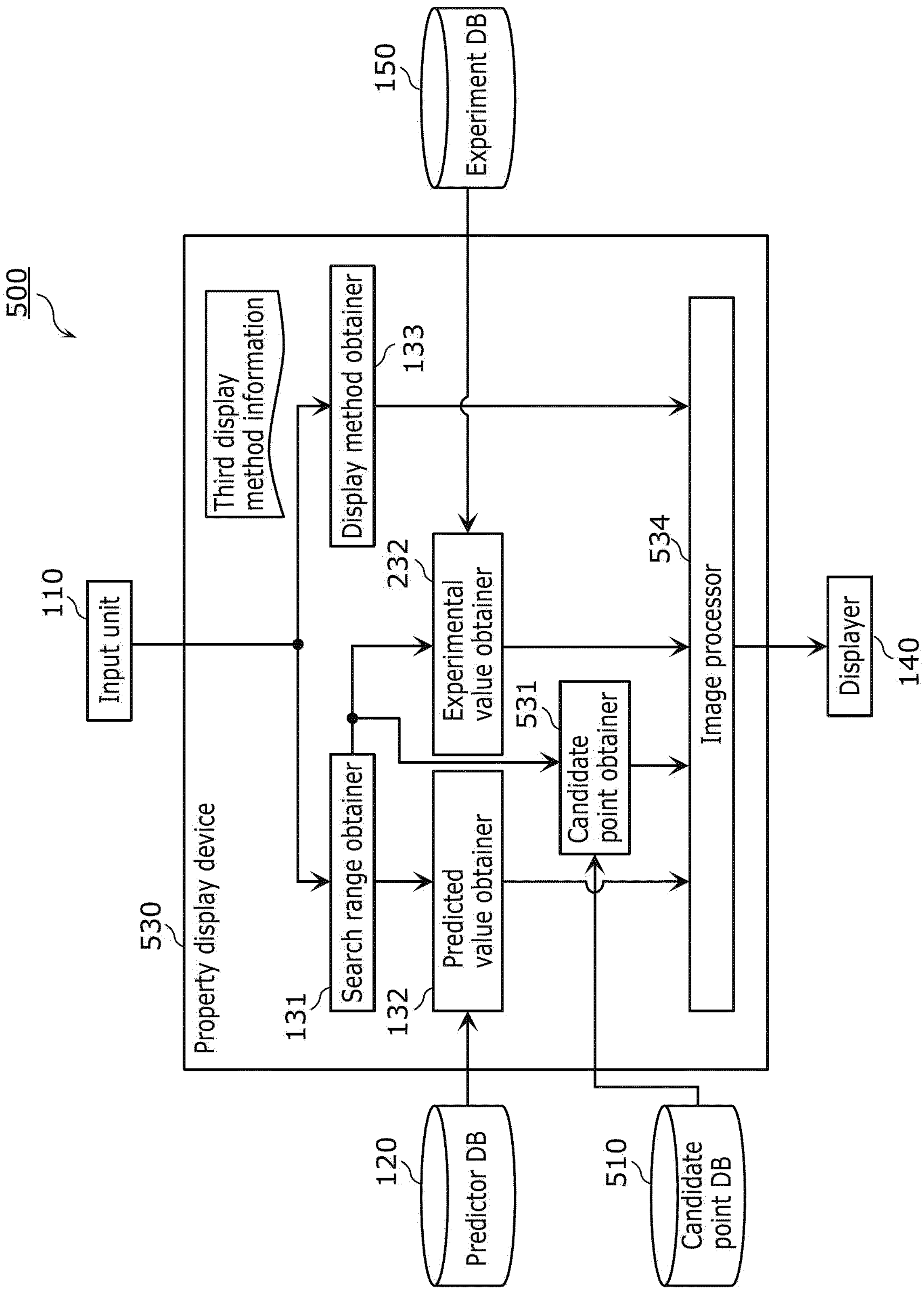
500
110
Input unit
530
Property display device
Third display method information
131
Search range obtainer
133
Display method obtainer
132
Predicted value obtainer
232
Experimental value obtainer
531
Candidate point obtainer
534
Image processor
140
Displayer
150
Experiment DB
120
Predictor DB
510
Candidate point DB

FIG. 46

Candidate point data

| No. | formula | Calcination method 1: Solid-phase method, 2: Ball mill | Calcination temperature [°C] | Calcination time [h] |
|---|---|---|---|---|
| 1 | Li1.45La0.90Ti1.0Ga0.10O3 | 1 | 100 | 3 |
| 2 | Li1.45La0.9Zr0.12Ti0.88Ga0.1O3 | 1 | 120 | 3 |
| 3 | Li1.45La0.9Zr0.45Ti0.55Ga0.1O3 | 2 | 100 | 4 |
| 4 | Li1.45La0.9Zr0.99Ti0.01Ga0.1O3 | 2 | 100 | 3 |
| 5 | Li1.45La0.9Zr1.0Ga0.1O3 | 2 | 120 | 3 |

FIG. 47

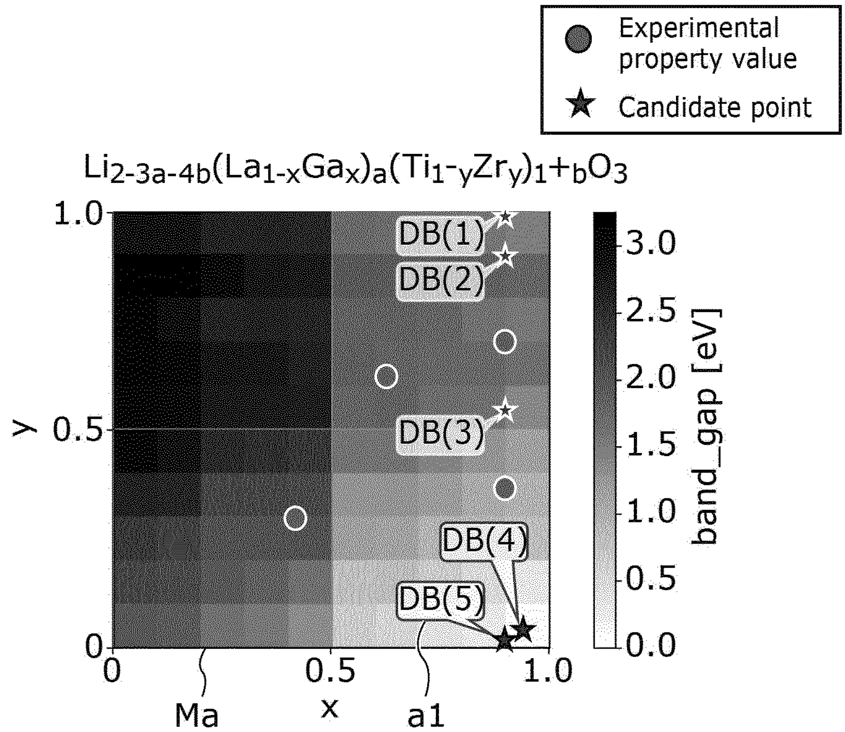

FIG. 48

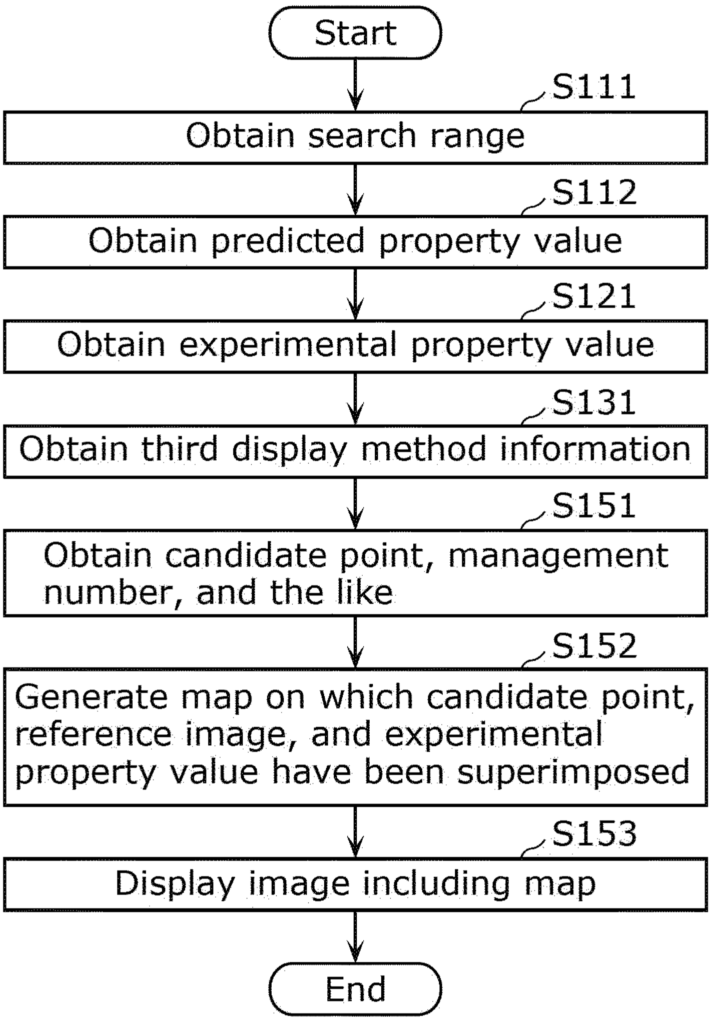
Start
S111
Obtain search range
S112
Obtain predicted property value
S121
Obtain experimental property value
S131
Obtain third display method information
S151
Obtain candidate point, management number, and the like
S152
Generate map on which candidate point, reference image, and experimental property value have been superimposed
S153
Display image including map
End

FIG. 49

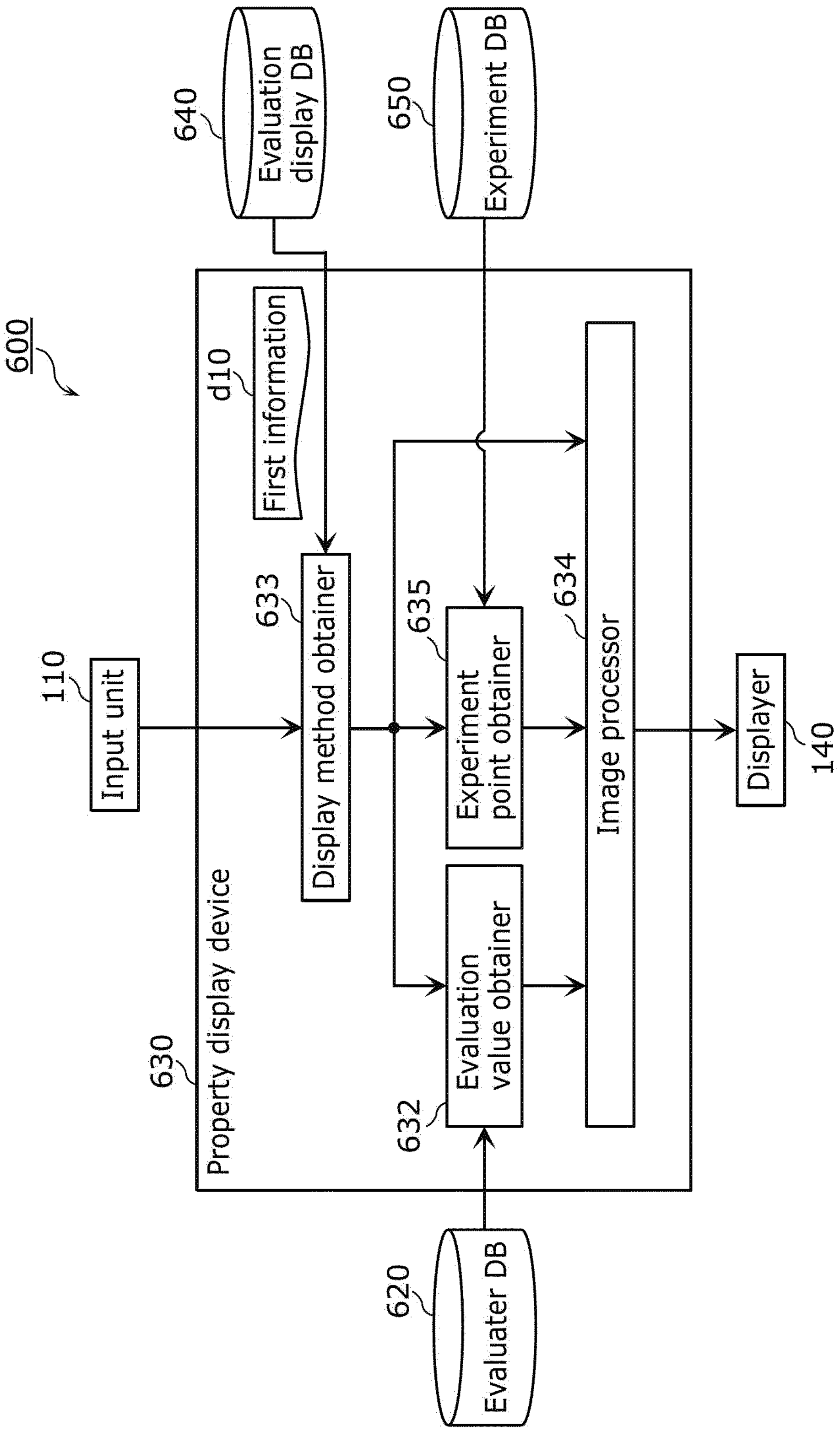
600
640
Evaluation display DB
650
Experiment DB
d10
First information
110
Input unit
630
Property display device
633
Display method obtainer
635
Experiment point obtainer
632
Evaluation value obtainer
634
Image processor
620
Evaluater DB
140
Displayer

FIG. 50

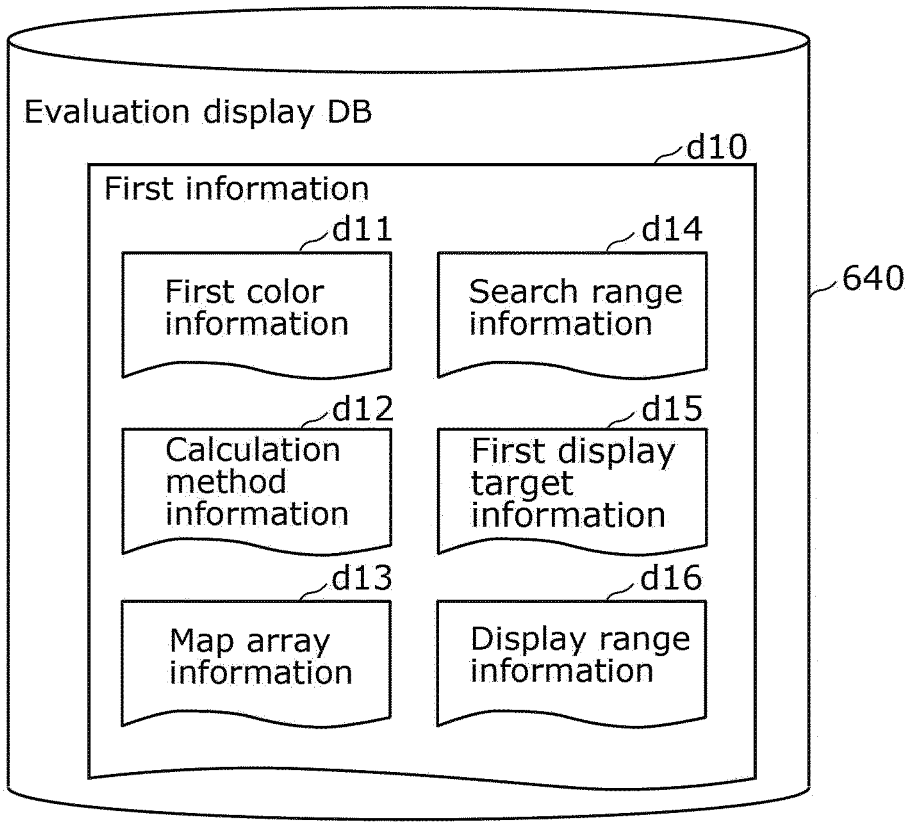
Evaluation display DB
d10
First information
d11
First color information
d14
Search range information
640
d12
Calculation method information
d15
First display target information
d13
Map array information
d16
Display range information

FIG. 51

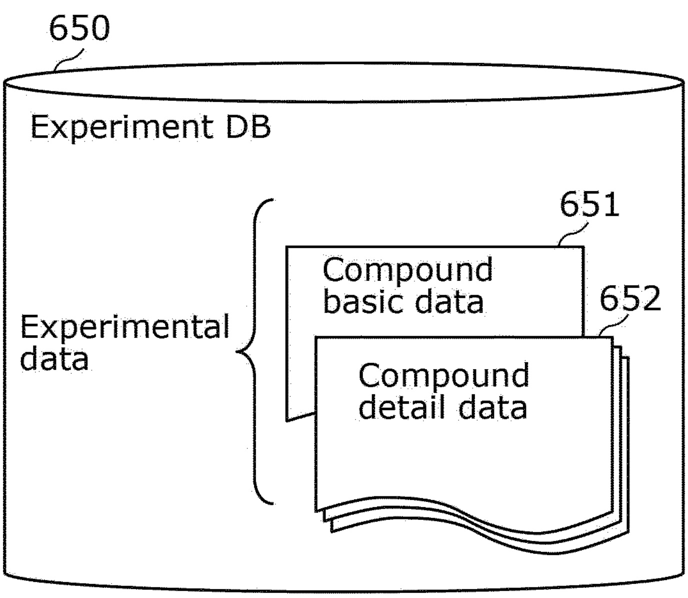
650
Experiment DB
651
Compound basic data
652
Experimental data
Compound detail data

# FIG. 52

Compound basic data 651

| ID | Composition formula | Processing condition | | Property 1 (band gap _eV) | Property 2 (heat-resistance temperature _°C) | Crystal phase |
|---|---|---|---|---|---|---|
| | | Calcination temperature _°C | Calcination time_h | | | |
| 000001-00001-001 | Li1.45La0.045Ti1.1Al0.005O3 | 400 | 3 | 2.349 | 213 | cubic-perovskite |
| 000002-00001-001 | Li1.45La0.045Zr0.22Ti0.88Al0.005O3 | 400 | 3 | 2.462 | 205 | cubic-perovskite |
| 000003-00001-001 | Li1.45La0.045Zr0.55Ti0.55Al0.005O3 | 400 | 3 | 2.578 | 210 | cubic-perovskite |
| 000004-00001-001 | Li1.45La0.045Zr0.99Ti0.11Al0.005O3 | 400 | 3 | 2.621 | 199 | cubic-perovskite |
| 000005-00001-001 | Li1.45La0.045Zr1.1Al0.005O3 | 400 | 3 | 2.594 | 206 | cubic-perovskite |

Experimental property values (Property 1, Property 2, Crystal phase)

FIG. 53

Compound detail data 652

| | | |
|---|---|---|
| | ID | 000001-00001-001 |
| | Researcher/ experimenter | Tanaka/sato |
| | Date of registration | 2020/02/03 |
| | Air temperature/ humidity | 24.8 / 69 |
| Processing information | Raw material | Li2O / La2O3 / Al2O3 / TiO2 |
| | Actual composition | Li1.451La0.0450Ti1.102Al0.005O3.01 |
| Measured data | Electrical resistance | {"10000/T / 1/K":[16, 17, 18, 19, 20, ...], "ln(1/R) / 1/Ohm":[-0.9, -2.0, -2.6, -3.8, -5.0, ...]} |
| | DSC | {"temperature / °C":[100, 101, 102, 103, 104, ...], "Heat flow / mW/mg": [0.001, 0.001, 0.002, 0.002, 0.002, ...]} |
| | XRD | {"2 theta / degree":[10, 10.01, 10.02, 10.03, ...], "Intensity / a.u.": [283, 294, 274, 291, ...]} |

FIG. 54

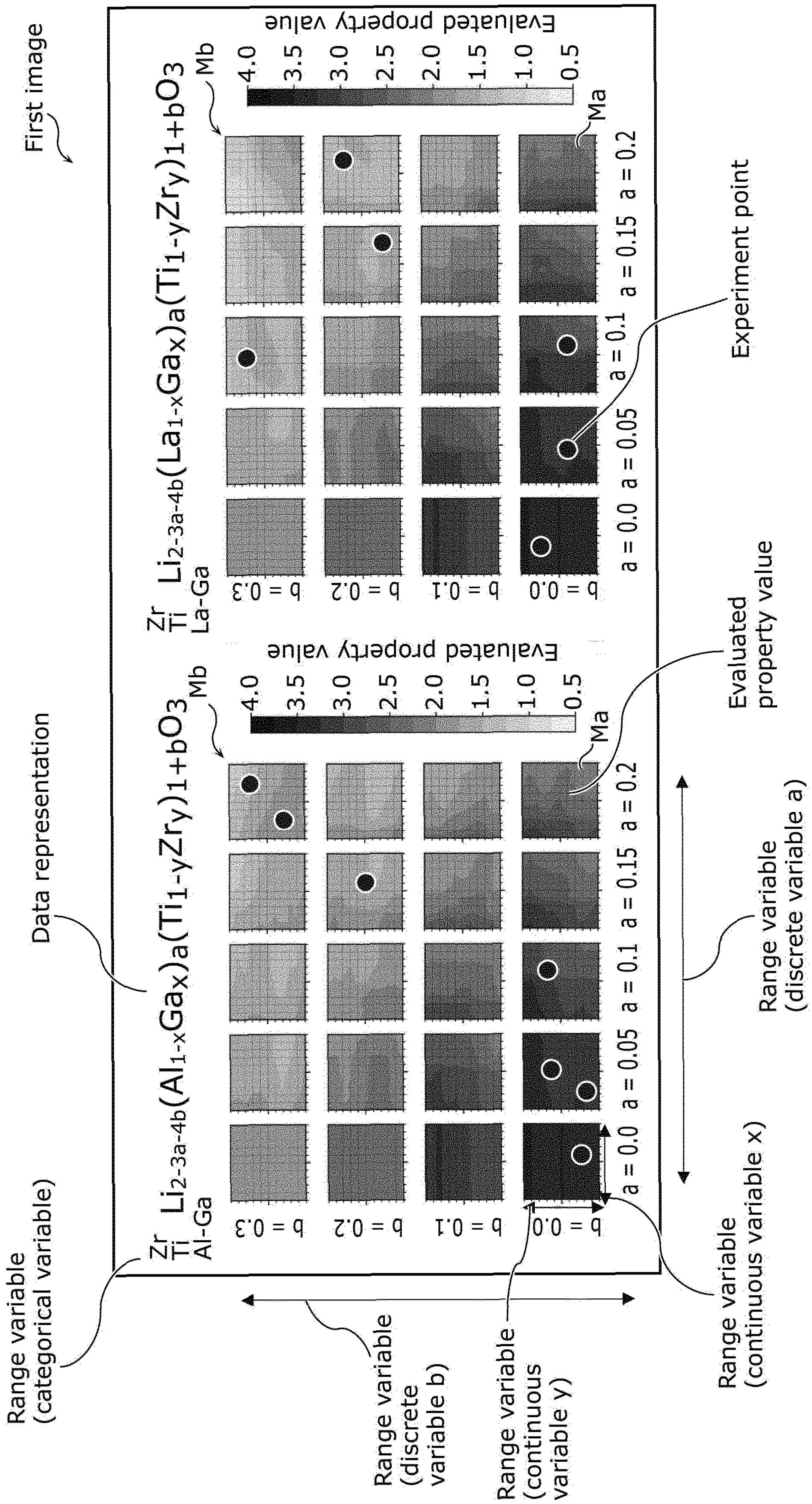

First image
Data representation
Range variable (categorical variable)
Zr Ti Al-Ga
$Li_{2-3a-4b}(Al_{1-x}Ga_x)_a(Ti_{1-y}Zr_y)_{1+b}O_3$
Zr Ti La-Ga
$Li_{2-3a-4b}(La_{1-x}Ga_x)_a(Ti_{1-y}Zr_y)_{1+b}O_3$
Mb
Ma
4.0
3.5
3.0
2.5
2.0
1.5
1.0
0.5
Evaluated property value
b = 0.3
b = 0.2
b = 0.1
b = 0.0
a = 0.0
a = 0.05
a = 0.1
a = 0.15
a = 0.2
Experiment point
Evaluated property value
Range variable (discrete variable a)
Range variable (discrete variable b)
Range variable (continuous variable y)
Range variable (continuous variable x)

FIG. 55

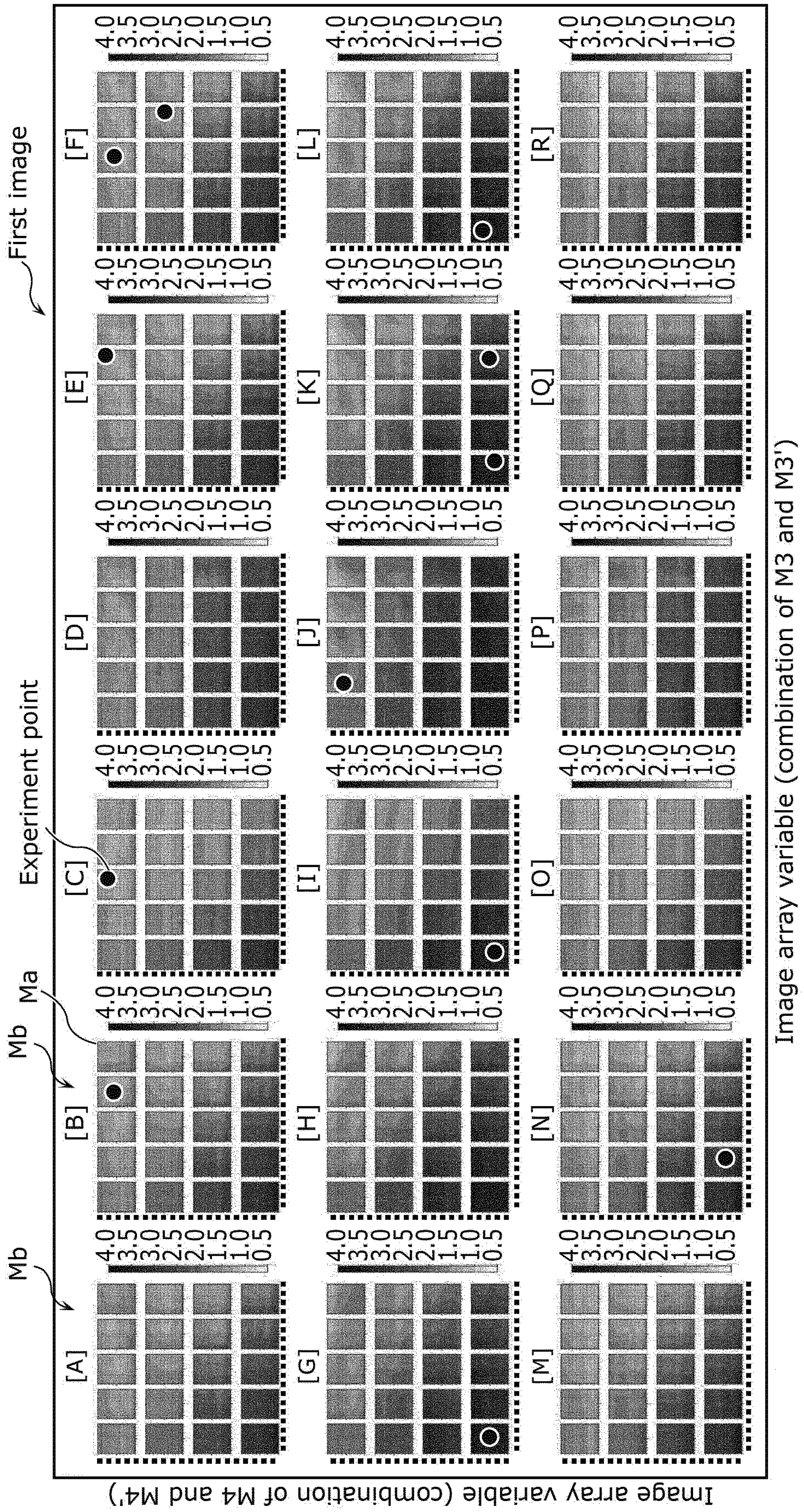
First image
Experiment point
Ma
Mb
Mb
[A]
[B]
[C]
[D]
[E]
[F]
[G]
[H]
[I]
[J]
[K]
[L]
[M]
[N]
[O]
[P]
[Q]
[R]
4.0
3.5
3.0
2.5
2.0
1.5
1.0
0.5
Image array variable (combination of M3 and M3')
Image array variable (combination of M4 and M4')

FIG. 56

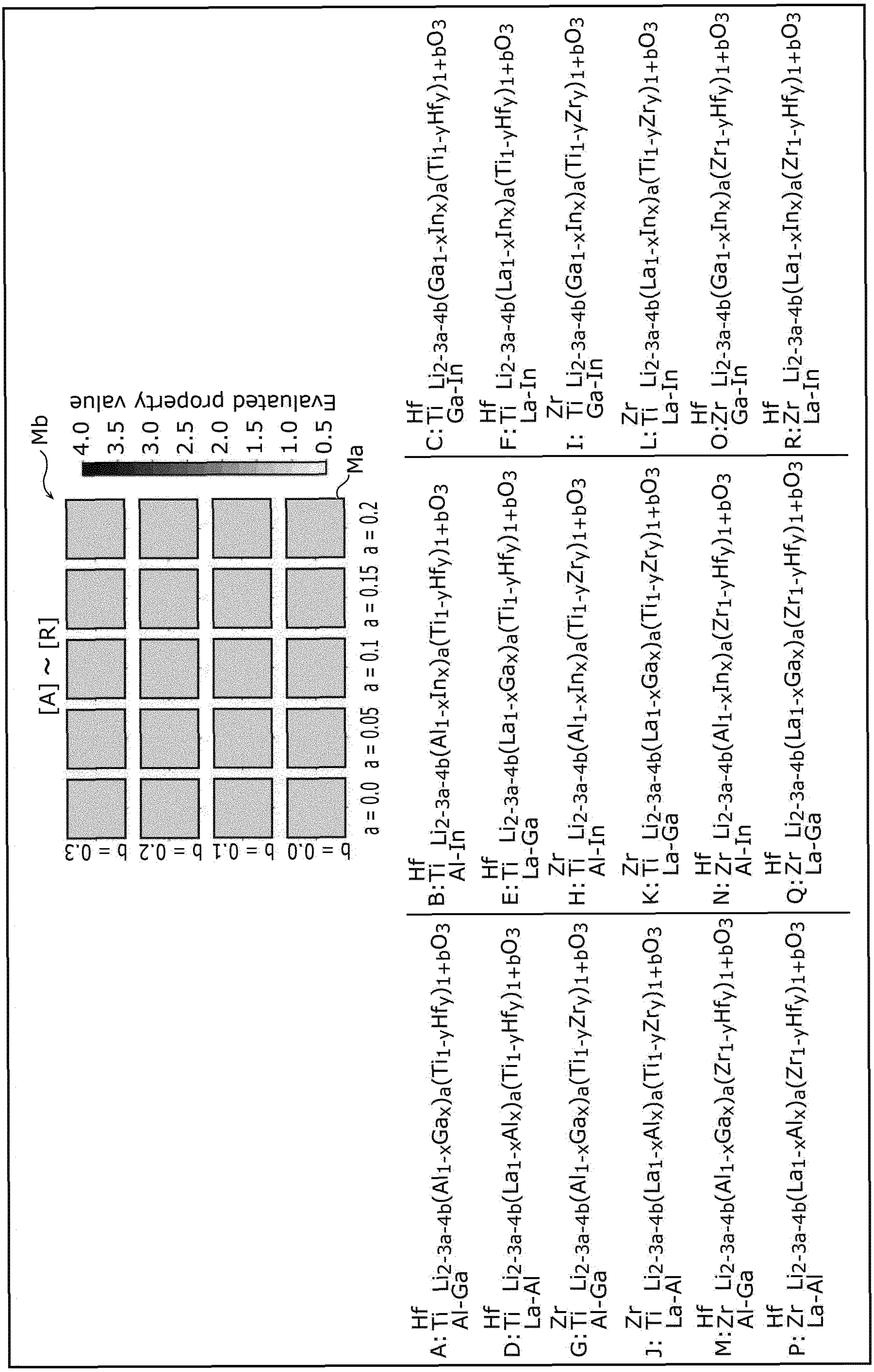
[A] ~ [R]
Mb
Ma
Evaluated property value
4.0
3.5
3.0
2.5
2.0
1.5
1.0
0.5
b = 0.3
b = 0.2
b = 0.1
b = 0.0
a = 0.0
a = 0.05
a = 0.1
a = 0.15
a = 0.2
A: Hf Ti Al-Ga $Li_{2-3a-4b}(Al_{1-x}Ga_x)_a(Ti_{1-y}Hf_y)_{1+b}O_3$
B: Hf Ti Al-In $Li_{2-3a-4b}(Al_{1-x}In_x)_a(Ti_{1-y}Hf_y)_{1+b}O_3$
C: Hf Ti Ga-In $Li_{2-3a-4b}(Ga_{1-x}In_x)_a(Ti_{1-y}Hf_y)_{1+b}O_3$
D: Hf Ti La-Al $Li_{2-3a-4b}(La_{1-x}Al_x)_a(Ti_{1-y}Hf_y)_{1+b}O_3$
E: Hf Ti La-Ga $Li_{2-3a-4b}(La_{1-x}Ga_x)_a(Ti_{1-y}Hf_y)_{1+b}O_3$
F: Hf Ti La-In $Li_{2-3a-4b}(La_{1-x}In_x)_a(Ti_{1-y}Hf_y)_{1+b}O_3$
G: Zr Ti Al-Ga $Li_{2-3a-4b}(Al_{1-x}Ga_x)_a(Ti_{1-y}Zr_y)_{1+b}O_3$
H: Zr Ti Al-In $Li_{2-3a-4b}(Al_{1-x}In_x)_a(Ti_{1-y}Zr_y)_{1+b}O_3$
I: Zr Ti Ga-In $Li_{2-3a-4b}(Ga_{1-x}In_x)_a(Ti_{1-y}Zr_y)_{1+b}O_3$
J: Zr Ti La-Al $Li_{2-3a-4b}(La_{1-x}Al_x)_a(Ti_{1-y}Zr_y)_{1+b}O_3$
K: Zr Ti La-Ga $Li_{2-3a-4b}(La_{1-x}Ga_x)_a(Ti_{1-y}Zr_y)_{1+b}O_3$
L: Zr Ti La-In $Li_{2-3a-4b}(La_{1-x}In_x)_a(Ti_{1-y}Zr_y)_{1+b}O_3$
M: Hf Zr Al-Ga $Li_{2-3a-4b}(Al_{1-x}Ga_x)_a(Zr_{1-y}Hf_y)_{1+b}O_3$
N: Hf Zr Al-In $Li_{2-3a-4b}(Al_{1-x}In_x)_a(Zr_{1-y}Hf_y)_{1+b}O_3$
O: Hf Zr Ga-In $Li_{2-3a-4b}(Ga_{1-x}In_x)_a(Zr_{1-y}Hf_y)_{1+b}O_3$
P: Hf Zr La-Al $Li_{2-3a-4b}(La_{1-x}Al_x)_a(Zr_{1-y}Hf_y)_{1+b}O_3$
Q: Hf Zr La-Ga $Li_{2-3a-4b}(La_{1-x}Ga_x)_a(Zr_{1-y}Hf_y)_{1+b}O_3$
R: Hf Zr La-In $Li_{2-3a-4b}(La_{1-x}In_x)_a(Zr_{1-y}Hf_y)_{1+b}O_3$

FIG. 57A

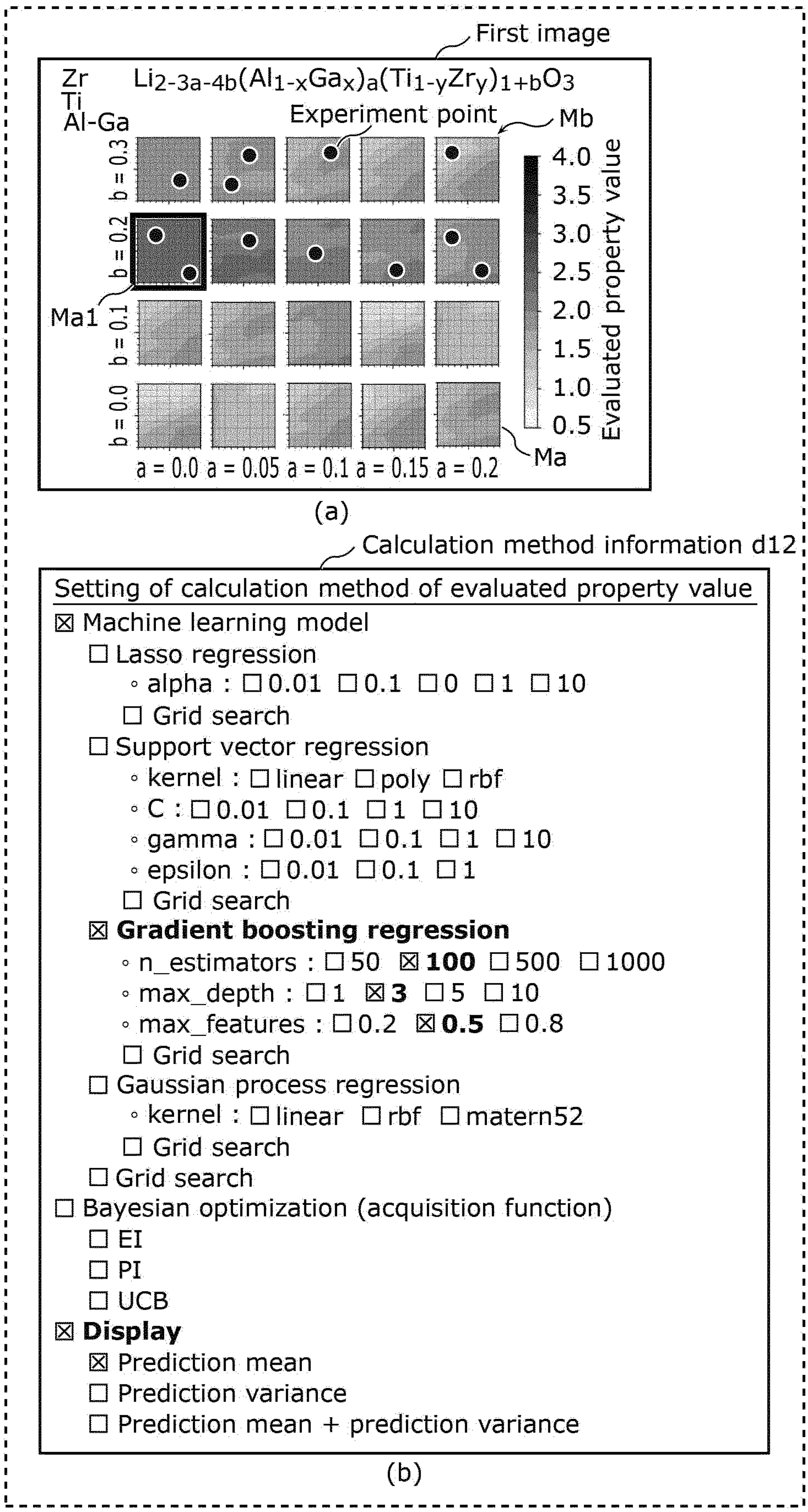
First image
Zr
Ti
Al-Ga
$Li_{2-3a-4b}(Al_{1-x}Ga_x)_a(Ti_{1-y}Zr_y)_{1+b}O_3$
Experiment point
Mb
b = 0.3
b = 0.2
b = 0.1
b = 0.0
Ma1
4.0
3.5
3.0
2.5
2.0
1.5
1.0
0.5
Evaluated property value
Ma
a = 0.0 a = 0.05 a = 0.1 a = 0.15 a = 0.2
(a)
Calculation method information d12
Setting of calculation method of evaluated property value
☒ Machine learning model
☐ Lasso regression
◦ alpha : ☐ 0.01 ☐ 0.1 ☐ 0 ☐ 1 ☐ 10
☐ Grid search
☐ Support vector regression
◦ kernel : ☐ linear ☐ poly ☐ rbf
◦ C : ☐ 0.01 ☐ 0.1 ☐ 1 ☐ 10
◦ gamma : ☐ 0.01 ☐ 0.1 ☐ 1 ☐ 10
◦ epsilon : ☐ 0.01 ☐ 0.1 ☐ 1
☐ Grid search
☒ **Gradient boosting regression**
◦ n_estimators : ☐ 50 ☒ **100** ☐ 500 ☐ 1000
◦ max_depth : ☐ 1 ☒ **3** ☐ 5 ☐ 10
◦ max_features : ☐ 0.2 ☒ **0.5** ☐ 0.8
☐ Grid search
☐ Gaussian process regression
◦ kernel : ☐ linear ☐ rbf ☐ matern52
☐ Grid search
☐ Grid search
☐ Bayesian optimization (acquisition function)
☐ EI
☐ PI
☐ UCB
☒ **Display**
☒ Prediction mean
☐ Prediction variance
☐ Prediction mean + prediction variance
(b)

# FIG. 57B

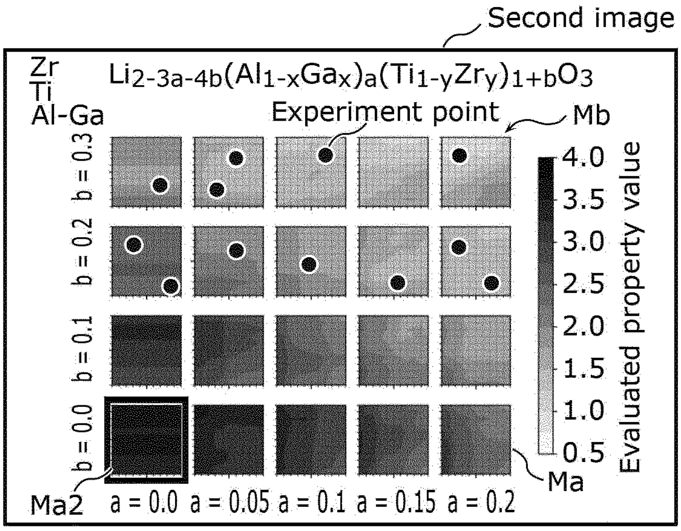


(a)

Calculation method information d12

Setting of calculation method of evaluated property value

☒ Machine learning model
- ☐ Lasso regression
  - ◦ alpha : ☐ 0.01 ☐ 0.1 ☐ 0 ☐ 1 ☐ 10
  - ☐ Grid search
- ☐ Support vector regression
  - ◦ kernel : ☐ linear ☐ poly ☐ rbf
  - ◦ C : ☐ 0.01 ☐ 0.1 ☐ 1 ☐ 10
  - ◦ gamma : ☐ 0.01 ☐ 0.1 ☐ 1 ☐ 10
  - ◦ epsilon : ☐ 0.01 ☐ 0.1 ☐ 1
  - ☐ Grid search
- ☐ Gradient boosting regression
  - ◦ n_estimators : ☐ 50 ☐ 100 ☐ 500 ☐ 1000
  - ◦ max_depth : ☐ 1 ☐ 3 ☐ 5 ☐ 10
  - ◦ max_features : ☐ 0.2 ☐ 0.5 ☐ 0.8
  - ☐ Grid search
- ☒ **Gaussian process regression**
  - ◦ kernel : ☐ linear ☒ **rbf** ☐ matern52
  - ☐ Grid search
- ☐ Grid search

☒ Bayesian optimization (acquisition function)
- ☐ EI
- ☐ PI
- ☒ **UCB** (coefficient of variance: **2.0**)

☒ Display
- ☐ Prediction mean
- ☐ Prediction variance
- ☒ Prediction mean + prediction variance (coefficient of variance : **2.0**)

(b)

# FIG. 58A

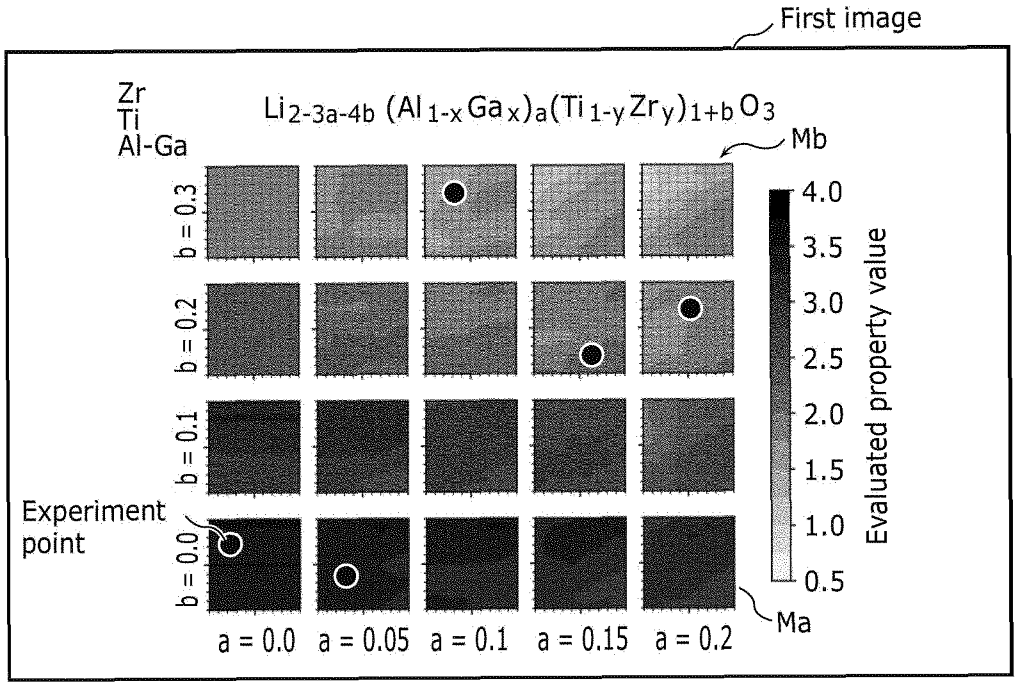


(a)

Search range information d14

Setting of non-visualization variable of evaluated property value

- Calcination temperature_°C: ☒ Minimum: **300** ☐ Maximum ☐ Best ☐ Mean ☐ User-defined option
- Calcination time_h : ☒ Minimum: **1** ☐ Maximum ☐ Best ☐ Mean ☐ User-defined option

(b)

FIG. 58B

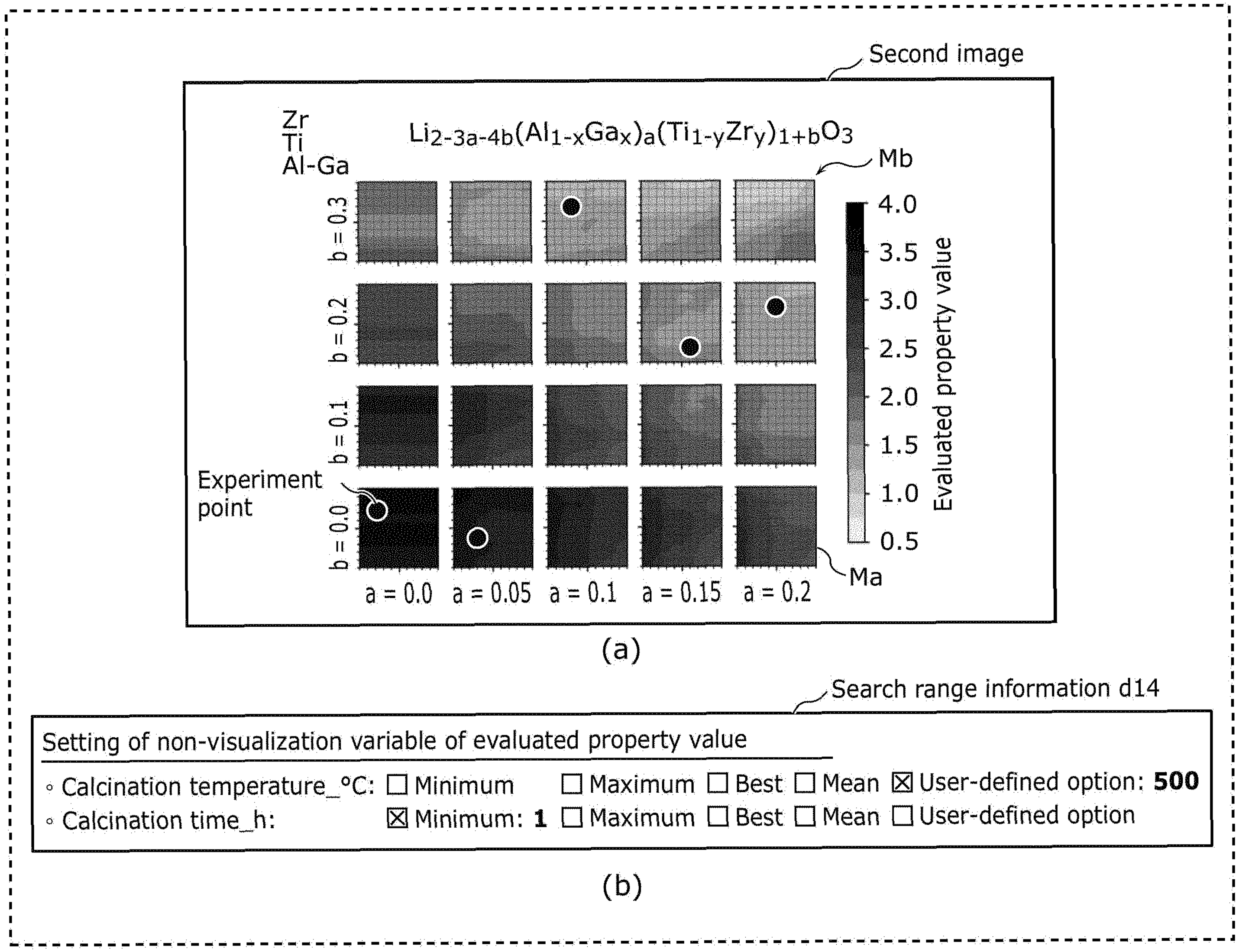
Second image
Zr
Ti
Al-Ga
Li2-3a-4b(Al1-xGax)a(Ti1-yZry)1+bO3
Mb
Ma
Experiment point
b = 0.3
b = 0.2
b = 0.1
b = 0.0
a = 0.0
a = 0.05
a = 0.1
a = 0.15
a = 0.2
4.0
3.5
3.0
2.5
2.0
1.5
1.0
0.5
Evaluated property value
(a)
Search range information d14
Setting of non-visualization variable of evaluated property value
◦ Calcination temperature_°C: ☐ Minimum ☐ Maximum ☐ Best ☐ Mean ☒ User-defined option: 500
◦ Calcination time_h: ☒ Minimum: 1 ☐ Maximum ☐ Best ☐ Mean ☐ User-defined option
(b)

FIG. 59A

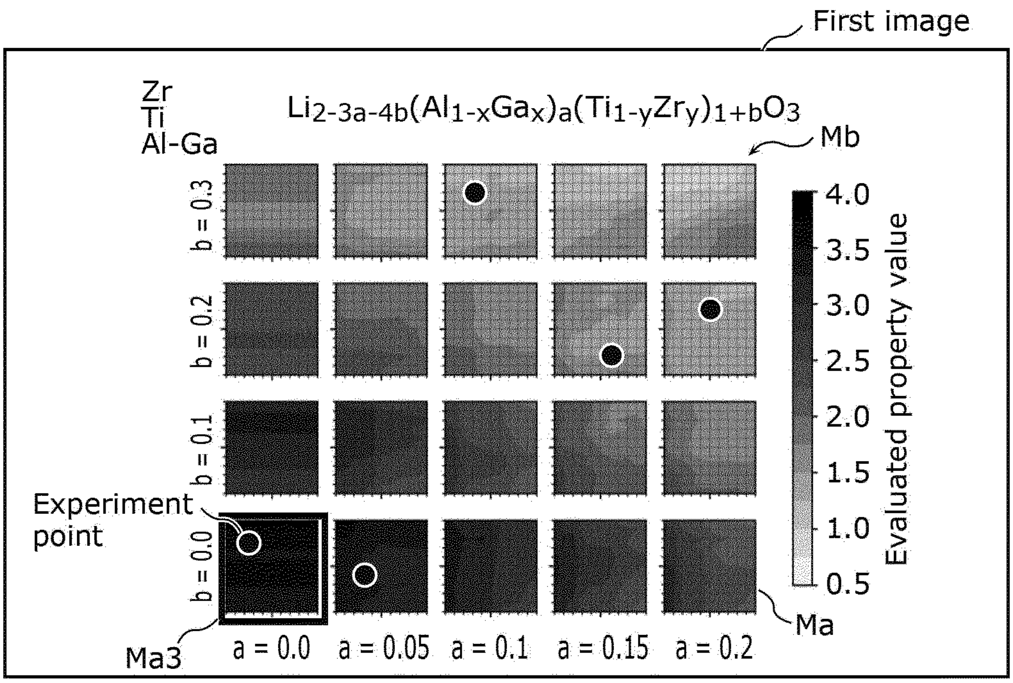


(a)

First display target information d15

Setting of display target of evaluated property value

- ☒ **Property 1 (band gap_eV)**
- ☐ Property 2 (heat-resistance temperature_°C)
- ☐ Crystal phase
- ☐ Superimposition

(b)

# FIG. 59B

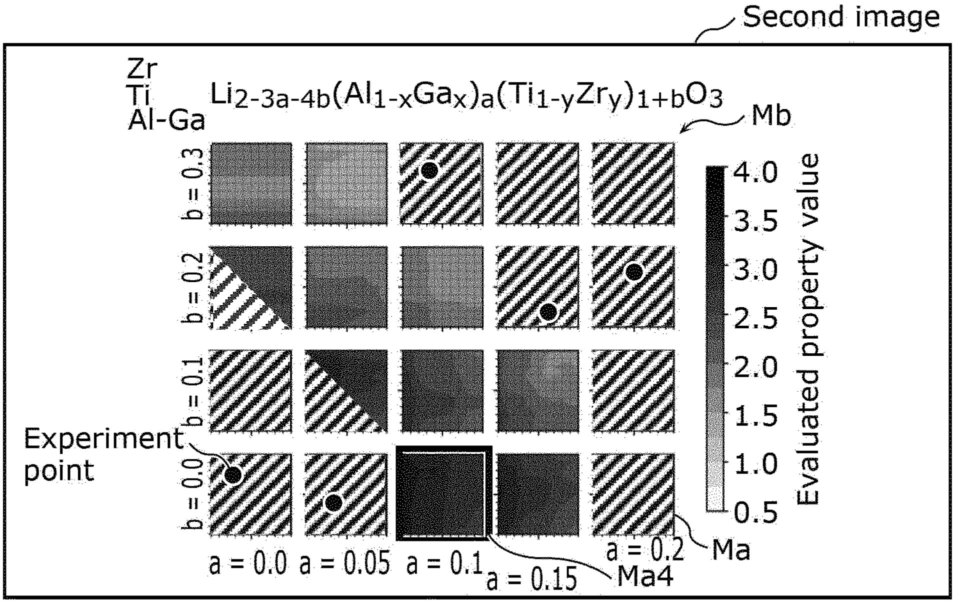


(a)

First display target information d15

Setting of display target of evaluated property value

☒ **Property 1 (band gap_eV)**

☐ Property 2 (heat-resistance temperature_°C)

☐ Crystal phase

☒ **Superimposition**

☐ Property 1 (band gap_eV)

☒ **Property 2 (heat-resistance temperature_°C)**

**Threshold** ☐ Disable ☒ **Enable : 200 or more**

☐ Crystal phase

(b)

FIG. 60A

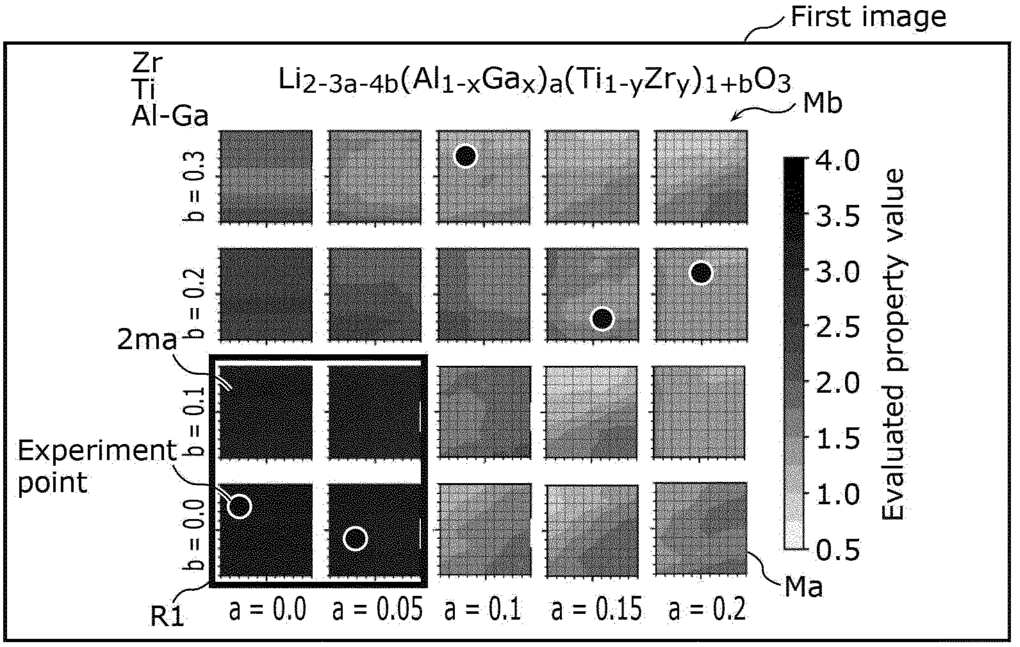


(a)

Display range information d16

Setting of display range of evaluated property value

- Upper limit
  - ☒ **Data maximum value**
  - ☐ User-defined option
- Lower limit
  - ☒ **Data minimum value**
  - ☐ User-defined option

(b)

FIG. 60B

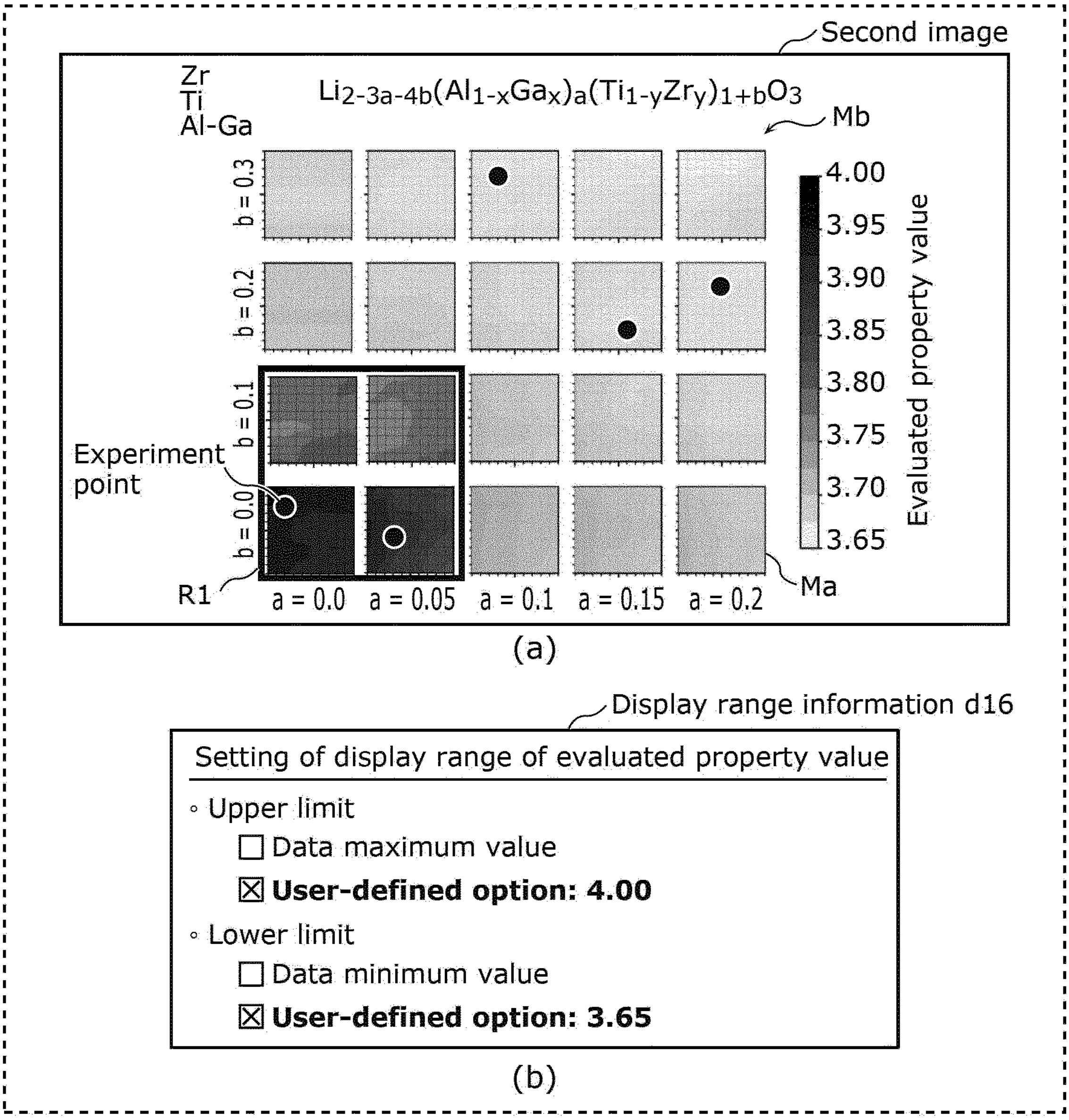
Second image
Zr
Ti
Al-Ga
$Li_{2-3a-4b}(Al_{1-x}Ga_x)_a(Ti_{1-y}Zr_y)_{1+b}O_3$
Mb
b = 0.3
b = 0.2
b = 0.1
b = 0.0
Experiment point
R1
a = 0.0
a = 0.05
a = 0.1
a = 0.15
a = 0.2
Ma
4.00
3.95
3.90
3.85
3.80
3.75
3.70
3.65
Evaluated property value
(a)
Display range information d16
Setting of display range of evaluated property value
◦ Upper limit
☐ Data maximum value
☒ User-defined option: 4.00
◦ Lower limit
☐ Data minimum value
☒ User-defined option: 3.65
(b)

# FIG. 61A

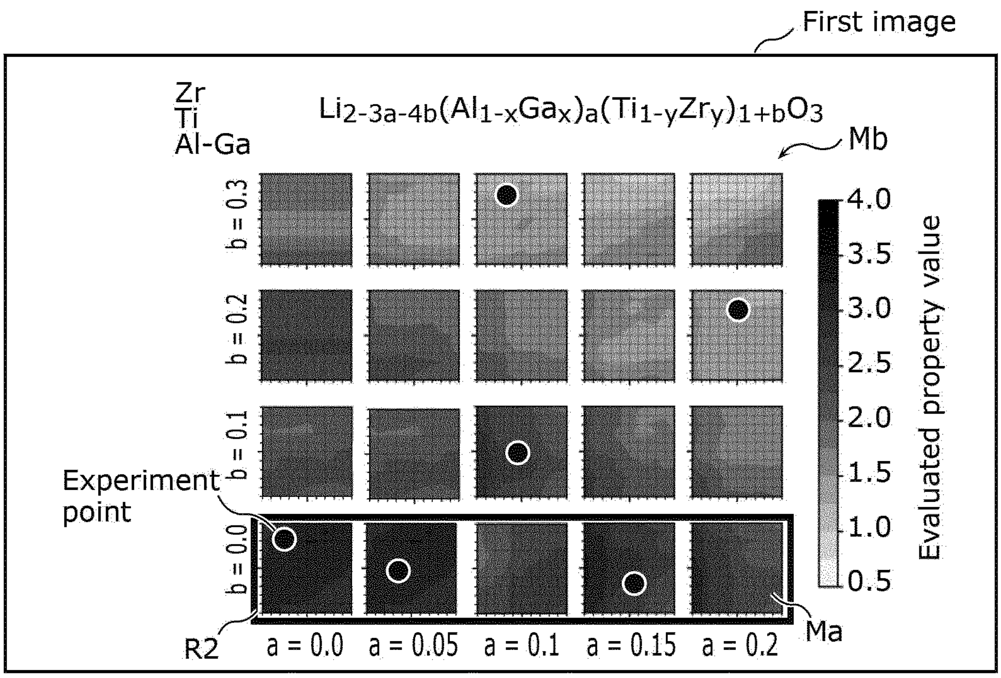


(a)

Search range information d14

Setting of search range

- Data representation :
  - Composition formula : $Li_{2-3a-4b}(M3_{1-x}M3'_x)_a(M4_{1-y}M4'_y)_{1+b}O_3$
  - Processing condition :
    - Calcination temperature_°C : Temp
    - Calcination time_h : Time
- Range variables:
  - M3, M3′ : La, Al, Ga, In
  - M4, M4′ : Ti, Zr, Hf
  - a : 0.0, 0.05, 0.1, 0.15, 0.2
  - b : 0.0, 0.1, 0.2, 0.3
  - Temp : 300, 400, 500, 600
  - Time : 1, 3, 5
  - x : Minimum value 0.0 Maximum value 1.0 Step width 0.1
  - y : Minimum value 0.0 Maximum value 1.0 Step width 0.1

(b)

# FIG. 61B

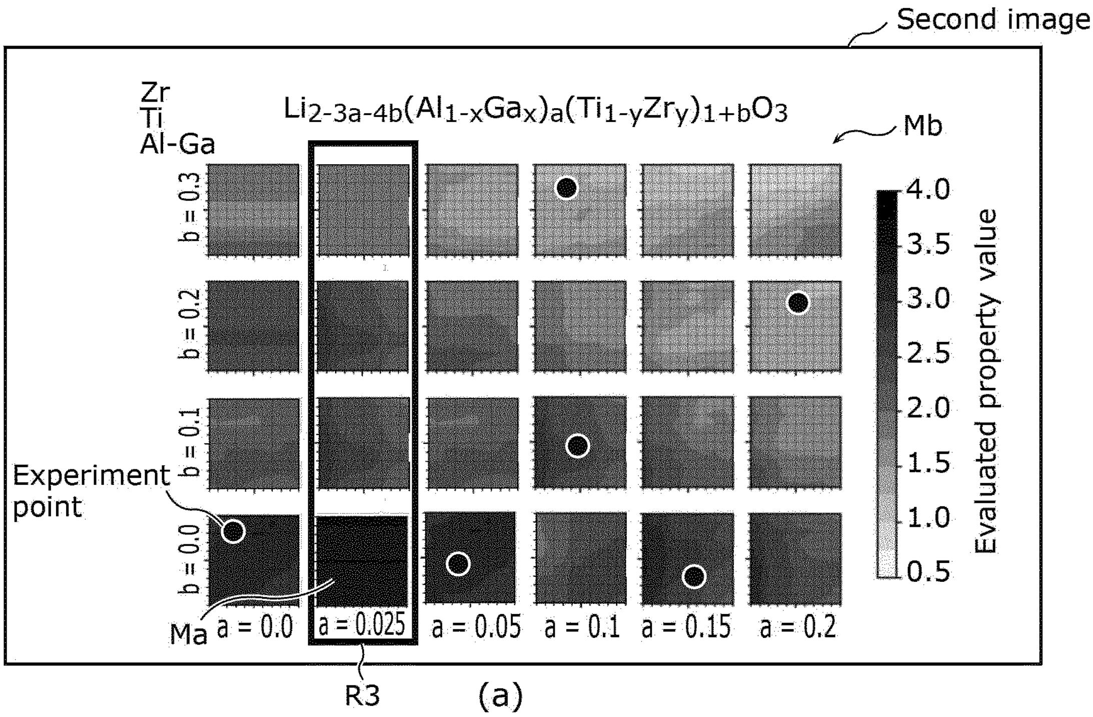


(a)

Search range information d14

**Setting of search range**

- Data representation :
  - Composition formula : $Li_{2-3a-4b}(M3_{1-x}M3'_x)_a(M4_{1-y}M4'_y)_{1+b}O_3$
  - Processing condition :
    - Calcination temperature_°C : Temp
    - Calcination time_h : Time
- Range variables:
  - M3, M3' : La, Al, Ga, In
  - M4, M4' : Ti, Zr, Hf
  - **a** : 0.0, **0.025**, 0.05, 0.1, 0.15, 0.2
  - b : 0.0, 0.1, 0.2, 0.3
  - Temp : 300, 400, 500, 600
  - Time : 1, 3, 5
  - x : Minimum value 0.0 Maximum value 1.0 Step width 0.1
  - y : Minimum value 0.0 Maximum value 1.0 Step width 0.1

(b)

FIG. 62

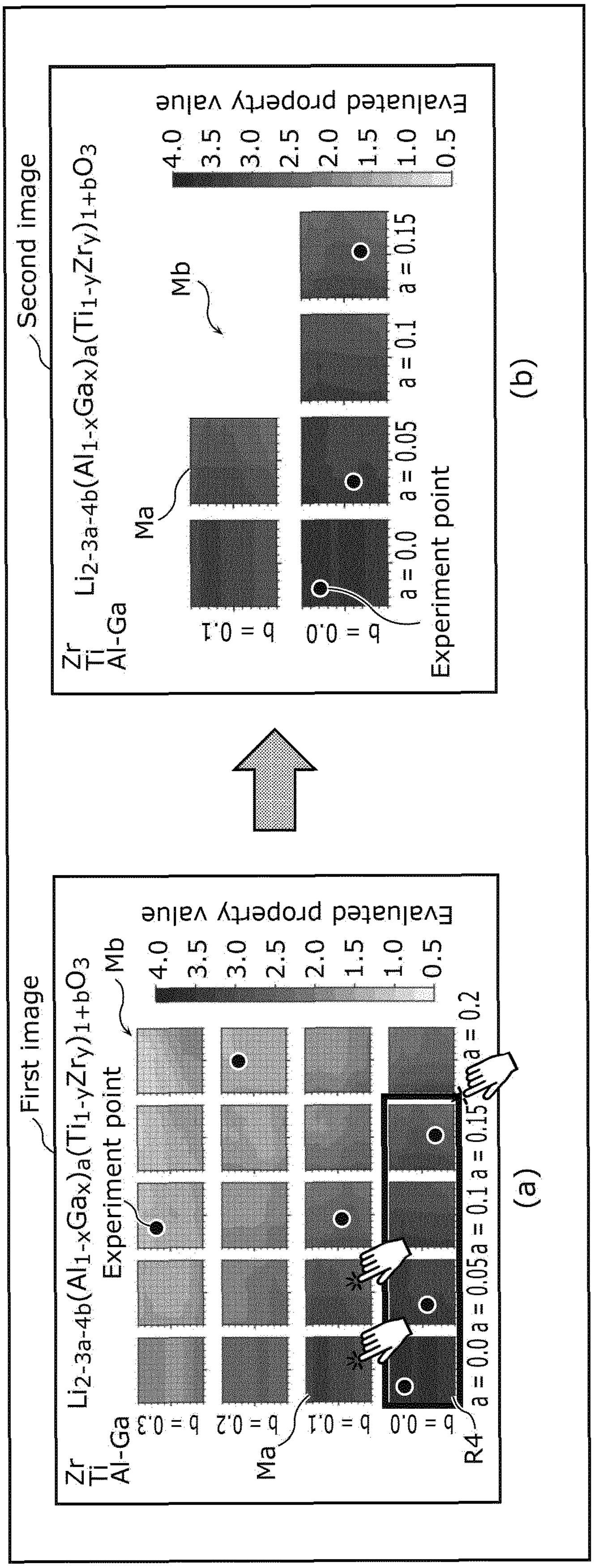
First image
Zr
Ti
Al-Ga
$Li_{2-3a-4b}(Al_{1-x}Ga_x)_a(Ti_{1-y}Zr_y)_{1+b}O_3$
Experiment point
Mb
Ma
R4
b = 0.3
b = 0.2
b = 0.1
b = 0.0
a = 0.0
a = 0.05
a = 0.1
a = 0.15
a = 0.2
4.0
3.5
3.0
2.5
2.0
1.5
1.0
0.5
Evaluated property value
(a)
Second image
Zr
Ti
Al-Ga
$Li_{2-3a-4b}(Al_{1-x}Ga_x)_a(Ti_{1-y}Zr_y)_{1+b}O_3$
Ma
Mb
b = 0.1
b = 0.0
a = 0.0
a = 0.05
a = 0.1
a = 0.15
Experiment point
4.0
3.5
3.0
2.5
2.0
1.5
1.0
0.5
Evaluated property value
(b)

FIG. 63

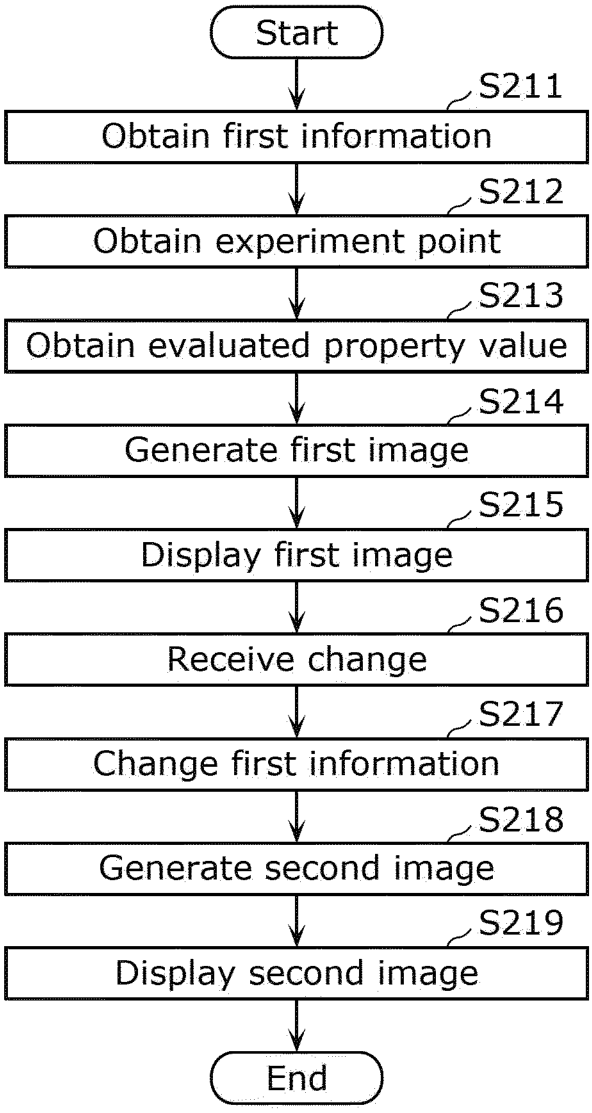
Start
S211
Obtain first information
S212
Obtain experiment point
S213
Obtain evaluated property value
S214
Generate first image
S215
Display first image
S216
Receive change
S217
Change first information
S218
Generate second image
S219
Display second image
End

FIG. 64

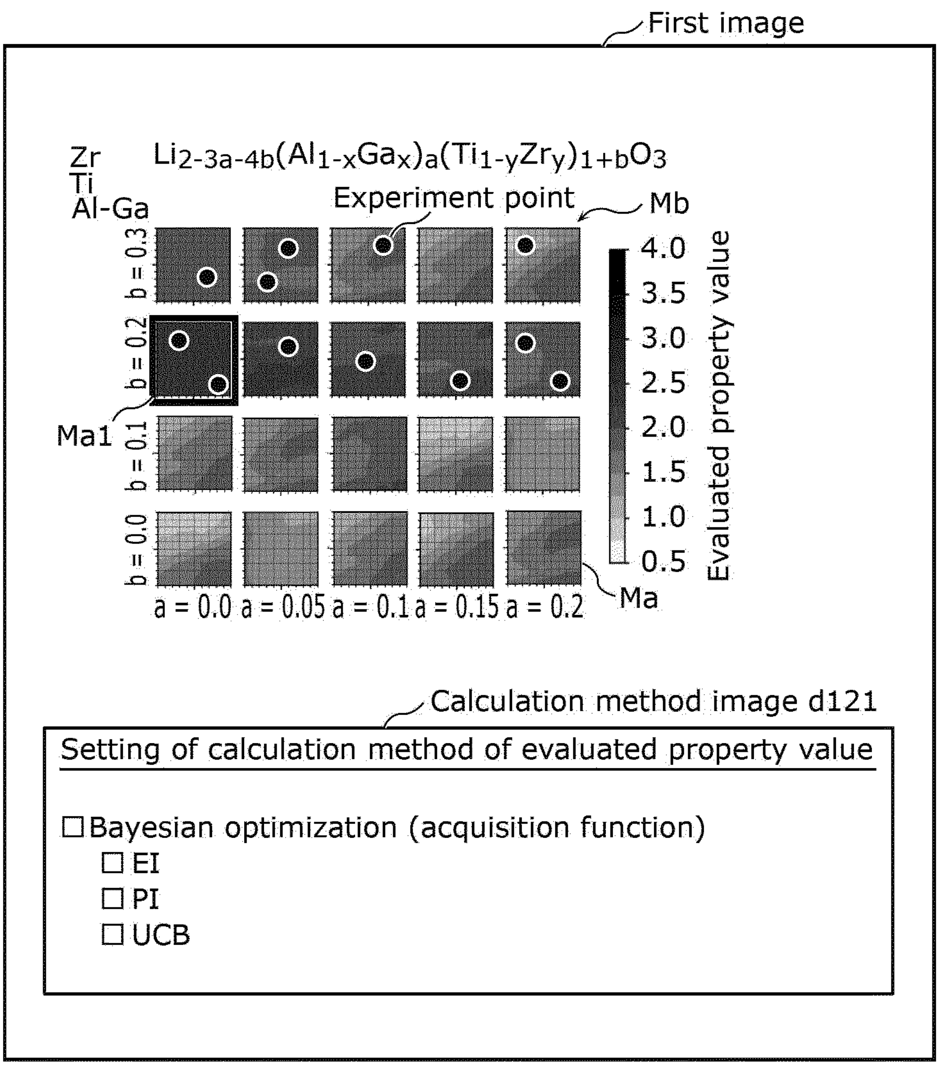
First image
Zr
Ti
Al-Ga
$Li_{2-3a-4b}(Al_{1-x}Ga_x)_a(Ti_{1-y}Zr_y)_{1+b}O_3$
Experiment point
Mb
b = 0.3
b = 0.2
b = 0.1
b = 0.0
Ma1
a = 0.0 a = 0.05 a = 0.1 a = 0.15 a = 0.2
Ma
4.0
3.5
3.0
2.5
2.0
1.5
1.0
0.5
Evaluated property value
Calculation method image d121
Setting of calculation method of evaluated property value
☐Bayesian optimization (acquisition function)
☐EI
☐PI
☐UCB

FIG. 65

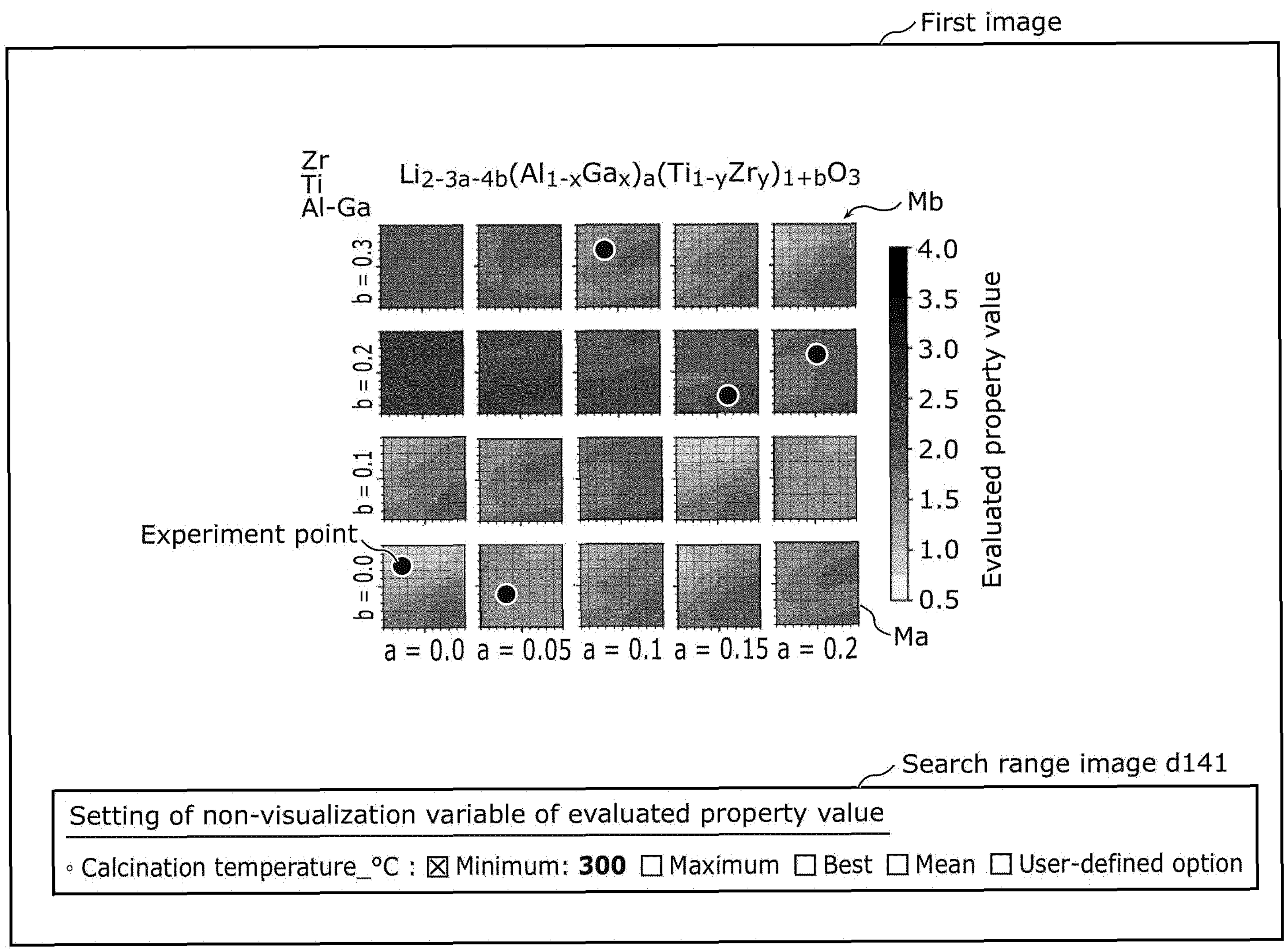
First image
Zr
Ti
Al-Ga
$Li_{2-3a-4b}(Al_{1-x}Ga_x)_a(Ti_{1-y}Zr_y)_{1+b}O_3$
Mb
b = 0.3
b = 0.2
b = 0.1
b = 0.0
Experiment point
a = 0.0 a = 0.05 a = 0.1 a = 0.15 a = 0.2
Ma
4.0
3.5
3.0
2.5
2.0
1.5
1.0
0.5
Evaluated property value
Search range image d141
Setting of non-visualization variable of evaluated property value
◦ Calcination temperature_°C : ☒ Minimum: **300** ☐ Maximum ☐ Best ☐ Mean ☐ User-defined option

FIG. 66

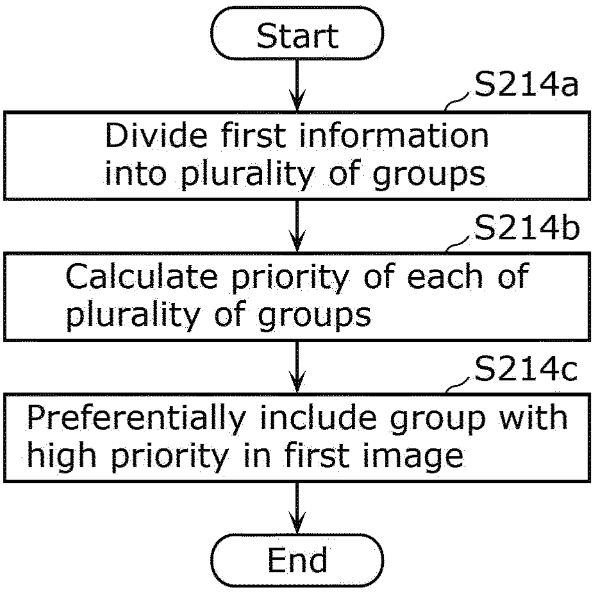
Start
S214a
Divide first information into plurality of groups
S214b
Calculate priority of each of plurality of groups
S214c
Preferentially include group with high priority in first image
End

FIG. 67

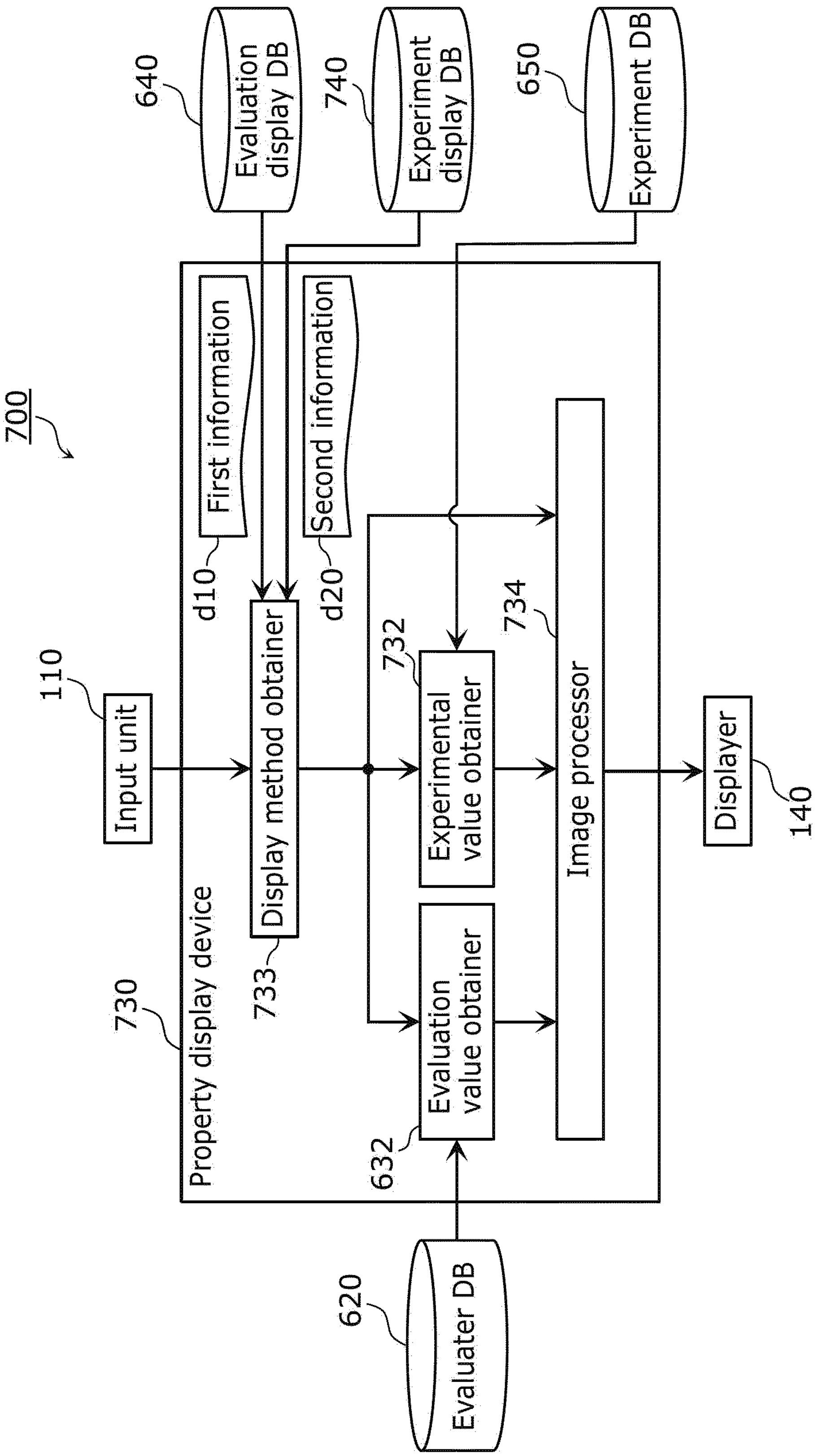
700
640
Evaluation display DB
740
Experiment display DB
650
Experiment DB
First information
Second information
d10
d20
110
Input unit
Display method obtainer
733
732
Experimental value obtainer
734
Image processor
Displayer
140
730
Property display device
Evaluation value obtainer
632
620
Evaluater DB

FIG. 68

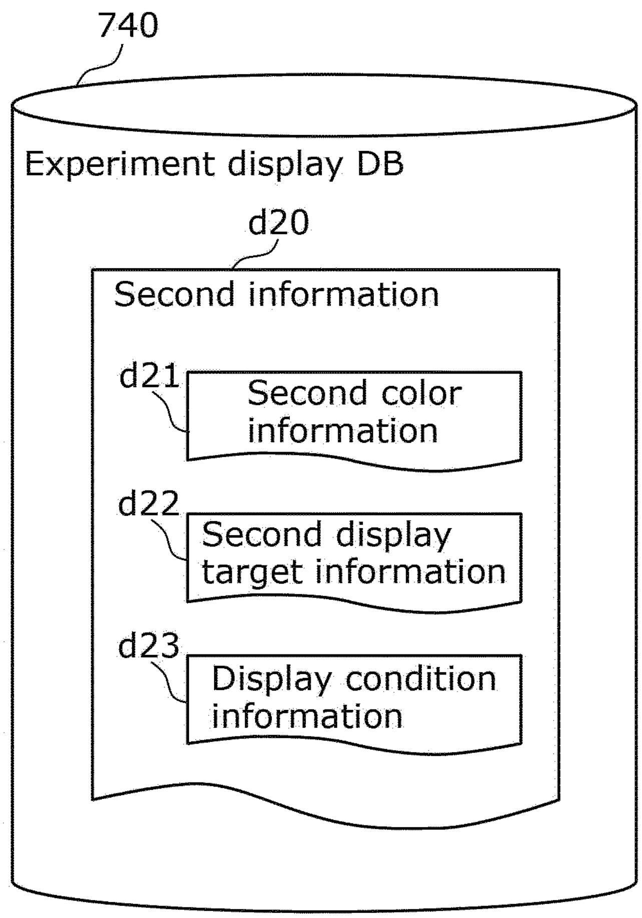
740
Experiment display DB
d20
Second information
d21
Second color information
d22
Second display target information
d23
Display condition information

# FIG. 69A

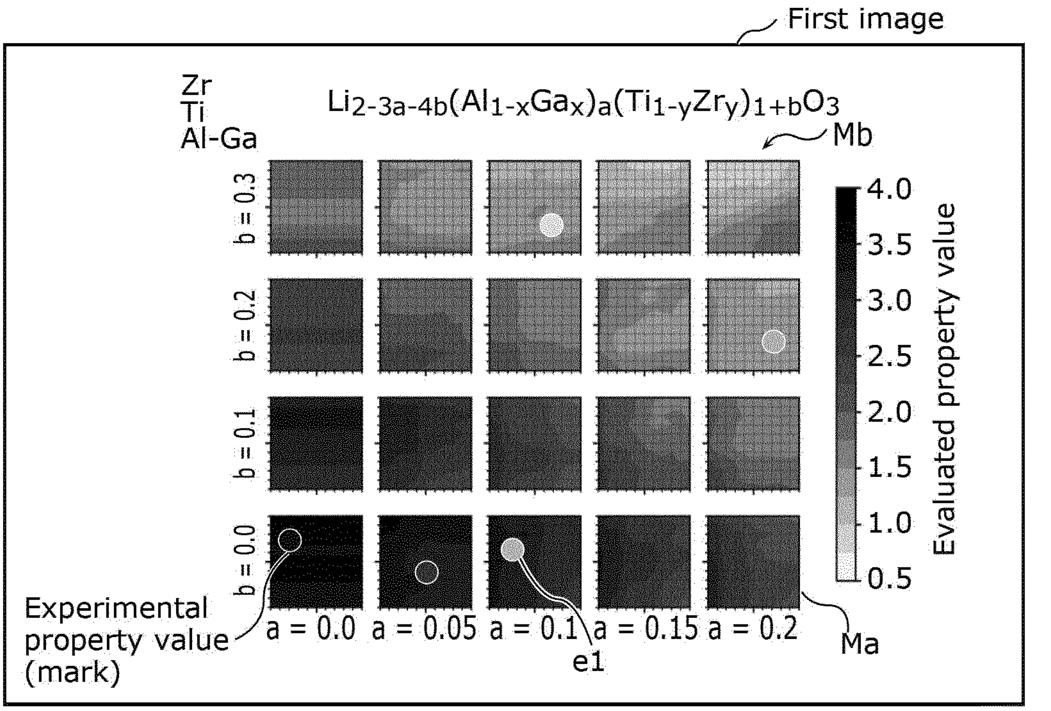


(a)

Second display target information d22

Setting of display target of experimental property value

- Shape
  - ☒ **Property 1 (band gap_eV)**
  - ☐ Property 2 (heat-resistance temperature_°C)
  - ☐ Crystal phase
- Shade of color
  - ☒ **Property 1 (band gap_eV)**
  - ☐ Property 2 (heat-resistance temperature_°C)
  - ☐ Crystal phase

(b)

# FIG. 69B

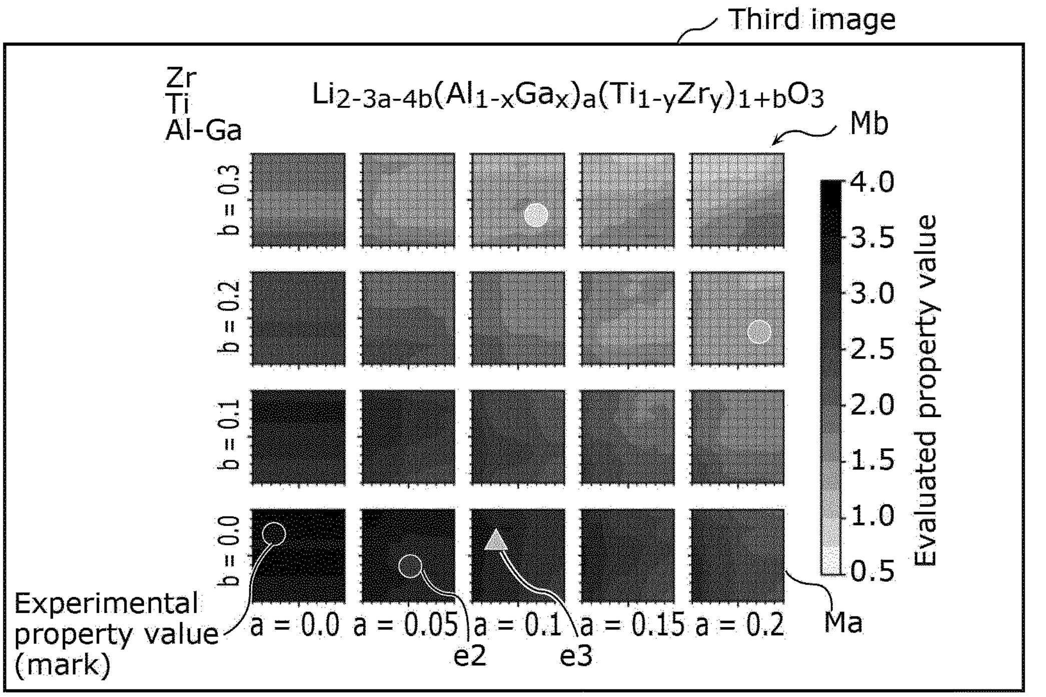


(a)

Second display target information d22

Setting of display target of experimental property value

- Shape
  - ☐ Property 1 (band gap_eV)
  - ☐ Property 2 (heat-resistance temperature_°C)
  - ☒ **Crystal phase**
- Shade of color
  - ☒ **Property 1 (band gap_eV)**
  - ☐ Property 2 (heat-resistance temperature_°C)
  - ☐ Crystal phase

(b)

FIG. 70A

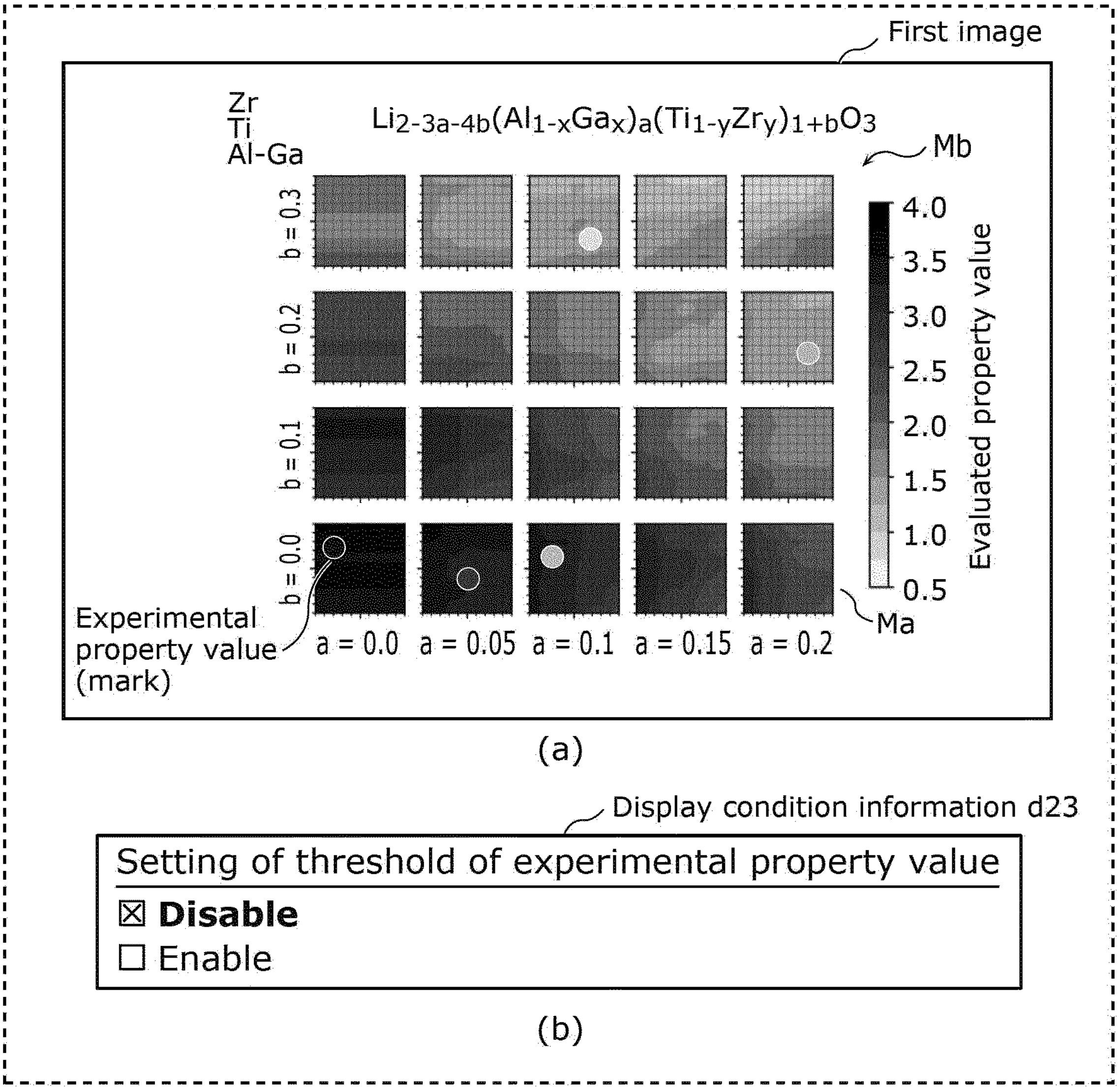
First image
Zr
Ti
Al-Ga
$Li_{2-3a-4b}(Al_{1-x}Ga_x)_a(Ti_{1-y}Zr_y)_{1+b}O_3$
Mb
b = 0.3
b = 0.2
b = 0.1
b = 0.0
4.0
3.5
3.0
2.5
2.0
1.5
1.0
0.5
Evaluated property value
Ma
Experimental property value (mark)
a = 0.0 a = 0.05 a = 0.1 a = 0.15 a = 0.2
(a)
Display condition information d23
Setting of threshold of experimental property value
☒ Disable
☐ Enable
(b)

FIG. 70B

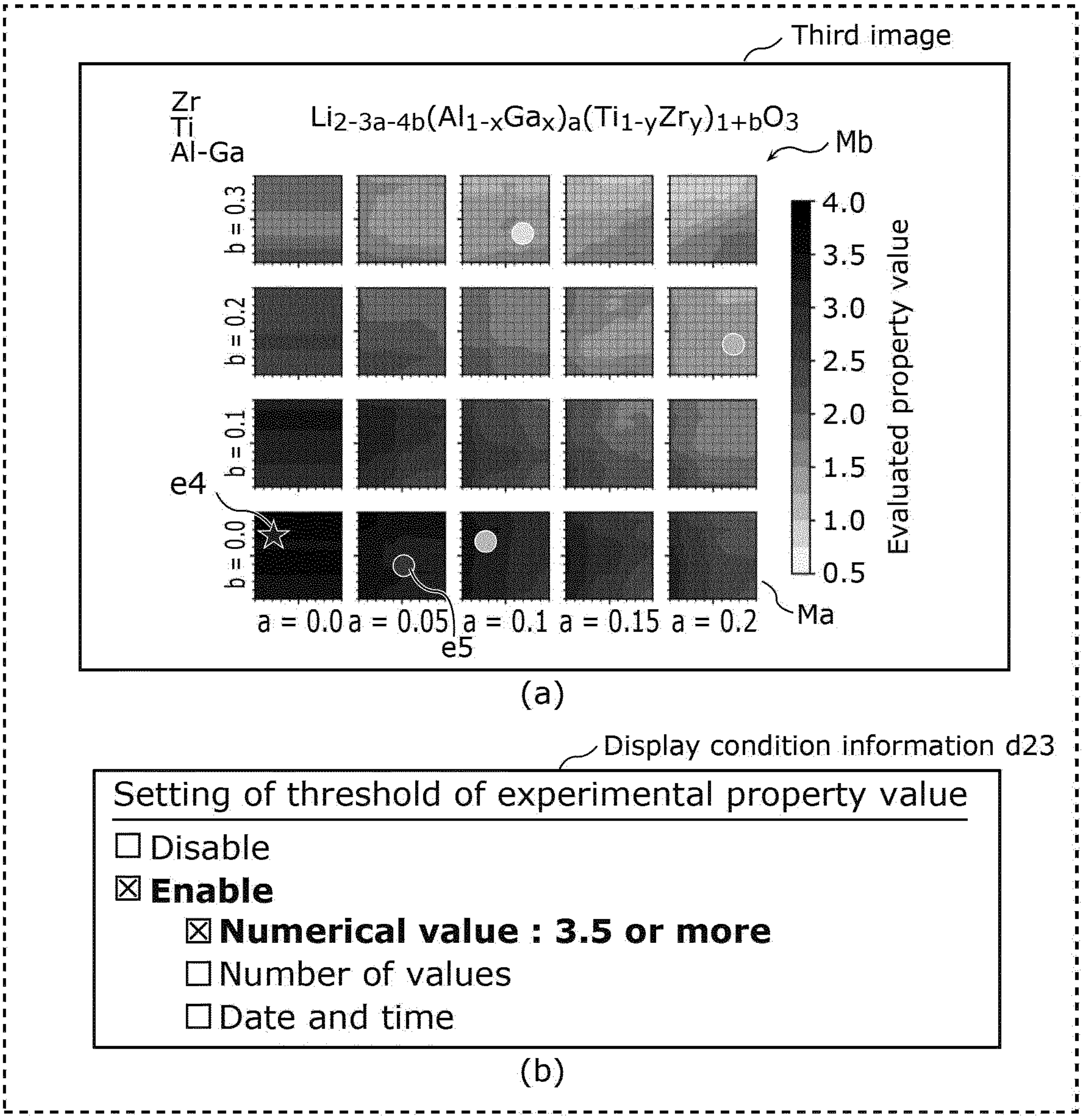
Third image
Zr
Ti
Al-Ga
Li2-3a-4b(Al1-xGax)a(Ti1-yZry)1+bO3
Mb
b = 0.3
b = 0.2
b = 0.1
b = 0.0
e4
4.0
3.5
3.0
2.5
2.0
1.5
1.0
0.5
Evaluated property value
a = 0.0
a = 0.05
a = 0.1
a = 0.15
a = 0.2
e5
Ma
(a)
Display condition information d23
Setting of threshold of experimental property value
☐ Disable
☒ Enable
☒ Numerical value : 3.5 or more
☐ Number of values
☐ Date and time
(b)

FIG. 71

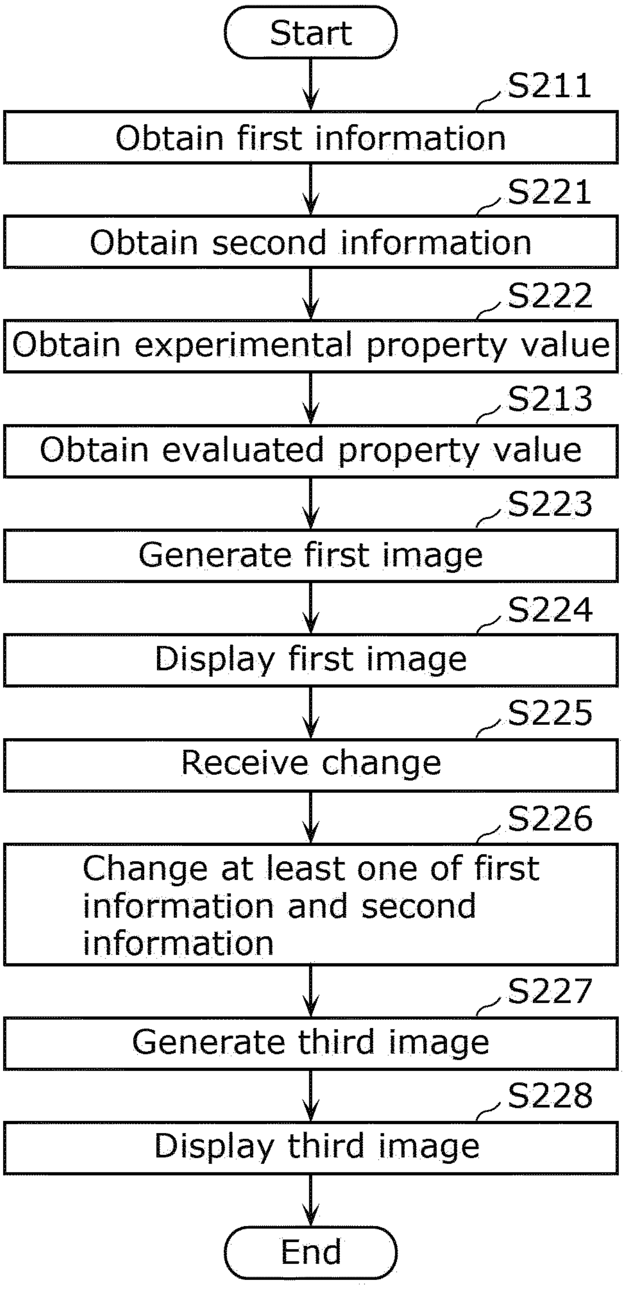
Start
S211
Obtain first information
S221
Obtain second information
S222
Obtain experimental property value
S213
Obtain evaluated property value
S223
Generate first image
S224
Display first image
S225
Receive change
S226
Change at least one of first information and second information
S227
Generate third image
S228
Display third image
End

FIG. 72

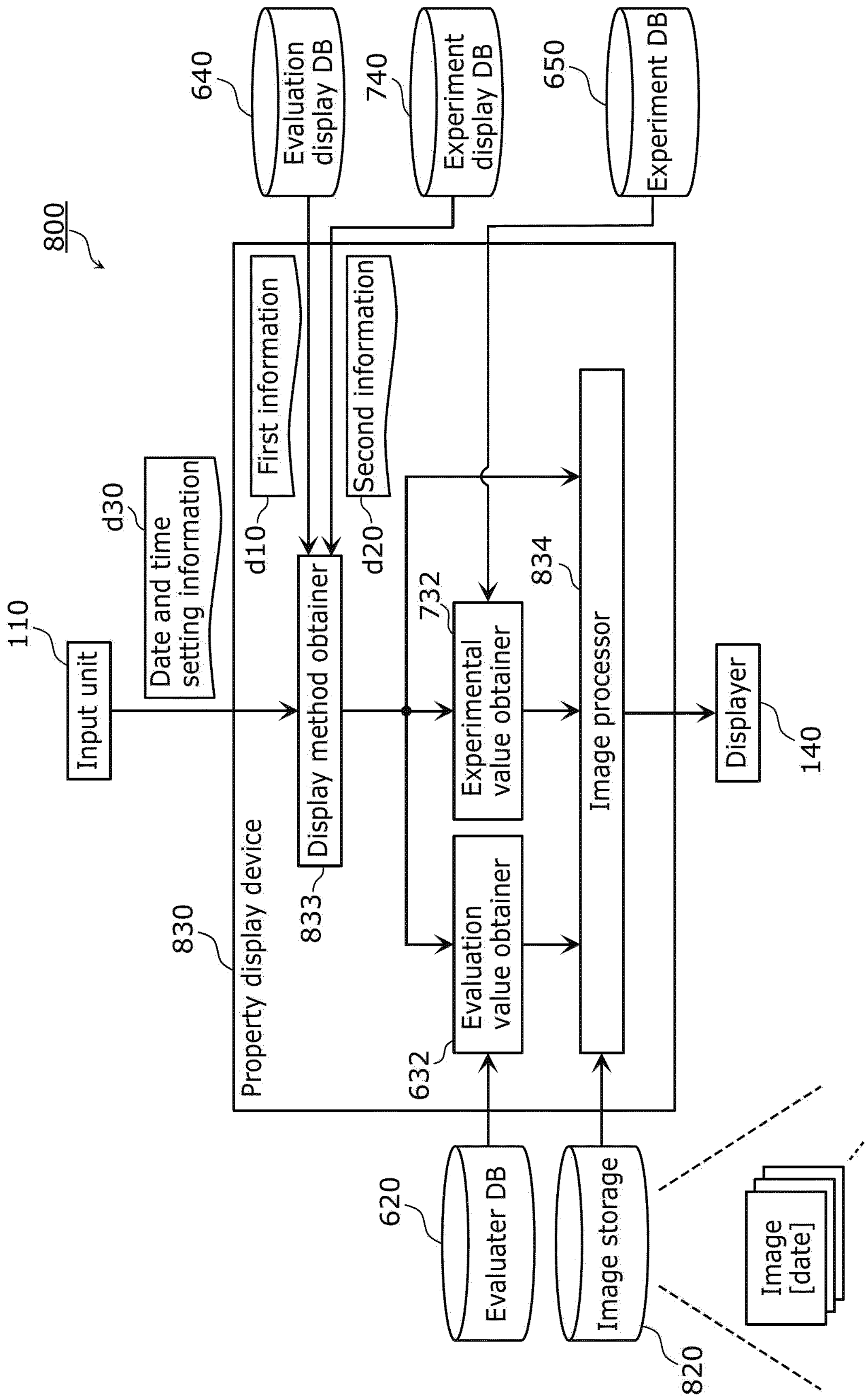
800
110
Input unit
d30
Date and time setting information
640
Evaluation display DB
740
Experiment display DB
650
Experiment DB
830
Property display device
d10
First information
d20
Second information
833
Display method obtainer
732
Experimental value obtainer
632
Evaluation value obtainer
834
Image processor
140
Displayer
620
Evaluater DB
820
Image storage
Image [date]

FIG. 73A

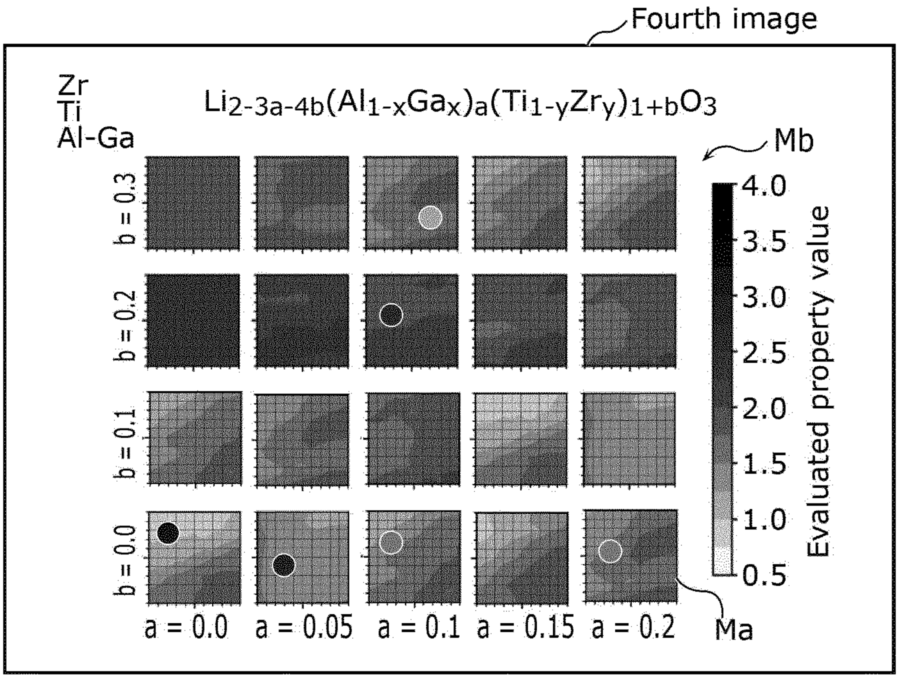
Fourth image
Zr
Ti
Al-Ga
Li2-3a-4b(Al1-xGax)a(Ti1-yZry)1+bO3
Mb
b = 0.3
b = 0.2
b = 0.1
b = 0.0
a = 0.0
a = 0.05
a = 0.1
a = 0.15
a = 0.2
Ma
4.0
3.5
3.0
2.5
2.0
1.5
1.0
0.5
Evaluated property value

(a)

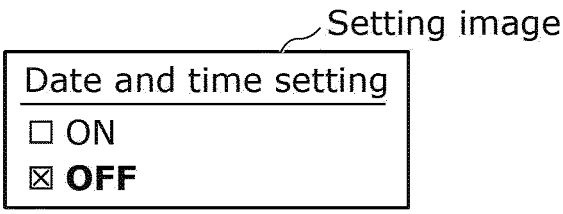
Setting image
Date and time setting
☐ ON
☒ OFF

(b)

FIG. 73B

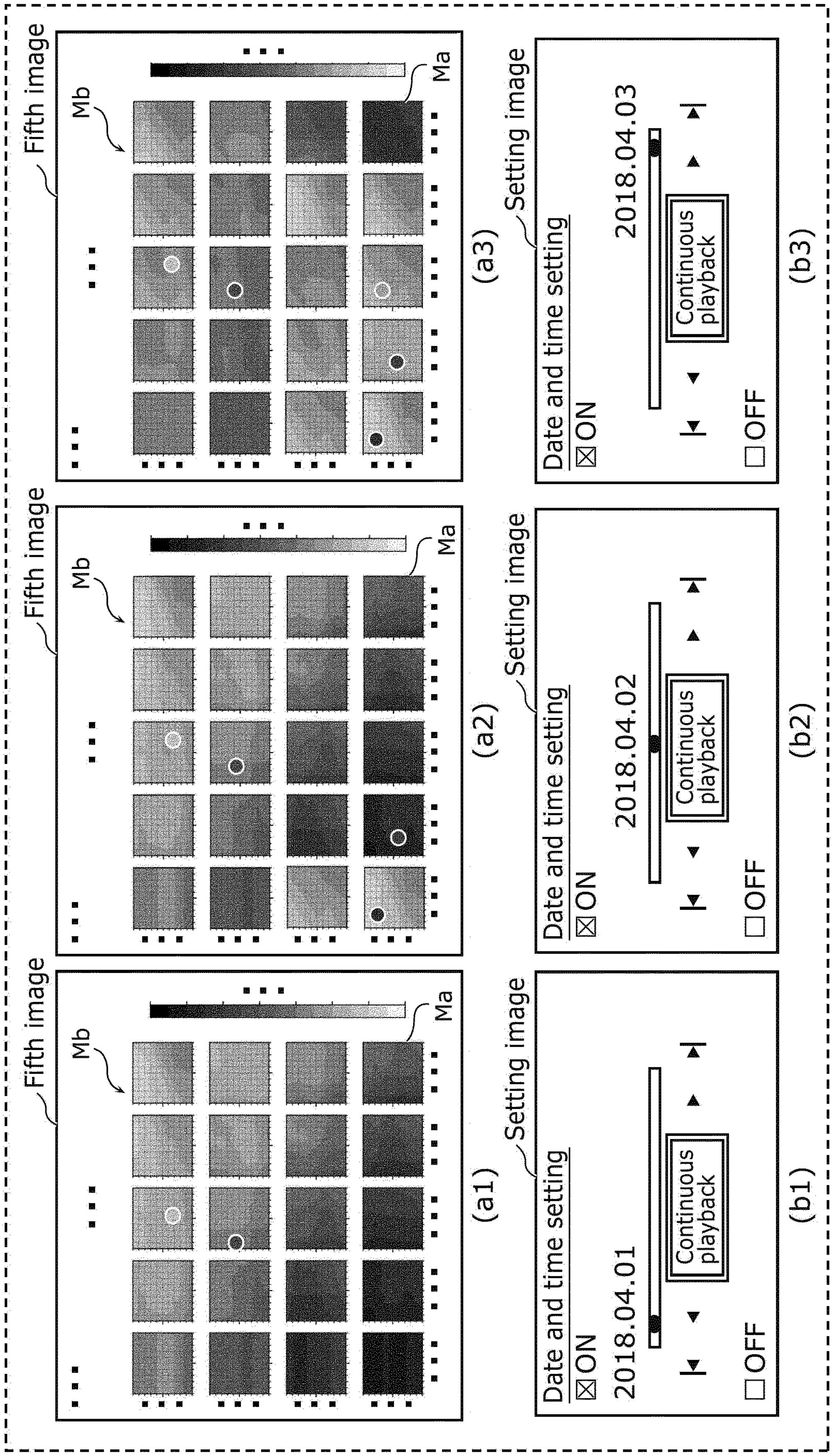
Fifth image
Mb
Ma
(a1)
Setting image
Date and time setting
☒ON
2018.04.01
Continuous playback
☐OFF
(b1)
Fifth image
Mb
Ma
(a2)
Setting image
Date and time setting
☒ON
2018.04.02
Continuous playback
☐OFF
(b2)
Fifth image
Mb
Ma
(a3)
Setting image
Date and time setting
☒ON
2018.04.03
Continuous playback
☐OFF
(b3)

FIG. 74

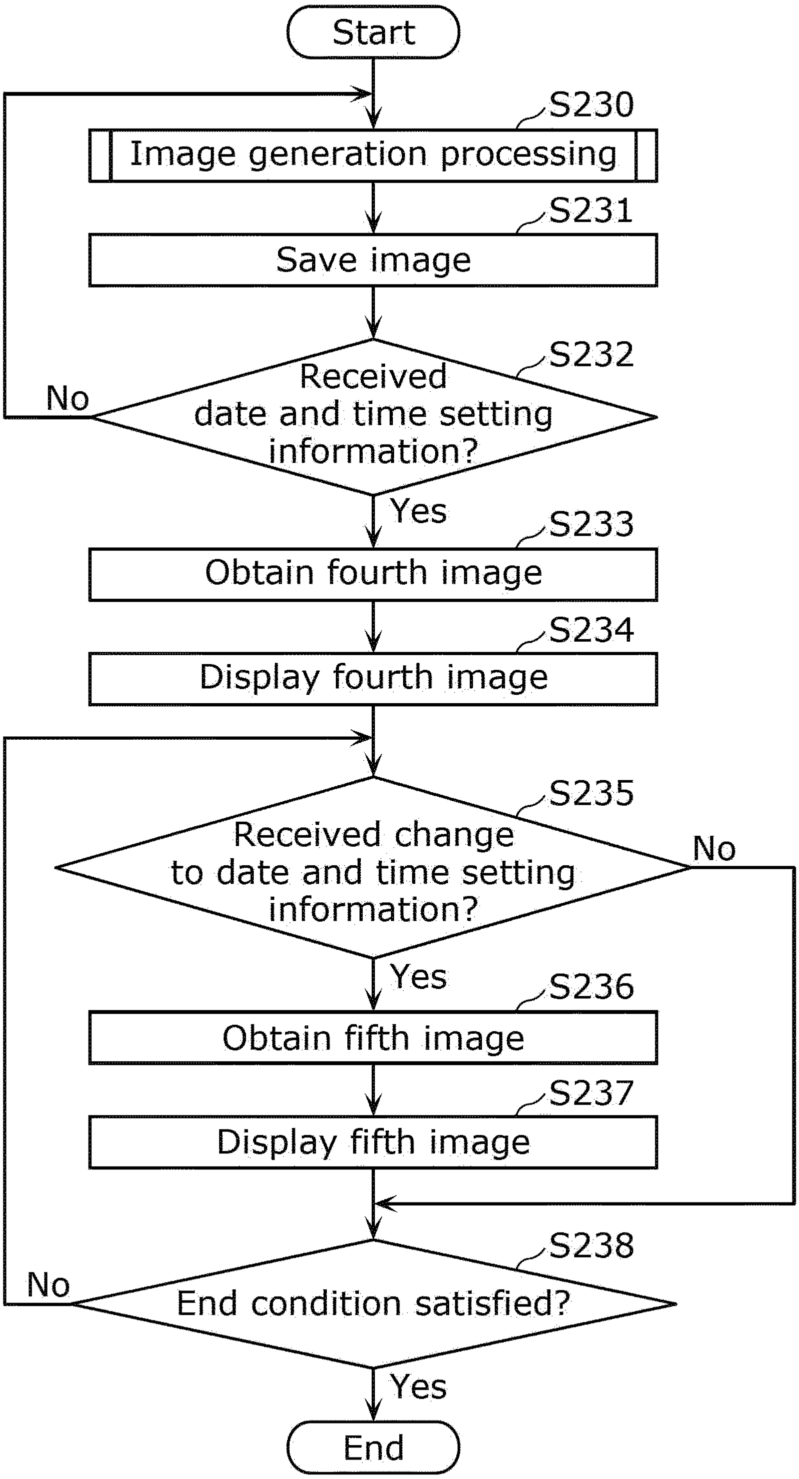
Start
S230
Image generation processing
S231
Save image
S232
Received date and time setting information?
No
Yes
S233
Obtain fourth image
S234
Display fourth image
S235
Received change to date and time setting information?
No
Yes
S236
Obtain fifth image
S237
Display fifth image
S238
End condition satisfied?
No
Yes
End

FIG. 75

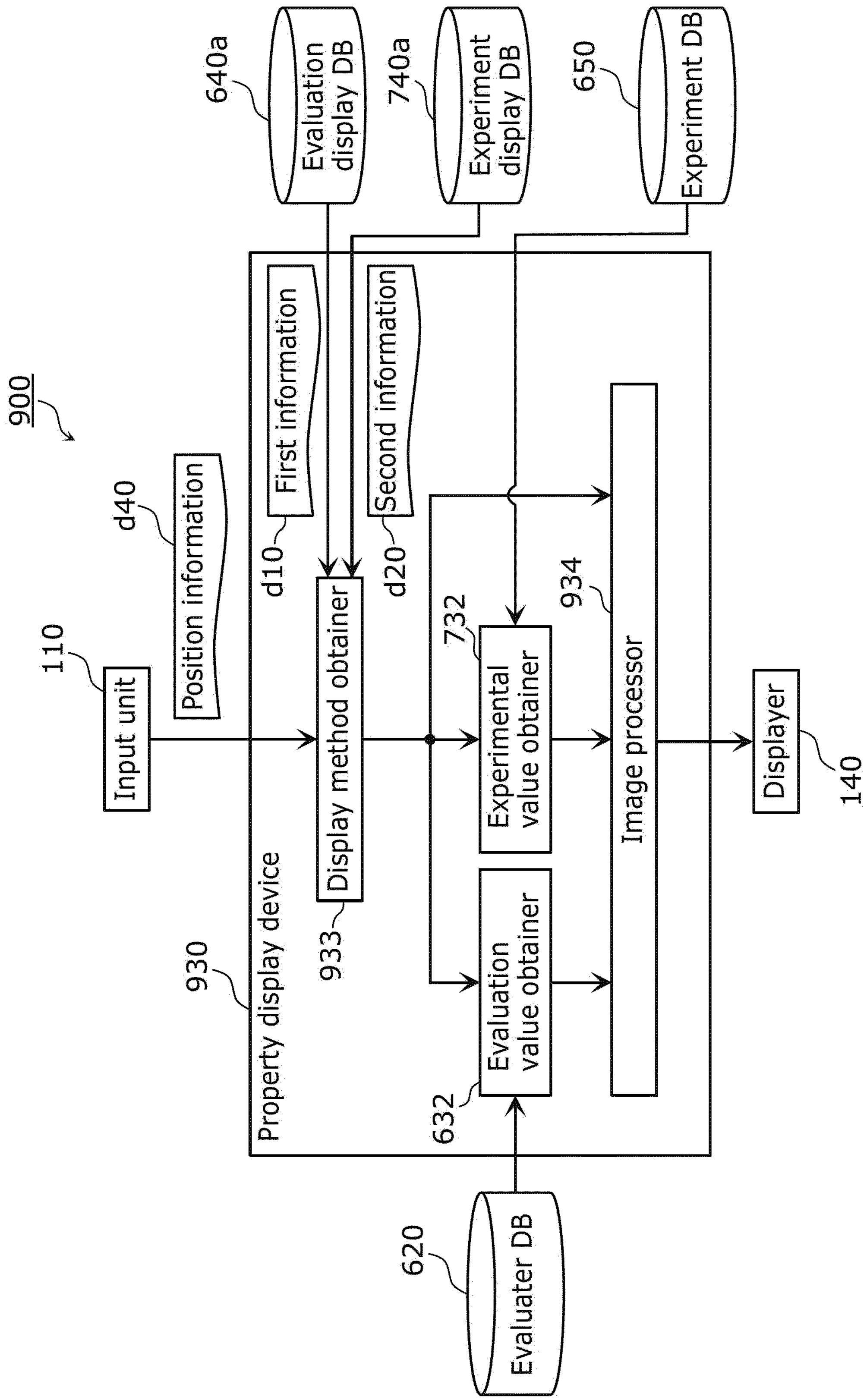
900
110
Input unit
d40
Position information
930
Property display device
933
Display method obtainer
d10
First information
d20
Second information
640a
Evaluation display DB
740a
Experiment display DB
650
Experiment DB
732
Experimental value obtainer
632
Evaluation value obtainer
934
Image processor
140
Displayer
620
Evaluater DB

FIG. 76

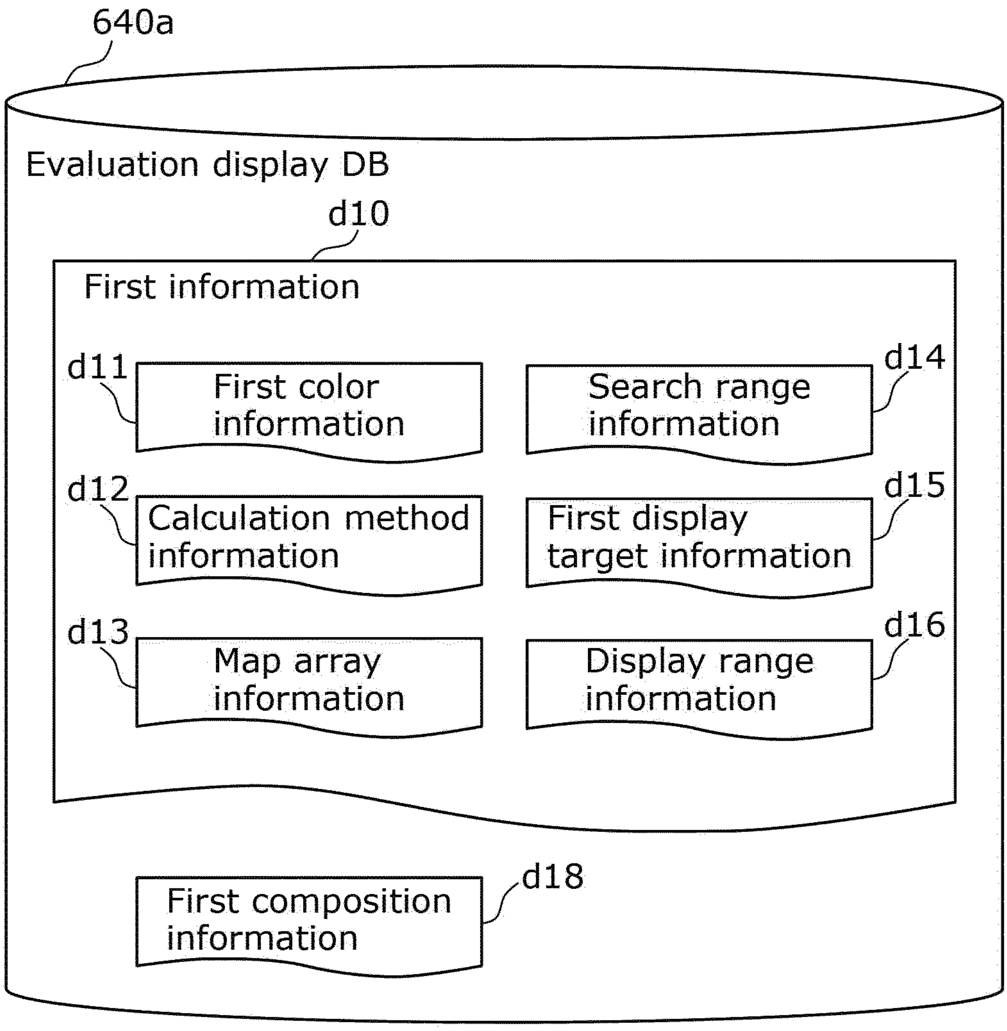
640a
Evaluation display DB
d10
First information
d11
First color information
d12
Calculation method information
d13
Map array information
d14
Search range information
d15
First display target information
d16
Display range information
First composition information
d18

FIG. 77

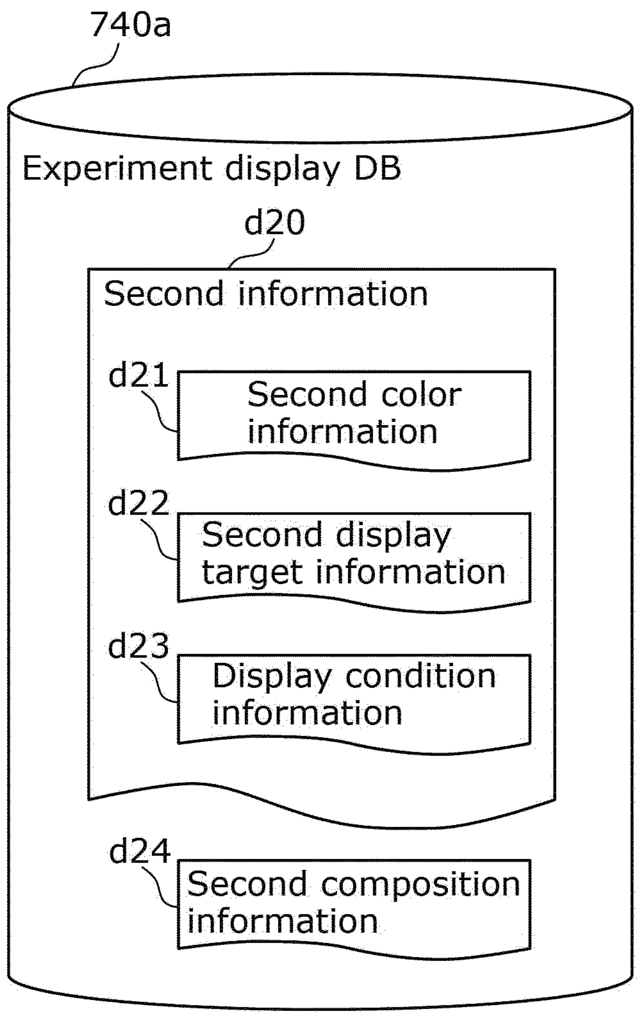
740a
Experiment display DB
d20
Second information
d21
Second color information
d22
Second display target information
d23
Display condition information
d24
Second composition information

FIG. 78

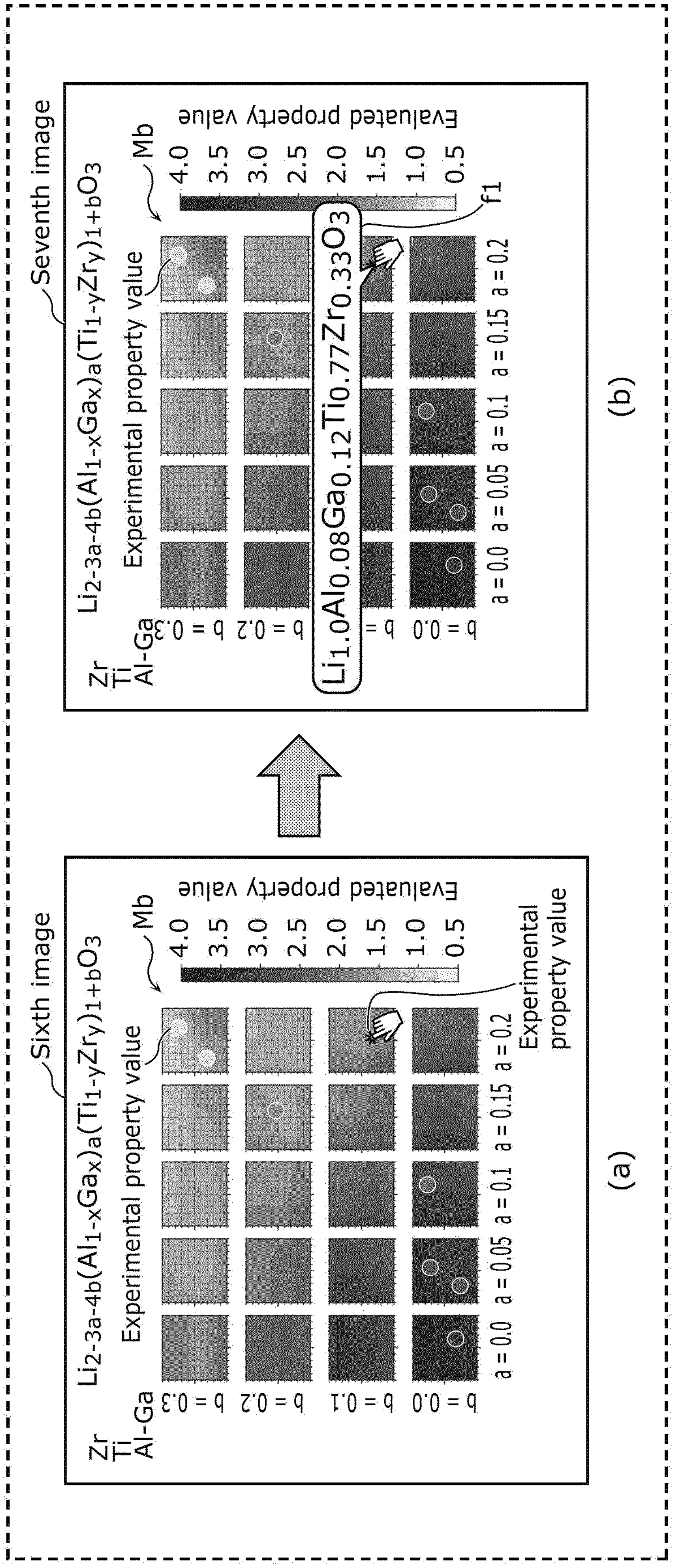

Sixth image
Li2-3a-4b(Al1-xGax)a(Ti1-yZry)1+bO3
Experimental property value
Zr
Ti
Al-Ga
b = 0.3
b = 0.2
b = 0.1
b = 0.0
a = 0.0
a = 0.05
a = 0.1
a = 0.15
a = 0.2
Mb
4.0
3.5
3.0
2.5
2.0
1.5
1.0
0.5
Evaluated property value
Experimental property value
(a)
Seventh image
Li2-3a-4b(Al1-xGax)a(Ti1-yZry)1+bO3
Experimental property value
Zr
Ti
Al-Ga
b = 0.3
b = 0.2
b =
b = 0.0
a = 0.0
a = 0.05
a = 0.1
a = 0.15
a = 0.2
Li1.0Al0.08Ga0.12Ti0.77Zr0.33O3
f1
Mb
4.0
3.5
3.0
2.5
2.0
1.5
1.0
0.5
Evaluated property value
(b)

FIG. 79

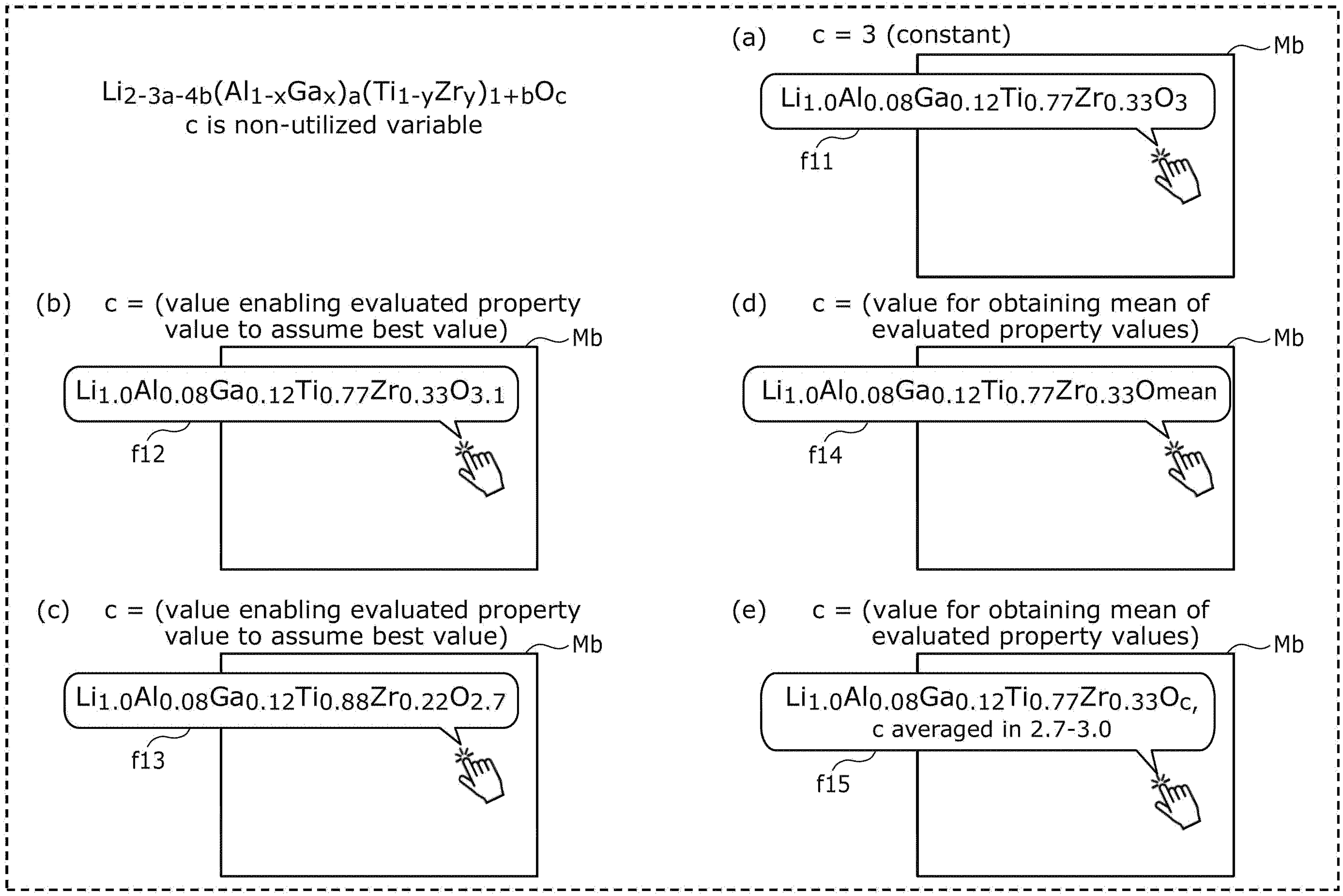
$Li_{2-3a-4b}(Al_{1-x}Ga_x)_a(Ti_{1-y}Zr_y)_{1+b}O_c$
c is non-utilized variable
(a) c = 3 (constant)
$Li_{1.0}Al_{0.08}Ga_{0.12}Ti_{0.77}Zr_{0.33}O_3$
f11
Mb
(b) c = (value enabling evaluated property value to assume best value)
$Li_{1.0}Al_{0.08}Ga_{0.12}Ti_{0.77}Zr_{0.33}O_{3.1}$
f12
Mb
(c) c = (value enabling evaluated property value to assume best value)
$Li_{1.0}Al_{0.08}Ga_{0.12}Ti_{0.88}Zr_{0.22}O_{2.7}$
f13
Mb
(d) c = (value for obtaining mean of evaluated property values)
$Li_{1.0}Al_{0.08}Ga_{0.12}Ti_{0.77}Zr_{0.33}O_{mean}$
f14
Mb
(e) c = (value for obtaining mean of evaluated property values)
$Li_{1.0}Al_{0.08}Ga_{0.12}Ti_{0.77}Zr_{0.33}O_c$, c averaged in 2.7-3.0
f15
Mb

FIG. 80

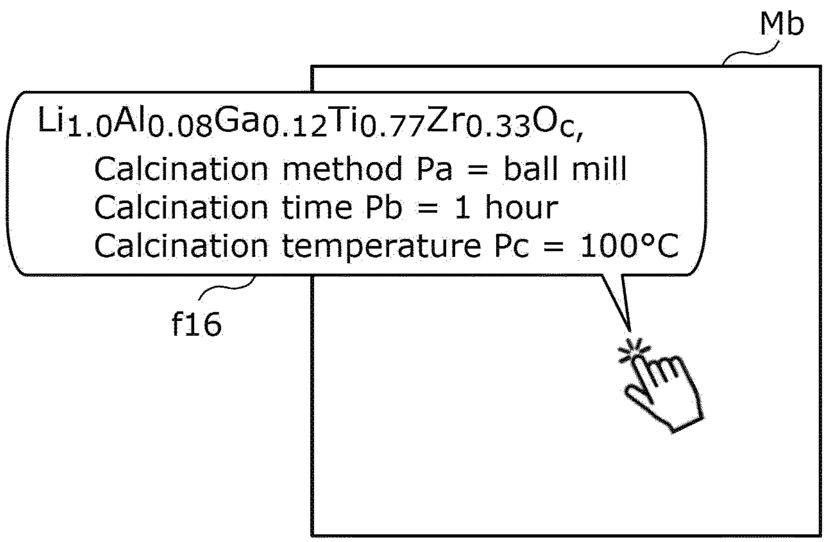
Mb
Li1.0Al0.08Ga0.12Ti0.77Zr0.33Oc,
Calcination method Pa = ball mill
Calcination time Pb = 1 hour
Calcination temperature Pc = 100°C
f16

FIG. 81

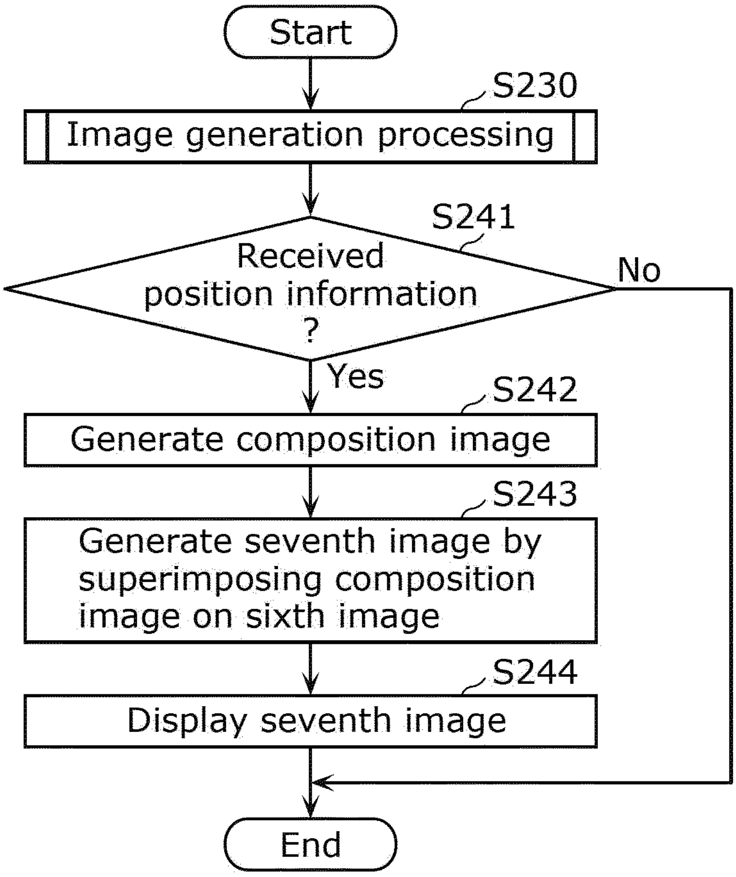
Start
S230
Image generation processing
S241
Received position information ?
No
Yes
S242
Generate composition image
S243
Generate seventh image by superimposing composition image on sixth image
S244
Display seventh image
End

## INTERNATIONAL SEARCH REPORT

International application No. **PCT/JP2022/028846**

**A. CLASSIFICATION OF SUBJECT MATTER**

***G16C 20/80***(2019.01)i; ***G01N 33/00***(2006.01)i
FI: G16C20/80; G01N33/00 D

According to International Patent Classification (IPC) or to both national classification and IPC

**B. FIELDS SEARCHED**

Minimum documentation searched (classification system followed by classification symbols)

G16C20/80; G01N33/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2020/263791 A1 (COVESTRO LLC) 30 December 2020 (2020-12-30)<br>paragraphs [0009], [0100], [0101], [0119]-[0121], [0126], [0127], fig. 3, 4A-4B, 10A-10B | 1-13, 20, 22-24<br>14-19, 21 |
| A | JP 4780554 B2 (YAMATO, Hiroshi) 28 September 2011 (2011-09-28)<br>entire text, all drawings | 1-24 |
| A | JP 2020-535566 A (COVESTRO LLC) 03 December 2020 (2020-12-03)<br>entire text, all drawings | 1-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 October 2022** | **25 October 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/028846**

| Patent document cited in search report | Publication date (day/month/year) | Patent family member(s) | Publication date (day/month/year) |
|---|---|---|---|
| WO 2020/263791 A1 | 30 December 2020 | US 2022/0318863 A1 | |
| JP 4780554 B2 | 28 September 2011 | (Family: none) | |
| JP 2020-535566 A | 03 December 2020 | US 2020/0210056 A1<br>entire text, all drawings<br>WO 2019/060268 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 6632412 B
- US 7199809 B
- JP 4009670 B
- JP 2020128962 A

### Non-patent literature cited in the description

- **JONATHAN SCHMIDT et al.** Predicting the Thermodynamic Stability of Solids Combining Density Functional Theory and Machine Learning. *Chemistry of Materials,* 2017, vol. 29 (12), 5090-5103
- **AUSTIN D. SENDEK et al.** Holistic computational structure screening of more than 12000 candidates for solid lithium-ion conductor materials. *Energy & Environmental Science,* 2017, vol. 10 (1), 306-320
- **XINGFENG HE et al.** Statistical variances of diffusional properties from ab initio molecular dynamics simulations. *Computational Materials,* 2018, vol. 4 (1), 1-9
- **KEI TERAYAMA ; KOJI TSUDA ; RYO TAMURA.** Efficient recommendation tool of materials by an executable file based on machine learning. *Japanese Journal of Applied Physics,* 2019, vol. 58 (098001